(19) **Europäisches Patentamt / European Patent Office / Office européen des brevets**

(11) **EP 4 372 103 A1**

(12) **EUROPEAN PATENT APPLICATION**
published in accordance with Art. 153(4) EPC

(43) Date of publication:
**22.05.2024 Bulletin 2024/21**

(21) Application number: **22824304.4**

(22) Date of filing: **17.06.2022**

(51) International Patent Classification (IPC):
**C12Q 1/6886** (2018.01)　　**C12Q 1/6883** (2018.01)
**G16H 50/20** (2018.01)

(52) Cooperative Patent Classification (CPC):
**C12Q 1/6883; C12Q 1/6886; G16H 50/20**

(86) International application number:
**PCT/CN2022/099311**

(87) International publication number:
**WO 2022/262831 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:　18.06.2021　CN 202110679281
　　　　　　　　18.06.2021　CN 202110680924
　　　　　　　　13.10.2021　CN 202111191903
　　　　　　　　24.12.2021　CN 202111598099
　　　　　　　　24.12.2021　CN 202111608215
　　　　　　　　24.12.2021　CN 202111600984
　　　　　　　　24.12.2021　CN 202111608328
　　　　　　　　17.01.2022　CN 202210047980
　　　　　　　　26.01.2022　CN 202210091957
　　　　　　　　26.01.2022　CN 202210092055
　　　　　　　　26.01.2022　CN 202210092038
　　　　　　　　26.01.2022　CN 202210092040

(71) Applicants:
• **Singlera Genomics (Jiangsu) Ltd.**
　**Yangzhou, Jiangsu 225012 (CN)**
• **Singlera Genomics (China) Ltd.**
　**Yangzhou, Jiangsu 225012 (CN)**

(72) Inventors:
• **LIU, Rui**
　**Shanghai 201318 (CN)**
• **MA, Chengcheng**
　**Shanghai 201318 (CN)**
• **XU, Minjie**
　**Shanghai 201318 (CN)**
• **SUN, Jin**
　**Shanghai 201318 (CN)**
• **LIU, Yiying**
　**Shanghai 201318 (CN)**
• **SU, Zhixi**
　**Shanghai 201318 (CN)**
• **SU, Mingyang**
　**Shanghai 201318 (CN)**
• **HE, Qiye**
　**Shanghai 201318 (CN)**
• **GONG, Chengxiang**
　**Shanghai 201318 (CN)**

(74) Representative: **Weickmann & Weickmann PartmbB**
**Postfach 860 820**
**81635 München (DE)**

Remarks:
The complete document including Reference Table(s) and the Sequence Listing(s) can be downloaded from the EPO website

(54) **SUBSTANCE AND METHOD FOR TUMOR ASSESSMENT**

(57)　The present application relates to a substance and method for assessing tumors. In particular, the present application provides substances, kits, devices, systems and methods for assessing tumor development risk and/or tumor progression in subjects. For example, the present application provides methods for assessing tumor formation risk and/or tumor progression in a subject based on the methylation status of a selected target polynucleotide sequence from the subject.

EP 4 372 103 A1

**Description**

**TECHNICAL FIELD**

The present application relates to the field of biomedicine, and specifically to a substance and method for assessing tumors.

**BACKGROUND**

Pancreatic cancer, such as pancreatic ductal adenocarcinoma (PDAC), is one of the most lethal diseases in the world. Its 5-year relative survival rate is 9%, and for patients with distant metastases, this rate is further reduced to only 3%. A major reason for the high mortality rate is that methods for early detection of PDAC remain limited, which is critical for PDAC patients to undergo surgical resection. Endoscopic ultrasound-guided fine-needle aspiration (EUS-FNA) is another common method to obtain pathological diagnosis without laparotomy, but it is invasive and requires clear imaging evidence, which usually means that PDAC has already progressed. During the occurrence and development of tumors, profound changes occur in the DNA methylation patterns and levels of genomic DNA in malignant cells. Some tumor-specific DNA methylations have been shown to occur early in tumorigenesis and may be a "driver" of tumorigenesis. Circulating tumor DNA (ctDNA) molecules are derived from apoptotic or necrotic tumor cells and carry tumor-specific DNA methylation markers from early malignant tumors. In recent years, they have been studied as a new promising target for the development of non-invasive early screening tools for various cancers. However, most of these studies have not yielded effective results.

Therefore, there is an urgent need in the art for a substance and method that can identify pancreatic cancer tumor-specific markers from plasma DNA.

**SUMMARY OF THE INVENTION**

The present application provides detection of the methylation level of a target gene and/or target sequence in a sample to identify pancreatic cancer using the differential gene methylation levels of the detection results, thereby achieving the purpose of non-invasive and precise diagnosis of pancreatic cancer with higher accuracy and lower cost.

In one aspect, the present application provides a reagent for detecting DNA methylation, wherein the reagent comprises a reagent for detecting the methylation level of a DNA sequence or a fragment thereof or the methylation status or level of one or more CpG dinucleotides in the DNA sequence or fragment thereof in a sample of a subject to be detected, and the DNA sequence is selected from one or more or all of the following gene sequences, or sequences within 20 kb upstream or downstream thereof: DMRTA2, FOXD3, TBX15, BCAN, TRIM58, SIX3, VAX2, EMX1, LBX2, TLX2, POU3F3, TBR1, EVX2, HOXD12, HOXD8, HOXD4, TOPAZ1, SHOX2, DRD5, RPL9, HOPX, SFRP2, IRX4, TBX18, OLIG3, ULBP1, HOXA13, TBX20, IKZF1, INSIG1, SOX7, EBF2, MOS, MKX, KCNA6, SYT10, AGAP2, TBX3, CCNA1, ZIC2, CLEC14A, OTX2, C14orf39, BNC1, AHSP, ZFHX3, LHX1, TIMP2, ZNF750, SIM2. The present application further provides methylation markers with the target sequences selected from the above-mentioned genes as pancreatic cancer-related genes, including the sequences set forth in SEQ ID NOs: 1-56. The present application further provides media and devices carrying the above-mentioned target gene and/or target sequence DNA sequence or fragments thereof and/or methylation information thereof. The present application further provides the use of the above-mentioned target gene and/or target sequence DNA sequence or fragments thereof and/or methylation information thereof in the preparation of a kit for diagnosing pancreatic cancer in a subject. The present application further provides the above-mentioned kit.

In another aspect, the present application provides a reagent for detecting DNA methylation, wherein the reagent comprises a reagent for detecting the methylation level of a DNA sequence or a fragment thereof or the methylation status or level of one or more CpG dinucleotides in the DNA sequence or fragment thereof in a sample of a subject to be detected, and the DNA sequence is selected from one or more (such as at least 7) or all of the following gene sequences, or sequences within 20 kb upstream or downstream thereof: SIX3, TLX2, and CILP2. The present application further provides methylation markers with the target sequences selected from the above-mentioned genes as pancreatic cancer-related genes, including the sequences set forth in SEQ ID NOs: 57-59. The present application further provides media and devices carrying the above-mentioned target gene and/or target sequence DNA sequence or fragments thereof and/or methylation information thereof. The present application further provides the use of the above-mentioned target gene and/or target sequence DNA sequence or fragments thereof and/or methylation information thereof in the preparation of a kit for diagnosing pancreatic cancer in a subject. The present application further provides the above-mentioned kit.

In another aspect, the present application provides a reagent for detecting DNA methylation, wherein the reagent comprises a reagent for detecting the methylation level of a DNA sequence or a fragment thereof or the methylation status or level of one or more CpG dinucleotides in the DNA sequence or fragment thereof in a sample of a subject to be detected, and the DNA sequence is selected from one or more (such as at least 7) or all of the following gene sequences, or sequences within 20 kb upstream or downstream thereof: ARHGEF16, PRDM16, NFIA, ST6GALNAC5, PRRX1, LHX4, ACBD6, FMN2, CHRM3, FAM150B, TMEM18, SIX3, CAMKMT, OTX1, WDPCP, CYP26B1, DYSF, HOXD1, HOXD4, UBE2F, RAMP1, AMT, PLSCR5, ZIC4, PEX5L, ETV5, DGKG, FGF12, FGFRL1, RNF212, DOK7, HGFAC, EVC, EVC2, HMX1, CPZ, IRX1, GDNF, AGGF1, CRHBP, PITX1, CATSPER3, NEUROG1, NPM1, TLX3, NKX2-5, BNIP1, PROP1, B4GALT7, IRF4, FOXF2, FOXQ1, FOXC1, GMDS, MOCS1, LRFN2, POU3F2, FBXL4, CCR6, GPR31, TBX20, HERPUD2, VIPR2, LZTS1, NKX2-6, PENK, PRDM14, VPS13B, OSR2, NEK6, LHX2, DDIT4, DNAJB12, CRTAC1, PAX2, HIF1AN, ELOVL3, INA, HMX2, HMX3, MKI67, DPYSL4, STK32C, INS, INS-IGF2, ASCL2, PAX6, RELT, FAM168A, OPCML, ACVR1B, ACVRL1, AVPR1A, LHX5, SDSL, RAB20, COL4A2, CARKD, CARS2, SOX1, TEX29, SPACA7, SFTA3, SIX6, SIX1, INF2, TMEM179, CRIP2, MTA1, PIAS1, SKOR1, ISL2, SCAPER, POLG, RHCG, NR2F2, RAB40C, PIGQ, CPNE2, NLRC5, PSKH1, NRN1L, SRR, HIC1, HOXB9, PRAC1, SMIM5, MYO15B, TNRC6C, 9-Sep, TBCD, ZNF750, KCTD1, SALL3, CTDP1, NFATC1, ZNF554, THOP1, CACTIN, PIP5K1C, KDM4B, PLIN3, EPS15L1, KLF2, EPS8L1, PPP1R12C, NKX2-4, NKX2-2, TFAP2C, RAE1, TNFRSF6B, ARFRP1, MYH9, and TXN2. The present application further provides methylation markers with the target sequences selected from the above-mentioned genes as pancreatic cancer-related genes, including the sequences set forth in SEQ ID NOs: 60-160. The present application further provides media and devices carrying the above-mentioned target gene and/or target sequence DNA sequence or fragments thereof and/or methylation information thereof. The present application further provides the use of the above-mentioned target gene and/or target sequence DNA sequence or fragments thereof and/or methylation information thereof in the preparation of a kit for diagnosing pancreatic cancer in a subject. The present application further provides the above-mentioned kit.

In another aspect, the present application provides detecting DNA methylation in plasma samples of patients, and constructing a machine learning model to diagnose pancreatic cancer based on the methylation level data of target methylation markers and the CA19-9 detection results, in order to achieve the purpose of non-invasive and precise diagnosis of pancreatic cancer with higher accuracy and lower cost. In addition, the present application provides a method for diagnosing pancreatic cancer or constructing a pancreatic cancer diagnostic model, comprising: (1) obtaining the methylation level of a DNA sequence or a fragment thereof or the methylation status or level of one or more CpG dinucleotides in the DNA sequence or fragment thereof in a sample of a subject, and the CA19-9 level of the subject, (2) using a mathematical model to calculate using the methylation status or level to obtain a methylation score, (3) combining the methylation score and the CA19-9 level into a data matrix, (4) constructing a pancreatic cancer diagnostic model based on the data matrix, and optionally (5) obtaining a pancreatic cancer score; and diagnosing pancreatic cancer based on the pancreatic cancer score. In one or more embodiments, the DNA sequence is selected from one or more (e.g., at least 2) or all of the following gene sequences, or sequences within 20 kb upstream or downstream thereof: SIX3, TLX2, CILP2. Preferably, the DNA sequence includes gene sequences selected from any of the following combinations: (1) SIX3, TLX2; (2) SIX3, CILP2; (3) TLX2, CILP2; (4) SIX3, TLX2, CILP2. In addition, the present application provides a method for diagnosing pancreatic cancer, comprising: (1) obtaining the methylation level of a DNA sequence or a fragment thereof or the methylation status or level of one or more CpG dinucleotides in the DNA sequence or fragment thereof in a sample of a subject, and the CA19-9 level of the subject, (2) using a mathematical model to calculate using the methylation status or level to obtain a methylation score, (3) obtaining a pancreatic cancer score based on the model shown below; and diagnosing pancreatic cancer based on the pancreatic cancer score:

$$y = \frac{1}{1 + e^{-(0.7032M + 0.6608C + 2.2243)}}$$

where M is the methylation score of the sample calculated in step (2), and C is the CA19-9 level of the sample. In one or more embodiments, the DNA sequence is selected from one or more (e.g., at least 2) or all of the following gene sequences, or sequences within 20 kb upstream or downstream thereof: SIX3, TLX2, CILP2. Preferably, the DNA sequence includes gene sequences selected from any of the following combinations: (1) SIX3, TLX2; (2) SIX3, CILP2; (3) TLX2, CILP2; (4) SIX3, TLX2, CILP2. In addition, the present application provides a method for constructing a pancreatic cancer diagnostic model, comprising: (1) obtaining the methylated haplotype fraction and sequencing depth of a genomic DNA segment in a subject, and optionally (2) pre-processing the methylated haplotype fraction and sequencing depth data, (3) performing cross-validation incremental feature selection to obtain feature methylated segments, (4) constructing a mathematic model for the methylation detection results of the feature methylated segments to obtain a methylation score, (5) constructing a pancreatic cancer diagnostic model based on the methylation score and the corresponding CA19-9 level. In one or more embodiments, step (1) comprises: 1.1) detecting DNA methylation of a sample of a subject to obtain sequencing read data, 1.2) optionally pre-processing the sequencing data, such as removing adapters and/or splicing, 1.3) aligning the sequencing data to a reference genome to obtain the location and sequencing depth information of the methylated segment, 1.4) calculating the methylated haplotype fraction (MHF) of the segment according to the following formula:

$$MHF_{i,h} = \frac{N_{i,h}}{N_i}$$

where $i$ represents the target methylated region, $h$ represents the target methylated haplotype, $Ni$ represents the number of reads located in the target methylated region, and $Ni_{,h}$ represents the number of reads containing the target methylated haplotype. The present application further provides the use of a reagent or device for detecting DNA methylation and a reagent or device for detecting CA19-9 levels in the preparation of a kit for diagnosing pancreatic cancer, wherein the reagent or device for detecting DNA methylation is used to determine the methylation level of a DNA sequence or a fragment thereof or the methylation status or level of one or more CpG dinucleotides in the DNA sequence or fragment thereof in a sample of a subject. The present application further provides the above-mentioned kit. The present application further provides a device for diagnosing pancreatic cancer or constructing a pancreatic cancer diagnostic model, including a memory, a processor, and a computer program stored in the memory and executable on the processor, wherein the above steps are implemented when the processor executes the program.

In another aspect, the present application provides a method for determining the presence of a pancreatic tumor, assessing the development or risk of development of a pancreatic tumor, and/or assessing the progression of a pancreatic tumor, comprising determining the presence and/or content of modification status of DNA regions with genes TLX2, EBF2, KCNA6, CCNA1, FOXD3, TRIM58, HOXD10, OLIG3, EN2, CLEC11A, and/or TWIST1 or fragments thereof in a sample to be tested. In addition, the present application provides a method for determining the presence of a disease, assessing the development or risk of development of a disease, and/or assessing the progression of a disease, comprising determining the presence and/or content of modification status of a DNA region selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277, or a complementary region thereof, or a fragment thereof in a sample to be tested. In addition, the present application provides a probe and/or primer combination for identifying the modification status of the above fragment. In addition, the present application provides a kit containing the above-mentioned substance. In another aspect, the present application provides the use of the nucleic acid of the present application, the nucleic acid combination of the present application and/or the kit of the present application in the preparation of a disease detection product. In another aspect, the present application provides the use of the nucleic acid of the present application, the nucleic acid combination of the present application and/or the kit of the present application in the preparation of a substance for determining the presence of a disease, assessing the development or risk of development of a disease and/or assessing the progression of a disease. In another aspect, the present application provides a storage medium recording a program capable of executing the method of the present application. In another aspect, the present application provides a device comprising the storage medium of the present application.

In another aspect, the present application provides a method for determining the presence of a pancreatic tumor, assessing the development or risk of development of a pancreatic tumor, and/or assessing the progression of a pancreatic tumor, comprising determining the presence and/or content of modification status of DNA regions with genes EBF2 and CCNA1, or KCNA6, TLX2 and EMX1, or TRIM58, TWIST1, FOXD3 and EN2, or TRIM58, TWIST1, CLEC11A, HOXD10 and OLIG3, or fragments thereof in a sample to be tested. In addition, the present application provides a method for determining the presence of a disease, assessing the development or risk of development of a disease, and/or assessing the progression of a disease, comprising determining the presence and/or content of modification status of a DNA region selected from the group consisting of DNA regions derived from human chr8:25907849-25907950, and derived from human chr13:37005635-37005754, or derived from human chr12:4919142-4919289, derived from human chr2:74743035-74743151, and derived from human chr2:73147525-73147644, or derived from human chr1:248020592-248020779, derived from human chr7:19156739-19157277, derived from human chr1:63788812-63788952, and derived from human chr7:155167513-155167628, or derived from human chr1:248020592-248020779, derived from human chr7:19156739-19157277, derived from human chr19:51228168-51228782, derived from human chr2:176945511-176945630, and derived from human chr6:137814700-137814853, or a complementary region thereof, or a fragment thereof in a sample to be tested. In addition, the present application provides a probe and/or primer combination for identifying the modification status of the above fragment. In addition, the present application provides a kit containing the above-mentioned substance combination. In another aspect, the present application provides the use of the nucleic acid of the present application, the nucleic acid combination of the present application and/or the kit of the present application in the preparation of a disease detection product. In another aspect, the present application provides the use of the nucleic acid of the present application, the nucleic acid combination of the present application and/or the kit of the present application in the preparation of a substance for determining the presence of a disease, assessing the development or risk of development of a disease and/or assessing the progression of a disease. In another aspect, the present application provides a storage medium recording a program capable of executing the method of the present application. In another aspect, the present application provides a device comprising the storage medium of the present application.

Those skilled in the art will readily appreciate other aspects and advantages of the present application from the detailed description below. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

**BRIEF DESCRIPTION OF DRAWINGS**

The specific features of the invention to which the present application relates are set forth in the appended claims. The features and advantages of the invention to which the present application relates can be better understood by reference to the exemplary embodiments described in detail below and the drawings. A brief description of the drawings is as follows:

Fig. 1 is a flow chart of a technical solution according to an embodiment of the present application.

Fig. 2 shows the ROC curves of a pancreatic cancer prediction model Model CN for diagnosing pancreatic cancer in the test group, with "false positive rate" on the abscissa, and "true positive rate" on the ordinate.

Fig. 3 shows the prediction score distribution of pancreatic cancer prediction model Model CN in the groups, with "model prediction value" on the ordinate.

Fig. 4 shows the methylation levels of 56 sequences of SEQ ID NOs: 1-56 in the training group, with "methylation level" on the ordinate.

Fig. 5 shows the methylation levels of 56 sequences of SEQ ID NOs: 1-56 in the test group, with "methylation level" on the ordinate.

Fig. 6 shows the classification ROC curves for CA19-9 alone, the SVM model Model CN constructed by the present application alone, and the model constructed by the present application combined with CA19-9, with "false positive rate" on the abscissa and "true positive rate" on the ordinate.

Fig. 7 shows the distribution of classification prediction scores for CA19-9 alone, the SVM model Model CN constructed by the present application alone, and the model constructed by the present application combined with CA19-9, with "model prediction value" on the ordinate.

Fig. 8 shows the ROC curves of the SVM model Model CN constructed in the present application in samples determined as negative with respect to tumor marker CA19-9 (with CA19-9 measurement value less than 37), with "false positive rate" on the abscissa and "true positive rate" on the ordinate.

Fig. 9 shows the ROC curves of the combination model of seven markers SEQ ID NOs: 9,14,13,26,40,43,52, with "false positive rate" on the abscissa, and "true positive rate" on the ordinate.

Fig. 10 shows the ROC curves of the combination model of seven markers SEQ ID NOs: 5,18,34,40,43,45,46, with "false positive rate" on the abscissa, and "true positive rate" on the ordinate.

Fig. 11 shows the ROC curves of the combination model of seven markers SEQ ID NOs: 11,8,20,44,48,51,54, with "false positive rate" on the abscissa, and "true positive rate" on the ordinate.

Fig. 12 shows the ROC curves of the combination model of seven markers SEQ ID NOs: 14,8,26,24,31,40,46, with "false positive rate" on the abscissa, and "true positive rate" on the ordinate.

Fig. 13 shows the ROC curves of the combination model of seven markers SEQ ID NOs: 3,9,8,29,42,40,41, with "false positive rate" on the abscissa, and "true positive rate" on the ordinate.

Fig. 14 shows the ROC curves of the combination model of seven markers SEQ ID NOs: 5,8,19,7,44,47,53, with "false positive rate" on the abscissa, and "true positive rate" on the ordinate.

Fig. 15 shows the ROC curves of the combination model of seven markers SEQ ID NOs: 12,17,24,28,40,42,47, with "false positive rate" on the abscissa, and "true positive rate" on the ordinate.

Fig. 16 shows the ROC curves of the combination model of seven markers SEQ ID NOs: 5,18,14,10,8,19,27, with "false positive rate" on the abscissa, and "true positive rate" on the ordinate.

Fig. 17 shows the ROC curves of the combination model of seven markers SEQ ID NOs: 6,12,20,26,24,47,50, with "false positive rate" on the abscissa, and "true positive rate" on the ordinate.

Fig. 18 shows the ROC curves of the combination model of seven markers SEQ ID NOs: 1,19,27,34,37,46,47, with "false positive rate" on the abscissa, and "true positive rate" on the ordinate.

Fig. 19 shows the ROC curves of the pancreatic cancer prediction model for differentiating chronic pancreatitis and pancreatic cancer in the training group and the test group, with "false positive rate" on the abscissa, and "true positive rate" on the ordinate.

Fig. 20 shows the prediction score distribution of the pancreatic cancer prediction model in the groups, with "model prediction value" on the ordinate.

Fig. 21 shows the methylation level of 3 methylation markers in the training group, with "methylation level" on the ordinate.

Fig. 22 shows the methylation level of 3 methylation markers in the test group, with "methylation level" on the ordinate.

Fig. 23 shows the ROC curves of the pancreatic cancer prediction model for diagnosing pancreatic cancer in negative samples as determined by traditional methods (i.e., with the CA19-9 measurement value less than 37), with "false positive rate" on the abscissa, and "true positive rate" on the ordinate.

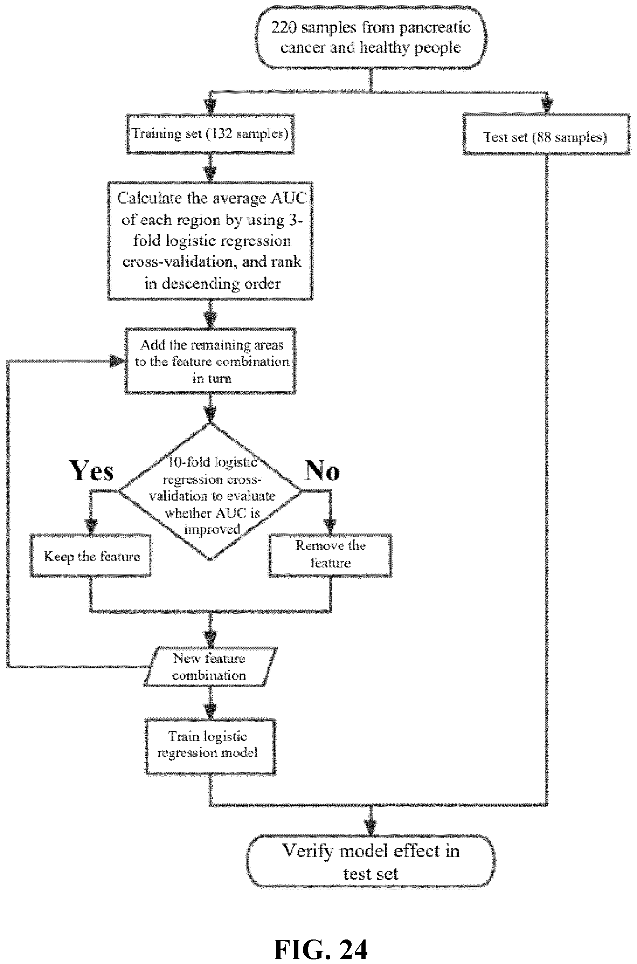

Fig. 24 shows a flow chart for screening methylation markers based on the feature matrix according to the present application.

Fig. 25 shows the distribution of the prediction scores of 101 markers.

Fig. 26 shows the ROC curves of 101 markers.

Fig. 27 shows the distribution of the prediction scores of 6 markers.

Fig. 28 shows the ROC curves of 6 markers.

Fig. 29 shows the distribution of the prediction scores of 7 markers.

Fig. 30 shows the ROC curves of 7 markers.

Fig. 31 shows the distribution of the prediction scores of 10 markers.

Fig. 32 shows the ROC curves of 10 markers.

Fig. 33 shows the distribution of the prediction scores of the DUALMODEL marker.

Fig. 34 shows the ROC curves of the DUALMODEL marker.

Fig. 35 shows the distribution of the prediction scores of the ALLMODEL marker.

Fig. 36 shows the ROC curves of the ALLMODEL marker.

Fig. 37 shows a flow chart of a technical solution according to an embodiment of the present invention.

Fig. 38 shows the distribution of methylation levels of 3 methylation markers in the training group.

Fig. 39 shows the distribution of methylation levels of 3 methylation markers in the test group.

Fig. 40 shows the ROC curves of CA19-9, pancreatic cancer and pancreatitis differentiation prediction models pp_model and cpp_model in the test set.

Fig. 41 shows the distribution of the prediction scores of CA19-9, pancreatic cancer and pancreatitis differentiation prediction models pp_model and cpp_model in the test set samples (the values are normalized using the maximum and minimum values).

## DETAILED DESCRIPTION OF THE INVENTION

The embodiments of the invention of the present application will be described below with specific examples. Those skilled in the art can easily understand other advantages and effects of the invention of the present application from the disclosure of the specification.

## Definition of Terms

In the present application, the term "sample to be tested" usually refers to a sample that needs to be tested. For example, it can be detected whether one or more gene regions on the sample to be tested are modified.

In the present application, the term "cell-free nucleic acid" or "cfDNA" generally refers to DNA in a sample that is not contained within the cell when collected. For example, cell-free nucleic acid may not refer to DNA that is rendered non-intracellular by in vitro disruption of cells or tissues. For example, cfDNA can include DNA derived from both normal cells and cancer cells. For example, cfDNA can be obtained from blood or plasma ("circulatory system"). For example, cfDNA can be released into the circulatory system through secretion or cell death processes such as necrosis or apoptosis.

In the present application, the term "complementary nucleic acid" generally refers to nucleotide sequences that are complementary to a reference nucleotide sequence. For example, complementary nucleic acids can be nucleic acid molecules that optionally have opposite orientations. For example, the complementarity may refer to having the following complementary associations: guanine and cytosine; adenine and thymine; adenine and uracil.

In the present application, the term "DNA region" generally refers to the sequence of two or more covalently bound naturally occurring or modified deoxyribonucleotides. For example, the DNA region of a gene may refer to the position of a specific deoxyribonucleotide sequence where the gene is located, for example, the deoxyribonucleotide sequence encodes the gene. For example, the DNA region of the present application includes the full length of the DNA region, complementary regions thereof, or fragments thereof. For example, a sequence of at least about 20 kb upstream and downstream of the detection region provided in the present application can be used as a detection site. For example, a sequence of at least about 20 kb, at least about 15 kb, at least about 10 kb, at least about 5 kb, at least about 3 kb, at least about 2 kb, at least about 1 kb, or at least about 0.5 kb upstream and downstream of the detection region provided in the present application can be used as a detection site. For example, appropriate primers and probes can be designed according to what's described above using a microcomputer to detect methylation of samples.

In the present application, the term" modification status" generally refer to the modification status of a gene fragment, a nucleotide, or a base thereof in the present application. For example, the modification status in the present application may refer to the modification status of cytosine. For example, a gene fragment with modification status in the present application may have altered gene expression activity. For example, the modification status in the present application may refer to the methylation modification of a base. For example, the modification status in the present application may refer to the covalent binding of a methyl group at the 5' carbon position of cytosine in the CpG region of genomic DNA, which may become 5-methylcytosine (5mC), for example. For example, the modification status may refer to the presence or absence of 5-methylcytosine ("5-mCyt") within the DNA sequence.

In the present application, the term "methylation" generally refers to the methylation status of a gene fragment, a nucleotide, or a base thereof in the present application. For example, the DNA segment in which the gene is located in the present application may have methylation on one or more strands. For example, the DNA segment in which the gene is located in the present application may have methylation on one or more sites.

In the present application, the term "conversion" generally refers to the conversion of one or more structures into another structure. For example, the conversion in the present application may be specific. For example, cytosine without methylation modification can turn into other structures (such as uracil) after conversion, and cytosine with methylation modification can remain basically unchanged after conversion. For example, cytosine without methylation modification can be cleaved after conversion, and cytosine with methylation modification can remain basically unchanged after conversion.

In the present application, the term "deamination reagent" generally refers to a substance that has the ability to remove amino groups. For example, deamination reagents can deaminate unmodified cytosine.

In the present application, the term "bisulfite" generally refers to a reagent that can differentiate a DNA region that has modification status from one that does not have modification status. For example, bisulfite may include bisulfite, or analogues thereof, or a combination thereof. For example, bisulfite can deaminate the amino group of unmodified cytosine to differentiate it from modified cytosine. In the present application, the term "analogue" generally refers to substances having a similar structure and/or function. For example, analogues of bisulfite may have a similar structure to bisulfite. For example, a bisulfite analogue may refer to a reagent that can also differentiate DNA regions that have modification status and those that do not have modification status.

In the present application, the term "methylation-sensitive restriction enzyme" generally refers to an enzyme that selectively digest nucleic acids according to the methylation status of its recognition site. For example, for a restriction enzyme that specifically cleaves when the recognition site is unmethylated, cleavage may not occur or occur with significantly reduced efficiency when the recognition site is methylated. For a restriction enzyme that specifically cleaves when the recognition site is methylated, cleavage may not occur or occur with significantly reduced efficiency when the recognition site is unmethylated. For example, methylation-specific restriction enzymes can recognize sequences containing CG dinucleotides (e.g., cgcg or cccggg).

In the present application, the term "tumor" generally refers to cells and/or tissues that exhibit at least partial loss of control during normal growth and/or development. For example, common tumors or cancer cells may often have lost contact inhibition and may be invasive and/or have the ability to metastasize. For example, the tumor of the present application may be benign or malignant.

In the present application, the term "progression" generally refers to a change in the disease from a less severe condition to a more severe condition. For example, tumor progression may include an increase in the number or severity of tumors, the extent of cancer cell metastasis, the rate at which the cancer grows or spreads. For example, tumor progression may include the progression of the cancer from a less severe state to a more severe state, such as from Stage I to Stage II, from Stage II to Stage III.

In the present application, the term "development" generally refers to the occurrence of a lesion in an individual. For example, when a tumor develops, the individual may be diagnosed as a tumor patient.

In the present application, the term "fluorescent PCR" generally refers to a quantitative or semi-quantitative PCR technique. For example, the PCR technique may be real-time quantitative polymerase chain reaction, quantitative polymerase chain reaction or kinetic polymerase chain reaction. For example, the initial amount of a target nucleic acid can be quantitatively detected by using PCR amplification with the aid of an intercalating fluorescent dye or a sequence-specific probe, and the sequence-specific probe can contain a fluorescent reporter that is detectable only if it hybridizes to the target nucleic acid.

In the present application, the term "PCR amplification" generally refers to a polymerase chain reaction. For example, PCR amplification in the present application may comprise any polymerase chain amplification reaction currently known for use in DNA amplification.

In the present application, the term" fluorescence Ct value" generally refer to a measurement value for the quantitative or semi-quantitative evaluation of the target nucleic acid. For example, it may refer to the number of amplification reaction cycles experienced when the fluorescence signal reaches a set threshold value.

**Detailed Description of the Invention**

Based on the methylation nucleic acid fragment markers of the present application, pancreatic cancer can be effectively identified; the present application provides a diagnostic model for the relationship between cfDNA methylation markers and pancreatic cancer based on plasma cfDNA high-throughput methylation sequencing. This model has the advantages of non-invasive, safe and convenient detection, high throughput and high detection specificity. Based on the optimal sequencing obtained in the present application, it can effectively control the detection cost while achieving good detection effects. Based on the DNA methylation markers of the present invention, it can effectively differentiate patients with pancreatic cancer and patients with chronic pancreatitis. The present invention provides a diagnostic model for the relationship between methylation level of cfDNA methylation markers and pancreatic cancer based on plasma cfDNA high-throughput methylation sequencing. This model has the advantages of non-invasive, safe and convenient detection, high throughput and high detection specificity. Based on the optimal sequencing obtained in the present invention, it can effectively control the detection cost while achieving good detection effects.

The present application found that the properties of pancreatic cancer are related to the methylation level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 genes selected from the following genes or sequences within 20 kb upstream or downstream thereof DMRTA2, FOXD3, TBX15, BCAN, TRIM58, SIX3, VAX2 , EMX1, LBX2, TLX2, POU3F3, TBR1, EVX2, HOXD12, HOXD8, HOXD4, TOPAZ1, SHOX2, DRD5, RPL9, HOPX, SFRP2, IRX4, TBX18, OLIG3, ULBP1, HOXA13, TBX20, IKZF1, INSIG1, SOX7, EBF2, MOS, MKX, KCNA6, SYT10, AGAP2, TBX3, CCNA1, ZIC2, CLEC14A, OTX2, C14orf39, BNC1, AHSP, ZFHX3, LHX1, TIMP2, ZNF750, SIM2. In one or more embodiments, the properties of pancreatic cancer are related to the methylation level of sequences of genes selected from any of the following combinations: (1) LBX2, TBR1, EVX2, SFRP2, SYT10, CCNA1, ZFHX3; (2) TRIM58, HOXD4, INSIG1, SYT10, CCNA1, ZIC2, CLEC14A; (3) EMX1, POU3F3, TOPAZ1, ZIC2, OTX2, AHSP, TIMP2; (4) EMX1, EVX2, RPL9, SFRP2, HOXA13, SYT10, CLEC14A; (5) TBX15, EMX1, LBX2, OLIG3, SYT10, AGAP2, TBX3; (6) TRIM58, VAX2, EMX1, HOXD4, ZIC2, CLEC14A, LHX1; (7) POU3F3, HOXD8, RPL9, TBX18, SYT10, TBX3, CLEC14A; (8) TRIM58, EMX1, TLX2, EVX2, HOXD4, HOXD4, IRX4; (9) SIX3, POU3F3, TOPAZ1, RPL9, SFRP2, CLEC14A, BNC1; (10) DMRTA2, HOXD4, IRX4, INSIG1, MOS, CLEC14A, CLEC14A. The present invention provides nucleic acid molecules containing one or more CpGs of the above-mentioned genes or fragments thereof. The present application found that the differentiation between pancreatic cancer and pancreatitis (such as chronic pancreatitis) is related to the methylation levels of 1, 2, 3 genes selected from the following genes or sequences within 20kb upstream or downstream thereof: SIX3, TLX2, CILP2.

In the present invention, the term "gene" includes both coding sequences and non-coding sequences of the gene of interest on the genome. Non-coding sequences include introns, promoters, regulatory elements or sequences, etc.

Further, the properties of pancreatic cancer are related to the methylation level of any one or random 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 segments or all 56 segments selected from: SEQ ID NO:1 in the DMRTA2 gene region, SEQ ID NO:2 in the FOXD3 gene region, SEQ ID NO:3 in the TBX15 gene region, SEQ ID NO:4 in the BCAN gene region, SEQ ID NO:5 in the TRIM58 gene region, SEQ ID NO:6 in the SIX3 gene region, SEQ ID NO:7 in the VAX2 gene region, SEQ ID NO:8 in the EMX1 gene region, SEQ ID NO:9 in the LBX2 gene region, SEQ ID NO:10 in the TLX2 gene region, SEQ ID NO:11 and SEQ ID NO:12 in the POU3F3 gene region, SEQ ID NO:13 in the TBR1 gene region, SEQ ID NO:14 and SEQ ID NO:15 in the EVX2 gene region, SEQ ID NO:16 in the HOXD12 gene region, SEQ ID NO:17 in the HOXD8 gene region, SEQ ID NO:18 and SEQ ID NO:19 in the HOXD4 gene region, SEQ ID NO:20 in the TOPAZ1 gene region, SEQ ID NO:21 in the SHOX2 gene region, SEQ ID NO:22 in the DRD5 gene region, SEQ ID NO:23 and SEQ ID NO:24 in the RPL9 gene region, SEQ ID NO:25 in the HOPX gene region, SEQ ID NO:26 in the SFRP2 gene region, SEQ ID NO:27 in the IRX4 gene region, SEQ ID NO:28 in the TBX18 gene region, SEQ ID NO:29 in the OLIG3 gene region, SEQ ID NO:30 in the ULBP1 gene region, SEQ ID NO:31 in the HOXA13 gene region, SEQ ID NO:32 in the TBX20 gene region, SEQ ID NO:33 in the IKZF1 gene region, SEQ ID NO:34 in the INSIG1 gene region, SEQ ID NO:35 in the SOX7 gene region, SEQ ID NO:36 in the EBF2 gene region, SEQ ID NO:37 in the MOS gene region, SEQ ID NO:38 in the MKX gene region, SEQ ID NO:39 in the KCNA6 gene region, SEQ ID NO:40 in the SYT10 gene region, SEQ ID NO:41 in the AGAP2 gene region, SEQ ID NO:42 in the TBX3 gene region, SEQ ID NO:43 in the CCNA1 gene region, SEQ ID NO:44 and SEQ ID NO:45 in the ZIC2 gene region, SEQ ID NO:46 and SEQ ID NO:47 in the CLEC14A gene region, SEQ ID NO:48 in the OTX2 gene region, SEQ ID NO:49 in the C14orf39 gene region, SEQ ID NO:50 in the BNC1 gene region, SEQ ID NO:51 in the AHSP gene region, SEQ ID NO:52 in the ZFHX3 gene region, SEQ ID NO:53 in the LHX1 gene region, SEQ ID NO:54 in the TIMP2 gene region, SEQ ID NO:55 in the ZNF750 gene region, and SEQ ID NO:56 in the SIM2 gene region.

In some embodiments, the properties of pancreatic cancer are related to the methylation level of sequences selected from any of the following combinations, or complementary sequences thereof: (1) SEQ ID NO:9, SEQ ID NO:13, SEQ ID NO:14, SEQ ID NO:26, SEQ ID NO:40, SEQ ID NO:43, SEQ ID NO:52, (2) SEQ ID NO:5, SEQ ID NO:18, SEQ ID NO:34, SEQ ID NO:40, SEQ ID NO:43, SEQ ID NO:45, SEQ ID NO:46, (3) SEQ ID NO:8, SEQ ID NO:11, SEQ ID NO:20, SEQ ID NO:44, SEQ ID NO:48, SEQ ID NO:51, SEQ ID NO:54, (4) SEQ ID NO:8, SEQ ID NO:14, SEQ ID NO:24, SEQ ID NO:26, SEQ ID NO:31, SEQ ID NO:40, SEQ ID NO:46, (5) SEQ ID NO:3, SEQ ID NO:8, SEQ ID NO:9, SEQ ID NO:29, SEQ ID NO:40, SEQ ID NO:41, SEQ ID NO:42, (6) SEQ ID NO:5, SEQ ID NO:7, SEQ ID NO:8, SEQ ID NO:19, SEQ ID NO:44, SEQ ID NO:47, SEQ ID NO:53, (7) SEQ ID NO:12, SEQ ID NO:17, SEQ ID NO:24, SEQ ID NO:28, SEQ ID NO:40, SEQ ID NO:42, SEQ ID NO:47, (8) SEQ ID NO:5, SEQ ID NO:8, SEQ ID NO:10, SEQ ID NO:14, SEQ ID NO:18, SEQ ID NO:19, SEQ ID NO:27, (9) SEQ ID NO:6, SEQ ID NO:12, SEQ ID NO:20, SEQ ID NO:24, SEQ ID NO:26, SEQ ID NO:47, SEQ ID NO:50, (10) SEQ ID NO:1, SEQ ID NO:19, SEQ ID NO:27, SEQ ID NO:34, SEQ ID NO:37, SEQ ID NO:46, SEQ ID NO:47.

"Pancreatic cancer-related sequences" described herein include the above-mentioned 50 genes, sequences within 20 kb upstream or downstream thereof, the above-mentioned 56 sequences (SEQ ID NOs:1-56) or complementary sequences, sub-regions, and/or treated sequences thereof.

The positions of the above-mentioned 56 sequences in human chromosomes are as follows: SEQ ID NO:1: chr1's 50884507-50885207bps, SEQ ID NO:2: chr1's 63788611-63789152bps, SEQ ID NO:3: chr1's 119522143-119522719bps, SEQ ID NO:4: chr1's 156611710-156612211bps, SEQ ID NO:5: chr1's 248020391-248020979bps, SEQ ID NO:6: chr2's 45028796-45029378bps, SEQ ID NO:7: chr2's 71115731-71116272bps, SEQ ID NO:8: chr2's 73147334-73147835bps, SEQ ID NO:9: chr2's 74726401-74726922bps, SEQ ID NO:10: chr2's 74742861-74743362bps, SEQ ID NO:11: chr2's 105480130-105480830bps, SEQ ID NO:12: chr2's 105480157-105480659bps, SEQ ID NO:13: chr2's 162280233-162280736bps, SEQ ID NO:14: chr2's 176945095-176945601bps, SEQ ID NO:15: chr2's 176945320-176945821bps, SEQ ID NO:16: chr2's 176964629-176965209bps, SEQ ID NO:17: chr2's 176994514-176995015bps, SEQ ID NO:18: chr2's 177016987-177017501bps, SEQ ID NO:19: chr2's 177024355-177024866bps, SEQ ID NO:20: chr3's 44063336-44063893bps, SEQ ID NO:21: chr3's 157812057-157812604bps, SEQ ID NO:22: chr4's 9783025-9783527bps, SEQ ID NO:23: chr4's 39448278-39448779bps, SEQ ID NO:24: chr4's 39448327-39448879bps, SEQ ID NO:25: chr4's 57521127-57521736bps, SEQ ID NO:26: chr4's 154709362-154709867bps, SEQ ID NO:27: chr5's 1876136-1876645bps, SEQ ID NO:28: chr6's 85476916-85477417bps, SEQ ID NO:29: chr6's 137814499-137815053bps, SEQ ID NO:30: chr6's 150285594-150286095bps, SEQ ID NO:31: chr7's 27244522-27245037bps, SEQ ID NO:32: chr7's 35293435-35293950bps, SEQ ID NO:33: chr7's 50343543-50344243bps, SEQ ID NO:34: chr7's 155167312-155167828bps, SEQ ID NO:35: chr8's 10588692-10589253bps, SEQ ID NO:36: chr8's 25907648-25908150bps, SEQ ID NO37: chr8's 57069450-57070150bps, SEQ ID NO:38: chr10's 28034404-28034908bps, SEQ ID NO:39: chr12's 4918941-4919489bps, SEQ ID NO:40: chr12's 33592612-33593117bps, SEQ ID NO:41: chr12's 58131095-58131654bps, SEQ ID NO:42: chr12's 115124763-115125348bps, SEQ ID NO:43: chr13's 37005444-37005945bps, SEQ ID NO:44: chr13's 100649468 - 100649995bps, SEQ ID NO:45: chr13's 100649513-100650027bps, SEQ ID NO:46: chr14's 38724419-38724935bps, SEQ ID NO:47: chr14's 38724602-38725108bps, SEQ ID NO:48: chr14's 57275646-57276162bps, SEQ ID NO:49: chr14's 60952384-60952933bps, SEQ ID NO:50: chr15's 83952059-83952595bps, SEQ ID NO:51: chr16's 31579970-31580561bps, SEQ ID NO:52: chr16's 73096773-73097473bps, SEQ ID NO:53: chr17's 35299694-35300224bps, SEQ ID NO:54: chr17's 76929623-76930176bps, SEQ ID NO:55: chr17's 80846617-80847210bps, SEQ ID NO:56: chr21's 38081247-38081752bps. Herein, the bases of the sequences and methylation sites are numbered corresponding to the reference genome HG19.

In one or more embodiments, the nucleic acid molecule described herein is a fragment of one or more genes selected from DMRTA2, FOXD3, TBX15, BCAN, TRIM58, SIX3, VAX2 , EMX1, LBX2, TLX2, POU3F3, TBR1, EVX2, HOXD12, HOXD8, HOXD4, TOPAZ1, SHOX2, DRD5, RPL9, HOPX, SFRP2, IRX4, TBX18, OLIG3, ULBP1, HOXA13, TBX20, IKZF1, INSIG1, SOX7, EBF2, MOS, MKX, KCNA6, SYT10, AGAP2, TBX3, CCNA1, ZIC2, CLEC14A, OTX2, C14orf39, BNC1, AHSP, ZFHX3, LHX1, TIMP2, ZNF750, SIM2; the length of the fragment is 1bp-1kb, preferably 1bp-700bp; the fragment comprises one or more methylation sites of the corresponding gene in the chromosomal region. The methylation sites in the genes or fragments thereof described herein include, but are not limited to: chr1 chromosome's 50884514, 50884531, 50884533, 50884541, 50884544, 50884547, 50884550, 50884552, 50884566, 50884582, 50884586, 50884589, 50884591, 50884598, 50884606, 50884610, 50884612, 50884615, 50884621, 50884633, 50884646, 50884649, 50884658, 50884662, 50884673, 50884682, 50884691, 50884699, 50884702, 50884724, 50884732, 50884735, 50884742, 50884751, 50884754, 50884774, 50884777, 50884780, 50884783, 50884786, 50884789, 50884792, 50884795, 50884798, 50884801, 50884804, 50884807, 50884809, 50884820, 50884822, 50884825, 50884849, 50884852, 50884868, 50884871, 50884885, 50884889, 50884902, 50884924, 50884939, 50884942, 50884945, 50884948, 50884975, 50884980, 50884983, 50884999, 50885001, 63788628, 63788660, 63788672, 63788685, 63788689, 63788703, 63788706, 63788709, 63788721, 63788741, 63788744, 63788747, 63788753, 63788759, 63788768, 63788776, 63788785, 63788789, 63788795, 63788804, 63788816, 63788822, 63788825, 63788828, 63788849, 63788852, 63788861, 63788870, 63788872, 63788878, 63788881, 63788889, 63788897, 63788902, 63788906, 63788917, 63788920, 63788933, 63788947, 63788983, 63788987, 63788993, 63788999, 63789004, 63789011, 63789014, 63789020, 63789022, 63789025, 63789031, 63789035, 63789047, 63789056, 63789059, 63789068, 63789071, 63789073, 63789077, 63789080, 63789083, 63789092, 63789094, 63789101, 63789106, 63789109, 63789124, 119522172, 119522188, 119522190, 119522233, 119522239, 119522313, 119522368, 119522386, 119522393, 119522409, 119522425, 119522427, 119522436, 119522440, 119522444, 119522446, 119522449, 119522451, 119522456, 119522459, 119522464, 119522469, 119522474, 119522486, 119522488, 119522500, 119522502, 119522516, 119522529, 119522537, 119522548, 119522550, 119522559, 119522563, 119522566, 119522571, 119522577, 119522579, 119522582, 119522594, 119522599, 119522607, 119522615, 119522621, 119522629, 119522631, 119522637, 119522665, 119522673, 156611713, 156611720, 156611733, 156611737, 156611749, 156611752, 156611761, 156611767, 156611784, 156611791, 156611797, 156611802, 156611811, 156611813, 156611819, 156611830, 156611836, 156611842, 156611851, 156611862, 156611890, 156611893, 156611902, 156611905, 156611915, 156611926, 156611945, 156611949, 156611951, 156611960, 156611963, 156611994, 156612002, 156612015, 156612024, 156612034, 156612042, 156612044, 156612079, 156612087, 156612090, 156612094, 156612097, 156612105, 156612140, 156612147, 156612166, 156612188, 156612191, 156612204, 156612209, 248020399, 248020410, 248020436, 248020447, 248020450, 248020453, 248020470, 248020495, 248020497, 248020507, 248020512, 248020516, 248020520, 248020526, 248020536, 248020543, 248020559, 248020562, 248020566, 248020573, 248020579, 248020581, 248020589, 248020591, 248020598, 248020625, 248020632, 248020641, 248020671, 248020680, 248020688, 248020692, 248020695, 248020697, 248020704, 248020707, 248020713, 248020721, 248020729, 248020741, 248020748, 248020756, 248020765, 248020775, 248020791, 248020795, 248020798, 248020812, 248020814, 248020821, 248020826, 248020828, 248020831, 248020836, 248020838, 248020840, 248020845, 248020848, 248020861, 248020869, 248020878, 248020883, 248020886, 248020902, 248020905, 248020908, 248020914, 248020925, 248020930, 248020934, 248020937, 248020940, 248020953, 248020956, 248020975; chr2 chromosome's 45028802, 45028816, 45028832, 45028839, 45028956, 45028961, 45028965, 45028973, 45029004, 45029017, 45029035, 45029046, 45029057, 45029060, 45029063, 45029065, 45029071, 45029106, 45029112, 45029117, 45029128, 45029146, 45029176, 45029179, 45029184, 45029189, 45029192, 45029195, 45029218, 45029226, 45029228, 45029231, 45029235, 45029263, 45029273, 45029285, 45029288, 45029295, 45029307, 45029317, 45029353, 45029357, 71115760, 71115787, 71115789, 71115837, 71115928, 71115936, 71115948, 71115962, 71115968, 71115978, 71115981, 71115983, 71115985, 71115987, 71115994, 71116000, 71116022, 71116024, 71116030, 71116036, 71116047, 71116054, 71116067, 71116096, 71116101, 71116103, 71116107, 71116117, 71116119, 71116130, 71116137, 71116141, 71116152, 71116154, 71116158, 71116174, 71116188, 71116190, 71116194, 71116203, 71116215, 71116226, 71116233, 71116242, 71116257, 71116259, 71116261, 71116268, 71116271, 73147340, 73147350, 73147364, 73147369, 73147382, 73147405, 73147408, 73147432, 73147438, 73147444, 73147481, 73147491, 73147493, 73147523, 73147529, 73147537, 73147559, 73147571, 73147582, 73147584, 73147592, 73147595, 73147598, 73147607, 73147613, 73147620, 73147623, 73147631, 73147644, 73147668, 73147673, 73147678, 73147687, 73147690, 73147693, 73147695, 73147710, 73147720, 73147738, 73147755, 73147767, 73147771, 73147789, 73147798, 73147803, 73147811, 73147814, 73147816, 73147822, 73147825, 73147827, 73147829, 74726438, 74726440, 74726449, 74726478, 74726480, 74726482, 74726484, 74726493, 74726495, 74726524, 74726526, 74726533, 74726536, 74726539, 74726548, 74726554, 74726569, 74726572, 74726585, 74726597, 74726599, 74726616, 74726633, 74726642, 74726649, 74726651, 74726656, 74726668, 74726672, 74726682, 74726687, 74726695, 74726700, 74726710, 74726716, 74726734, 74726746, 74726760, 74726766, 74726772, 74726784, 74726791, 74726809, 74726828, 74726833, 74726835, 74726861, 74726892, 74726894, 74726908, 74742879, 74742882, 74742891, 74742913, 74742922, 74742925, 74742942, 74742950, 74742953, 74742967, 74742981, 74742984, 74742996, 74743004, 74743006, 74743009, 74743011, 74743015, 74743021, 74743035, 74743056, 74743059, 74743061, 74743064, 74743068, 74743073, 74743082, 74743084, 74743101, 74743108, 74743111, 74743119, 74743121, 74743127, 74743131, 74743137, 74743139, 74743141, 74743146, 74743172, 74743174, 74743182, 74743186, 74743191, 74743195, 74743198, 74743207, 74743231, 74743234, 74743241, 74743243, 74743268, 74743295, 74743301, 74743306, 74743318, 74743321, 74743325, 74743329, 74743333, 74743336, 74743343, 74743346, 74743352, 74743357, 105480130, 105480161, 105480179, 105480198, 105480207, 105480210, 105480212, 105480226, 105480254, 105480258, 105480272, 105480291, 105480337, 105480360, 105480377, 105480383, 105480387, 105480390, 105480407, 105480409, 105480412, 105480424, 105480426, 105480429, 105480433, 105480438, 105480461, 105480464, 105480475, 105480481, 105480488, 105480490, 105480503, 105480546, 105480556, 105480571, 105480577, 105480581, 105480604, 105480621, 105480623, 105480630, 105480634, 105480637, 162280237, 162280239, 162280242, 162280245, 162280249, 162280257, 162280263, 162280289, 162280293, 162280297, 162280306, 162280309, 162280314, 162280317, 162280327, 162280331, 162280341, 162280351, 162280362, 162280368, 162280393, 162280396, 162280398, 162280402, 162280405, 162280407, 162280409, 162280417, 162280420, 162280438, 162280447, 162280459, 162280462, 162280466, 162280470, 162280473, 162280479, 162280483, 162280486, 162280489, 162280492, 162280498, 162280519, 162280534, 162280539, 162280548, 162280561, 162280570, 162280575, 162280585, 162280598, 162280604, 162280611, 162280614, 162280618, 162280623, 162280627, 162280633, 162280641, 162280647, 162280657, 162280673, 162280681, 162280693, 162280708, 162280728, 176945102, 176945119, 176945122, 176945132, 176945134, 176945137, 176945141, 176945144, 176945147, 176945150, 176945159, 176945165, 176945170, 176945177, 176945179, 176945186, 176945188, 176945198, 176945200, 176945213, 176945215, 176945218, 176945222, 176945224, 176945250, 176945270, 176945274, 176945288, 176945296, 176945298, 176945316, 176945329, 176945336, 176945339, 176945345, 176945347, 176945351, 176945354, 176945356, 176945372, 176945374, 176945378, 176945381, 176945384, 176945387, 176945392, 176945398, 176945402, 176945417, 176945422, 176945426, 176945452, 176945458, 176945462, 176945464, 176945468, 176945497, 176945507, 176945526, 176945532, 176945547, 176945550, 176945570, 176945580, 176945582, 176945585, 176945604, 176945609, 176945647, 176945679, 176945695, 176945732, 176945747, 176945750, 176945761, 176945770, 176945789, 176945791, 176945795, 176964640, 176964642, 176964663, 176964665, 176964667, 176964670, 176964672, 176964685, 176964690, 176964694, 176964703, 176964709, 176964711, 176964720, 176964724, 176964736, 176964739, 176964747, 176964769, 176964778, 176964805, 176964811, 176964834, 176964838, 176964843, 176964847, 176964863, 176964865, 176964869, 176964875, 176964879, 176964886, 176964892, 176964930, 176964946, 176964959, 176964966, 176964969, 176964978, 176965003, 176965021, 176965035, 176965062, 176965065, 176965069, 176965085, 176965099, 176965102, 176965109, 176965125, 176965130, 176965140, 176965186, 176965196, 176994516, 176994525, 176994528, 176994531, 176994537, 176994546, 176994557, 176994559, 176994568, 176994570, 176994583, 176994586, 176994623, 176994637, 176994654, 176994661, 176994665, 176994682, 176994688, 176994728, 176994738, 176994747, 176994750, 176994753, 176994764, 176994768, 176994773, 176994778, 176994780, 176994783, 176994793, 176994801, 176994804, 176994807, 176994809, 176994811, 176994822, 176994830, 176994832, 176994837, 176994839, 176994848, 176994851, 176994853, 176994859, 176994864, 176994867, 176994871, 176994880, 176994890, 176994905, 176994909, 176994911, 176994931, 176994934, 176994936, 176994938, 176994942, 176994944, 176994948, 176994952, 176994961, 176994964, 176994971, 176994974, 176994980, 176994983, 176994986, 176994996, 176995011, 176995013, 177017050, 177017079, 177017124, 177017173, 177017179, 177017182, 177017193, 177017211, 177017223, 177017225, 177017227, 177017237, 177017239, 177017246, 177017251, 177017253, 177017267, 177017270, 177017276, 177017296, 177017300, 177017331, 177017352, 177017368, 177017374, 177017378, 177017389, 177017446, 177017449, 177017452, 177017463, 177017483, 177017488, 177024359, 177024367, 177024415, 177024502, 177024514, 177024528, 177024531, 177024540, 177024548, 177024550, 177024558, 177024582, 177024605, 177024616, 177024619, 177024634, 177024642, 177024655, 177024698, 177024709, 177024714, 177024723, 177024725, 177024748, 177024756, 177024769, 177024771, 177024776, 177024783, 177024800, 177024836, 177024838, 177024856, 177024861; chr3 chromosome's 44063356, 44063391, 44063404, 44063411, 44063417, 44063423, 44063450, 44063516, 44063541, 44063544, 44063559, 44063565, 44063567, 44063574, 44063586, 44063593, 44063602, 44063606, 44063620, 44063633, 44063638, 44063643, 44063649, 44063657, 44063660, 44063662, 44063682, 44063686, 44063719, 44063745, 44063756, 44063768, 44063779, 44063807, 44063821, 44063832, 44063836, 44063858, 44063877, 157812071, 157812085, 157812092, 157812117, 157812131, 157812152, 157812170, 157812173, 157812175, 157812184, 157812206, 157812212, 157812226, 157812256, 157812259, 157812275, 157812277, 157812287, 157812294, 157812296, 157812302, 157812305, 157812307, 157812312, 157812319, 157812321, 157812329, 157812331, 157812334, 157812354, 157812358, 157812369, 157812380, 157812383, 157812385, 157812404, 157812411, 157812414, 157812420, 157812437, 157812442, 157812457, 157812468, 157812470, 157812475, 157812498, 157812542, 157812548; chr4 chromosome's 9783036, 9783050, 9783059, 9783075, 9783080, 9783097, 9783105, 9783112, 9783120, 9783126, 9783142, 9783144, 9783153, 9783160, 9783166, 9783185, 9783192, 9783196, 9783198, 9783206, 9783213, 9783218, 9783220, 9783233, 9783244, 9783246, 9783252, 9783271, 9783275, 9783277, 9783304, 9783322, 9783327, 9783342, 9783348, 9783354, 9783358, 9783361, 9783363, 9783376, 9783398, 9783409, 9783425, 9783427, 9783442, 9783449, 9783467, 9783492, 9783494, 9783496, 9783501, 9783508, 9783511, 39448284, 39448302, 39448320, 39448323, 39448340, 39448343, 39448347, 39448365, 39448422, 39448432, 39448453, 39448464, 39448473, 39448478, 39448481, 39448503, 39448516, 39448524, 39448528, 39448549, 39448551, 39448557, 39448562, 39448568, 39448575, 39448577, 39448586, 39448593, 39448613, 39448625, 39448629, 39448633, 39448647, 39448653, 39448662, 39448665, 39448670, 39448683, 39448695, 39448697, 39448729, 39448732, 39448748, 39448757, 39448759, 39448767, 39448773, 39448796, 39448800, 39448809, 39448811, 39448836, 39448845, 39448857, 39448864, 39448869, 39448874, 57521138, 57521209, 57521237, 57521297, 57521304, 57521310, 57521336, 57521348, 57521377, 57521397, 57521411, 57521419, 57521426, 57521442, 57521449, 57521486, 57521506, 57521518, 57521537, 57521545, 57521581, 57521603, 57521622, 57521631, 57521652, 57521657, 57521665, 57521680, 57521687, 57521701, 57521716, 57521725, 57521733, 154709378, 154709414, 154709425, 154709441, 154709492, 154709513, 154709522, 154709540, 154709557, 154709561, 154709576, 154709591, 154709597, 154709607, 154709612, 154709617, 154709633, 154709640, 154709663, 154709675, 154709684, 154709690, 154709697, 154709721, 154709745, 154709756, 154709759, 154709789, 154709812, 154709828, 154709834; chr5 chromosome's 1876139, 1876168, 1876200, 1876208, 1876213, 1876215, 1876286, 1876290, 1876298, 1876308, 1876311, 1876337, 1876339, 1876347, 1876354, 1876368, 1876372, 1876374, 1876386, 1876395, 1876397, 1876399, 1876403, 1876420, 1876424, 1876432, 1876436, 1876449, 1876456, 1876459, 1876463, 1876483, 1876498, 1876525, 1876527, 1876557, 1876563, 1876570, 1876576, 1876605, 1876630, 1876634, 1876638; chr6 chromosome's 85476921, 85476930, 85476974, 85477014, 85477032, 85477035, 85477070, 85477083, 85477106, 85477124, 85477151, 85477153, 85477166, 85477175, 85477186, 85477217, 85477228, 85477230, 85477236, 85477245, 85477249, 85477251, 85477253, 85477261, 85477283, 137814512, 137814516, 137814523, 137814548, 137814558, 137814561, 137814564, 137814567, 137814620, 137814636, 137814638, 137814642, 137814645, 137814654, 137814666, 137814679, 137814689, 137814695, 137814707, 137814710, 137814717, 137814723, 137814728, 137814744, 137814746, 137814749, 137814768, 137814776, 137814786, 137814788, 137814792, 137814794, 137814803, 137814807, 137814818, 137814824, 137814837, 137814860, 137814920, 137814935, 137814952, 137814957, 137814960, 137814969, 137814971, 137814986, 137814988, 137814995, 137815016, 137815024, 137815030, 137815034, 137815036, 137815040, 150285620, 150285634, 150285641, 150285652, 150285659, 150285661, 150285670, 150285677, 150285688, 150285695, 150285697, 150285706, 150285713, 150285715, 150285724, 150285731, 150285733, 150285742, 150285760, 150285767, 150285769, 150285775, 150285778, 150285788, 150285813, 150285815, 150285826, 150285829, 150285844, 150285860, 150285887, 150285890, 150285892, 150285901, 150285908, 150285910, 150285926, 150285928, 150285937, 150285944, 150285956, 150285963, 150285966, 150285974, 150285981, 150285983, 150285992, 150285999, 150286001, 150286010, 150286017, 150286019, 150286028, 150286035, 150286038, 150286046, 150286055, 150286063, 150286073, 150286082, 150286089, 150286091; chr7 chromosome's 27244531, 27244533, 27244537, 27244555, 27244564, 27244578, 27244603, 27244609, 27244612, 27244619, 27244621, 27244627, 27244631, 27244657, 27244673, 27244702, 27244704, 27244714, 27244723, 27244755, 27244772, 27244780, 27244787, 27244789, 27244798, 27244800, 27244810, 27244833, 27244856, 27244869, 27244874, 27244881, 27244885, 27244887, 27244892, 27244897, 27244907, 27244911, 27244917, 27244920, 27244931, 27244948, 27244951, 27244980, 27244982, 27244986, 27245014, 27245018, 35293441, 35293451, 35293470, 35293479, 35293482, 35293488, 35293492, 35293497, 35293502, 35293506, 35293514, 35293531, 35293537, 35293543, 35293588, 35293590, 35293621, 35293652, 35293656, 35293658, 35293670, 35293676, 35293685, 35293687, 35293690, 35293692, 35293700, 35293717, 35293721, 35293731, 35293747, 35293750, 35293753, 35293759, 35293767, 35293780, 35293783, 35293790, 35293796, 35293809, 35293812, 35293815, 35293821, 35293827, 35293829, 35293834, 35293838, 35293840, 35293847, 35293849, 35293860, 35293863, 35293867, 35293869, 35293879, 35293884, 35293892, 35293940, 50343545, 50343548, 50343552, 50343555, 50343562, 50343566, 50343572, 50343574, 50343577, 50343579, 50343587, 50343603, 50343605, 50343608, 50343611, 50343624, 50343628, 50343630, 50343635, 50343637, 50343639, 50343648, 50343651, 50343654, 50343656, 50343659, 50343663, 50343669, 50343672, 50343674, 50343678, 50343682, 50343693, 50343696, 50343699, 50343702, 50343714, 50343719, 50343725, 50343728, 50343731, 50343736, 50343739, 50343758, 50343765, 50343768, 50343770, 50343785, 50343789, 50343791, 50343805, 50343813, 50343822, 50343824, 50343826, 50343829, 50343831, 50343833, 50343838, 50343847, 50343850, 50343853, 50343858, 50343864, 50343869, 50343872, 50343883, 50343890, 50343897, 50343907, 50343909, 50343914, 50343926, 50343934, 50343939, 50343946, 50343950, 50343959, 50343961, 50343963, 50343969, 50343974, 50343980, 50343990, 50344001, 50344007, 50344011, 50344028, 50344041, 155167320, 155167333, 155167340, 155167343, 155167345, 155167347, 155167350, 155167357, 155167379, 155167382, 155167394, 155167401, 155167423, 155167430, 155167467, 155167478, 155167480, 155167486, 155167499, 155167505, 155167507, 155167511, 155167513, 155167516, 155167518, 155167528, 155167543, 155167552, 155167555, 155167560, 155167562, 155167568, 155167570, 155167578, 155167602, 155167608, 155167611, 155167617, 155167662, 155167702, 155167707, 155167716, 155167718, 155167739, 155167750, 155167753, 155167757, 155167759, 155167771, 155167773, 155167791, 155167801, 155167803, 155167805, 155167813, 155167819, 155167821, 155167827; chr8 chromosome's 10588729, 10588742, 10588820, 10588833, 10588841, 10588851, 10588857, 10588865, 10588867, 10588883, 10588888, 10588895, 10588938, 10588942, 10588946, 10588948, 10588951, 10588959, 10588992, 10589003, 10589007, 10589009, 10589016, 10589034, 10589060, 10589062, 10589076, 10589079, 10589093, 10589152, 10589193, 10589206, 10589241, 25907660, 25907702, 25907709, 25907724, 25907747, 25907752, 25907754, 25907757, 25907769, 25907796, 25907800, 25907814, 25907818, 25907821, 25907824, 25907838, 25907848, 25907866, 25907874, 25907880, 25907884, 25907893, 25907898, 25907900, 25907902, 25907906, 25907918, 25907947, 25907976, 25908055, 25908057, 25908064, 25908071, 25908098, 25908101, 57069480, 57069544, 57069569, 57069606, 57069631, 57069648, 57069688, 57069698, 57069709, 57069712, 57069722, 57069735, 57069739, 57069755, 57069764, 57069773, 57069775, 57069784, 57069786, 57069791, 57069793, 57069800, 57069812, 57069816, 57069823, 57069825, 57069827, 57069839, 57069842, 57069847, 57069851, 57069853, 57069884, 57069889, 57069894, 57069907, 57069914, 57069919, 57069931, 57069940, 57069948, 57069958, 57069968, 57069973, 57069978, 57070013, 57070035, 57070038, 57070042, 57070046, 57070066, 57070079, 57070087, 57070091, 57070126, 57070143; chr10 chromosome's 28034412, 28034415, 28034418, 28034442, 28034444, 28034467, 28034469, 28034494, 28034501, 28034505, 28034545, 28034556, 28034559, 28034568, 28034582, 28034591, 28034596, 28034599, 28034605, 28034616, 28034619, 28034622, 28034624, 28034645, 28034651, 28034654, 28034658, 28034669, 28034682, 28034687, 28034697, 28034711, 28034714, 28034727, 28034729, 28034739, 28034741, 28034751, 28034757, 28034760, 28034763, 28034768, 28034787, 28034790, 28034792, 28034794, 28034797, 28034801, 28034816, 28034843, 28034853, 28034856, 28034867, 28034871, 28034873, 28034882, 28034888, 28034892, 28034907; chr12 chromosome's 4918962, 4918966, 4918968, 4918975, 4918982, 4919001, 4919056, 4919065, 4919079, 4919081, 4919086, 4919095, 4919097, 4919118, 4919124, 4919138, 4919145, 4919147, 4919164, 4919170, 4919173, 4919184, 4919191, 4919199, 4919215, 4919230, 4919236, 4919239, 4919242, 4919253, 4919260, 4919281, 4919293, 4919300, 4919303, 4919309, 4919327, 4919331, 4919351, 4919358, 4919376, 4919386, 4919395, 4919401, 4919408, 4919421, 4919424, 4919430, 4919438, 4919453, 4919465, 4919469, 4919475, 4919486, 33592615, 33592629, 33592635, 33592642, 33592659, 33592661, 33592663, 33592674, 33592681, 33592683, 33592692, 33592704, 33592707, 33592709, 33592711, 33592715, 33592720, 33592725, 33592727, 33592744, 33592774, 33592798, 33592803, 33592811, 33592831, 33592848, 33592859, 33592862, 33592865, 33592867, 33592875, 33592882, 33592885, 33592887, 33592891, 33592905, 33592908, 33592913, 33592915, 33592923, 33592931, 33592933, 33592953, 33592955, 33592977, 33592981, 33592986, 33592989, 33592998, 33593004, 33593017, 33593035, 33593049, 33593090, 33593093, 58131100, 58131102, 58131111, 58131133, 58131154, 58131168, 58131175, 58131181, 58131224, 58131242, 58131261, 58131277, 58131300, 58131303, 58131306, 58131309, 58131312, 58131318, 58131321, 58131331, 58131345, 58131348, 58131384, 58131390, 58131404, 58131412, 58131414, 58131426, 58131429, 58131445, 58131453, 58131475, 58131478, 58131487, 58131503, 58131510, 58131523, 58131546, 58131549, 58131553, 58131557, 58131564, 58131571, 58131576, 58131586, 58131605, 58131608, 58131624, 58131642, 115124768, 115124773, 115124782, 115124811, 115124838, 115124853, 115124871, 115124874, 115124894, 115124904, 115124924, 115124930, 115124933, 115124935, 115124946, 115124970, 115124973, 115124981, 115124999, 115125013, 115125034, 115125053, 115125060, 115125098, 115125107, 115125114, 115125121, 115125131, 115125141, 115125151, 115125177, 115125192, 115125225, 115125305, 115125335; chr13 chromosome's 37005452, 37005489, 37005501, 37005520, 37005551, 37005553, 37005557, 37005562, 37005566, 37005570, 37005582, 37005596, 37005608, 37005629, 37005633, 37005635, 37005673, 37005678, 37005686, 37005694, 37005704, 37005706, 37005721, 37005732, 37005738, 37005741, 37005745, 37005773, 37005778, 37005794, 37005801, 37005805, 37005814, 37005816, 37005821, 37005833, 37005835, 37005844, 37005855, 37005857, 37005878, 37005881, 37005883, 37005892, 37005899, 37005909, 37005924, 37005929, 37005934, 37005939, 37005941, 100649486, 100649489, 100649519, 100649538, 100649567, 100649569, 100649577, 100649584, 100649601, 100649603, 100649605, 100649623, 100649625, 100649628, 100649648, 100649671, 100649673, 100649686, 100649689, 100649691, 100649701, 100649705, 100649715, 100649718, 100649721, 100649725, 100649731, 100649734, 100649738, 100649740, 100649745, 100649763, 100649769, 100649777, 100649785, 100649792, 100649800, 100649847, 100649886, 100649912, 100649915, 100649917, 100649941, 100649945, 100649949, 100649965, 100649975, 100649982, 100650005; chr14 chromosome's 38724435, 38724459, 38724473, 38724486, 38724507, 38724511, 38724527, 38724531, 38724534, 38724540, 38724544, 38724546, 38724565, 38724578, 38724586, 38724597, 38724624, 38724627, 38724646, 38724648, 38724650, 38724669, 38724675, 38724680, 38724682, 38724685, 38724726, 38724732, 38724734, 38724746, 38724765, 38724771, 38724780, 38724796, 38724798, 38724806, 38724808, 38724810, 38724821, 38724847, 38724852, 38724858, 38724864, 38724867, 38724873, 38724896, 38724906, 38724929, 38724935, 38724945, 38724978, 38724995, 38725003, 38725005, 38725014, 38725016, 38725023, 38725026, 38725030, 38725034, 38725038, 38725048, 38725058, 38725077, 38725081, 38725088, 38725101, 57275669, 57275674, 57275677, 57275681, 57275683, 57275687, 57275690, 57275706, 57275725, 57275749, 57275752, 57275761, 57275768, 57275772, 57275778, 57275785, 57275821, 57275823, 57275827, 57275829, 57275831, 57275835, 57275852, 57275874, 57275876, 57275885, 57275896, 57275908, 57275912, 57275914, 57275924, 57275956, 57275967, 57275969, 57275971, 57275981, 57275988, 57275993, 57275995, 57276000, 57276031, 57276035, 57276039, 57276057, 57276066, 57276073, 57276090, 60952394, 60952398, 60952405, 60952418, 60952421, 60952425, 60952464, 60952468, 60952482, 60952500, 60952503, 60952505, 60952517, 60952522, 60952544, 60952550, 60952554, 60952593, 60952599, 60952615, 60952618, 60952634, 60952658, 60952683, 60952687, 60952730, 60952738, 60952755, 60952762, 60952781, 60952791, 60952799, 60952827, 60952829, 60952836, 60952839, 60952841, 60952848, 60952855, 60952857, 60952870, 60952876, 60952878, 60952887, 60952896, 60952898, 60952908, 60952919, 60952921, 60952931; chr15 chromosome's 83952068, 83952081, 83952084, 83952087, 83952095, 83952105, 83952108, 83952114, 83952125, 83952135, 83952140, 83952156, 83952160, 83952162, 83952175, 83952178, 83952181, 83952184, 83952188, 83952200, 83952206, 83952209, 83952214, 83952220, 83952225, 83952229, 83952236, 83952238, 83952242, 83952266, 83952285, 83952291, 83952298, 83952309, 83952314, 83952317, 83952345, 83952352, 83952358, 83952360, 83952367, 83952406, 83952411, 83952414, 83952418, 83952420, 83952425, 83952430, 83952453, 83952464, 83952472, 83952486, 83952496, 83952498, 83952500, 83952506, 83952508, 83952527, 83952553, 83952559, 83952566, 83952570, 83952582, 83952592; chr16 chromosome's 31579976, 31580071, 31580078, 31580081, 31580089, 31580100, 31580110, 31580117, 31580138, 31580150, 31580153, 31580159, 31580165, 31580220, 31580246, 31580254, 31580269, 31580287, 31580296, 31580299, 31580309, 31580311, 31580316, 31580343, 31580424, 31580496, 31580524, 31580560, 73096786, 73096842, 73096889, 73096894, 73096903, 73096914, 73096923, 73096929, 73096934, 73096943, 73096948, 73096966, 73096970, 73096979, 73097000, 73097015, 73097017, 73097019, 73097028, 73097037, 73097045, 73097057, 73097060, 73097066, 73097069, 73097078, 73097080, 73097082, 73097084, 73097108, 73097114, 73097142, 73097156, 73097183, 73097260, 73097267, 73097284, 73097296, 73097301, 73097329, 73097357, 73097364, 73097377, 73097381, 73097387, 73097470; chr17 chromosome's 35299698, 35299703, 35299710, 35299719, 35299729, 35299731, 35299741, 35299746, 35299776, 35299813, 35299816, 35299822, 35299837, 35299850, 35299877, 35299885, 35299913, 35299915, 35299926, 35299928, 35299933, 35299935, 35299944, 35299946, 35299963, 35299966, 35299972, 35299974, 35299990, 35299996, 35299999, 35300006, 35300010, 35300020, 35300027, 35300036, 35300039, 35300044, 35300059, 35300068, 35300074, 35300086, 35300097, 35300109, 35300115, 35300146, 35300151, 35300163, 35300167, 35300172, 35300196, 35300202, 35300214, 35300217, 35300221, 76929645, 76929709, 76929713, 76929742, 76929769, 76929829, 76929873, 76929926, 76929982, 76930043, 76930095, 76930148, 76930169, 80846623, 80846652, 80846683, 80846709, 80846717, 80846730, 80846745, 80846763, 80846794, 80846860, 80846867, 80846886, 80846960, 80846965, 80847079, 80847092, 80847115, 80847128, 80847137, 80847153, 80847158, 80847209; chr21 chromosome's 38081248, 38081253, 38081300, 38081303, 38081306, 38081321, 38081327, 38081333, 38081341, 38081344, 38081352, 38081354, 38081356, 38081363, 38081394, 38081396, 38081407, 38081421, 38081430, 38081443, 38081454, 38081461, 38081478, 38081480, 38081492, 38081497, 38081499, 38081502, 38081514, 38081517, 38081520, 38081537, 38081557, 38081563, 38081566, 38081577, 38081583, 38081586, 38081606, 38081625, 38081642, 38081665, 38081695, 38081707, 38081719, 38081725, 38081732. The bases of the above-mentioned methylation sites are numbered corresponding to the reference genome HG19.

In one or more embodiments, the differentiation between pancreatic cancer and pancreatitis is correlated with the methylation level of sequences from genes selected from any of the following combinations: (1) SIX3, TLX2; (2) SIX3, CILP2; (3) TLX2, CILP2; (4) SIX3, TLX2, CILP2. The present invention provides nucleic acid molecules containing one or more CpGs of the above-mentioned genes or fragments thereof.

Further, the differentiation between pancreatic cancer and pancreatitis is related to the methylation level of any one segment or random two or all three segments selected from: SEQ ID NO:57 in the SIX3 gene region, SEQ ID NO:58 in the TLX2 gene region and SEQ ID NO:59 in the CILP2 gene region.

In some embodiments, the differentiation between pancreatic cancer and pancreatitis correlates with the methylation level of a sequence selected from any one of the group consisting of (1) SEQ ID NO:57, SEQ ID NO:58, (2) SEQ ID NO:57, SEQ ID NO:59, (3) SEQ ID NO:58, SEQ ID NO:59, (4) SEQ ID NO:57, SEQ ID NO:58, SEQ ID NO:59, or complementary sequences thereof.

The "sequence related to differentiation between pancreatic cancer and pancreatitis" described herein includes the above-mentioned 3 genes, sequences within 20kb upstream or downstream thereof, the above 3 sequences (SEQ ID NOs:57-59) or complementary sequences thereof.

The positions of the above-mentioned 3 sequences in the human chromosome are as follows: SEQ ID NO:57: chr2's 45028785-45029307, SEQ ID NO:58: chr2's 74742834-74743351, SEQ ID NO:59: chr19's 19650745-19651270. Herein, the bases of the sequences and methylation sites are numbered corresponding to the reference genome HG19.

In one or more embodiments, the nucleic acid molecule described herein is a fragment of one or more genes selected from SIX3, TLX2, CILP2; the length of the fragment is 1bp-1kb, preferably 1bp-700bp; the fragment comprises one or more methylation sites of the corresponding gene in the chromosomal region. The methylation sites in the genes or fragments thereof described herein include, but are not limited to: chr2's 45028802, 45028816, 45028832, 45028839, 45028956, 45028961, 45028965, 45028973, 45029004, 45029017, 45029035, 45029046, 45029057, 45029060, 45029063, 45029065, 45029071, 45029106, 45029112, 45029117, 45029128, 45029146, 45029176, 45029179, 45029184, 45029189, 45029192, 45029195, 45029218, 45029226, 45029228, 45029231, 45029235, 45029263, 45029273, 45029285, 45029288, 45029295,74742838, 74742840, 74742844, 74742855, 74742879, 74742882, 74742891, 74742913, 74742922, 74742925, 74742942, 74742950, 74742953, 74742967, 74742981, 74742984, 74742996, 74743004, 74743006, 74743009, 74743011, 74743015, 74743021, 74743035, 74743056, 74743059, 74743061, 74743064, 74743068, 74743073, 74743082, 74743084, 74743101, 74743108, 74743111, 74743119, 74743121, 74743127, 74743131, 74743137, 74743139, 74743141, 74743146, 74743172, 74743174, 74743182, 74743186, 74743191, 74743195, 74743198, 74743207, 74743231, 74743234, 74743241, 74743243, 74743268, 74743295, 74743301, 74743306, 74743318, 74743321, 74743325, 74743329, 74743333, 74743336, 74743343, 74743346; chr19's 19650766, 19650791, 19650796, 19650822, 19650837, 19650839, 19650874, 19650882, 19650887, 19650893, 19650895, 19650899, 19650907, 19650917, 19650955, 19650978, 19650981, 19650995, 19650997, 19651001, 19651008, 19651020, 19651028, 19651041, 19651053, 19651059, 19651062, 19651065, 19651071, 19651090, 19651101, 19651109, 19651111, 19651113, 19651121, 19651123, 19651127, 19651133, 19651142, 19651144, 19651151, 19651166, 19651170, 19651173, 19651176, 19651179, 19651183, 19651185, 19651202, 19651204, 19651206, 19651225, 19651227, 19651235, 19651237, 19651243, 19651246, 19651263, 19651267. The unmutated bases of the above methylation sites are numbered corresponding to the reference genome HG19.

In one or more embodiments, the differentiation between pancreatic cancer and pancreatitis is related to the methylation level of sequences from genes selected from any one of: ARHGEF16, PRDM16, NFIA, ST6GALNAC5, PRRX1, LHX4, ACBD6, FMN2, CHRM3, FAM150B, TMEM18, SIX3, CAMKMT, OTX1, WDPCP, CYP26B1, DYSF, HOXD1, HOXD4, UBE2F, RAMP1, AMT, PLSCR5, ZIC4, PEX5L, ETV5, DGKG, FGF12, FGFRL1, RNF212, DOK7, HGFAC, EVC, EVC2, HMX1, CPZ, IRX1, GDNF, AGGF1, CRHBP, PITX1, CATSPER3, NEUROG1, NPM1, TLX3, NKX2-5, BNIP1, PROP1, B4GALT7, IRF4, FOXF2, FOXQ1, FOXC1, GMDS, MOCS1, LRFN2, POU3F2, FBXL4, CCR6, GPR31, TBX20, HERPUD2, VIPR2, LZTS1, NKX2-6, PENK, PRDM14, VPS13B, OSR2, NEK6, LHX2, DDIT4, DNAJB12, CRTAC1, PAX2, HIF1AN, ELOVL3, INA, HMX2, HMX3, MKI67, DPYSL4, STK32C, INS, INS-IGF2, ASCL2, PAX6, RELT, FAM168A, OPCML, ACVR1B, ACVRL1, AVPR1A, LHX5, SDSL, RAB20, COL4A2, CARKD, CARS2, SOX1, TEX29, SPACA7, SFTA3, SIX6, SIX1, INF2, TMEM179, CRIP2, MTA1, PIAS1, SKOR1, ISL2, SCAPER, POLG, RHCG, NR2F2, RAB40C, PIGQ, CPNE2, NLRC5, PSKH1, NRN1L, SRR, HIC1, HOXB9, PRAC1, SMIM5, MYO15B, TNRC6C, 9-Sep, TBCD, ZNF750, KCTD1, SALL3, CTDP1, NFATC1, ZNF554, THOP1, CACTIN, PIP5K1C, KDM4B, PLIN3, EPS15L1, KLF2, EPS8L1, PPP1R12C, NKX2-4, NKX2-2, TFAP2C, RAE1, TNFRSF6B, ARFRP1, MYH9, and TXN2. The present invention provides nucleic acid molecules containing one or more CpGs of the above-mentioned genes or fragments thereof.

In some embodiments, the differentiation between pancreatic cancer and pancreatitis is correlated with the methylation level of sequences selected from any of the group consisting of SEQ ID NOs: 60-160, or complementary sequences thereof.

The "sequence related to differentiation between pancreatic cancer and pancreatitis" described herein includes the above-mentioned 101 genes, sequences within 20kb upstream or downstream thereof, the above-mentioned 101 sequences (SEQ ID NOs:60-160) or complementary sequences thereof. Herein, the bases of the sequences and methylation sites are numbered corresponding to the reference genome HG19.

In one or more embodiments, the length of the nucleic acid molecule is 1bp-1000bp, 1bp-900bp, 1bp-800bp, 1bp-700bp. The length of the nucleic acid molecule may be a range between any of the above end values.

As used herein, methods for detecting DNA methylation are well known in the art, such as bisulfite conversion-based PCR (e.g., methylation-specific PCR (MSP)), DNA sequencing, whole-genome methylation sequencing, simplified methylation sequencing, methylation-sensitive restriction enzyme assay, fluorescence quantitation, methylation-sensitive high-resolution melting curve assay, chip-based methylation atlas, mass spectrometry. In one or more embodiments, the detection includes detecting any strand at a gene or site.

Accordingly, the present invention relates to reagents for detecting DNA methylation. The reagents used in the above-mentioned methods of detecting DNA methylation are well known in the art. In detection methods involving DNA amplification, reagents for detecting DNA methylation include primers. The sequence of the primer is methylation specific or non-specific. The sequence of the primer may include a non-methylation specific blocker. The blocker can improve the specificity of methylation detection. Reagents for detecting DNA methylation may also include probes. Typically, the 5' end of the probe sequence is labeled with a fluorescent reporter and the 3' end is labeled with a quencher. Exemplarily, the sequence of the probe includes MGB (minor groove binder) or LNA (locked nucleic acid). MGB and LNA are used to increase the Tm value, increase the specificity of the assay, and increase the flexibility of probe design. "Primer" as used herein refers to a nucleic acid molecule with a specific nucleotide sequence that guides synthesis when nucleotide polymerization is initiated. Primers are usually two artificially synthesized oligonucleotide sequences. One primer is complementary to a DNA template strand at one end of the target region, the other primer is complementary to another DNA template strand at the other end of the target region, and they serve as the starting point of nucleotide polymerization. Primers are usually at least 9bp. In vitro artificially designed primers are widely used in polymerase chain reaction (PCR), qPCR, sequencing and probe synthesis. Typically, primers are designed to make the amplified product have a length of 1-2000 bp, 10-1000 bp, 30-900 bp, 40-800 bp, 50-700 bp, or at least 150 bp, at least 140 bp, at least 130 bp, at least 120 bp.

The term "variant" or" mutant" herein refers to a polynucleotide whose nucleic acid sequence is changed by insertion, deletion or substitution of one or more nucleotides compared with a reference sequence while retaining its ability to hybridize with other nucleic acids. Mutants according to any of embodiments herein include nucleotide sequences having at least 70%, preferably at least 80%, preferably at least 85%, preferably at least 90%, preferably at least 95%, preferably at least 97% sequence identity to a reference sequence while retaining the biological activity of the reference sequence. Sequence identity between two aligned sequences can be calculated using, for example, NCBI's BLASTn. Mutants also include nucleotide sequences that have one or more mutations (insertions, deletions, or substitutions) in the nucleotide sequence of the reference sequence while still retaining the biological activity of the reference sequence. The plurality of mutations usually refer to mutations within 1-10, such as 1-8, 1-5 or 1-3. The substitution may be between purine nucleotides and pyrimidine nucleotides, or between purine nucleotides or between pyrimidine nucleotides. Substitutions are preferably conservative substitutions. For example, in the art, conservative substitutions with nucleotides with like or similar properties generally do not alter the stability and function of the polynucleotide. Conservative substitutions include the exchange between purine nucleotides (A and G) and the exchange between pyrimidine nucleotides (T or U and C). Therefore, substitution of one or several sites in a polynucleotide of the present invention with residues from the same side chain will not materially affect its activity. Furthermore, methylation sites (such as consecutive CGs) are not mutated in the variants of the present invention. That is, the method of the present invention detects the methylation status of methylatable sites in the corresponding sequence, and mutations can occur in bases at non-methylatable sites. Typically, methylation sites are consecutive CpG dinucleotides.

As described herein, conversions can occur between bases of DNA or RNA. The "conversion", "cytosine conversion" or "CT conversion" described herein is the process of converting an unmodified cytosine (C) to a base (e.g., uracil (U)) that is less capable of binding to guanine than cytosine by treating DNA using a non-enzymatic or enzymatic method. Non-enzymatic or enzymatic methods for converting cytosine are well known in the art. Exemplarily, non-enzymatic methods include treatment with conversion reagents such as bisulfite, acid sulfite or metabisulfite, such as calcium bisulfite, sodium bisulfite, potassium bisulfite, ammonium bisulfite, sodium bisulfate, potassium bisulfate and ammonium bisulfate. Exemplarily, enzymatic methods include deaminase treatment. The converted DNA is optionally purified. DNA purification methods suitable for use herein are well known in the art.

The present invention further provides a methylation detection kit for diagnosing pancreatic cancer. The kit comprises the primers and/or probes described herein and is used to detect the methylation level of pancreatic cancer-related sequences discovered by the inventors. The kit may also comprise a nucleic acid molecule described herein, particularly as described in the first aspect, as an internal standard or positive control. The term "hybridization" described herein mainly refers to the pairing of nucleic acid sequences under stringent conditions. Exemplary stringent conditions are hybridization and membrane washing at 65°C in a solution of $0.1 \times$ SSPE (or $0.1 \times$ SSC) and 0.1% SDS.

In addition to the primers, probes, and nucleic acid molecules, the kit also comprises other reagents required for detecting DNA methylation. Exemplarily, other reagents for detecting DNA methylation may include one or more of the following: bisulfite and derivatives thereof, PCR buffers, polymerase, dNTPs, primers, probes, methylation-sensitive or insensitive restriction endonucleases, digestion buffers, fluorescent dyes, fluorescent quenchers, fluorescent reporters, exonucleases, alkaline phosphatases, internal standards, and controls.

The kit may also comprise a converted positive standard in which unmethylated cytosine is converted to a base that does not bind to guanine. The positive standard may be fully methylated. The kit may also comprise PCR reaction reagents. Preferably, the PCR reaction reagents include Taq DNA polymerase, PCR buffer, dNTPs, and $Mg^{2+}$.

The present invention further provides a method for screening pancreatic cancer, comprising: (1) detecting the methylation level of the pancreatic cancer-related sequence described herein in a sample of a subject; (2) obtaining a score by comparing it with the control sample and/or reference level or by calculation; (3) identifying whether the subject has pancreatic cancer based on the score. Usually, before step (1), the method further comprises: extraction and quality inspection of sample DNA, and/or converting unmethylated cytosine on the DNA into bases that do not bind to guanine.

In a specific embodiment, step (1) comprises: treating genomic DNA or cfDNA with a conversion reagent to convert unmethylated cytosine into a base (such as uracil) with a lower binding capacity to guanine than to cytosine; performing PCR amplification using primers suitable for amplifying the converted sequences of pancreatic cancer-related sequences described herein; determining the methylation status or level of at least one CpG by the presence or absence of amplified products, or by sequence identification (e.g., probe-based PCR identification or DNA sequencing identification).

Alternatively, step (1) may further comprise: treating genomic DNA or cfDNA with a methylation-sensitive restriction endonuclease; performing PCR amplification using primers suitable for amplifying the sequence of at least one CpG of the pancreatic cancer-related sequences described herein; determining the methylation status or level of at least one CpG by the presence or absence of amplification products. The "methylation level" described herein includes the relationship of methylation status of any number of CpGs at any position in the sequence of interest. The relationship may be the addition or subtraction of methylation status parameters (e.g., 0 or 1) or the calculation result of a mathematical algorithm (e.g., mean, percentage, fraction, ratio, degree, or calculation using a mathematical model), including but not limited to methylation level measure, methylated haplotype fraction, or methylated haplotype load. The term "methylation status" displays the methylation of specific CpG sites, typically including methylated or unmethylated (e.g., methylation status parameter 0 or 1).

In one or more embodiments, the methylation level in the sample of the subject is increased or decreased when compared to control samples and/or reference levels. When methylation marker levels meet a certain threshold, pancreatic cancer is identified. Alternatively, the methylation levels of the tested genes can be mathematically analyzed to obtain a score. For the tested samples, when the score is greater than the threshold, the determination result is positive, that is, pancreatic cancer is present; otherwise, it is negative, that is, there is no pancreatic cancer plasma. Conventional mathematical analysis methods and the process of determining thresholds are known in the art. An exemplary method is a mathematical model. For example, for differential methylation markers, a support vector machine (SVM) model is constructed for two groups of samples, and the model is used to statistically analyze the precision, sensitivity and specificity of the detection results as well as the area under the prediction value characteristic curve (ROC) (AUC), and statistically analyze the prediction scores of the test set samples.

In one or more embodiments, the methylation level in the sample of the subject is increased or decreased when compared to control samples and/or reference levels. When methylation marker levels meet a certain threshold, pancreatic cancer is identified, otherwise it is chronic pancreatitis. Alternatively, the methylation levels of the tested genes can be mathematically analyzed to obtain a score. For the tested sample, when the score is greater than the threshold, the differentiation result is positive, that is, pancreatic cancer is present; otherwise, it is negative, that is, it is pancreatitis. Conventional mathematical analysis methods and processes for determining thresholds are known in the art, and an exemplary method is the support vector machine (SVM) mathematical model. For example, for differential methylation markers, a support vector machine (SVM) is constructed for the samples of the training group, and the precision, sensitivity and specificity of the detection results as well as the area under the prediction value characteristic curve (ROC) (AUC) are statistically analyzed using the model, and the prediction scores of the samples of the test set are statistically analyzed. In an embodiment of the support vector machine, the score threshold is 0.897. If the score is greater than 0.897, the subject is considered to be a patient with pancreatic cancer; otherwise, the subject is a patient with chronic pancreatitis.

In a preferred embodiment, the model training process is as follows: first, obtaining differentially methylated segments according to the methylation level of each site and constructing a differentially methylated region matrix, for example, constructing a methylation data matrix from the methylation level data of a single CpG dinucleotide position in the HG19 genome through, for example, samtools software; then training the SVM model.

The exemplary SVM model training process is as follows:

a) A training model mode is constructed. The sklearn software package (0.23.1) of python software (v3.6.9) is used to construct the training model and cross-validate the training mode of the training model, command line: model = SVR().

b) The sklearn software package (0.23.1) is used to input the data matrix to construct the SVM model, model.fit(x_train, y_train), where x_train represents the training set data matrix, and y_train represents the phenotypic information of the training set.

Typically, during model construction, the category with pancreatic cancer can be coded as 1 and the category without pancreatic cancer as 0. In the present invention, the threshold is set as 0.895 by python software (v3.6.9) and sklearn software package (0.23.1). The constructed model finally differentiates samples with or without pancreatic cancer by 0.895.

Here, the sample is from a mammal, preferably a human. The sample can be from any organ (e.g., pancreas), tissue (e.g., epithelial tissue, connective tissue, muscle tissue, and neural tissue), cell (e.g., pancreatic cancer biopsy), or body fluid (e.g., blood, plasma, serum, interstitial fluid, urine). Generally, it is sufficient as long as the sample contains genomic DNA or cfDNA (circulating-free DNA or cell-free DNA). cfDNA, called circulating-free DNA or cell-free DNA, is degraded DNA fragments released into plasma. Exemplarily, the sample is a pancreatic cancer biopsy, preferably a fine needle aspiration biopsy. Alternatively, the sample is plasma or cfDNA.

The present application further relates to methods for obtaining methylated haplotype fractions associated with pancreatic cancer. Taking the methylation data obtained by methylation-targeted sequencing (MethylTitan) as an example, the process of screening and testing marker sites is as follows: original paired-end sequencing reads - combining the reads to obtain combined single-end reads - removing the adapters to obtain adapter-free reads - Bismark aligning to the human DNA genome to form a BAM file - extracting the CpG site methylation level of each read by samtools to form a haplotype file - statistically analyzing the C site methylated haplotype fraction to form meth file - calculating MHF (methylated haplotype fraction - using Coverage 200 to filter sites to form meth.matrix matrix file - filtering based on NA value greater than 0.1 to filter sites - pre-dividing samples into training set and test set - constructing a logistic regression model of phenotype for each haplotype in the training set, selecting the regression P value of each methylated haplotype fraction - statistically analyzing each MethylTitan amplification region and selecting the methylated haplotype with the most significant P value to represent the methylation level of the region and modeling through support vector machine - forming the results of the training set (ROC plot) and predicting the test set using the model for validation. Specifically, the method for obtaining methylated haplotypes related to pancreatic cancer comprises the following steps: (1) obtaining plasma samples from patients with or without pancreatic cancer to be tested, extracting cfDNA, using the MethylTitan method to perform library constructing and sequencing, and obtaining sequencing reads; (2) pre-processing sequencing data, including adapter-removing and splicing of the sequencing data generated by the sequencer; (3) aligning the sequencing data after the above pre-processing to the HG19 reference genome sequence of the human genome to determine the position of each fragment. The data in step (2) can come from Illumina sequencing platform paired-end 150bp sequencing. The adapter-removing in step (2) is to remove the sequencing adapters at the 5' end and 3' end of the two paired-end sequencing data respectively, as well as remove the low-quality bases after removing the adapters. The splicing process in step (2) is to combine the paired-end sequencing data and restore them to the original library fragments. This allows for better alignment and accurate positioning of sequencing fragments. For example, the length of the sequencing library is about 180 bp, and the paired ends of 150 bp can completely cover the entire library fragment. Step (3) comprises: (a) performing CT and GA conversion on the HG19 reference genome data respectively to construct two sets of converted reference genomes, and construct alignment indexes for the converted reference genomes respectively; (b) performing CT and GA conversion on the upper combined sequencing sequence data as well; (c) aligning the above converted reference genome sequences, respectively, and finally summarizing the alignment results to determine the position of the sequencing data in the reference genome.

In addition, the method for obtaining methylation values related to pancreatic cancer also comprises (4) calculation of MHF; (5) construction of methylated haplotype MHF data matrix; and (6) construction of logistic regression model of each methylated haplotype according to sample grouping. Step (4) involves obtaining the methylated haplotype status and sequencing depth information at the position of the HG19 reference genome based on the alignment results obtained in step (3). Step (5) involves combining methylated haplotype status and sequencing depth information data into a data matrix. Among them, each data point with a depth less than 200 is treated as a missing value, and the K nearest neighbor (KNN) method is used to fill the missing values. Step (6) consists of screening haplotypes with significant regression coefficients between the two groups based on statistical modeling of each position in the above matrix using logistic regression.

The present invention explores the relationship between DNA methylation and CA19-9 levels and pancreatic cancer and pancreatitis. It is intended to use the marker cluster DNA methylation level and the CA19-9 level as markers for differentiation between pancreatic cancer and chronic pancreatitis through non-invasive methods to improve the accuracy of non-invasive diagnosis of pancreatic cancer.

The inventors found that if the CA19-9 level is combined in pancreatic cancer marker screening and diagnosis, the diagnostic accuracy can be significantly improved.

The present invention first provides a method for screening pancreatic cancer methylation markers, comprising: (1) obtaining the methylated haplotype fraction and sequencing depth of the DNA segment of a genome (such as cfDNA) of a subject, optionally (2) pre-processing the methylated haplotype fraction and sequencing depth data, and (3) performing cross-validation incremental feature selection to obtain feature methylated segments.

The data acquisition in step (1) can be data analysis after methylation detection or reading directly from the file. In embodiments where methylation detection is carried out, step (1) comprises: 1.1) detecting DNA methylation of a sample of a subject to obtain sequencing read data, 1.3) aligning the sequencing data to a reference genome to obtain the location and sequencing depth information of the methylated segment, 1.4) calculating the methylated haplotype fraction (MHF) of the segment according to the following formula:

$$MHF_{i,h} = \frac{N_{i,h}}{N_i}$$

where $i$ represents the target methylated region, $h$ represents the target methylated haplotype, $N_i$ represents the number of reads located in the target methylated region, and $N_{i,h}$ represents the number of reads containing the target methylated haplotype. Typically, methylated haplotype fraction need to be calculated for each methylated haplotype within the target region. This step may also comprise 1.2) steps of pre-processing the sequencing data, such as adapter removing and/or splicing.

Step (2) comprises a step of combining methylated haplotype ratio and sequencing depth information data into a data matrix. In addition, in order to make the results more accurate, step (2) also comprises: removing sites with a missing value proportion higher than 5-15% (for example, 10%) in the data matrix, and for each data point with a depth less than 300 (for example, less than 200), it is treated as a missing value, and the missing values are imputed using the K nearest neighbor method.

In one or more embodiments, step (3) comprises: using a mathematical model to perform cross-validation incremental feature selection in the training data, wherein the DNA segments that increase the AUC of the mathematical model are feature methylated segments. Among them, the mathematical model can be a support vector machine model (SVM) or a random forest model. Preferably, step (3) comprises: (3.1) ranking the relevance of DNA segments according to their methylated haplotype fraction and sequencing depth to obtain highly relevant candidate methylated segments, and (3.2) performing cross-validation incremental feature selection, wherein the candidate methylated segments are ranked according to relevance (for example, according to regression coefficient in descending order), one or more candidate methylated segment data are added each time, and the test data are predicted, wherein candidate methylated segments whose mean cross-validation AUC increases are feature methylated segments. Among them, step (3.1) can specifically invovle: constructing a logistic regression model based on the methylated haplotype fraction and sequencing depth of the DNA segment with respect to the subject's phenotype, and screening out the DNA segments with large regression coefficients to form candidate methylated segments. The prediction in step (3.2) can be made by constructing a model (such as a support vector machine model or a random forest model).

After obtaining the feature methylated segments, they can be combined with CA19-9 levels to build a more accurate pancreatic cancer diagnostic model. Therefore, in the method of constructing a pancreatic cancer diagnostic model, in addition to the above steps (1)-(3), it also comprises (4) constructing a mathematical model for the data of the feature methylated segment to obtain methylation scores, and (5) combining the methylation score and CA19-9 level into a data matrix, and constructing a pancreatic cancer diagnostic model based on the data matrix. The "data" in step (4) are the methylation detection results of feature methylated segments, preferably a matrix combining methylated haplotype fraction with sequencing depth.

The mathematical model in step (4) can be any mathematical model commonly used for diagnostic data analysis, such as support vector machine (SVM) model, random forest, and regression model. Herein, an exemplary mathematical model is a vector machine (SVM) model.

The pancreatic cancer diagnostic model in step (5) can be any mathematical model used for diagnostic data analysis, such as support vector machine (SVM) model, random forest, and regression model. Herein, an exemplary pancreatic cancer diagnostic model is the logistic regression pancreatic cancer model shown below:

$$y = \frac{1}{1 + e^{-(0.7032M + 0.6608C + 2.2243)}}$$

where M is the methylation score of the sample, and C is the CA19-9 level of the sample. In one or more embodiments, the model threshold is 0.885, a value higher than this value is determined to indicate pancreatic cancer, and a value lower than or equal to this value is determined to indicate absence of pancreatic cancer.

In specific embodiments, a machine learning-based method for differentiating pancreatitis and pancreatic cancer comprises:

(1) extracting the blood of a patient with pancreatic cancer or pancreatitis to be tested, and collecting patient age, gender, CA19-9 test value and other information; (2) obtaining plasma samples from the patient with pancreatic cancer or pancreatitis to be tested, extracting cfDNA, and using the MethylTitan method to create library and perform sequencing to obtain sequencing reads; (3) pre-processing sequencing data, including performing adapter removal and splicing on the sequencing data generated by the sequencer; (4) aligning the above-mentioned pre-processed sequencing data to the reference genome sequence to determine the position of each fragment; (5) calculation of the MHF (Methylated Haplotype Fraction) methylation numerical matrix: a target methylated region may have multiple methylated haplotypes, for each methylated haplotype in the target region, it needs to calculate this value, and the MHF calculation formula is illustrated as follows:

$$MHF_{i,h} = \frac{N_{i,h}}{N_i}$$

where i represents the target methylated region, h represents the target methylated haplotype, Ni represents the number of reads located in the target methylated region, Ni,h represents the number of reads containing the target methylated haplotype; (6) for a position in the reference genome, obtaining the methylated haplotype fraction and sequencing depth information at that position, and combining the methylated haplotype fraction and sequencing depth information data into a data matrix; removing sites with a missing value proportion higher than 10%, taking each data point with a depth less than 200 as a missing value, and using the K nearest neighbor (KNN) method to impute the missing values; (7) dividing all samples into two parts, one being the training set and the other being the test set; (8) discovering feature methylated segments according to the training set sample group: constructing a logistic regression model for each methylated segment for the phenotype, and for each amplified target region, screening to select methylated segments with the most significant regression coefficient to form candidate methylated segments. The training set is randomly divided into ten parts for ten-fold cross-validation incremental feature selection. The candidate methylated segments in each region are ranked in descending order according to the significance of the regression coefficient, and the data of one methylated segment is added each time to predict the test data (constructing a vector machine (SVM) model for prediction). The differentiation index is the mean value of the 10-time cross-validation AUCs. If the AUC of the training data increases, the candidate methylated segment will be retained as the feature methylated segment, otherwise it will be discarded; (9) incorporating the data of the characteristic methylated region in the training set screened in step (8) into the support vector machine (SVM) model, and verifying the performance of the model in the test set; (10) incorporating the data matrix combining the prediction score of the training set SVM model in step (9) and the CA19-9 measurements corresponding to the training set samples into the logistic regression model, and verifying the performance of the model combined with CA19-9 in the test set.

The present invention further provides a kit for diagnosing pancreatic cancer, wherein the kit includes a reagent or device for detecting DNA methylation and a reagent or device for detecting CA19-9 level.

Reagents for detecting DNA methylation are used to determine the methylation level of a DNA sequence or a fragment thereof or the methylation status or level of one or more CpG dinucleotides in the DNA sequence or fragment thereof in a sample of a subject. Exemplary reagents for detecting DNA methylation include primers and/or probes described herein for detecting methylation levels of sequences related to differentiation between pancreatic cancer and pancreatitis found by the inventors.

The CA19-9 level described herein mainly refers to the CA19-9 level in body fluids (such as blood or plasma). Reagents for detecting CA19-9 levels can be any reagents known in the art that can be used in CA19-9 detection methods, such as detection reagents based on immune reactions, including but not limited to: antibodies against CA19-9, and optional buffers, washing liquids, etc. The exemplary detection method used in the present invention detects the content of CA19-9 through chemiluminescence immunoassay. The specific steps are as follows: first, an antibody against CA19-9 is labeled with a chemiluminescence marker (acridinium ester), and the labeled antibody and CA19-9 antigen undergo an immune reaction to form a CA19-9 antigen-acridinium ester labeled antibody complex, and then an oxidizing agent ($H_2O_2$) and NaOH are added to form an alkaline environment. At this time, the acridinium ester can decompose and emit light without a catalyst. The photon energy generated per unit time is received and recorded by the light collector and photomultiplier tube (chemiluminescence detector). The integral of this light is proportional to the amount of CA19-9 antigen, and the content of CA19-9 can be calculated according to the standard curve.

The present invention further includes a method for diagnosing pancreatic cancer, comprising: (1) obtaining the methylation level of a DNA sequence or a fragment thereof or the methylation status or level of one or more CpG dinucleotides in the DNA sequence or fragment thereof in a sample of a subject, and the CA19-9 level of the subject, (2) using a mathematical model (e.g., support vector machine model or random forest model) to calculate using the methylation status or level to obtain a methylation score, (3) combining the methylation score and the CA19-9 level into a data matrix, (4) constructing a pancreatic cancer diagnostic model (e.g., logistic regression model) based on the data matrix, and optionally (5) obtaining a pancreatic cancer score; and diagnosing pancreatic cancer according to whether the pancreatic cancer score reaches the threshold. The method may further include DNA extraction and/or quality inspection before step (1). The present invention is particularly suitable for identifying pancreatic cancer from patients with pancreatitis, that is, differentiating between pancreatic cancer and pancreatitis.

The subject is, for example, a patient diagnosed with pancreatitis or a patient who has been diagnosed with pancreatitis (previous diagnosis). That is, in one or more embodiments, the method identifies pancreatic cancer in patients diagnosed with chronic pancreatitis, including previously diagnosed patients. Of course, the method of the present invention is not limited to the above-mentioned subjects, and can also be used to directly diagnose and identify pancreatitis or pancreatic cancer in undiagnosed subjects.

In a specific embodiment, step (1) comprises detecting the methylation level of a DNA sequence or a fragment thereof or the methylation status or level of one or more CpG dinucleotides in the DNA sequence or fragment thereof in a sample of a subject, for example, detecting the methylation status or level using primer molecules and/or probe molecules described herein.

Methods for detecting methylation status or level and detecting CA19-9 level are described elsewhere herein. A specific method for detecting methylation status or level comprises: treating genomic DNA or cfDNA with a conversion reagent to convert unmethylated cytosine into a base (such as uracil) with a lower binding capacity to guanine than to cytosine; performing PCR amplification using primers suitable for amplifying the converted sequences of sequences related to the differentiation between pancreatic cancer and pancreatitis described herein; determining the methylation level of at least one CpG by the presence or absence of amplified products, or by sequence identification (e.g., probe-based PCR identification or DNA sequencing identification).

In a preferred embodiment, the model training process is as follows: first, obtaining differentially methylated segments according to the methylation level of each site and constructing a differentially methylated region matrix, for example, constructing a methylation data matrix from the methylation level data of a single CpG dinucleotide position in the HG19 genome through, for example, samtools software; then training the SVM model.

The exemplary SVM model training process is as follows:

a) The sklearn software package (v0.23.1) of python software (v3.6.9) is used to construct the training model and cross-validate the training mode of the training model, command line: model = SVR().

b) The sklearn software package (v0.23.1) is used to input the data matrix to construct the SVM model, model.fit(x_train, y_train), where x_train represents the training set data matrix, and y_train represents the phenotypic information of the training set.

According to the inventors' findings, combining methylation scores with CA19-9 levels can significantly improve diagnostic accuracy. Specifically, the methylation score and CA19-9 level are combined into a data matrix, and then a pancreatic cancer diagnostic model (such as a logistic regression model) is built based on the data matrix to obtain a pancreatic cancer score.

The data matrix of methylation scores and CA19-9 levels is optionally normalized. Standardization can be performed using conventional standardization methods in the art. In the embodiments of the present invention, the RobustScaler standardization method is used as an example, and the standardization formula is as follows:

$$x' = \frac{x - median}{IQR}$$

where x and x' are the sample data before and after normalization respectively, median is the median of the sample, and IQR is the interquartile range of the sample.

Similar to methylation scores, methods of conventional mathematical modeling and the process of determining thresholds through data matrices are known in the art, for example through support vector machine (SVM) mathematical models, random forest models or logistic regression models. An exemplary approach is a logistic regression model. For example, for differential methylation markers, a logistic regression model is constructed for the samples of the training group, and the precision, sensitivity and specificity of the detection results as well as the area under the prediction value characteristic curve (ROC) (AUC) are statistically analyzed using the model, and the prediction scores of the samples of the test set are statistically analyzed. When the pancreatic cancer score combining methylation levels and CA19-9 levels meets a certain threshold, pancreatic cancer is identified, otherwise chronic pancreatitis is identified.

In another aspect, the present application provides a method for determining the presence of a pancreatic tumor, assessing the development or risk of development of a pancreatic tumor, and/or assessing the progression of a pancreatic tumor, comprising determining the presence and/or content of modification status of a DNA region with genes TLX2, EBF2, KCNA6, CCNA1, FOXD3, TRIM58, HOXD10, OLIG3, EN2, CLEC11A, TWIST1 and/or EMX1, or a fragment thereof in a sample to be tested. For example, the method of the present application may comprise determining whether a pancreatic tumor exists based on a determination result of the presence and/or content of modification status of a DNA region with genes TLX2, EBF2, KCNA6, CCNA1, FOXD3, TRIM58, HOXD10, OLIG3, EN2, CLEC11A, TWIST1, and/or EMX1, or a fragment thereof in a sample to be tested. For example, the method of the present application may comprise assessing whether the development of a pancreatic tumor is diagnosed based on a determination result of the presence and/or content of modification status of a DNA region with genes TLX2, EBF2, KCNA6, CCNA1, FOXD3, TRIM58, HOXD10, OLIG3, EN2, CLEC11A, TWIST1, and/or EMX1, or a fragment thereof in a sample to be tested. For example, the method of the present application may comprise whether there is a risk of being diagnosed with the development of a pancreatic tumor and/or the level of risk based on a determination result of the presence and/or content of modification status of a DNA region with genes TLX2, EBF2, KCNA6, CCNA1, FOXD3, TRIM58, HOXD10, OLIG3, EN2, CLEC11A, TWIST1, and/or EMX1, or a fragment thereof in a sample to be tested. For example, the method of the present application may comprise assessing the progression of a pancreatic tumor based on a determination result of the presence and/or content of modification status of a DNA region with genes TLX2, EBF2, KCNA6, CCNA1, FOXD3, TRIM58, HOXD10, OLIG3, EN2, CLEC11A, TWIST1, and/or EMX1, or a fragment thereof in a sample to be tested.

In another aspect, the present application provides a method for assessing the methylation status of a pancreatic tumor-related DNA region, which may comprise determining the presence and/or content of modification status of a DNA region with genes TLX2, EBF2, KCNA6, CCNA1, FOXD3, TRIM58, HOXD10, OLIG3, EN2, CLEC11A, TWIST1, and/or EMX1, or a fragment thereof in a sample to be tested. For example, it comprises assessing the methylation status of a pancreatic tumor-related DNA region based on the determination result concerning the presence and/or content of modification status of a DNA region with genes TLX2, EBF2, KCNA6, CCNA1, FOXD3, TRIM58, HOXD10, OLIG3, EN2, CLEC11A, TWIST1, and/or EMX1, or a fragment thereof in a sample to be tested. For example, the methylation status of a pancreatic tumor-related DNA region may refer to the confirmed presence or increased content of methylation relative to the reference level in that DNA region, which may be associated with the occurrence of pancreatic tumors.

For example, the DNA region of the present application can be derived from human chr2:74740686-74744275, derived from human chr8:25699246-25907950, derived from human chr12:4918342-4960278, derived from human chr13:37005635-37017019, derived from human chr1:63788730-63790797, derived from human chr1:248020501-248043438, derived from human chr2:176945511-176984670, derived from human chr6:137813336-137815531, derived from human chr7:155167513-155257526, derived from human chr19:51226605-51228981, derived from human chr7:19155091-19157295, and derived from human chr2:73147574-73162020. For example, the genes of the present application can be described by their names and their chromosomal coordinates. For example, chromosomal coordinates can be consistent with the Hg 19 version of the human genome database (or "Hg19 coordinates"), published in February 2009. For example, the DNA region of the present application may be derived from a region defined by Hg 19 coordinates.

In another aspect, the present application provides a method for determining the presence of a disease, assessing the development or risk of development of a disease, and/or assessing the progression of a disease, comprising determining the presence and/or content of modification status of a specific sub-region of a DNA region with genes TLX2, EBF2, KCNA6, CCNA1, FOXD3, TRIM58, HOXD10, OLIG3, EN2, CLEC11A, TWIST1 and/or EMX1, or complementary regions thereof or fragments thereof in a sample to be tested.

In another aspect, the present application provides a method for determining the presence of a disease, assessing the development or risk of development of a disease, and/or assessing the progression of a disease, which may comprise determining the presence and/or content of modification status of a DNA region selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or a complementary region thereof, or a fragment thereof in a sample to be tested. For example, the method of the present application may comprise identifying whether the disease exists based on the determination result of the presence and/or content of modification status of the DNA region, or complementary regions thereof, or fragments thereof in the sample to be tested. For example, the method of the present application may comprise assessing whether the development of a disease is diagnosed or not based on the determination result of the presence and/or content of modification status of the DNA region, or complementary regions thereof, or fragments thereof in the sample to be tested. For example, the method of the present application may comprise assessing whether there is a risk of being diagnosed with a disease and/or the level of risk based on the determination result of the presence and/or content of modification status of the DNA region, or complementary region thereof, or fragments thereof in the sample to be tested. For example, the method of the present application may comprise assessing the progression of a disease based on the determination result of the presence and/or content of modification status of the DNA region, or complementary regions thereof, or fragments thereof in the sample to be tested.

In another aspect, the present application provides a method for determining the methylation status of a DNA region, which may comprise determining the presence and/or content of modification status of a DNA region selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or a complementary region thereof, or a fragment thereof in a sample to be tested. For example, the confirmed presence or increased content relative to reference levels of methylation in that DNA region can be associated with the occurrence of diseases. For example, the DNA region in the present application may refer to a specific segment of genomic DNA. For example, the DNA region of the present application may be designated by a gene name or a set of chromosomal coordinates. For example, a gene can have its sequence and chromosomal location determined by reference to its name, or have its sequence and chromosomal location determined by reference to its chromosomal coordinates. The present application uses the methylation status of these specific DNA regions as a series of analytical indicators, which can provide significant improvement in sensitivity and/or specificity and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

For example, a variant may comprise at least 80%, at least 85%, at least 90%, 95%, 98%, or 99% sequence identity to the DNA region described herein, and a variant may comprise one or more deletions, additions, substitutions, inverted sequences, etc. For example, the modification status of the variants in the present application can achieve the same evaluation results. The DNA region of the present application may comprise any other mutation, polymorphic variation or allelic variation in all forms.

For example, the method of the present application may comprise: providing a nucleic acid capable of binding to a DNA region selected from the group consisting of SEQ ID NOs: 164, 168, 172, 176, 180, 184, 188, 192, 196, 200, 204, 208, 212, 216, 220, 224, 228, and 232, or a complementary region thereof, or a converted region thereof, or a fragment thereof.

In another aspect, the present application provides a method for determining the presence of a disease, assessing the development or risk of development of a disease, and/or assessing the progression of a disease, which may comprise determining the presence and/or content of modification status of a DNA region selected from the group consisting of DNA regions derived from human chr2:74743042-74743113 and derived form human chr2:74743157-74743253, derived form human chr2:74743042-74743113 and derived from human chr2:74743157-74743253, derived form human chr8:25907865-25907930 and derived from human chr8:25907698-25907814, derived form human chr12:4919188-4919272, derived form human chr12:4919036-4919164 and derived from human chr12:4919341-4919438, derived form human chr13:37005652-37005721, derived form human chr13:37005458-37005596 and derived from human chr13:37005694-37005824, derived form human chr1:63788850-63788913, derived form human chr1:248020635-248020731, derived form human chr2:176945521-176945603, derived form human chr6:137814750-137814815, derived form human chr7:155167531-155167610, derived form human chr19:51228620-51228722, and derived from human chr7:19156779-19157914, and derived from human chr2:73147571-73147626, or a complementary region thereof, or a fragment thereof in a sample to be tested.

For example, one or more of the above regions can serve as amplification regions and/or detection regions.

For example, the method of the present application may comprise: providing a nucleic acid selected from the group consisting of SEQ ID NOs: 165, 169, 173, 177, 181, 185, 189, 193, 197, 201, 205, 209, 213, 217, 221, 225, 229, and 233, or a complementary nucleic acid thereof, or a fragment thereof. For example, the nucleic acid may be used to detect a target region. For example, the nucleic acid may be used as a probe.

For example, the method of the present application may comprise: providing a nucleic acid combination selected from the group consisting of SEQ ID NOs: 166 and 167, 170 and 171, 174 and 175, 178 and 179, 182 and 183, 186 and 187, 190 and 191, 194 and 195, 198 and 199, 202 and 203, 206 and 207, 210 and 211, 214 and 215, 218 and 219, 222 and 223, 226 and 227, 230 and 231, and 234 and 235, or a complementary nucleic acid combination thereof, or a fragment thereof. For example, the nucleic acid combination may be used to amplify a target region. For example, the nucleic acid combination can serve as a primer combination.

For example, the disease may include tumors. For example, the disease may include solid tumors. For example, the disease may include any tumor such as pancreatic tumors. For example, optionally the disease of the present application may include pancreatic cancer. For example, optionally the disease of the present application may include pancreatic ductal adenocarcinoma. For example, optionally the pancreatic tumor of the present application may include pancreatic ductal adenocarcinoma.

For example, "complementary" and "substantially complementary" in the present application may include hybridization or base pairing or formation of a double strand between nucleotides or nucleic acids, for example between two strands of a double strand DNA molecule, or between oligonucleotide primers and primer binding sites on a single strand nucleic acid. Complementary nucleotides may typically be A and T (or A and U) or C and G. For two single-stranded RNA or DNA molecules, when the nucleotides of one strand are paired with at least about 80% (usually at least about 90% to about 95%, or even about 98% to about 100%) of those of the other strand when they are optimally aligned and compared and have appropriate nucleotide insertions or deletions, they can be considered to be substantially complementary. In one aspect, two complementary nucleotide sequences are capable of hybridizing with less than 25% mismatch, more preferably less than 15% mismatch, and less than 5% mismatch or without mismatch between reverse nucleotides. For example, two molecules can hybridize under highly stringent conditions.

For example, the modification status in the present application may refer to the presence, absence and/or content of modification status at a specific nucleotide or multiple nucleotides within a DNA region. For example, the modification status in the present application may refer to the modification status of each base or each specific base (e.g., cytosine) in a specific DNA sequence. For example, the modification status in the present application may refer to the modification status of base pair combinations and/or base combinations in a specific DNA sequence. For example, the modification status in the present application may refer to information about the density of region modifications in a specific DNA sequence (including the DNA region where the gene is located or specific region fragments thereof), but may not provide precise location information on where modifications occur in the sequence.

For example, the modification status of the present application may be a methylation status or a state similar to methylation. For example, a state of being methylated or being highly methylated can be associated with transcriptional silencing of a specific region. For example, a state of being methylated or being highly methylated may be associated with being able to be converted by a methylation-specific conversion reagent (such as a deamination reagent and/or a methylation-sensitive restriction enzyme). For example, conversion may refer to being converted into other substances and/or being cleaved or digested.

For example, the method may further comprise obtaining the nucleic acid in the sample to be tested. For example, the nucleic acid may include a cell-free nucleic acid. For example, the sample to be tested may include tissue, cells and/or body fluids. For example, the sample to be tested may include plasma. For example, the detection method of the present application can be performed on any suitable biological sample. For example, the sample to be tested can be any sample of biological materials, such as it can be derived from an animal, but is not limited to cellular materials, biological fluids (such as blood), discharge, tissue biopsy specimens, surgical specimens, or fluids that have been introduced into the body of an animal and subsequently removed. For example, the sample to be tested in the present application may include a sample that has been processed in any form after the sample is isolated.

For example, the method may further comprise converting the DNA region or fragment thereof. For example, through the conversion step of the present application, the bases with the modification and the bases without the modification can form different substances after conversion. For example, the base with the modification status is substantially unchanged after conversion, and the base without the modification status is changed to other bases (for example, the other base may include uracil) different from the base after conversion or is cleaved after conversion. For example, the base may include cytosine. For example, the modification may include methylation modification. For example, the conversion may comprise conversion by a deamination reagent and/or a methylation-sensitive restriction enzyme. For example, the deamination reagent may include bisulfite or analogues thereof. For example, it is sodium bisulfite or potassium bisulfite.

For example, the method may further comprise amplifying the DNA region or fragment thereof in the sample to be tested before determining the presence and/or content of modification status of the DNA region or fragment thereof. For example, the amplification may include PCR amplification. For example, the amplification in the present application may include any known amplification system. For example, the amplification step in the present application may be optional. For example, "amplification" may refer to the process of producing multiple copies of a desired sequence. "Multiple copies" may refer to at least two copies. "Copy" may not imply perfect sequence complementarity or identity to the template sequence. For example, copies may include nucleotide analogs such as deoxyinosine, intentional sequence changes (such as those introduced by primers containing sequences that are hybridizable but not complementary to the template), and/or may occur during amplification Sequence error.

For example, the method for determining the presence and/or content of modification status may comprise determining the presence and/or content of a substance formed by a base with the modification status after the conversion. For example, the method for determining the presence and/or content of modification status may comprise determining the presence and/or content of a DNA region with the modification status or a fragment thereof. For example, the presence and/or content of a DNA region with the modification status or a fragment thereof can be directly detected. For example, it can be detected in the following manner: a DNA region with the modification status or a fragment thereof may have different characteristics from a DNA region without the modification status or a fragment thereof during a reaction (e.g., an amplification reaction). For example, in a fluorescent PCR method, a DNA region with the modification status or a fragment thereof can be specifically amplified and emit fluorescence; a DNA region without the modification status or a fragment thereof can be substantially not amplified, and basically do not emit fluorescence. For example, alternative methods of determining the presence and/or content of species formed upon conversion of bases with the modification status may be included within the scope of the present application.

For example, the presence and/or content of the DNA region with the modification status or fragment thereof is determined by the fluorescence Ct value detected by the fluorescence PCR method. For example, the presence of a pancreatic tumor, or the development or risk of development of a pancreatic tumor is determined by determining the presence of modification status of the DNA region or fragment thereof and/or a higher content of modification status of the DNA region or fragment thereof relative to the reference level. For example, when the fluorescence Ct value of the sample to be tested is lower than the reference fluorescence Ct value, the presence of modification status of the DNA region or fragment thereof can be determined and/or it can be determined that the content of modification status of the DNA region or fragment thereof is higher than the content of modification status in the reference sample. For example, the reference fluorescence Ct value can be determined by detecting the reference sample. For example, when the fluorescence Ct value of the sample to be tested is higher than or substantially equivalent to the reference fluorescence Ct value, the presence of modification status of the DNA region or fragment thereof may not be ruled out; when the fluorescence Ct value of the sample to be tested is higher than or substantially equivalent to the reference fluorescence Ct value, it can be confirmed that the content of modification status of the DNA region or fragment thereof is lower than or substantially equal to the content of modification status in the reference sample.

For example, the present application can represent the presence and/or content of modification status of a specific DNA region or fragment thereof through a cycle threshold (i.e., Ct value), which, for example, includes the methylation level of a sample to be tested and a reference level. For example, the Ct value may refer to the number of cycles at which fluorescence of the PCR product can be detected above the background signal. For example, there can be a negative correlation between the Ct value and the starting content of the target marker in the sample, that is, the lower the Ct value, the greater the content of modification status of the DNA region or fragment thereof in the sample to be tested.

For example, when the Ct value of the sample to be tested is the same as or lower than its corresponding reference Ct value, it can be confirmed as the presence of a specific disease, diagnosed as the development or risk of development of a specific disease, or assessed as certain progression of a specific disease. For example, when the Ct value of the sample to be tested is lower than its corresponding reference Ct value by at least 1 cycle, at least 2 cycles, at least 5 cycles, at least 10 cycles, at least 20 cycles, or at least 50 cycles, it can be confirmed as the presence of a specific disease, diagnosed as the development or risk of development of a specific disease, or assessed as certain progression of a specific disease.

For example, when the Ct value of a cell sample, a tissue sample or a sample derived from a subject is the same as or higher than its corresponding reference Ct value, it can be confirmed as the absence of a specific disease, not diagnosed as the development or risk of development of a specific disease, or not assessed as certain progression of a specific disease. For example, when the Ct value of a cell sample, a tissue sample or a sample derived from a subject is higher than its corresponding reference Ct value by at least 1 cycle, at least 2 cycles, at least 5 cycles, at least 10 cycles, at least 20 cycles, or at least 50 cycles, it can be confirmed as the absence of a specific disease, not diagnosed as the development or risk of development of a specific disease, or not assessed as certain progression of a specific disease. For example, when the Ct value of a cell sample, a tissue sample or a sample derived from a subject is the same as or its corresponding reference Ct value, it can be confirmed as the presence or absence of a specific disease, diagnosed as developing or not developing, having or not having risk of development of a specific disease, or assessed as having or not having certain progression of a specific disease, and at the same time, suggestions for further testing can be given.

For example, the reference level or control level in the present application may refer to a normal level or a healthy level. For example, the normal level may be the modification level of a DNA region of a sample derived from cells, tissues or individuals free of the disease. For example, when used for the evaluation of a tumor, the normal level may be the modification level of a DNA region of a sample derived from cells, tissues or individuals free of the tumor. For example, when used for the evaluation of a pancreatic tumor, the normal level may be the modification level of a DNA region of a sample derived from cells, tissues or individuals without the pancreatic tumor.

For example, the reference level in the present application may refer to a threshold level at which the presence or absence of a particular disease is confirmed in a subject or sample. For example, the reference level in the present application may refer to a threshold level at which a subject is diagnosed as developing or at risk of developing a particular disease. For example, the reference level in the present application may refer to a threshold level at which a subject is assessed as having certain progression of a particular disease. For example, when the modification status of a DNA region in a cell sample, a tissue sample or a sample derived from a subject is higher than or substantially equal to the corresponding reference level (for example, the reference level here may refer to the modification status of a DNA region of a patient without a specific disease), it can be confirmed as the presence of a specific disease, diagnosed as developing or at risk of developing a specific disease, or assessed as certain progression of a specific disease. For example, A and B are "substantially equal" in the present application may mean that the difference between A and B is 1% or less, 0.5% or less, 0.1% or less, 0.01% or less, 0.001% or less, or 0.0001% or less. For example, when the modification status of a DNA region in a cell sample, a tissue sample, or a sample derived from a subject is higher than the corresponding reference level by at least 1%, at least 5%, at least 10%, at least 20%, at least 50%, at least 1 times, at least 2 times, at least 5 times, at least 10 times, or at least 20 times, it can be confirmed as the presence of a specific disease, diagnosed as the development or risk of development of a specific disease, or assessed as certain progression of a specific disease. For example, in at least one, at least two, or at least three times of detection among many times of detection, when the modification status of a DNA region in a cell sample, a tissue sample, or a sample derived from a subject is higher than the corresponding reference level by at least 1%, at least 5%, at least 10%, at least 20%, at least 50%, at least 1 times, at least 2 times, at least 5 times, at least 10 times, or at least 20 times, it can be confirmed as the presence of a specific disease, diagnosed as the development or risk of development of a specific disease, or assessed as a certain progression of a specific disease.

For example, when the modification status of a DNA region in a cell sample, a tissue sample or a sample derived from a subject is lower than or substantially equal to the corresponding reference level (for example, the reference level here may refer to the modification status of a DNA region of a patient with a specific disease), it can be not confirmed as the absence of a specific disease, not diagnosed as developing or at risk of developing a specific disease, or not assessed as certain progression of a specific disease. For example, when the modification status of a DNA region in a cell sample, a tissue sample, or a sample derived from a subject is lower than the corresponding reference level by at least 1%, at least 5%, at least 10%, at least 20%, at least 50%, and at least 100%, it can be confirmed as the absence of a specific disease, not diagnosed as the development or risk of development of a specific disease, or not assessed as certain progression of a specific disease.

Reference levels can be selected by those skilled in the art based on the desired sensitivity and specificity. For example, the reference levels in various situations in the present application may be readily identifiable by those skilled in the art. For example, appropriate reference levels and/or appropriate means of obtaining the reference levels can be identified based on a limited number of attempts. For example, the reference levels may be derived from one or more reference samples, where the reference levels are obtained from experiments performed in parallel with experiments testing the sample of interest. Alternatively, reference levels may be obtained in a database that includes a collection of data, standards or levels from one or more reference samples or disease reference samples. In some embodiments, a set of data, standards or levels can be standardized or normalized so that it can be compared with data from one or more samples and thereby used to reduce errors arising from different detection conditions.

For example, the reference levels may be derived from a database, which may be a reference database that includes, for example, modification levels of target markers from one or more reference samples and/or other laboratories and clinical data. For example, a reference database can be established by aggregating reference level data from reference samples obtained from healthy individuals and/or individuals not suffering from the corresponding disease (i.e., individuals known not to have the disease). For example, a reference database can be established by aggregating reference level data from reference samples obtained from individuals with the corresponding disease under treatment. For example, a reference database can be built by aggregating data from reference samples obtained from individuals at different stages of the disease. For example, different stages may be evidenced by different modification levels of the marker of interest of the present application. Those skilled in the art can also determine whether an individual suffers from the corresponding disease or is at risk of suffering from the corresponding disease based on various factors, such as age, gender, medical history, family history, symptoms.

For example, the present application can use cycle thresholds (i.e., Ct values) to represent the presence and/or content of modification status in specific DNA regions or fragments thereof. The determination method can be as follows: a score is calculated based on the methylation level of each sequence selected from the gene, and if the score is greater than 0, the result is positive, that is, the result corresponding to the sample can be a malignant nodule; in one or more embodiments, if the score is less than 0, the result is negative, that is, the result corresponding to the pancreatic sample can be a benign nodule. For example, in the PCR embodiment, the methylation level can be calculated as follows: methylation level = $2^{\wedge}(-\Delta Ct$ sample to be tested$)/2^{\wedge}(-\Delta Ct$ positive standard$) \times 100\%$, where, $\Delta Ct = Ct$ target gene - Ct internal reference gene. In sequencing embodiments, methylation level can be calculated as follows: methylation level = number of methylated bases/number of total bases.

For example, the method of the present application may comprise the following steps: obtaining the nucleic acid in the sample to be tested; converting the DNA region or fragment thereof; determining the presence and/or content of the substance formed by the base with the modification status after the conversion.

For example, the method of the present application may comprise the following steps: obtaining the nucleic acid in the sample to be tested; converting the DNA region or fragment thereof; amplifying the DNA region or fragment thereof in the sample to be detected; determining the presence and/or content of the substance formed by the base with the modification status after the conversion.

For example, the method of the present application may comprise the following steps: obtaining the nucleic acid in the sample to be tested; treating the DNA obtained from the sample to be tested with a reagent capable of differentiating unmethylated sites and methylated sites in the DNA, thereby obtaining treated DNA; optionally amplifying the DNA region or fragment thereof in the sample to be tested; quantitatively, semi-quantitatively or qualitatively analyzing the presence and/or content of methylation status of the treated DNA in the sample to be tested; comparing the methylation level of the treated DNA in the sample to be tested with the corresponding reference level. When the methylation status of the DNA region in the sample to be tested is higher than or basically equal to the corresponding reference level, it can be confirmed as presence of a specific disease, diagnosed as the development or risk of development of a specific disease, or assessed as certain progression of a specific disease.

In another aspect, the present application provides a nucleic acid, which may comprise a sequence capable of binding to a DNA region with genes TLX2, EBF2, KCNA6, CCNA1, FOXD3, TRIM58, HOXD10, OLIG3, EN2, CLEC11A, TWIST1, and/or EMX1, or a complementary region thereof, or a converted region thereof, or a fragment thereof. For example, the nucleic acid can be any probe of the present application. In another aspect, the present application provides a method for preparing a nucleic acid, which may comprise designing a nucleic acid capable of binding to a DNA region with genes TLX2, EBF2, KCNA6, CCNA1, FOXD3, TRIM58, HOXD10, OLIG3, EN2, CLEC11A, TWIST1, and/or EMX1, or a complementary region thereof, or a converted region thereof, or a fragment thereof, based on the modification status of the DNA region, or complementary region thereof, or converted region thereof, or fragment thereof. For example, the method of preparing nucleic acids can be any suitable method known in the art.

In another aspect, the present application provides a nucleic acid combination, which may comprise sequences capable of binding to a DNA region with genes TLX2, EBF2, KCNA6, CCNA1, FOXD3, TRIM58, HOXD10, OLIG3, EN2, CLEC11A, TWIST1, and/or EMX1, or a complementary region thereof, or a converted region thereof, or a fragment thereof. For example, the nucleic acid combination can be any primer combination of the present application. In another aspect, the present application provides a method for preparing a nucleic acid combination, which may comprise designing a nucleic acid combination capable of amplifying a DNA region with genes TLX2, EBF2, KCNA6, CCNA1, FOXD3, TRIM58, HOXD10, OLIG3, EN2, CLEC11A, TWIST1, and/or EMX1, or a complementary region thereof, or a converted region thereof, or a fragment thereof, based on the modification status of the DNA region, or complementary region thereof, or converted region thereof, or fragment thereof. For example, the method of preparing the nucleic acids in the nucleic acid combination can be any suitable method known in the art. For example, the methylation status of a target polynucleotide can be assessed using a single probe or primer configured to hybridize with the target polynucleotide. For example, the methylation status of a target polynucleotide can be assessed using multiple probes or primers configured to hybridize with the target polynucleotide.

In another aspect, the present application provides a kit, which may comprise the nucleic acid of the present application and/or the nucleic acid combination of the present application. For example, the kit of the present application may optionally comprise reference samples for corresponding uses or provide reference levels for corresponding uses.

In another aspect, the probes in the present application may also contain detectable substances. In one or more embodiments, the detectable substance may be a 5' fluorescent reporter and a 3' labeling quencher. In one or more embodiments, the fluorescent reporter gene can be selected from Cy5, Texas Red, FAM, and VIC.

In another aspect, the kit of the present application may also comprise a converted positive standard in which unmethylated cytosine is converted to a base that does not bind to guanine. In one or more embodiments, the positive standard can be fully methylated.

In another aspect, the kit of the present application can also comprise one or more substances selected from the following: PCR buffer, polymerase, dNTP, restriction endonuclease, enzyme digestion buffer, fluorescent dye, fluorescence quencher, fluorescent reporter, exonuclease, alkaline phosphatase, internal standard, control, KCl, $MgCl_2$ and $(NH_4)_2SO_4$.

In another aspect, the reagents used to detect DNA methylation in the present application may be reagents used in one or more of the following methods: bisulfite conversion-based PCR (e.g., methylation-specific PCR), DNA sequencing (e.g., bisulfite sequencing, whole-genome methylation sequencing, simplified methylation sequencing), methylation-sensitive restriction endonuclease assay, fluorescence quantitation, methylation-sensitive high-resolution melting curve assay, chip-based methylation atlas, and mass spectrometry (e.g., flight mass spectrometry). For example, the reagent may be selected from one or more of the following: bisulfite and derivatives thereof, fluorescent dyes, fluorescent quenchers, fluorescent reporters, internal standards, and controls.

Diagnostic methods, preparation uses

In another aspect, the present application provides the use of the nucleic acid of the present application, the nucleic acid combination of the present application and/or the kit of the present application in the preparation of a disease detection product.

In another aspect, the present application provides a disease detection method, which may include providing the nucleic acid of the present application, the nucleic acid combination of the present application and/or the kit of the present application.

In another aspect, the present application provides the nucleic acid of the present application, the nucleic acid combination of the present application and/or the kit of the present application for use in disease detection.

In another aspect, the present application provides the use of the nucleic acid of the present application, the nucleic acid combination of the present application and/or the kit of the present application in the preparation of a substance for determining the presence of a disease, assessing the development or risk of development of a disease and/or assessing the progression of a disease.

In another aspect, the present application provides a method for determining the presence of a disease, assessing the development or risk of development of a disease and/or assessing the progression of a disease, which may comprise providing the nucleic acid of the present application, the nucleic acid combination of the present application and/or the kit of the present application.

In another aspect, the present application provides the nucleic acid of the present application, the nucleic acid combination of the present application and/or the kit of the present application, which may be used for determining the presence of a disease, assessing the development or risk of development of a disease and/or assessing the progression of a disease.

In another aspect, the present application provides the use of the nucleic acid of the present application, the nucleic acid combination of the present application and/or the kit of the present application in the preparation of a substance that can determine the modification status of the DNA region or fragment thereof.

In another aspect, the present application provides a method for determining the modification status of the DNA region or fragment thereof, which may comprise providing the nucleic acid of the present application, the nucleic acid combination of the present application and/or the kit of the present application.

In another aspect, the present application provides the nucleic acid of the present application, the nucleic acid combination of the present application and/or the kit of the present application, which may be used for determining the modification status of the DNA region or fragment thereof.

In another aspect, the present application provides the use of a nucleic acid, a nucleic acid combination and/or a kit for determining the modification status of a DNA region in the preparation of a substance for determining the presence of a pancreatic tumor, assessing the development or risk of development of a pancreatic tumor and/or assessing the progression of a pancreatic tumor, wherein the DNA region for determination includes DNA regions with genes TLX2, EBF2, KCNA6, CCNA1, FOXD3, TRIM58, HOXD10, OLIG3, EN2, CLEC11A, TWIST1, and/or EMX1, or fragments thereof.

In another aspect, the present application provides a method for determining the presence of a pancreatic tumor, assessing the development or risk of development of a pancreatic tumor and/or assessing the progression of a pancreatic tumor, which may comprise providing a nucleic acid, a nucleic acid combination and/or a kit for determining the modification status of a DNA region, wherein the DNA region for determination includes DNA regions with genes TLX2, EBF2, KCNA6, CCNA1, FOXD3, TRIM58, HOXD10, OLIG3, EN2, CLEC11A, TWIST1, and/or EMX1, or fragments thereof.

In another aspect, the present application provides a nucleic acid, a nucleic acid combination and/or a kit for determining the modification status of a DNA region, which may be used for determining the presence of a pancreatic tumor, assessing the development or risk of development of a pancreatic tumor and/or assessing the progression of a pancreatic tumor, wherein the DNA region for determination includes DNA regions with genes TLX2, EBF2, KCNA6, CCNA1, FOXD3, TRIM58, HOXD10, OLIG3, EN2, CLEC11A, TWIST1, and/or EMX1, or fragments thereof.

In another aspect, the present application provides the use of a nucleic acid, a nucleic acid combination and/or a kit for determining the modification status of a DNA region in the preparation of a substance for determining the presence of a disease, assessing the development or risk of development of a disease, and/or assessing the progression of a disease, wherein the DNA region may include a DNA region selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or a complementary region thereof, or a fragment thereof.

In another aspect, the present application provides a method for determining the presence of a pancreatic tumor, assessing the development or risk of development of a pancreatic tumor, and/or assessing the progression of a pancreatic tumor, which may comprise providing a nucleic acid, a nucleic acid combination and/or a kit for determining the modification status of a DNA region, wherein the DNA region may include a DNA region selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or a complementary region thereof, or a fragment thereof.

In another aspect, the present application provides a nucleic acid, a nucleic acid combination and/or a kit for determining the modification status of a DNA region, which may be used for determining the presence of a pancreatic tumor, assessing the development or risk of development of a pancreatic tumor, and/or assessing the progression of a pancreatic tumor, wherein the DNA region may include a DNA region selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or a complementary region thereof, or a fragment thereof.

In another aspect, the present application provides nucleic acids of DNA regions with genes TLX2, EBF2, KCNA6, CCNA1, FOXD3, TRIM58, HOXD10, OLIG3, EN2, CLEC11A, TWIST1, and/or EMX1, or converted regions thereof, or fragments thereof, and combinations of the above-mentioned nucleic acids.

In another aspect, the present application provides the use of nucleic acids of DNA regions with genes TLX2, EBF2, KCNA6, CCNA1, FOXD3, TRIM58, HOXD10, OLIG3, EN2, CLEC11A, TWIST1, and/or EMX1, or converted regions thereof, or fragments thereof, and combinations of the above-mentioned nucleic acids, in the preparation of a substance for determining the presence of a pancreatic tumor, assessing the development or risk of development of a pancreatic tumor, and/or assessing the progression of a pancreatic tumor.

In another aspect, the present application provides a method for determining the presence of a pancreatic tumor, assessing the development or risk of development of a pancreatic tumor, and/or assessing the progression of a pancreatic tumor, which comprises providing nucleic acids of DNA regions with genes TLX2, EBF2, KCNA6, CCNA1, FOXD3, TRIM58, HOXD10, OLIG3, EN2, CLEC11A, TWIST1, and/or EMX1, or converted regions thereof, or fragments thereof, and combinations of the above-mentioned nucleic acids.

In another aspect, the present application provides nucleic acids of DNA regions with genes TLX2, EBF2, KCNA6, CCNA1, FOXD3, TRIM58, HOXD10, OLIG3, EN2, CLEC11A, TWIST1, and/or EMX1, or converted regions thereof, or fragments thereof, and combinations of the above-mentioned nucleic acids, which may be used for determining the presence of a pancreatic tumor, assessing the development or risk of development of a pancreatic tumor, and/or assessing the progression of a pancreatic tumor.

In another aspect, the present application provides nucleic acids of DNA regions selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or complementary regions thereof, or converted regions thereof, or fragments thereof, and combinations of the above-mentioned nucleic acids.

In another aspect, the present application provides the use of nucleic acids of DNA regions selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or complementary regions thereof, or converted regions thereof, or fragments thereof, and combinations of the above-mentioned nucleic acids, in the preparation of a substance for determining the presence of a disease, assessing the development or risk of development of a disease, and/or assessing the progression of a disease.

In another aspect, the present application provides a method for determining the presence of a disease, assessing the development or risk of development of a disease, and/or assessing the progression of a disease, which comprises providing nucleic acids of DNA regions selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or complementary regions thereof, or converted regions thereof, or fragments thereof, and combinations of the above-mentioned nucleic acids.

In another aspect, the present application provides nucleic acids of DNA regions selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or complementary regions thereof, or converted regions thereof, or fragments thereof, and combinations of the above-mentioned nucleic acids, which may be used for determining the presence of a disease, assessing the development or risk of development of a disease, and/or assessing the progression of a disease.

For example, the DNA region used for determination in the present application comprises two genes selected from the group consisting of DNA regions with EBF2 and CCNA1, or fragments thereof. For example, it comprises determining the presence and/or content of modification status of two DNA regions selected from the group consisting of DNA regions derived from human chr8:25907849-25907950, and derived from human chr13:37005635-37005754, or complementary regions thereof, or fragments thereof in a sample to be tested.

For example, in the method of the present application, the target gene may include 2 genes selected from the group consisting of KCNA6, TLX2, and EMX1. For example, in the method of the present application, the target gene may include KCNA6 and TLX2.

For example, in the method of the present application, the target gene may include KCNA6 and EMX1. For example, in the method of the present application, the target gene may include TLX2 and EMX1. For example, in the method of the present application, the target gene may include 3 genes selected from the group consisting of KCNA6, TLX2, and EMX1. For example, in the method of the present application, the target gene may include KCNA6, TLX2 and EMX1. For example, it comprises determining the presence and/or content of modification status of two or more DNA regions selected from the group consisting of DNA regions derived from human chr12:4919142-4919289, derived from human chr2:74743035-74743151, and derived from human chr2:73147525-73147644, or complementary regions thereof, or fragments thereof in a sample to be tested.

For example, in the method of the present application, the target gene may include 2 genes selected from the group consisting of TRIM58, TWIST1, FOXD3 and EN2. For example, in the method of the present application, the target gene may include TRIM58 and TWIST 1. For example, in the method of the present application, the target gene may include TRIM58 and FOXD3. For example, in the method of the present application, the target gene may include TRIM58 and EN2. For example, in the method of the present application, the target gene may include TWIST1 and FOXD3. For example, in the method of the present application, the target gene may include TWIST1 and EN2. For example, in the method of the present application, the target gene may include FOXD3 and EN2. For example, in the method of the present application, the target gene may include 3 genes selected from the group consisting of TRIM58, TWIST 1, FOXD3 and EN2. For example, in the method of the present application, the target gene may include TRIM58, TWIST1 and FOXD3. For example, in the method of the present application, the target gene may include TRIM58, TWIST1 and EN2. For example, in the method of the present application, the target gene may include TRIM58, FOXD3 and EN2. For example, in the method of the present application, the target gene may include TWIST1, FOXD3 and EN2. For example, in the method of the present application, the target gene may include 4 genes selected from the group consisting of TRIM58, TWIST1, FOXD3 and EN2. For example, in the method of the present application, the target gene may include TRIM58, TWIST1, FOXD3 and EN2. For example, it comprises determining the presence and/or content of modification status of two or more DNA regions selected from the group consisting of DNA regions derived from human chr1:248020592-248020779, derived from human chr7:19156739-19157277, derived from human chr1:63788812-63788952, and derived from human chr7:155167513-155167628, or complementary regions thereof, or fragments thereof in a sample to be tested.

For example, in the method of the present application, the target gene may include 2 genes selected from the group consisting of TRIM58, TWIST1, CLEC11A, HOXD10, and OLIG3. For example, in the method of the present application, the target gene may include TRIM58 and TWIST1. For example, in the method of the present application, the target gene may include TRIM58 and CLEC11A. For example, in the method of the present application, the target gene may include TRIM58 and HOXD10. For example, in the method of the present application, the target gene may include TRIM58 and OLIG3. For example, in the method of the present application, the target gene may include TWIST1 and CLEC11A. For example, in the method of the present application, the target gene may include TWIST1 and HOXD10. For example, in the method of the present application, the target gene may include TWIST 1 and OLIG3. For example, in the method of the present application, the target gene may include CLEC11A and HOXD10. For example, in the method of the present application, the target gene may include CLEC11A and OLIG3. For example, in the method of the present application, the target gene may include HOXD10 and OLIG3. For example, in the method of the present application, the target gene may include 3 genes selected from the group consisting of TRIM58, TWIST1, CLEC11A, HOXD10, and OLIG3. For example, in the method of the present application, the target gene may include TRIM58, TWIST 1 and CLEC11A. For example, in the method of the present application, the target gene may include TRIM58, TWIST1 and HOXD10. For example, in the method of the present application, the target gene may include TRIM58, TWIST1 and OLIG3. For example, in the method of the present application, the target gene may include TRIM58, CLEC11A and HOXD10. For example, in the method of the present application, the target gene may include TRIM58, CLEC11A and OLIG3. For example, in the method of the present application, the target gene may include TRIM58, HOXD10 and OLIG3. For example, in the method of the present application, the target gene may include TWIST1, CLEC11A and HOXD10. For example, in the method of the present application, the target gene may include TWIST1, CLEC11A and OLIG3. For example, in the method of the present application, the target gene may include TWIST 1, HOXD10 and OLIG3. For example, in the method of the present application, the target gene may include CLEC11A, HOXD10 and OLIG3. For example, in the method of the present application, the target gene may include 4 genes selected from the group consisting of TRIM58, TWIST1, CLEC11A, HOXD10, and OLIG3. For example, in the method of the present application, the target gene may include TRIM58, TWIST1, CLEC11A and HOXD10. For example, in the method of the present application, the target gene may include TRIM58, TWIST1, CLEC11A and OLIG3. For example, in the method of the present application, the target gene may include TRIM58, TWIST 1, HOXD10 and OLIG3. For example, in the method of the present application, the target gene may include TRIM58, CLEC11A, HOXD10 and OLIG3. For example, in the method of the present application, the target gene may include TWIST1, CLEC11A, HOXD10 and OLIG3. For example, in the method of the present application, the target gene may include 5 genes selected from the group consisting of TRIM58, TWIST1, CLEC11A, HOXD10, and OLIG3. For example, in the method of the present application, the target gene may include TRIM58, TWIST1, CLEC11A, HOXD10 and OLIG3.

For example, it comprises determining the presence and/or content of modification status of two or more DNA regions selected from the group consisting of DNA regions derived from human chr1:248020592-248020779, derived from human chr7:19156739-19157277, derived from human chr19:51228168-51228782, derived from human chr2:176945511-176945630, and derived from human chr6:137814700-137814853, or complementary regions thereof, or fragments thereof in a sample to be tested.

For example, the nucleic acid of the present application may refer to an isolated nucleic acid. For example, an isolated polynucleotide can be a DNA molecule, an RNA molecule, or a combination thereof. For example, the DNA molecule may be a genomic DNA molecule or a fragment thereof.

In another aspect, the present application provides a storage medium recording a program capable of executing the method of the present application.

In another aspect, the present application provides a device which may comprises the storage medium of the present application. In another aspect, the present application provides a non-volatile computer-readable storage medium on which a computer program is stored, and the program is executed by a processor to implement any one or more methods of the present application. For example, the non-volatile computer-readable storage medium may include floppy disks, flexible disks, hard disks, solid state storage (SSS) (such as solid state drives (SSD)), solid state cards (SSC), solid state modules (SSM)), enterprise flash drives, magnetic tapes, or any other non-transitory magnetic media, etc. Non-volatile computer-readable storage media may also include punched card, paper tape, optical mark card (or any other physical media having a hole pattern or other optically identifiable markings), compact disk read-only memory (CD-ROM), compact disc rewritable (CD-RW), digital versatile disc (DVD), blu-ray disc (BD) and/or any other non-transitory optical media.

For example, the device of the present application may further include a processor coupled to the storage medium, and the processor is configured to execute based on a program stored in the storage medium to implement the method of the present application. For example, the device may implement various mechanisms to ensure that the method of the present application when executed on a database system produce correct results. In the present application, the device may use magnetic disks as permanent data storage. In the present application, the device can provide database storage and processing services for multiple database clients. The device may store database data across multiple shared storage devices and/or may utilize one or more execution platforms with multiple execution nodes. The device can be organized so that storage and computing resources can be expanded effectively infinitely.

"Multiple" as described herein means any integer. Preferably, "more" in "one or more" may be, for example, any integer greater than or equal to 2, including 2, 3, 4, 5, 6, 7, 8, 9, 10, 11, 12, 13, 14, 15, 16, 17, 18, 19, 20, 30, 40, 50, 60 or more.

Embodiment 1

1. An isolated nucleic acid molecule from a mammal, wherein the nucleic acid molecule is a methylation marker of a pancreatic cancer-related gene, and the sequence of the nucleic acid molecule includes (1) one or more or all of the following sequences or variants having at least 70% identity thereto: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, wherein the methylation sites in the variants are not mutated, (2) complementary sequences of (1), (3) sequences of (1) or (2) that have been treated to convert unmethylated cytosine into a base with a lower binding capacity to guanine than to cytosine,

preferably, the nucleic acid molecule is used as an internal standard or control for detecting the DNA methylation level of the corresponding sequence in the sample.

2. A reagent for detecting DNA methylation, wherein the reagent comprises a reagent for detecting the methylation level of a DNA sequence or a fragment thereof or the methylation status or level of one or more CpG dinucleotides in the DNA sequence or fragment thereof in a sample of a subject to be detected, and the DNA sequence is selected from one or more or all of the following gene sequences, or sequences within 20 kb upstream or downstream thereof: DMRTA2, FOXD3, TBX15, BCAN, TRIM58, SIX3, VAX2, EMX1, LBX2, TLX2, POU3F3, TBR1, EVX2, HOXD12, HOXD8, HOXD4, TOPAZ1, SHOX2, DRD5, RPL9, HOPX, SFRP2, IRX4, TBX18, OLIG3, ULBP1, HOXA13, TBX20, IKZF1, INSIG1, SOX7, EBF2, MOS, MKX, KCNA6, SYT10, AGAP2, TBX3, CCNA1, ZIC2, CLEC14A, OTX2, C14orf39, BNC1, AHSP, ZFHX3, LHX1, TIMP2, ZNF750, SIM2,
preferably,

the DNA sequence is selected from one or more or all of the following sequences or complementary sequences thereof: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ IDNO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, or variants having at least 70% identity thereto, wherein the methylation sites in the variants are not mutated, and/or
the reagent is a primer molecule that hybridizes with the DNA sequence or fragment thereof, and the primer molecule can amplify the DNA sequence or fragment thereof after sulfite treatment, and/or
the reagent is a probe molecule that hybridizes with the DNA sequence or fragment thereof.

3. A medium recording DNA sequences or fragments thereof and/or methylation information thereof, wherein the DNA sequence is (i) selected from one, more or all of the following gene sequences, or sequences within 20 kb upstream or downstream thereof: DMRTA2, FOXD3, TBX15, BCAN, TRIM58, SIX3, VAX2, EMX1, LBX2, TLX2, POU3F3, TBR1, EVX2, HOXD12, HOXD8, HOXD4, TOPAZ1, SHOX2, DRD5, RPL9, HOPX, SFRP2, IRX4, TBX18, OLIG3, ULBP1, HOXA13, TBX20, IKZF1, INSIG1, SOX7, EBF2, MOS, MKX, KCNA6, SYT10, AGAP2, TBX3, CCNA1, ZIC2, CLEC14A, OTX2, C14orf39, BNC1, AHSP, ZFHX3, LHX1, TIMP2, ZNF750, SIM2, or (ii) sequences of (i) that have been treated to convert unmethylated cytosine into a base with a lower binding capacity to guanine than to cytosine, preferably,

the medium is used for alignment with the gene methylation sequencing data to determine the presence, content and/or methylation level of nucleic acid molecules comprising the sequence or fragment thereof, and/or

the DNA sequence comprises a sense strand or an antisense strand of DNA, and/or

the length of the fragment is 1-1000bp, and/or

the DNA sequence is selected from one or more or all of the following sequences or complementary sequences thereof: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO: 10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, or variants having at least 70% identity thereto, wherein the methylation sites in the variants are not mutated, more preferably,

the medium is a carrier printed with the DNA sequence or fragment thereof and/or methylation information thereof, and/or

the medium is a computer-readable medium storing the sequence or fragment thereof and/or methylation information thereof and a computer program, and when the computer program is executed by a processor, the following steps are implemented: comparing the methylation sequencing data of a sample with the sequence or fragment thereof to obtain the presence, content and/or methylation level of nucleic acid molecules containing the sequence or fragment thereof in the sample, wherein the presence, content and/or methylation level are used to diagnose pancreatic cancer.

4. Use of the following items (a) and/or (b) in the preparation of a kit for diagnosing pancreatic cancer in a subject,

(a) reagents or devices for determining the methylation level of a DNA sequence or a fragment thereof or the methylation status or level of one or more CpG dinucleotides in the DNA sequence or fragment thereof in a sample of a subject,

(b) a nucleic acid molecule of the DNA sequence or fragment thereof that has been treated to convert unmethylated cytosine into a base with a lower binding capacity to guanine than to cytosine,

wherein, the DNA sequence is selected from one, more or all of the following gene sequences, or sequences within 20 kb upstream or downstream thereof: DMRTA2, FOXD3, TBX15, BCAN, TRIM58, SIX3, VAX2 , EMX1, LBX2, TLX2, POU3F3, TBR1, EVX2, HOXD12, HOXD8, HOXD4, TOPAZ1, SHOX2, DRD5, RPL9, HOPX, SFRP2, IRX4, TBX18, OLIG3, ULBP1, HOXA13, TBX20, IKZF1, INSIG1, SOX7, EBF2, MOS, MKX, KCNA6, SYT10, AGAP2, TBX3, CCNA1, ZIC2, CLEC14A, OTX2, C14orf39, BNC1, AHSP, ZFHX3, LHX1, TIMP2, ZNF750, SIM2, preferably, the length of the fragment is 1-1000 bp.

5. The use of embodiment 4, wherein the DNA sequence is selected from one or more or all of the following sequences or complementary sequences thereof: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, or variants having at least 70% identity thereto, wherein the methylation sites in the variants are not mutated.

6. The use of embodiment 4 or 5, wherein,

the reagent comprises a primer molecule that hybridizes with the DNA sequence or fragment thereof, and/or

the reagent comprises a probe molecule that hybridizes with the DNA sequence or fragment thereof, and/or

the reagents comprise the medium of embodiment 3.

7. The use of embodiment 4 or 5, wherein,

the sample is from mammalian tissues, cells or body fluids, for example from pancreatic tissue or blood, and/or

the sample includes genomic DNA or cfDNA, and/or

the DNA sequence is converted in which unmethylated cytosine is converted into a base that has a lower binding capacity to guanine than to cytosine, and/or
the DNA sequence is treated with methylation-sensitive restriction enzymes.

8. The use according to embodiment 4 or 5, wherein the diagnosis involves: obtaining a score by comparing with a control sample and/or a reference level or by calculation, and diagnosing pancreatic cancer based on the score; preferably, the calculation is performed by constructing a support vector machine model.

9. A kit for identifying pancreatic cancer, including:

(a) reagents or devices for determining the methylation level of a DNA sequence or a fragment thereof or the methylation status or level of one or more CpG dinucleotides in the DNA sequence or fragment thereof in a sample of a subject, and
optionally, (b) a nucleic acid molecule of the DNA sequence or fragment thereof that has been processed to convert unmethylated cytosine into a base with a lower binding capacity to guanine than to cytosine,
wherein, the DNA sequence is selected from one, more (e.g., at least 7) or all of the following gene sequences, or sequences within 20 kb upstream or downstream thereof: DMRTA2, FOXD3, TBX15, BCAN, TRIM58, SIX3, VAX2 , EMX1, LBX2, TLX2, POU3F3, TBR1, EVX2, HOXD12, HOXD8, HOXD4, TOPAZ1, SHOX2, DRD5, RPL9, HOPX, SFRP2, IRX4, TBX18, OLIG3, ULBP1, HOXA13, TBX20, IKZF1, INSIG1, SOX7, EBF2, MOS, MKX, KCNA6, SYT10, AGAP2, TBX3, CCNA1, ZIC2, CLEC14A, OTX2, C14orf39, BNC1, AHSP, ZFHX3, LHX1, TIMP2, ZNF750, SIM2, preferably,
the DNA sequence is selected from one or more or all of the following sequences or complementary sequences thereof: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, or variants having at least 70% identity thereto, wherein the methylation sites in the variants are not mutated, and/or
the kit is suitable for the use of any one of embodiments 6-8, and/or
the reagent comprises a primer molecule that hybridizes with the DNA sequence or fragment thereof, and/or
the reagent comprises a probe molecule that hybridizes with the DNA sequence or fragment thereof, and/or
the reagents comprise the medium of embodiment 3, and/or
the sample is from mammalian tissues, cells or body fluids, for example from pancreatic tissue or blood, and/or
the DNA sequence is converted in which unmethylated cytosine is converted into a base that has a lower binding capacity to guanine than to cytosine, and/or
the DNA sequence is treated with methylation-sensitive restriction enzymes.

10. A device for diagnosing pancreatic cancer, including a memory, a processor, and a computer program stored in the memory and executable on the processor, wherein, the following steps are implemented when the processor executes the program:

(1) obtaining the methylation level of a DNA sequence or a fragment thereof or the methylation status or level of one or more CpG dinucleotides in the DNA sequence or fragment thereof in a sample of a subject to be detected, wherein the DNA sequence is selected from one or more or all of the following gene sequences: DMRTA2, FOXD3, TBX15, BCAN, TRIM58, SIX3, VAX2, EMX1, LBX2, TLX2, POU3F3, TBR1, EVX2, HOXD12, HOXD8, HOXD4, TOPAZ1, SHOX2, DRD5, RPL9, HOPX, SFRP2, IRX4, TBX18, OLIG3, ULBP1, HOXA13, TBX20, IKZF1, INSIG1, SOX7, EBF2, MOS, MKX, KCNA6, SYT10, AGAP2, TBX3, CCNA1, ZIC2, CLEC14A, OTX2, C14orf39, BNC1, AHSP, ZFHX3, LHX1, TIMP2, ZNF750, SIM2,
(2) obtaining a score by comparing with a control sample and/or a reference level or by calculation, and
(3) diagnosing pancreatic cancer based on the score,
preferably,
the DNA sequence is selected from one or more or all of the following sequences or complementary sequences thereof: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, or variants having at least 70% identity thereto, wherein the methylation sites in the variants are not mutated, and/or step (1) comprises detecting the methylation level of the sequence in the sample by means of the nucleic acid molecule of embodiment 1 and/or the reagent of embodiment 2 and/or the medium of embodiment 3, and/or the sample includes genomic DNA or cfDNA, and/or

the sequence is converted in which unmethylated cytosine is converted into a base that has a lower binding capacity to guanine than to cytosine, and/or

the DNA sequence is treated with methylation-sensitive restriction enzymes, and/or

the score in step (2) is calculated by constructing a support vector machine model.

Embodiment 2

1. An isolated nucleic acid molecule from a mammal, wherein the nucleic acid molecule is a methylation marker related to the differentiation between pancreatic cancer and pancreatitis, the sequence of the nucleic acid molecule includes (1) one or more or all of the sequences selected from the group consisting of SEQ ID NO:57, SEQ ID NO:58, SEQ ID NO:59, or variants having at least 70% identity thereto, the methylation sites in the variants are not mutated, (2) complementary sequences of (1), (3) sequences of (1) or (2) that have been treated to convert unmethylated cytosine into a base with a lower binding capacity to guanine than to cytosine,

preferably, the nucleic acid molecule is used as an internal standard or control for detecting the DNA methylation level of the corresponding sequence in the sample.

2. A reagent for detecting DNA methylation, wherein the reagent comprises a reagent for detecting the methylation level of a DNA sequence or a fragment thereof or the methylation status or level of one or more CpG dinucleotides in the DNA sequence or fragment thereof in a sample of a subject to be detected, and the DNA sequence is selected from one or more or all of the following gene sequences, or sequences within 20 kb upstream or downstream thereof: SIX3, TLX2, CILP2,

preferably,

the DNA sequence is selected from one or more or all of the following sequences or complementary sequences thereof: SEQ ID NO:57, SEQ ID NO:58, SEQ ID NO:59, or variants having at least 70% identity thereto, the methylation sites in the variants are not mutated, and/or

the reagent is a primer molecule that hybridizes with the DNA sequence or fragment thereof, and the primer molecule can amplify the DNA sequence or fragment thereof after sulfite treatment, and/or

the reagent is a probe molecule that hybridizes with the DNA sequence or fragment thereof.

3. A medium recording DNA sequences or fragments thereof and/or methylation information thereof, wherein the DNA sequence is (i) selected from one, more or all of the following gene sequences, or sequences within 20 kb upstream or downstream thereof: SIX3, TLX2, CILP2, or (ii) sequences of (i) that have been treated to convert unmethylated cytosine into a base with a lower binding capacity to guanine than to cytosine,

preferably,

the medium is used for alignment with the gene methylation sequencing data to determine the presence, content and/or methylation level of nucleic acid molecules comprising the sequence or fragment thereof, and/or

the DNA sequence comprises a sense strand or an antisense strand of DNA, and/or

the length of the fragment is 1-1000bp, and/or

the DNA sequence is selected from one or more or all of the following sequences or complementary sequences thereof: SEQ ID NO:57, SEQ ID NO:58, SEQ ID NO:59, or variants having at least 70% identity thereto, the methylation sites in the variants are not mutated,

more preferably,

the medium is a carrier printed with the DNA sequence or fragment thereof and/or methylation information thereof, and/or

the medium is a computer-readable medium storing the sequence or fragment thereof and/or methylation information thereof and a computer program, and when the computer program is executed by a processor, the following steps are implemented: comparing the methylation sequencing data of a sample with the sequence or fragment thereof to obtain the presence, content and/or methylation level of nucleic acid molecules containing the sequence or fragment thereof in the sample, wherein the presence, content and/or methylation level are used for differentiating between pancreatic cancer and pancreatitis.

4. Use of the following items (a) and/or (b) in the preparation of a kit for differentiating between pancreatic cancer and pancreatitis,

(a) reagents or devices for determining the methylation level of a DNA sequence or a fragment thereof or the methylation status or level of one or more CpG dinucleotides in the DNA sequence or fragment thereof in a sample of a subject,
(b) a nucleic acid molecule of the DNA sequence or fragment thereof that has been treated to convert unmethylated cytosine into a base with a lower binding capacity to guanine than to cytosine,

wherein, the DNA sequence is selected from one, more or all of the following gene sequences, or sequences within 20 kb upstream or downstream thereof: SIX3, TLX2, CILP2,
preferably, the length of the fragment is 1-1000 bp.

5. The use of embodiment 4, wherein the DNA sequence is selected from one or more or all of the following sequences or complementary sequences thereof: SEQ ID NO:57, SEQ ID NO:58, SEQ ID NO:59, or variants having at least 70% identity thereto, the methylation sites in the variants are not mutated.
6. The use of embodiment 4 or 5, wherein,

the reagent comprises a primer molecule that hybridizes with the DNA sequence or fragment thereof, and/or
the reagent comprises a probe molecule that hybridizes with the DNA sequence or fragment thereof, and/or
the reagents comprise the medium of embodiment 3.

7. The use of embodiment 4 or 5, wherein,

the sample is from mammalian tissues, cells or body fluids, for example from pancreatic tissue or blood, and/or
the sample includes genomic DNA or cfDNA, and/or
the DNA sequence is converted in which unmethylated cytosine is converted into a base that has a lower binding capacity to guanine than to cytosine, and/or
the DNA sequence is treated with methylation-sensitive restriction enzymes.

8. The use according to embodiment 4 or 5, wherein the diagnosis involves: obtaining a score by comparing with a control sample and/or a reference level or by calculation, and differentiating between pancreatic cancer and pancreatitis based on the score; preferably, the calculation is performed by constructing a support vector machine model.
9. A kit for differentiating between pancreatic cancer and pancreatitis, comprising:

(a) reagents or devices for determining the methylation level of a DNA sequence or a fragment thereof or the methylation status or level of one or more CpG dinucleotides in the DNA sequence or fragment thereof in a sample of a subject, and

optionally, (b) a nucleic acid molecule of the DNA sequence or fragment thereof that has been processed to convert unmethylated cytosine into a base with a lower binding capacity to guanine than to cytosine,
wherein, the DNA sequence is selected from one, more or all of the following gene sequences, or sequences within 20 kb upstream or downstream thereof: SIX3, TLX2, CILP2,
preferably,
the DNA sequence is selected from one or more or all of the following sequences or complementary sequences thereof: SEQ ID NO:57, SEQ ID NO:58, SEQ ID NO:59, or variants having at least 70% identity thereto, the methylation sites in the variants are not mutated, and/or
the kit is suitable for the use of any one of embodiments 6-8, and/or
the reagent comprises a primer molecule that hybridizes with the DNA sequence or fragment thereof, and/or
the reagent comprises a probe molecule that hybridizes with the DNA sequence or fragment thereof, and/or
the reagents comprise the medium of embodiment 3, and/or
the sample is from mammalian tissues, cells or body fluids, for example from pancreatic tissue or blood, and/or
the DNA sequence is converted in which unmethylated cytosine is converted into a base that has a lower binding capacity to guanine than to cytosine, and/or
the DNA sequence is treated with methylation-sensitive restriction enzymes.

10. A device for differentiating between pancreatic cancer and pancreatitis, including a memory, a processor, and a computer program stored in the memory and executable on the processor, wherein, the following steps are implemented when the processor executes the program:

(1) obtaining the methylation level of a DNA sequence or a fragment thereof or the methylation status or level of one or more CpG dinucleotides in the DNA sequence or fragment thereof in a sample of a subject to be detected, wherein the DNA sequence is selected from one or more or all of the following gene sequences: SIX3, TLX2, CILP2,
(2) obtaining a score by comparing with a control sample and/or a reference level or by calculation, and
(3) differentiating between pancreatic cancer and pancreatitis based on the score,
preferably,

the DNA sequence is selected from one or more or all of the following sequences or complementary sequences thereof: SEQ ID NO:57, SEQ ID NO:58, SEQ ID NO:59, or variants having at least 70% identity thereto, the methylation sites in the variants are not mutated, and/or
step (1) comprises detecting the methylation level of the sequence in the sample by means of the nucleic acid molecule of embodiment 1 and/or the reagent of embodiment 2 and/or the medium of embodiment 3, and/or the sample includes genomic DNA or cfDNA, and/or
the sequence is converted in which unmethylated cytosine is converted into a base that has a lower binding capacity to guanine than to cytosine, and/or
the DNA sequence is treated with methylation-sensitive restriction enzymes, and/or the score in step (2) is calculated by constructing a support vector machine model.

Embodiment 3

1. A method for assessing the presence and/or progression of a pancreatic tumor, comprising determining the presence and/or content of modification status of a DNA region selected from the following DNA regions, or complementary regions thereof, or fragments thereof in a sample to be tested:

| Chromosome range number | Chromosome range |
|---|---|
| 1 | derived from human chr1: 3310705-3310905 |
| 2 | derived from human chr1: 61520321-61520632 |
| 3 | derived from human chr1: 77333096-77333296 |
| 4 | derived from human chr1: 170630461-170630661 |
| 5 | derived from human chr1: 180202481-180202846 |
| 6 | derived from human chr1: 240161230-240161455 |
| 7 | derived from human chr2: 468096-468607 |
| 8 | derived from human chr2: 469568-469933 |
| 9 | derived from human chr2: 45155938-45156214 |
| 10 | derived from human chr2: 63285937-63286137 |
| 11 | derived from human chr2: 63286154-63286354 |
| 12 | derived from human chr2: 72371208-72371433 |
| 13 | derived from human chr2: 177043062-177043477 |
| 14 | derived from human chr2: 238864855-238865085 |
| 15 | derived from human chr3: 49459532-49459732 |
| 16 | derived from human chr3: 147109862-147110062 |
| 17 | derived from human chr3: 179754913-179755264 |
| 18 | derived from human chr3: 185973717-185973917 |
| 19 | derived from human chr3: 192126117-192126324 |
| 20 | derived from human chr4: 1015773-1015973 |
| 21 | derived from human chr4: 3447856-3448097 |

(continued)

| Chromosome range number | Chromosome range |
|---|---|
| 22 | derived from human chr4: 5710006-5710312 |
| 23 | derived from human chr4: 8859842-8860042 |
| 24 | derived from human chr5: 3596560-3596842 |
| 25 | derived from human chr5: 3599720-3599934 |
| 26 | derived from human chr5: 37840176-37840376 |
| 27 | derived from human chr5: 76249591-76249791 |
| 28 | derived from human chr5: 134364359-134364559 |
| 29 | derived from human chr5: 134870613-134870990 |
| 30 | derived from human chr5: 170742525-170742728 |
| 31 | derived from human chr5: 172659554-172659918 |
| 32 | derived from human chr5: 177411431-177411827 |
| 33 | derived from human chr6: 391439-391639 |
| 34 | derived from human chr6: 1378941-1379141 |
| 35 | derived from human chr6: 1625294-1625494 |
| 36 | derived from human chr6: 40308768-40308968 |
| 37 | derived from human chr6: 99291616-99291816 |
| 38 | derived from human chr6: 167544878-167545117 |
| 39 | derived from human chr7: 35297370-35297570 |
| 40 | derived from human chr7: 35301095-35301411 |
| 41 | derived from human chr7: 158937005-158937205 |
| 42 | derived from human chr8: 20375580-20375780 |
| 43 | derived from human chr8: 23564023-23564306 |
| 44 | derived from human chr8: 23564051-23564251 |
| 45 | derived from human chr8: 57358434-57358672 |
| 46 | derived from human chr8: 70983528-70983793 |
| 47 | derived from human chr8: 99986831-99987031 |
| 48 | derived from human chr9: 126778194-126778644 |
| 49 | derived from human chr10: 74069147-74069510 |
| 50 | derived from human chr10: 99790636-99790963 |
| 51 | derived from human chr10: 102497304-102497504 |
| 52 | derived from human chr10: 103986463-103986663 |
| 53 | derived from human chr10: 105036590-105036794 |
| 54 | derived from human chr10: 124896740-124897020 |
| 55 | derived from human chr10: 124905504-124905704 |
| 56 | derived from human chr10: 130084908-130085108 |
| 57 | derived from human chr10: 134016194-134016408 |
| 58 | derived from human chr11: 2181981-2182295 |
| 59 | derived from human chr11: 2292332-2292651 |

(continued)

| Chromosome range number | Chromosome range |
|---|---|
| 60 | derived from human chr11: 31839396-31839726 |
| 61 | derived from human chr11: 73099779-73099979 |
| 62 | derived from human chr11: 132813724-132813924 |
| 63 | derived from human chr12: 52311647-52311991 |
| 64 | derived from human chr12: 63544037-63544348 |
| 65 | derived from human chr12: 113902107-113902307 |
| 66 | derived from human chr13: 111186630-111186830 |
| 67 | derived from human chr13: 111277395-111277690 |
| 68 | derived from human chr13: 112711391-112711603 |
| 69 | derived from human chr13: 112758741-112758954 |
| 70 | derived from human chr13: 112759950-112760185 |
| 71 | derived from human chr14: 36986598-36986864 |
| 72 | derived from human chr14: 60976665-60976952 |
| 73 | derived from human chr14: 105102449-105102649 |
| 74 | derived from human chr14: 105933655-105933855 |
| 75 | derived from human chr15: 68114350-68114550 |
| 76 | derived from human chr15: 68121381-68121679 |
| 77 | derived from human chr15: 68121923-68122316 |
| 78 | derived from human chr15: 76635120-76635744 |
| 79 | derived from human chr15: 89952386-89952646 |
| 80 | derived from human chr15: 96856960-96857162 |
| 81 | derived from human chr16: 630128-630451 |
| 82 | derived from human chr16: 57025884-57026193 |
| 83 | derived from human chr16: 67919979-67920237 |
| 84 | derived from human chr17: 2092044-2092244 |
| 85 | derived from human chr17: 46796653-46796853 |
| 86 | derived from human chr17: 73607909-73608115 |
| 87 | derived from human chr17: 75369368-75370149 |
| 88 | derived from human chr17: 80745056-80745446 |
| 89 | derived from human chr18: 24130835-24131035 |
| 90 | derived from human chr18: 76739171-76739371 |
| 91 | derived from human chr18: 77256428-77256628 |
| 92 | derived from human chr19: 2800642-2800863 |
| 93 | derived from human chr19: 3688030-3688230 |
| 94 | derived from human chr19: 4912069-4912269 |
| 95 | derived from human chr19: 16511819-16512143 |
| 96 | derived from human chr19: 55593132-55593428 |
| 97 | derived from human chr20: 21492735-21492935 |

(continued)

| Chromosome range number | Chromosome range |
|---|---|
| 98 | derived from human chr20: 55202107-55202685 |
| 99 | derived from human chr20: 55925328-55925530 |
| 100 | derived from human chr20: 62330559-62330808 |
| 101 | derived from human chr22: 36861325-36861709 |

2. A method for assessing the presence and/or progression of a pancreatic tumor, comprising determining the presence and/or content of modification status of a DNA region selected from any one of SEQ ID NOs: 60 to 160, or complementary regions thereof, or fragments thereof in a sample to be tested.
A method for assessing the existence and/or progression of a pancreatic tumor, comprising determining the existence and/or content of modification status of a DNA region with genes selected from the group consisting of ARHGEF16, PRDM16, NFIA, ST6GALNAC5, PRRX1, LHX4, ACBD6, FMN2, CHRM3, FAM150B, TMEM18, SIX3, CAMKMT, OTX1, WDPCP, CYP26B1, DYSF, HOXD1, HOXD4, UBE2F, RAMP1, AMT, PLSCR5, ZIC4, PEX5L, ETV5, DGKG, FGF12, FGFRL1, RNF212, DOK7, HGFAC, EVC, EVC2, HMX1, CPZ, IRX1, GDNF, AGGF1, CRHBP, PITX1, CATSPER3, NEUROG1, NPM1, TLX3, NKX2-5, BNIP1, PROP1, B4GALT7, IRF4, FOXF2, FOXQ1, FOXC1, GMDS, MOCS1, LRFN2, POU3F2, FBXL4, CCR6, GPR31, TBX20, HERPUD2, VIPR2, LZTS1, NKX2-6, PENK, PRDM14, VPS13B, OSR2, NEK6, LHX2, DDIT4, DNAJB12, CRTAC1, PAX2, HIF1AN, ELOVL3, INA, HMX2, HMX3, MKI67, DPYSL4, STK32C, INS, INS-IGF2, ASCL2, PAX6, RELT, FAM168A, OPCML, ACVR1B, ACVRL1, AVPR1A, LHX5, SDSL, RAB20, COL4A2, CARKD, CARS2, SOX1, TEX29, SPACA7, SFTA3, SIX6, SIX1, INF2, TMEM179, CRIP2, MTA1, PIAS1, SKOR1, ISL2, SCAPER, POLG, RHCG, NR2F2, RAB40C, PIGQ, CPNE2, NLRC5, PSKH1, NRN1L, SRR, HIC1, HOXB9, PRAC1, SMIM5, MYO15B, TNRC6C, 9-Sep, TBCD, ZNF750, KCTD1, SALL3, CTDP1, NFATC1, ZNF554, THOP1, CACTIN, PIP5K1C, KDM4B, PLIN3, EPS15L1, KLF2, EPS8L1, PPP1R12C, NKX2-4, NKX2-2, TFAP2C, RAE1, TNFRSF6B, ARFRP1, MYH9, and TXN2, or a fragment thereof in a sample to be tested.
3. The method of any one of embodiments 1-2, further comprising obtaining a nucleic acid in the sample to be tested.
4. The method of embodiment 3, wherein the nucleic acid includes a cell-free nucleic acid.
5. The method of any one of embodiments 1-4, wherein the sample to be tested includes tissue, cells and/or body fluids.
6. The method of any one of embodiments 1-5, wherein the sample to be tested includes plasma.
7. The method of any one of embodiments 1-6, further comprising converting the DNA region or fragment thereof.
8. The method of embodiment 7, wherein the base with the modification status and the base without the modification status form different substances after the conversion, respectively.
9. The method of any one of embodiments 7-8, wherein the base with the modification status is substantially unchanged after conversion, and the base without the modification status is changed to other bases different from the base after conversion or is cleaved after conversion.
10. The method of any one of embodiments 8-9, wherein the base includes cytosine.
11. The method of any one of embodiments 1-10, wherein the modification status includes methylation modification.
12. The method of any one of embodiments 9-11, wherein the other base includes cytosine.
13. The method of any one of embodiments 7-12, wherein the conversion comprises conversion by a deamination reagent and/or a methylation-sensitive restriction enzyme.
14. The method of embodiment 13, wherein the deamination reagent includes bisulfite or analogues thereof.
15. The method of any one of embodiments 1-14, wherein the method for determining the presence and/or content of modification status comprises determining the presence and/or content of a DNA region with the modification status or a fragment thereof.
16. The method of any one of embodiments 1-15, wherein the presence and/or content of the DNA region with the modification status or fragment thereof is detected by sequencing.
17. The method of embodiments 1-16, wherein the presence or progression of a pancreatic tumor is determined by determining the presence of modification status of the DNA region or fragment thereof and/or a higher content of modification status of the DNA region or fragment thereof relative to the reference level.
18. A nucleic acid comprising a sequence capable of binding to the DNA region of embodiment 1, or a complementary region thereof, or a converted region thereof, or a fragment thereof.
19. A nucleic acid comprising a sequence capable of binding to the DNA region selected from any one of SEQ ID NO: 60 to 160, or a complementary region thereof, or a converted region thereof, or a fragment thereof.

20. A nucleic acid comprising a sequence capable of binding to a DNA region with the genes selected from embodiment 2, or a complementary region thereof, or a converted region thereof, or a fragment thereof:

21. A kit comprising the nucleic acid of any one of embodiments 18-20.

22. Use of the nucleic acid of any one of embodiments 18-20 and/or the kit of embodiment 21 in the preparation of a disease detection product.

23. Use of the nucleic acid of any one of embodiments 18-20, and/or the kit according to embodiment 21, in the preparation of a substance for assessing the presence and/or progression of a pancreatic tumor.

24. Use of the nucleic acid of any one of embodiments 18-20, and/or the kit of embodiment 21, in the preparation of a substance for determining the modification status of the DNA region or fragment thereof.

25. A method for preparing a nucleic acid, comprising designing a nucleic acid capable of binding to the DNA region selected from embodiment 1, or complementary region thereof, or converted region thereof, or fragment thereof, based on the modification status of the DNA region, or complementary region thereof, or converted region thereof, or fragment thereof.

26. A method for preparing a nucleic acid, comprising designing a nucleic acid capable of binding to a DNA region selected from any one of SEQ ID NO: 60 to 160, or a complementary region thereof, or a converted region thereof, or a fragment thereof, based on the modification status of the DNA region, or complementary region thereof, or converted region thereof, or fragment thereof.

27. A method for preparing a nucleic acid, comprising designing a nucleic acid capable of binding to a DNA region with genes of embodiment 2, or a complementary region thereof, or a converted region thereof, or a fragment thereof, based on the modification status of the DNA region, or complementary region thereof, or converted region thereof, or fragment thereof.

28. Use of nucleic acids, nucleic acid combinations and/or kits for determining the modification status of a DNA region in the preparation of a substance for assessing the presence and/or progression of a pancreatic tumor, wherein the DNA region for determination comprises a sequence of a DNA region selected from embodiment 1, or a complementary region thereof, or a converted region thereof, or a fragment thereof.

29. Use of nucleic acids, nucleic acid combinations and/or kits for determining the modification status of a DNA region in the preparation of a substance for assessing the presence and/or progression of a pancreatic tumor, wherein the DNA region for determination comprises a sequence of a DNA region selected from any one of SEQ ID NOs: 60 to 160, or a complementary region thereof, or a converted region thereof, or a fragment thereof.

30. Use of nucleic acids, nucleic acid combinations and/or kits for determining the modification status of a DNA region in the preparation of a substance for assessing the presence and/or progression of a pancreatic tumor, wherein the DNA region for determination comprises a sequence of a DNA region with genes selected from embodiment 2, or a complementary region thereof, or a converted region thereof, or a fragment thereof.

31. The use of any one of embodiments 29-30, wherein the modification status includes methylation modification.

32. A storage medium recording a program capable of executing the method of any one of embodiments 1-17.

33. A device comprising the storage medium of embodiment 32, and optionally further comprising a processor coupled to the storage medium, wherein the processor is configured to execute based on a program stored in the storage medium to implement the method of any one of embodiments 1-17.

Embodiment 4

1. A method for constructing a pancreatic cancer diagnostic model, comprising:

(1) obtaining the methylation level of a DNA sequence or a fragment thereof or the methylation status or level of one or more CpG dinucleotides in the DNA sequence or fragment thereof in a sample of a subject, and the CA19-9 level of the subject,
(2) obtaining a methylation score by calculation using a mathematical model using the methylation status or level,
(3) combining the methylation score and the CA19-9 level into a data matrix,
(4) constructing a pancreatic cancer diagnostic model based on the data matrix.

2. The method of embodiment 1, wherein the method further includes one or more features selected from the following:

the DNA sequence is selected from one or more of the following gene sequences, or sequences within 20 kb upstream or downstream thereof: SIX3, TLX2, CILP2,
the fragment comprise at least one CpG dinucleotide,
step (1) comprises detecting the methylation level of a DNA sequence or a fragment thereof or the methylation status or level of one or more CpG dinucleotides in the DNA sequence or fragment thereof in a sample of a subject,

the sample is from mammalian tissues, cells or body fluids, for example, pancreatic tissue or blood,
the CA19-9 level is blood or plasma CA19-9 level,
the mathematical model in step (2) is a support vector machine model,
the pancreatic cancer diagnostic model in step (4) is a logistic regression model.

3. A method for constructing a pancreatic cancer diagnostic model, comprising:

(1) obtaining the methylated haplotype fraction and sequencing depth of a subject's genomic DNA segment,
optionally (2) pre-processing the methylated haplotype fraction and sequencing depth data,
(3) performing cross-validation incremental feature selection to obtain feature methylated segments,
(4) constructing a mathematical model for the methylation detection results of the feature methylated segments to obtain a methylation score,
(5) constructing a pancreatic cancer diagnostic model based on the methylation score and the corresponding CA19-9 level.

4. The method of embodiment 3, wherein the method further includes one or more features selected from the following: step (1) comprises:

1.1) detecting the DNA methylation of a sample of a subject to obtain sequencing read data,
1.2) optional pre-processing of the sequencing data, such as adapter removal and/or splicing,
1.3) aligning the sequencing data with the reference genome to obtain the location and sequencing depth information of the methylated segment,
1.4) calculating the methylated haplotype fraction (MHF) of the segment according to the following formula:

$$MHF_{i,h} = \frac{N_{i,h}}{N_i}$$

where $i$ represents the target methylated region, $h$ represents the target methylated haplotype, $Ni$ represents the number of reads located in the target methylated region, and $Ni_{,h}$ represents the number of reads containing the target methylated haplotype;
step (2) comprises: (2.1) combining the methylated haplotype fraction and sequencing depth information data into a data matrix; preferably, step (2) further comprises: 2.2) removing sites with a missing value proportion higher than 5-15% (e.g., 10%) from the data matrix, and/or 2.3) taking each data point with a depth less than 300 (e.g., less than 200) as a missing value, and imputing the missing values (e.g., using the K nearest neighbor method),
step (3) comprises: using a mathematical model to perform cross-validation incremental feature selection in the training data, wherein the DNA segments that increase the AUC of the mathematical model are feature methylated segments,
step (5) comprises: combining the methylation score and CA19-9 level into a data matrix, and constructing a pancreatic cancer diagnostic model based on the data matrix.

5. The method of embodiment 3 or 4, wherein the method further includes one or more features selected from the following:

the mathematical model in step (4) is a vector machine (SVM) model,
the methylation detection result in step (4) is a combined matrix of methylated haplotype fraction and sequencing depth,
the pancreatic cancer diagnostic model in step (5) is a logistic regression model.

6. Use of a reagent or device for detecting DNA methylation and a reagent or device for detecting CA19-9 levels in the preparation of a kit for diagnosing pancreatic cancer, wherein the reagent or device for detecting DNA methylation is used to determine the methylation level of a DNA sequence or a fragment thereof or the methylation status or level of one or more CpG dinucleotides in the DNA sequence or fragment thereof in a sample of a subject.
7. The use of embodiment 6, wherein the use further includes one or more features selected from the following:

the DNA sequence is selected from one or more of the following gene sequences, or sequences within 20 kb upstream or downstream thereof: SIX3, TLX2, CILP2,
the fragment comprise at least one CpG dinucleotide,

the reagent for detecting DNA methylation includes a primer molecule that hybridizes with the DNA sequence or fragment thereof, and the primer molecule can amplify the DNA sequence or fragment thereof after sulfite treatment, the reagent for detecting DNA methylation comprises a probe molecule that hybridizes with the DNA sequence or fragment thereof,

the reagent for detecting CA19-9 level is a detection reagent based on immune response,

the kit also comprises a PCR reaction reagent,

the kit also comprises other reagents for detecting DNA methylation, which are reagents used in one or more of methods selected from: bisulfite conversion-based PCR, DNA sequencing, methylation-sensitive restriction endonuclease assay, fluorescence quantification, methylation-sensitive high-resolution melting curve assay, chip-based methylation atlas, mass spectrometry,

the diagnosis includes: performing calculation by constructing the pancreatic cancer diagnostic model of any one of embodiments 1-5, and diagnosing pancreatic cancer based on the score.

8. A kit for diagnosing pancreatic cancer, comprising:

(a) reagents or devices for detecting DNA methylation, used to determine the methylation level of a DNA sequence or a fragment thereof or the methylation status or level of one or more CpG dinucleotides in the DNA sequence or fragment thereof in a sample of a subject, and
(b) reagents or devices for detecting CA19-9 level.

9. The kit of embodiment 8, wherein the kit further includes one or more features selected from the following:

the DNA sequence is selected from one or more of the following gene sequences, or sequences within 20 kb upstream or downstream thereof: SIX3, TLX2, CILP2,

the fragment comprise at least one CpG dinucleotide,

the reagent for detecting DNA methylation includes a primer molecule that hybridizes with the DNA sequence or fragment thereof, and the primer molecule can amplify the DNA sequence or fragment thereof after sulfite treatment, the reagent for detecting DNA methylation comprises a probe molecule that hybridizes with the DNA sequence or fragment thereof,

the reagent for detecting CA19-9 level is a detection reagent based on immune response,

the kit also comprises a PCR reaction reagent,

the kit also comprises other reagents for detecting DNA methylation, which are reagents used in one or more of the following methods: bisulfite conversion-based PCR, DNA sequencing, methylation-sensitive restriction endonuclease assay, fluorescence quantification, methylation-sensitive high-resolution melting curve assay, chip-based methylation atlas, mass spectrometry.

10. A device for diagnosing pancreatic cancer or constructing a pancreatic cancer diagnostic model, including a memory, a processor, and a computer program stored in the memory and executable on the processor, wherein the following steps are implemented when the processor executes the program:

(1) obtaining the methylation level of a DNA sequence or a fragment thereof or the methylation status or level of one or more CpG dinucleotides in the DNA sequence or fragment thereof in a sample of a subject, and the CA19-9 level of the subject,
(2) obtaining a methylation score by calculation using a mathematical model using the methylation status or level,
(3) combining the methylation score and the CA19-9 level into a data matrix,
(4) constructing a pancreatic cancer diagnostic model based on the data matrix,
optionally (5) obtaining a pancreatic cancer score; diagnosing pancreatic cancer based on the pancreatic cancer score,
or

(1) obtaining the methylation level of a DNA sequence or a fragment thereof or the methylation status or level of one or more CpG dinucleotides in the DNA sequence or fragment thereof in a sample of a subject, and the CA19-9 level of the subject,
(2) obtaining a methylation score by calculation using a mathematical model using the methylation status or level,
(3) obtaining a pancreatic cancer score according to the model shown below, and diagnosing pancreatic cancer based on the pancreatic cancer score:

$$y = \frac{1}{1 + e^{-(0.7032M + 0.6608C + 2.2243)}}$$

where M is the methylation score of the sample calculated in step (2), and C is the CA19-9 level of the sample, preferably, the device further includes one or more features selected from:

the DNA sequence is selected from one or more of the following gene sequences, or sequences within 20 kb upstream or downstream thereof: SIX3, TLX2, CILP2,
the fragment comprise at least one CpG dinucleotide,
step (1) comprises detecting the methylation level of a DNA sequence or a fragment thereof or the methylation status or level of one or more CpG dinucleotides in the DNA sequence or fragment thereof in a sample of a subject,
the sample is from mammalian tissues, cells or body fluids, for example, pancreatic tissue or blood,
the CA19-9 level is blood or plasma CA19-9 level,
the mathematical model in step (2) is a support vector machine model,
the pancreatic cancer diagnostic model in step (4) is a logistic regression model.

Embodiment 5

1. A method for determining the presence of a pancreatic tumor, assessing the development or risk of development of a pancreatic tumor, and/or assessing the progression of a pancreatic tumor, comprising determining the presence and/or content of modification status of a DNA region with genes TLX2, EBF2, KCNA6, CCNA1, FOXD3, TRIM58, HOXD10, OLIG3, EN2, CLEC11A, TWIST 1 and/or EMX1 or fragments thereof in a sample to be tested.
2. A method for assessing the methylation status of a pancreatic tumor-related DNA region, comprising determining the presence and/or content of modification status of a DNA region with genes TLX2, EBF2, KCNA6, CCNA1, FOXD3, TRIM58, HOXD10, OLIG3, EN2, CLEC11A, TWIST1, and/or EMX1, or fragments thereof in a sample to be tested.
3. The method of any one of embodiments 1-2, wherein the DNA region is derived from human chr2:74740686-74744275, derived from human chr8:25699246-25907950, derived from human chr12:4918342-4960278, derived from human chr13:37005635-37017019, derived from human chr1:63788730-63790797, derived from human chr1:248020501-248043438, derived from human chr2:176945511-176984670, derived from human chr6:137813336-137815531, derived from human chr7:155167513-155257526, derived from human chr19:51226605-51228981, derived from human chr7:19155091-19157295, and derived from human chr2:73147574-73162020.
4. The method of any one of embodiments 1-3, further comprising obtaining a nucleic acid in the sample to be tested.
5. The method of embodiment 4, wherein the nucleic acid includes a cell-free nucleic acid.
6. The method of any one of embodiments 1-5, wherein the sample to be tested includes tissue, cells and/or body fluids.
7. The method of any one of embodiments 1-6, wherein the sample to be tested includes plasma.
8. The method of any one of embodiments 1-7, further comprising converting the DNA region or fragment thereof.
9. The method of embodiment 8, wherein the base with the modification status and the base without the modification status form different substances after conversion.
10. The method of any one of embodiments 1-9, wherein the base with the modification status is substantially unchanged after conversion, and the base without the modification status is changed to other bases different from the base after conversion or is cleaved after conversion.
11. The method of any one of embodiments 9-10, wherein the base includes cytosine.
12. The method of any one of embodiments 1-11, wherein the modification status includes methylation modification.
13. The method of any one of embodiments 10-12, wherein the other base includes cytosine.
14. The method of any one of embodiments 8-13, wherein the conversion comprises conversion by a deamination reagent and/or a methylation-sensitive restriction enzyme.
15. The method of embodiment 14, wherein the deamination reagent includes bisulfite or analogues thereof.
16. The method of any one of embodiments 1-15, wherein the method for determining the presence and/or content of modification status comprises determining the presence and/or content of a substance formed by a base with the modification status after the conversion.
17. The method of any one of embodiments 1-16, wherein the method for determining the presence and/or content of modification status comprises determining the presence and/or content of a DNA region with the modification status or a fragment thereof.

18. The method of any one of embodiments 1-17, wherein the presence and/or content of the DNA region with the modification status or fragment thereof is determined by the fluorescence Ct value detected by the fluorescence PCR method.

19. The method of any one of embodiments 1-18, wherein the presence of a pancreatic tumor, or the development or risk of development of a pancreatic tumor is determined by determining the presence of modification status of the DNA region or fragment thereof and/or a higher content of modification status of the DNA region or fragment thereof relative to the reference level.

20. The method of any one of embodiments 1-19, further comprising amplifying the DNA region or fragment thereof in the sample to be tested before determining the presence and/or content of modification status of the DNA region or fragment thereof.

21. The method of embodiment 20, wherein the amplification comprises PCR amplification.

22. A method for determining the presence of a disease, assessing the development or risk of development of a disease, and/or assessing the progression of a disease, comprising determining the presence and/or content of modification status of a DNA region selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or a complementary region thereof, or a fragment thereof in a sample to be tested.

23. A method for determining the methylation status of a DNA region, comprising determining the presence and/or content of modification status of a DNA region selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or a complementary region thereof, or a fragment thereof in a sample to be tested.

24. The method of any one of embodiments 22-23, comprising providing a nucleic acid capable of binding to a DNA region selected from the group consisting of SEQ ID NOs: 164, 168, 172, 176, 180, 184, 188, 192, 196, 200, 204, 208, 212, 216, 220, 224, 228, and 232, or a complementary region thereof, or a converted region thereof, or a fragment thereof.

25. The method of any one of embodiments 22-24, comprising providing a nucleic acid capable of binding to a DNA region selected from the group consisting of DNA regions derived from human chr2:74743042-74743113 and derived form human chr2:74743157-74743253, derived form human chr2:74743042-74743113 and derived from human chr2:74743157-74743253, derived form human chr8:25907865-25907930 and derived from human chr8:25907698-25907814, derived form human chr12:4919188-4919272, derived form human chr12:4919036-4919164 and derived from human chr12:4919341-4919438, derived form human chr13:37005652-37005721, derived form human chr13:37005458-37005596 and derived from human chr13:37005694-37005824, derived form human chr1:63788850-63788913, derived form human chr1:248020635-248020731, derived form human chr2:176945521-176945603, derived form human chr6:137814750-137814815, derived form human chr7:155167531-155167610, derived form human chr19:51228620-51228722, and derived from human chr7:19156779-19157914, and derived from human chr2:73147571-73147626, or a complementary region thereof, or a converted region thereof, or a fragment thereof.

26. The method of any one of embodiments 22-25, comprising providing a nucleic acid selected from the group consisting of SEQ ID NOs: 165, 169, 173, 177, 181, 185, 189, 193, 197, 201, 205, 209, 213, 217, 221, 225, 229, and 233, or a complementary nucleic acid thereof, or a fragment thereof.

27. The method of any one of embodiments 22-26, comprising providing a nucleic acid combination selected from the group consisting of SEQ ID NOs: 166 and 167, 170 and 171, 174 and 175, 178 and 179, 182 and 183, 186 and 187, 190 and 191, 194 and 195, 198 and 199, 202 and 203, 206 and 207, 210 and 211, 214 and 215, 218 and 219, 222 and 223, 226 and 227, 230 and 231, and 234 and 235, or a complementary nucleic acid combination thereof, or a fragment thereof.

28. The method of any one of embodiments 22-27, wherein the disease includes a tumor.

29. The method of any one of embodiments 22-28, further comprising obtaining a nucleic acid in the sample to be tested.

30. The method of embodiment 29, wherein the nucleic acid includes a cell-free nucleic acid.

31. The method of any one of embodiments 22-30, wherein the sample to be tested includes tissue, cells and/or body fluids.

32. The method of any one of embodiments 22-31, wherein the sample to be tested includes plasma.

33. The method of any one of embodiments 22-32, further comprising converting the DNA region or fragment thereof.

34. The method of embodiment 33, wherein the base with the modification status and the base without the modification status form different substances after conversion.

35. The method of any one of embodiments 22-34, wherein the base with the modification status is substantially unchanged after conversion, and the base without the modification status is changed to other bases different from the base after conversion or is cleaved after conversion.

36. The method of any one of embodiments 34-35, wherein the base includes cytosine.

37. The method of any one of embodiments 22-36, wherein the modification status includes methylation modification.

38. The method of any one of embodiments 35-37, wherein the other base includes cytosine.

39. The method of any one of embodiments 33-38, wherein the conversion comprises conversion by a deamination reagent and/or a methylation-sensitive restriction enzyme.

40. The method of embodiment 39, wherein the deamination reagent includes bisulfite or analogues thereof.

41. The method of any one of embodiments 22-40, wherein the method for determining the presence and/or content of modification status comprises determining the presence and/or content of a substance formed by a base with the modification status after the conversion.

42. The method of any one of embodiments 22-41, wherein the method for determining the presence and/or content of modification status comprises determining the presence and/or content of a DNA region with the modification status or a fragment thereof.

43. The method of any one of embodiments 22-42, wherein the presence and/or content of the DNA region with the modification status or fragment thereof is determined by the fluorescence Ct value detected by the fluorescence PCR method.

44. The method of any one of embodiments 22-43, wherein the presence of a pancreatic tumor, or the development or risk of development of a pancreatic tumor is determined by determining the presence of modification status of the DNA region or fragment thereof and/or a higher content of modification status of the DNA region or fragment thereof relative to the reference level.

45. The method of any one of embodiments 22-44, further comprising amplifying the DNA region or fragment thereof in the sample to be tested before determining the presence and/or content of modification status of the DNA region or fragment thereof.

46. The method of embodiment 45, wherein the amplification comprises PCR amplification.

47. A nucleic acid, comprising a sequence capable of binding to a DNA region with genes TLX2, EBF2, KCNA6, CCNA1, FOXD3, TRIM58, HOXD10, OLIG3, EN2, CLEC11A, TWIST1, and/or EMX1, or a complementary region thereof, or a converted region thereof, or a fragment thereof.

48. A method for preparing a nucleic acid, comprising designing a nucleic acid capable of binding to a DNA region with genes TLX2, EBF2, KCNA6, CCNA1, FOXD3, TRIM58, HOXD10, OLIG3, EN2, CLEC11A, TWIST1, and/or EMX1, or a complementary region thereof, or a converted region thereof, or a fragment thereof, based on the modification status of the DNA region, or complementary region thereof, or converted region thereof, or fragment thereof.

49. A nucleic acid combination, comprising a sequence capable of binding to a DNA region with genes TLX2, EBF2, KCNA6, CCNA1, FOXD3, TRIM58, HOXD10, OLIG3, EN2, CLEC11A, TWIST1, and/or EMX1, or a complementary region thereof, or a converted region thereof, or a fragment thereof.

50. A method for preparing a nucleic acid combination, comprising designing a nucleic acid combination capable of amplifying a DNA region with genes TLX2, EBF2, KCNA6, CCNA1, FOXD3, TRIM58, HOXD10, OLIG3, EN2, CLEC11A, TWIST1, and/or EMX1, or a complementary region thereof, or a converted region thereof, or a fragment thereof, based on the modification status of the DNA region, or complementary region thereof, or converted region thereof, or fragment thereof.

51. A kit, comprising the nucleic acid of embodiment 47 and/or the nucleic acid combination of embodiment 49.

52. Use of the nucleic acid of embodiment 47, the nucleic acid combination of embodiment 49, and/or the kit of embodiment 51 in the preparation of a disease detection product.

53. Use of the nucleic acid of embodiment 47, the nucleic acid combination of embodiment 49 and/or the kit of embodiment 51 in the preparation of a substance for determining the presence of a disease, assessing the development or risk of development of a disease and/or assessing the progression of a disease.

54. Use of the nucleic acid of embodiment 47, the nucleic acid combination of embodiment 49 and/or the kit of embodiment 51 in the preparation of a substance for determining the modification status of the DNA region or fragment thereof.

55. Use of a nucleic acid, a nucleic acid combination and/or a kit for determining the modification status of a DNA region in the preparation of a substance for determining the presence of a pancreatic tumor, assessing the development or risk of development of a pancreatic tumor and/or assessing the progression of a pancreatic tumor, wherein the DNA region for determination includes DNA regions with genes TLX2, EBF2, KCNA6, CCNA1, FOXD3, TRIM58, HOXD10, OLIG3, EN2, CLEC11A, TWIST1, and/or EMX1, or fragments thereof.

56. Use of a nucleic acid, a nucleic acid combination and/or a kit for determining the modification status of a DNA region in the preparation of a substance for determining the presence of a disease, assessing the development or risk of development of a disease, and/or assessing the progression of a disease, wherein the DNA region includes a DNA region selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or a complementary region thereof, or a fragment thereof.

57. Use of nucleic acids of DNA regions with genes TLX2, EBF2, KCNA6, CCNA1, FOXD3, TRIM58, HOXD10, OLIG3, EN2, CLEC11A, TWIST1, and/or EMX1, or converted regions thereof, or fragments thereof, and combinations of the above-mentioned nucleic acids, in the preparation of a substance for determining the presence of a pancreatic tumor, assessing the development or risk of development of a pancreatic tumor, and/or assessing the progression of a pancreatic tumor.

58. Use of nucleic acids of DNA regions selected from the group consisting of DNA regions derived from human chr2:74743035-74743151 and derived from human chr2:74743080-74743301, derived from human chr8:25907849-25907950 and derived from human chr8:25907698-25907894, derived from human chr12:4919142-4919289, derived from human chr12:4918991-4919187 and derived from human chr12:4919235-4919439, derived from human chr13:37005635-37005754, derived from human chr13:37005458-37005653 and derived from human chr13:37005680-37005904, derived from human chr1:63788812-63788952, derived from human chr1:248020592-248020779, derived from human chr2:176945511-176945630, derived from human chr6:137814700-137814853, derived from human chr7:155167513-155167628, derived from human chr19:51228168-51228782, and derived from human chr7:19156739-19157277 and derived from human chr2:73147525-73147644, or complementary regions thereof, or converted regions thereof, or fragments thereof, and combinations of the above-mentioned nucleic acids, in the preparation of a substance for determining the presence of a disease, assessing the development or risk of development of a disease, and/or assessing the progression of a disease.

59. A storage medium recording a program capable of executing the method of any one of embodiments 1-46.

60. A device comprising the storage medium of embodiment 59.

61. The device of embodiment 60, further comprising a processor coupled to the storage medium, wherein the processor is configured to execute based on a program stored in the storage medium to implement the method as claimed in any one of embodiments 1-46.

Embodiment 6

1. A method for determining the presence of a pancreatic tumor, assessing the development or risk of development of a pancreatic tumor, and/or assessing the progression of a pancreatic tumor, comprising determining the presence and/or content of modification status of a DNA region with two genes selected from the group consisting of EBF2, and CCNA1, KCNA6, TLX2, and EMX1, TRIM58, TWIST1, FOXD3, and EN2, TRIM58, TWIST1, CLEC11A, HOXD10, and OLIG3, or fragments thereof in a sample to be tested.

2. A method for assessing the methylation status of a pancreatic tumor-related DNA region, comprising determining the presence and/or content of modification status of a DNA region with two genes selected from the group consisting of EBF2, and CCNA1, KCNA6, TLX2, and EMX1, TRIM58, TWIST1, FOXD3, and EN2, TRIM58, TWIST1, CLEC11A, HOXD10, and OLIG3, or fragments thereof in a sample to be tested.

3. The method of any one of embodiments 1-2, wherein the DNA region is selected from two of the group consisting of DNA regions derived from human chr8:25699246-25907950, and derived from human chr13:37005635-37017019, derived from human chr12:4918342-4960278, derived from human chr2:74740686-74744275, and derived from human chr2:73147574-73162020, derived from human chr1:248020501-248043438, derived from human chr7:19155091-19157295, derived from human chr1:63788730-63790797, and derived from human chr7:155167513-155257526, derived from human chr1:248020501-248043438, derived from human chr7:19155091-19157295, derived from human chr19:51226605-51228981, derived from human chr2:176945511-176984670, and derived from human chr6:137813336-137815531.

4. The method of any one of embodiments 1-3, further comprising obtaining a nucleic acid in the sample to be tested.

5. The method of embodiment 4, wherein the nucleic acid includes a cell-free nucleic acid.

6. The method of any one of embodiments 1-5, wherein the sample to be tested includes tissue, cells and/or body fluids.

7. The method of any one of embodiments 1-6, wherein the sample to be tested includes plasma.

8. The method of any one of embodiments 1-7, further comprising converting the DNA region or fragment thereof.

9. The method of embodiment 8, wherein the base with the modification status and the base without the modification status form different substances after conversion.

10. The method of any one of embodiments 1-9, wherein the base with the modification status is substantially unchanged after conversion, and the base without the modification status is changed to other bases different from the base after conversion or is cleaved after conversion.

11. The method of any one of embodiments 9-10, wherein the base includes cytosine.

12. The method of any one of embodiments 1-11, wherein the modification status includes methylation modification.

13. The method of any one of embodiments 10-12, wherein the other base includes cytosine.

14. The method of any one of embodiments 8-13, wherein the conversion comprises conversion by a deamination reagent and/or a methylation-sensitive restriction enzyme.

15. The method of embodiment 14, wherein the deamination reagent includes bisulfite or analogues thereof.

16. The method of any one of embodiments 1-15, wherein the method for determining the presence and/or content of modification status comprises determining the presence and/or content of a substance formed by a base with the modification status after the conversion.

17. The method of any one of embodiments 1-16, wherein the method for determining the presence and/or content of modification status comprises determining the presence and/or content of a DNA region with the modification status or a fragment thereof.

18. The method of any one of embodiments 1-17, wherein the presence and/or content of the DNA region with the modification status or fragment thereof is determined by the fluorescence Ct value detected by the fluorescence PCR method.

19. The method of any one of embodiments 1-18, wherein the presence of a pancreatic tumor, or the development or risk of development of a pancreatic tumor is determined by determining the presence of modification status of the DNA region or fragment thereof and/or a higher content of modification status of the DNA region or fragment thereof relative to the reference level.

20. The method of any one of embodiments 1-19, further comprising amplifying the DNA region or fragment thereof in the sample to be tested before determining the presence and/or content of modification status of the DNA region or fragment thereof.

21. The method of embodiment 20, wherein the amplification comprises PCR amplification.

22. A method for determining the presence of a disease, assessing the development or risk of development of a disease, and/or assessing the progression of a disease, comprising determining the presence and/or content of modification status of two DNA regions selected from the group consisting of DNA regions derived from human chr8:25907849-25907950, and derived from human chr13:37005635-37005754, derived from human chr12:4919142-4919289, derived from human chr2:74743035-74743151, and derived from human chr2:73147525-73147644, derived from human chr1:248020592-248020779, derived from human chr7:19156739-19157277, derived from human chr1:63788812-63788952, and derived from human chr7:155167513-155167628, derived from human chr1:248020592-248020779, derived from human chr7:19156739-19157277, derived from human chr19:51228168-51228782, derived from human chr2:176945511-176945630, and derived from human chr6:137814700-137814853, or complementary regions thereof, or fragments thereof in a sample to be tested.

23. A method for determining the methylation status of a DNA region, comprising determining the presence and/or content of modification status of two DNA regions selected from the group consisting of DNA regions derived from human chr8:25907849-25907950, and derived from human chr13:37005635-37005754, or derived from human chr12:4919142-4919289, derived from human chr2:74743035-74743151, and derived from human chr2:73147525-73147644, or derived from human chr1:248020592-248020779, derived from human chr7:19156739-19157277, derived from human chr1:63788812-63788952, and derived from human chr7:155167513-155167628, or derived from human chr1:248020592-248020779, derived from human chr7:19156739-19157277, derived from human chr19:51228168-51228782, derived from human chr2:176945511-176945630, and derived from human chr6:137814700-137814853, or complementary regions thereof, or fragments thereof in a sample to be tested.

24. The method of any one of embodiments 22-23, comprising providing a nucleic acid capable of binding to two DNA regions selected from the group consisting of SEQ ID NOs: 1 and 5, or complementary regions thereof, or converted regions thereof, or fragments thereof.

25. The method of any one of embodiments 22-24, comprising providing a nucleic acid capable of binding to two DNA regions selected from the group consisting of DNA regions derived from human chr8:25907865-25907930, and derived from human chr13:37005652-37005721, derived from human chr12:4919188-4919272, derived from human chr2:74743042-74743113, and derived from human chr2:73147571-73147626, derived from human chr1:248020635-248020731, derived from human chr7:19156779-19157914, derived from human chr1:63788850-63788913, and derived from human chr7:155167531-155167610, derived from human chr1:248020635-248020731, derived from human chr7:19156779-19157914, derived from human chr19:51228620-51228722, derived from human chr2:176945521-176945603, and derived from human chr6:137814750-137814815, or complementary regions thereof, or converted regions thereof, or fragments thereof.

26. The method of any one of embodiments 22-25, comprising providing two nucleic acids selected from the group consisting of SEQ ID NO: 173 and 193, 181, 165 and 233, 209, 229, 205 and 221, 209, 229, 225, 213 and 217, or complementary nucleic acids thereof, or fragments thereof.

27. The method of any one of embodiments 22-26, comprising providing two nucleic acid combinations selected from the group consisting of SEQ ID NOs: 174 and 175, and 194 and 195, 182 and 183, 166 and 167, and 234 and 235, 210 and 211, 230 and 231, 206 and 207, and 222 and 223, 210 and 211, 230 and 231, 226 and 227, 214 and 215, and 218 and 219, or complementary nucleic acid combinations thereof, or fragments thereof.

28. The method of any one of embodiments 22-27, wherein the disease includes a tumor.

29. The method of any one of embodiments 22-28, further comprising obtaining a nucleic acid in the sample to be tested.

30. The method of embodiment 29, wherein the nucleic acid includes a cell-free nucleic acid.

31. The method of any one of embodiments 22-30, wherein the sample to be tested includes tissue, cells and/or body fluids.

32. The method of any one of embodiments 22-31, wherein the sample to be tested includes plasma.

33. The method of any one of embodiments 22-32, further comprising converting the DNA region or fragment thereof.

34. The method of embodiment 33, wherein the base with the modification status and the base without the modification status form different substances after conversion.

35. The method of any one of embodiments 22-34, wherein the base with the modification status is substantially unchanged after conversion, and the base without the modification status is changed to other bases different from the base after conversion or is cleaved after conversion.

36. The method of any one of embodiments 34-35, wherein the base includes cytosine.

37. The method of any one of embodiments 22-36, wherein the modification status includes methylation modification.

38. The method of any one of embodiments 35-37, wherein the other base includes cytosine.

39. The method of any one of embodiments 33-38, wherein the conversion comprises conversion by a deamination reagent and/or a methylation-sensitive restriction enzyme.

40. The method of embodiment 39, wherein the deamination reagent includes bisulfite or analogues thereof.

41. The method of any one of embodiments 22-40, wherein the method for determining the presence and/or content of modification status comprises determining the presence and/or content of a substance formed by a base with the modification status after the conversion.

42. The method of any one of embodiments 22-41, wherein the method for determining the presence and/or content of modification status comprises determining the presence and/or content of a DNA region with the modification status or a fragment thereof.

43. The method of any one of embodiments 22-42, wherein the presence and/or content of the DNA region with the modification status or fragment thereof is determined by the fluorescence Ct value detected by the fluorescence PCR method.

44. The method of any one of embodiments 22-43, wherein the presence of a pancreatic tumor, or the development or risk of development of a pancreatic tumor is determined by determining the presence of modification status of the DNA region or fragment thereof and/or a higher content of modification status of the DNA region or fragment thereof relative to the reference level.

45. The method of any one of embodiments 22-44, further comprising amplifying the DNA region or fragment thereof in the sample to be tested before determining the presence and/or content of modification status of the DNA region or fragment thereof.

46. The method of embodiment 45, wherein the amplification comprises PCR amplification.

47. A nucleic acid, comprising a sequence capable of binding to a DNA region with two genes selected from the group consisting of EBF2, and CCNA1, KCNA6, TLX2, and EMX1, TRIM58, TWIST1, FOXD3, and EN2, TRIM58, TWIST1, CLEC11A, HOXD10, and OLIG3, or a complementary region thereof, or a converted region thereof, or a fragment thereof.

48. A method for preparing a nucleic acid, comprising designing a nucleic acid capable of binding to a DNA region with two genes selected from the group consisting of EBF2, and CCNA1, KCNA6, TLX2, and EMX1, TRIM58, TWIST1, FOXD3, and EN2, TRIM58, TWIST1, CLEC11A, HOXD10, and OLIG3, or a complementary region thereof, or a converted region thereof, or a fragment thereof, based on the modification status of the DNA region, or complementary region thereof, or converted region thereof, or fragment thereof.

49. A nucleic acid combination, comprising a sequence capable of binding to a DNA region with two genes selected from the group consisting of EBF2, and CCNA1, KCNA6, TLX2, and EMX1, TRIM58, TWIST1, FOXD3, and EN2, TRIM58, TWIST1, CLEC11A, HOXD10, and OLIG3, or a complementary region thereof, or a converted region thereof, or a fragment thereof.

50. A method for preparing a nucleic acid combination, comprising designing a nucleic acid combination capable of amplifying a DNA region with two genes selected from the group consisting of EBF2, and CCNA1, KCNA6, TLX2, and EMX1, TRIM58, TWIST1, FOXD3, and EN2, TRIM58, TWIST1, CLEC11A, HOXD10, and OLIG3, or a complementary region thereof, or a converted region thereof, or a fragment thereof, based on the modification status of the DNA region, or complementary region thereof, or converted region thereof, or fragment thereof.

51. A kit, comprising the nucleic acid of embodiment 47 and/or the nucleic acid combination of embodiment 49.

52. Use of the nucleic acid of embodiment 47, the nucleic acid combination of embodiment 49, and/or the kit of embodiment 51 in the preparation of a disease detection product.

53. Use of the nucleic acid of embodiment 47, the nucleic acid combination of embodiment 49 and/or the kit of embodiment 51 in the preparation of a substance for determining the presence of a disease, assessing the development or risk of development of a disease and/or assessing the progression of a disease.

54. Use of the nucleic acid of embodiment 47, the nucleic acid combination of embodiment 49 and/or the kit of embodiment 51 in the preparation of a substance for determining the modification status of the DNA region or fragment thereof.

55. Use of a nucleic acid, a nucleic acid combination and/or a kit for determining the modification status of a DNA region in the preparation of a substance for determining the presence of a pancreatic tumor, assessing the development or risk of development of a pancreatic tumor and/or assessing the progression of a pancreatic tumor, wherein the DNA region for determination includes DNA regions with two genes selected from the group consisting of EBF2, and CCNA1, KCNA6, TLX2, and EMX1, TRIM58, TWIST1, FOXD3, and EN2, TRIM58, TWIST1, CLEC11A, HOXD10, and OLIG3, or fragments thereof.

56. Use of a nucleic acid, a nucleic acid combination and/or a kit for determining the modification status of a DNA region in the preparation of a substance for determining the presence of a disease, assessing the development or risk of development of a disease, and/or assessing the progression of a disease, wherein the DNA region comprises two DNA regions selected from the group consisting of DNA regions derived from human chr8:25907849-25907950, and derived from human chr13:37005635-37005754, derived from human chr12:4919142-4919289, derived from human chr2:74743035-74743151, and derived from human chr2:73147525-73147644, derived from human chr1:248020592-248020779, derived from human chr7:19156739-19157277, derived from human chr1:63788812-63788952, and derived from human chr7:155167513-155167628, derived from human chr1:248020592-248020779, derived from human chr7:19156739-19157277, derived from human chr19:51228168-51228782, derived from human chr2:176945511-176945630, and derived from human chr6:137814700-137814853, or complementary regions thereof, or fragments thereof.

57. Use of nucleic acids of DNA regions with two genes selected from the group consisting of EBF2, and CCNA1, KCNA6, TLX2, and EMX1, TRIM58, TWIST1, FOXD3, and EN2, TRIM58, TWIST1, CLEC11A, HOXD10, and OLIG3, or converted regions thereof, or fragments thereof, and combinations of the above-mentioned nucleic acids, in the preparation of a substance for determining the presence of a pancreatic tumor, assessing the development or risk of development of a pancreatic tumor, and/or assessing the progression of a pancreatic tumor.

58. Use of nucleic acids of two DNA regions selected from the group consisting of DNA regions derived from human chr8:25907849-25907950, and derived from human chr13:37005635-37005754, derived from human chr12:4919142-4919289, derived from human chr2:74743035-74743151, and derived from human chr2:73147525-73147644, derived from human chr1:248020592-248020779, derived from human chr7:19156739-19157277, derived from human chr1:63788812-63788952, and derived from human chr7:155167513-155167628, derived from human chr1:248020592-248020779, derived from human chr7:19156739-19157277, derived from human chr19:51228168-51228782, derived from human chr2:176945511-176945630, and derived from human chr6:137814700-137814853, or complementary regions thereof, or converted regions thereof, or fragments thereof, and combinations of the above-mentioned nucleic acids, in the preparation of a substance for determining the presence of a disease, assessing the development or risk of development of a disease, and/or assessing the progression of a disease.

59. A storage medium recording a program capable of executing the method of any one of embodiments 1-46.

60. A device comprising the storage medium of embodiment 59.

61. The device of embodiment 60, further comprising a processor coupled to the storage medium, wherein the processor is configured to execute based on a program stored in the storage medium to implement the method as claimed in any one of embodiments 1-46.

Without intending to be limited by any theory, the following examples are only for illustrating the methods and uses of the present application, and are not intended to limit the scope of the invention of the present application.

**EXAMPLES**

**Example 1**

**1-1: Screening of differentially methylated sites for pancreatic cancer by targeted methylation sequencing**

The inventors collected a total of 94 pancreatic cancer blood samples and 80 pancreatic cancer-free blood samples, and all enrolled patients signed informed consent forms. See the table below for sample information.

|  | Training set | Test set |
|---|---|---|
| Sample type |  |  |
| Pancreatic cancer | 63 | 31 |
| Without pancreatic cancer | 54 | 26 |
| Age |  |  |
|  | 58(18-80) | 58(27-79) |
| Gender |  |  |
| Male | 62 | 29 |
| Female | 55 | 28 |
| Pathological stage |  |  |
| I | 18 | 7 |
| II | 30 | 14 |
| III or IV | 14 | 9 |
| Unknown | 1 | 1 |
| CA19-9 |  |  |
| Distribution (mean, maximum and minimum) | 324(1-1200) | 331(1-1200) |
| >37 | 52 | 24 |
| ≤37 | 33 | 21 |

The methylation sequencing data of plasma DNA were obtained by the MethylTitan assay to identify methylation classification markers therein. The process is as follows:

1. Extraction of plasma cfDNA samples

A 2 ml whole blood sample was collected from the patient using a Streck blood collection tube, the plasma was separated by centrifugation timely (within 3 days), transported to the laboratory, and then cfDNA was extracted using the QIAGEN QIAamp Circulating Nucleic Acid Kit according to the instructions.

2. Sequencing and data pre-processing

1) The library was paired-end sequenced using an Illumina Nextseq 500 sequencer.

2) Pear (v0.6.0) software combined the paired-end sequencing data of the same paired-end 150bp sequenced fragment from the Illumina Hiseq X10/ Nextseq 500/Nova seq sequener into one sequence, with the shortest overlapping length of 20 bp and the shortest length of 30bp after combination.

3) Trim_galore v 0.6.0 and cutadapt v1.8.1 software were used to perform adapter removal on the combined sequencing data. The adapter sequence "AGATCGGAAGAGCAC" was removed from the 5' end of the sequence, and bases with sequencing quality value lower than 20 at both ends were removed.

3. Sequencing data alignment

The reference genome data used herein were from the UCSC database (UCSC: HG19, hgdownload.soe.ucsc.edu/goldenPath/hg19/bigZips/hg19.fa.gz).

1) First, HG19 was subjected to conversion from cytosine to thymine (CT) and adenine to guanine (GA) using Bismark software, and an index for the converted genome was constructed using Bowtie2 software.
2) The pre-processed data were also subjected to conversions of CT and GA.
3) The converted sequences were aligned to the converted HG19 reference genome using Bowtie2 software. The minimum seed sequence length was 20, and no mismatching was allowed in the seed sequence.

4. Calculation of MHF

For the CpG sites in each target region HG19, the methylation level corresponding to each site was obtained based on the above alignment results. The nucleotide numbering of sites herein corresponds to the nucleotide position numbering of HG19. One target methylated region may have multiple methylated haplotypes. This value needs to be calculated for each methylated haplotype in the target region. An example of the MHF calculation formula is as follows:

$$MHFi,h = \frac{Ni,h}{Ni}$$

where i represents the target methylated region, h represents the target methylated haplotype, $N_i$ represents the number of reads located in the target methylated region, and $N_{i,h}$ represents the number of reads containing the target methylated haplotype.

5. Methylation data matrix

1) The methylation sequencing data of each sample in the training set and the test set were combined into a data matrix, and each site with a depth less than 200 was taken as a missing value.
2) Sites with a missing value proportion higher than 10% were removed.
3) For missing values in the data matrix, the KNN algorithm was used to interpolate the missing data.

6. Discovering feature methylated segments based on training set sample group

1) A logistic regression model was constructed for each methylated segment with regard to the phenotype, and the methylated segment with the most significant regression coefficient was screened out for each amplified target region to form candidate methylated segments.

2) The training set was randomly divided into ten parts for ten-fold cross-validation incremental feature selection.

3) The candidate methylated segments in each region were ranked in descending order according to the significance of the regression coefficient, and the data of one methylated segment was added each time to predict the test data.

4) In step 3), 10 copies of data generated in step 2) were used. For each copy of data, 10 times of calculation were conducted, and the final AUC was the average of 10 calculations. If the AUC of the training data increases, the candidate methylated segment is retained as the feature methylated segment, otherwise it is discarded.

5) The feature combination corresponding to the average AUC median under different number of features in the training set was taken as the final combination of feature methylated segments.

The distribution of the selected characteristic methylation nucleic acid sequences is as follows: SEQ ID NO:1 in the DMRTA2 gene region, SEQ ID NO:2 in the FOXD3 gene region, SEQ ID NO:3 in the TBX15 gene region, SEQ ID NO:4 in the BCAN gene region, SEQ ID NO:5 in the TRIM58 gene region, SEQ ID NO:6 in the SIX3 gene region, SEQ ID NO:7 in the VAX2 gene region, SEQ ID NO:8 in the EMX1 gene region, SEQ ID NO:9 in the LBX2 gene region, SEQ ID NO:10 in the TLX2 gene region, SEQ ID NO:11 and SEQ ID NO:12 in the POU3F3 gene region, SEQ ID N0:13 in the TBR1 gene region, SEQ ID NO:14 and SEQ ID NO:15 in the EVX2 gene region, SEQ ID NO:16 in the HOXD12 gene region, SEQ ID NO:17 in the HOXD8 gene region, SEQ ID NO:18 and SEQ ID NO:19 in the HOXD4 gene region, SEQ ID NO:20 in the TOPAZ 1 gene region, SEQ ID NO:21 in the SHOX2 gene region, SEQ ID NO:22 in the DRD5 gene region, SEQ ID NO:23 and SEQ ID NO:24 in the RPL9 gene region, SEQ ID NO:25 in the HOPX gene region, SEQ ID NO:26 in the SFRP2 gene region, SEQ ID NO:27 in the IRX4 gene region, SEQ ID NO:28 in the TBX18 gene region, SEQ ID NO:29 in the OLIG3 gene region, SEQ ID NO:30 in the ULBP1 gene region, SEQ ID NO:31 in the HOXA13 gene region, SEQ ID NO:32 in the TBX20 gene region, SEQ ID NO:33 in the IKZF1 gene region, SEQ ID NO:34 in the INSIG1 gene region, SEQ ID NO:35 in the SOX7 gene region, SEQ ID NO:36 in the EBF2 gene region, SEQ ID NO:37 in the MOS gene region, SEQ ID NO:38 in the MKX gene region, SEQ ID NO:39 in the KCNA6 gene region, SEQ ID NO:40 in the SYT10 gene region, SEQ ID NO:41 in the AGAP2 gene region, SEQ ID NO:42 in the TBX3 gene region, SEQ ID NO:43 in the CCNA1 gene region, SEQ ID NO:44 and SEQ ID NO:45 in the ZIC2 gene region, SEQ ID NO:46 and SEQ ID NO:47 in the CLEC14A gene region, SEQ ID NO:48 in the OTX2 gene region, SEQ ID NO:49 in the C14orf39 gene region, SEQ ID NO:50 in the BNC1 gene region, SEQ ID NO:51 in the AHSP gene region, SEQ ID NO:52 in the ZFHX3 gene region, SEQ ID NO:53 in the LHX1 gene region, SEQ ID NO:54 in the TIMP2 gene region, SEQ ID NO:55 in the ZNF750 gene region, and SEQ ID NO:56 in the SIM2 gene region. The levels of the above methylation markers increased or decreased in cfDNA of the patients with pancreatic cancer (Table 1-1). The sequences of the above 56 marker regions are set forth in SEQ ID NOs: 1-56. The methylation levels of all CpG sites in each marker region can be obtained by MethylTitan sequencing. The average methylation level of all CpG sites in each region, as well as the methylation level of a single CpG site, can both be used as a marker for the diagnosis of pancreatic cancer.

**Table 1-1: Average levels of methylation markers in the training set**

| Sequence | Gene region | Number of CGs | Pancreatic cancer | Without pancreatic cancer |
|---|---|---|---|---|
| SEQ ID NO:1 | DMRTA2 | 68 | 0.805118 | 0.846704212 |
| SEQ ID NO:2 | FOXD3 | 66 | 0.533626 | 0.631423118 |
| SEQ ID NO:3 | TBX15 | 49 | 0.46269 | 0.598647228 |
| SEQ ID NO:4 | BCAN | 51 | 0.895958 | 0.93205906 |
| SEQ ID NO:5 | TRIM58 | 75 | 0.781674 | 0.885116786 |
| SEQ ID NO:6 | SIX3 | 42 | 0.47867 | 0.530648758 |
| SEQ ID NO:7 | VAX2 | 49 | 0.754202 | 0.822800234 |
| SEQ ID NO:8 | EMX1 | 52 | 0.031272 | 0.015568518 |
| SEQ ID NO:9 | LBX2 | 50 | 0.804002 | 0.888596008 |
| SEQ ID NO:10 | TLX2 | 65 | 0.094431 | 0.046327063 |
| SEQ ID NO:11 | POU3F3 | 41 | 0.742934 | 0.79432709 |
| SEQ ID NO:12 | POU3F3 | 43 | 0.873117 | 0.907378674 |
| SEQ ID N0:13 | TBR1 | 66 | 0.83205 | 0.881520895 |
| SEQ ID NO:14 | EVX2 | 66 | 0.867162 | 0.914658287 |

(continued)

| Sequence | Gene region | Number of CGs | Pancreatic cancer | Without pancreatic cancer |
|----------|-------------|---------------|-------------------|---------------------------|
| SEQ ID NO:15 | EVX2 | 48 | 0.189907 | 0.134652946 |
| SEQ ID NO:16 | HOXD12 | 54 | 0.528523 | 0.59532531 |
| SEQ ID NO:17 | HOXD8 | 71 | 0.081469 | 0.04359926 |
| SEQ ID NO:18 | HOXD4 | 33 | 0.874582 | 0.916354164 |
| SEQ ID NO:19 | HOXD4 | 34 | 0.922386 | 0.947447638 |
| SEQ ID NO:20 | TOPAZ1 | 39 | 0.814131 | 0.887701025 |
| SEQ ID NO:21 | SHOX2 | 48 | 0.579209 | 0.670680638 |
| SEQ ID NO:22 | DRD5 | 53 | 0.896517 | 0.933959939 |
| SEQ ID NO:23 | RPL9 | 47 | 0.335709 | 0.189887387 |
| SEQ ID NO:24 | RPL9 | 53 | 0.255473 | 0.114913562 |
| SEQ ID NO:25 | HOPX | 33 | 0.867922 | 0.92600206 |
| SEQ ID NO:26 | SFRP2 | 31 | 0.874256 | 0.91995393 |
| SEQ ID NO:27 | IRX4 | 43 | 0.895035 | 0.936693651 |
| SEQ ID NO:28 | TBX18 | 25 | 0.842926 | 0.890887017 |
| SEQ ID NO:29 | OLIG3 | 54 | 0.505465 | 0.58611049 |
| SEQ ID NO:30 | ULBP1 | 62 | 0.96065 | 0.986061614 |
| SEQ ID NO:31 | HOXA13 | 48 | 0.849438 | 0.901184354 |
| SEQ ID NO:32 | TBX20 | 58 | 0.853916 | 0.919348754 |
| SEQ ID NO:33 | IKZF1 | 89 | 0.002234 | 7.42E-06 |
| SEQ ID NO:34 | INSIG1 | 58 | 0.778164 | 0.834092757 |
| SEQ ID NO:35 | SOX7 | 33 | 0.762759 | 0.833374722 |
| SEQ ID NO:36 | EBF2 | 35 | 0.006304 | 0.001619493 |
| SEQ ID NO:37 | MOS | 56 | 0.041915 | 0.028504837 |
| SEQ ID NO:38 | MKX | 59 | 0.945305 | 0.967669383 |
| SEQ ID NO:39 | KCNA6 | 54 | 0.91901 | 0.955657579 |
| SEQ ID NO:40 | SYT10 | 55 | 0.876289 | 0.911901265 |
| SEQ ID NO:41 | AGAP2 | 49 | 0.71894 | 0.789339811 |
| SEQ ID NO:42 | TBX3 | 35 | 0.591944 | 0.704717363 |
| SEQ ID NO:43 | CCNA1 | 51 | 0.051066 | 0.025112299 |
| SEQ ID NO:44 | ZIC2 | 48 | 0.371048 | 0.456316055 |
| SEQ ID NO:45 | ZIC2 | 47 | 0.74489 | 0.82642923 |
| SEQ ID NO:46 | CLEC14A | 48 | 0.79031 | 0.870664251 |
| SEQ ID NO:47 | CLEC14A | 51 | 0.903921 | 0.953341879 |
| SEQ ID NO:48 | OTX2 | 47 | 0.811418 | 0.861958339 |
| SEQ ID NO:49 | C14orf39 | 50 | 0.824815 | 0.919119502 |
| SEQ ID NO:50 | BNC1 | 64 | 0.939319 | 0.969846657 |
| SEQ ID NO:51 | AHSP | 28 | 0.669693 | 0.78221847 |
| SEQ ID NO:52 | ZFHX3 | 46 | 0.269205 | 0.155691343 |

(continued)

| Sequence | Gene region | Number of CGs | Pancreatic cancer | Without pancreatic cancer |
|---|---|---|---|---|
| SEQ ID NO:53 | LHX1 | 55 | 0.814173 | 0.894836486 |
| SEQ ID NO:54 | TIMP2 | 13 | 0.734619 | 0.782587252 |
| SEQ ID NO:55 | ZNF750 | 22 | 0.643534 | 0.809896825 |
| SEQ ID NO:56 | SIM2 | 47 | 0.861297 | 0.915016312 |

The methylation levels of methylation markers of people with pancreatic cancer and those without pancreatic cancer in the test set are shown in Table 1-2. As can be seen from the table, the distribution of the selected methylation markers was significantly different between people with pancreatic cancer and those without pancreatic cancer, achieving good differentiating effects.

**Table 1-2: Methylation levels of methylation markers in the test set**

| Sequence | Gene region | Number of CGs | Pancreatic cancer | Without pancreatic cancer |
|---|---|---|---|---|
| SEQ ID NO:1 | DMRTA2 | 68 | 0.80821 | 0.841562 |
| SEQ ID NO:2 | FOXD3 | 66 | 0.532689 | 0.608005 |
| SEQ ID NO:3 | TBX15 | 49 | 0.456977 | 0.583602 |
| SEQ ID NO:4 | BCAN | 51 | 0.886301 | 0.928237 |
| SEQ ID NO:5 | TRIM58 | 75 | 0.757257 | 0.865708 |
| SEQ ID NO:6 | SIX3 | 42 | 0.45768 | 0.507013 |
| SEQ ID NO:7 | VAX2 | 49 | 0.743388 | 0.823884 |
| SEQ ID NO:8 | EMX1 | 52 | 0.057218 | 0.018418 |
| SEQ ID NO:9 | LBX2 | 50 | 0.802808 | 0.886972 |
| SEQ ID NO:10 | TLX2 | 65 | 0.121389 | 0.052678 |
| SEQ ID NO:11 | POU3F3 | 41 | 0.729466 | 0.786569 |
| SEQ ID NO:12 | POU3F3 | 43 | 0.854963 | 0.902213 |
| SEQ ID NO:13 | TBR1 | 66 | 0.818731 | 0.883992 |
| SEQ ID NO:14 | EVX2 | 66 | 0.85586 | 0.911954 |
| SEQ ID NO:15 | EVX2 | 48 | 0.194409 | 0.145985 |
| SEQ ID NO:16 | HOXD12 | 54 | 0.464472 | 0.504838 |
| SEQ ID NO:17 | HOXD8 | 71 | 0.103311 | 0.053572 |
| SEQ ID NO:18 | HOXD4 | 33 | 0.856557 | 0.905414 |
| SEQ ID NO:19 | HOXD4 | 34 | 0.910568 | 0.940956 |
| SEQ ID NO:20 | TOPAZ1 | 39 | 0.789318 | 0.900009 |
| SEQ ID NO:21 | SHOX2 | 48 | 0.588091 | 0.644361 |
| SEQ ID NO:22 | DRD5 | 53 | 0.876745 | 0.929319 |
| SEQ ID NO:23 | RPL9 | 47 | 0.324825 | 0.185376 |
| SEQ ID NO:24 | RPL9 | 53 | 0.282492 | 0.11378 |
| SEQ ID NO:25 | HOPX | 33 | 0.866604 | 0.916437 |
| SEQ ID NO:26 | SFRP2 | 31 | 0.85147 | 0.911779 |
| SEQ ID NO:27 | IRX4 | 43 | 0.872813 | 0.924474 |
| SEQ ID NO:28 | TBX18 | 25 | 0.831686 | 0.891538 |

(continued)

| Sequence | Gene region | Number of CGs | Pancreatic cancer | Without pancreatic cancer |
|---|---|---|---|---|
| SEQ ID NO:29 | OLIG3 | 54 | 0.508308 | 0.582988 |
| SEQ ID NO:30 | ULBP1 | 62 | 0.94355 | 0.980948 |
| SEQ ID NO:31 | HOXA13 | 48 | 0.841288 | 0.893729 |
| SEQ ID NO:32 | TBX20 | 58 | 0.829121 | 0.914558 |
| SEQ ID NO:33 | IKZF1 | 89 | 0.017736 | 8.01E-06 |
| SEQ ID NO:34 | INSIG1 | 58 | 0.774911 | 0.832428 |
| SEQ ID NO:35 | SOX7 | 33 | 0.751425 | 0.808935 |
| SEQ ID NO:36 | EBF2 | 35 | 0.015764 | 0.004153 |
| SEQ ID NO:37 | MOS | 56 | 0.068217 | 0.028952 |
| SEQ ID NO:38 | MKX | 59 | 0.906794 | 0.960283 |
| SEQ ID NO:39 | KCNA6 | 54 | 0.897371 | 0.940083 |
| SEQ ID NO:40 | SYT10 | 55 | 0.862951 | 0.913739 |
| SEQ ID NO:41 | AGAP2 | 49 | 0.710999 | 0.776851 |
| SEQ ID NO:42 | TBX3 | 35 | 0.609331 | 0.704816 |
| SEQ ID NO:43 | CCNA1 | 51 | 0.065936 | 0.026731 |
| SEQ ID NO:44 | ZIC2 | 48 | 0.352573 | 0.434612 |
| SEQ ID NO:45 | ZIC2 | 47 | 0.736551 | 0.814384 |
| SEQ ID NO:46 | CLEC14A | 48 | 0.767731 | 0.874676 |
| SEQ ID NO:47 | CLEC14A | 51 | 0.869351 | 0.943006 |
| SEQ ID NO:48 | OTX2 | 47 | 0.784839 | 0.845296 |
| SEQ ID NO:49 | C14orf39 | 50 | 0.815521 | 0.908652 |
| SEQ ID NO:50 | BNC1 | 64 | 0.918581 | 0.965099 |
| SEQ ID NO:51 | AHSP | 28 | 0.647706 | 0.764136 |
| SEQ ID NO:52 | ZFHX3 | 46 | 0.298317 | 0.155255 |
| SEQ ID NO:53 | LHX1 | 55 | 0.791322 | 0.862229 |
| SEQ ID NO:54 | TIMP2 | 13 | 0.71954 | 0.77554 |
| SEQ ID NO:55 | ZNF750 | 22 | 0.650884 | 0.763429 |
| SEQ ID NO:56 | SIM2 | 47 | 0.876345 | 0.867791 |

Table 1-3 lists the correlation (Pearson correlation coefficient) between the methylation levels of 10 random CpG sites or combinations thereof and the methylation level of the entire marker in each selected marker, as well as the corresponding significance p value. It can be seen that the methylation level of a single CpG site or a combination of multiple CpG sites within the marker had a significant correlation with the methylation level of the entire region ($p < 0.05$), and the correlation coefficients were all above 0.8. This strong or extremely strong correlation indicates that a single CpG site or a combination of multiple CpG sites within the marker has the same good differentiating effect as the entire marker.

**Table 1-3: Correlation between the methylation level of random CpG sites or combinations of multiple sites and the methylation level of the entire marker in 56 markers**

| CpG sites and combinations | SEQ ID | Training set correlation | Training set p-value | Test set correlation | Test set p-value |
|---|---|---|---|---|---|
| chr1:50884902 | SEQ ID NO:1 | 0.8337 | 1.74E-16 | 0.8493 | 1.71E-14 |
| chr1:50884924 | SEQ ID NO:1 | 0.8111 | 8.72E-16 | 0.8316 | 1.16E-14 |
| chr1:50884889 | SEQ ID NO:1 | 0.8119 | 2.08E-15 | 0.8376 | 2.59E-13 |
| chr1:50884939 | SEQ ID NO:1 | 0.8042 | 2.59E-12 | 0.8433 | 4.14E-14 |
| chr1:50884942,50884945 | SEQ ID NO:1 | 0.8083 | 2.87E-12 | 0.8212 | 3.54E-13 |
| chr1:50884945 | SEQ ID NO:1 | 0.8172 | 5.01E-12 | 0.813 | 6.46E-14 |
| chr1:50884942 | SEQ ID NO:1 | 0.8232 | 4.55E-11 | 0.8085 | 5.16E-14 |
| chr1:50884948 | SEQ ID NO:1 | 0.8129 | 5.90E-11 | 0.8067 | 4.09E-14 |
| chr1:50884885 | SEQ ID NO:1 | 0.8221 | 2.96E-10 | 0.8447 | 4.30E-13 |
| chr1:50884942,50884945,50884 948 | SEQ ID NO:1 | 0.8262 | 3.18E-10 | 0.8241 | 8.06E-14 |
| chr1:63788861 | SEQ ID NO:2 | 0.837 | 2.27E-36 | 0.848 | 5.00E-19 |
| chr1:63788852 | SEQ ID NO:2 | 0.8116 | 4.06E-26 | 0.809 | 9.86E-14 |
| chr1:63788881 | SEQ ID NO:2 | 0.8103 | 1.19E-24 | 0.8357 | 1.74E-08 |
| chr1:63788902 | SEQ ID NO:2 | 0.8443 | 5.41E-24 | 0.8186 | 1.13E-06 |
| chr1:63788897 | SEQ ID NO:2 | 0.8345 | 1.55E-23 | 0.8283 | 1.03E-07 |

(continued)

| CpG sites and combinations | SEQ ID | Training set correlation | Training set p-value | Test set correlation | Test set p-value |
|---|---|---|---|---|---|
| chr1:63788852,63788861 | SEQ ID NO:2 | 0.8175 | 2.28E-23 | 0.8103 | 1.55E-09 |
| chr1:63788849 | SEQ ID NO:2 | 0.8365 | 3.39E-21 | 0.8341 | 4.06E-12 |
| chr1:63788849,63788852 | SEQ ID NO:2 | 0.8297 | 4.10E-20 | 0.8437 | 1.01E-07 |
| chr1:63788906 | SEQ ID NO:2 | 0.8486 | 5.08E-20 | 0.807 | 2.72E-08 |
| chr1:63788902,63788906 | SEQ ID NO:2 | 0.8018 | 1.80E-19 | 0.8349 | 3.71E-04 |
| chr1:119522449 | SEQ ID NON | 0.8397 | 2.04E-30 | 0.8345 | 1.45E-12 |
| chr1:119522456 | SEQ ID NON | 0.8267 | 6.67E-27 | 0.8392 | 1.15E-11 |
| chr1:119522446 | SEQ ID NON | 0.8279 | 2.56E-25 | 0.8072 | 8.45E-11 |
| chr1:119522451 | SEQ ID NON | 0.8342 | 3.68E-25 | 0.8403 | 3.93E-11 |
| chr1:119522469 | SEQ ID NON | 0.8197 | 9.72E-25 | 0.8162 | 7.31E-10 |
| chr1:119522459 | SEQ ID NON | 0.8103 | 1.80E-24 | 0.8081 | 1.14E-11 |
| chr1:119522474 | SEQ ID NON | 0.8103 | 1.82E-24 | 0.8218 | 8.44E-10 |
| chr1:119522464 | SEQ ID NON | 0.8116 | 1.35E-22 | 0.8239 | 2.62E-10 |
| chr1:119522440 | SEQ ID NON | 0.8233 | 1.45E-22 | 0.8269 | 5.94E-14 |
| chr1:119522449,119522451 | SEQ ID NON | 0.8062 | 5.93E-22 | 0.8129 | 2.49E-09 |

(continued)

| CpG sites and combinations | SEQ ID | Training set correlation | Training set p-value | Test set correlation | Test set p-value |
|---|---|---|---|---|---|
| chr1:156611960 | SEQ ID NO:4 | 0.8047 | 5.13E-35 | 0.811 | 0.00E+00 |
| chr1:156611963 | SEQ ID NO:4 | 0.9205 | 9.82E-56 | 0.9079 | 1.81E-25 |
| chr1:156611960,156611963 | SEQ ID NO:4 | 0.9146 | 9.68E-54 | 0.8855 | 1.21E-22 |
| chr1:156611951,156611960 | SEQ ID NO:4 | 0.8968 | 1.40E-48 | 0.8803 | 4.44E-22 |
| chr1:156611951 | SEQ ID NO:4 | 0.8947 | 4.96E-48 | 0.9058 | 3.54E-25 |
| chr1:156611951,156611960,156 611963 | SEQ ID NO:4 | 0.8504 | 1.27E-38 | 0.8339 | 6.55E-18 |
| chr1:156611949,156611951 | SEQ ID NO:4 | 0.8226 | 1.54E-28 | 0.8231 | 4.01E-17 |
| chr1:156611949 | SEQ ID NO:4 | 0.8381 | 3.01E-28 | 0.8553 | 1.19E-19 |
| chr1:156611949,156611951,156 611960 | SEQ ID NO:4 | 0.841 | 2.87E-23 | 0.805 | 6.41E-16 |
| chr1:156611949,156611951,156 611960,156611963 | SEQ ID NO:4 | 0.8126 | 1.38E-19 | 0.8231 | 2.37E-15 |
| chr1:248020641 | SEQ ID NO:5 | 0.8433 | 2.07E-37 | 0.8449 | 8.91E-19 |
| chr1:248020795 | SEQ ID NO:5 | 0.8163 | 2.89E-33 | 0.8342 | 2.27E-15 |
| chr1:248020798 | SEQ ID NO:5 | 0.8032 | 1.72E-31 | 0.802 | 9.91E-16 |
| chr1:248020812 | SEQ ID NO:5 | 0.8318 | 2.33E-23 | 0.8215 | 3.65E-11 |
| chr1:248020795,248020798 | SEQ ID NO:5 | 0.8238 | 1.20E-21 | 0.8329 | 2.63E-09 |

(continued)

| CpG sites and combinations | SEQ ID | Training set correlation | Training set p-value | Test set correlation | Test set p-value |
|---|---|---|---|---|---|
| chr1:248020713 | SEQ ID NO:5 | 0.8027 | 5.61E-19 | 0.8178 | 1.47E-11 |
| chr1:248020704 | SEQ ID NO:5 | 0.8356 | 4.74E-18 | 0.8199 | 2.26E-11 |
| chr1:248020791 | SEQ ID NO:5 | 0.8403 | 2.59E-17 | 0.8142 | 3.38E-10 |
| chr1:248020625 | SEQ ID NO:5 | 0.8015 | 2.24E-16 | 0.8414 | 1.38E-10 |
| chr1:248020680 | SEQ ID NO:5 | 0.8011 | 4.58E-15 | 0.8166 | 8.80E-10 |
| chr2:45029071 | SEQ ID NO:6 | 0.8419 | 1.55E-27 | 0.8046 | 4.38E-09 |
| chr2:45029060 | SEQ ID NO:6 | 0.819 | 6.20E-26 | 0.8111 | 1.23E-08 |
| chr2:45029046 | SEQ ID NO:6 | 0.8438 | 2.66E-25 | 0.8008 | 1.49E-08 |
| chr2:45029065 | SEQ ID NO:6 | 0.8173 | 8.08E-18 | 0.8319 | 2.69E-06 |
| chr2:45029117 | SEQ ID NO:6 | 0.8091 | 4.47E-17 | 0.8253 | 1.12E-06 |
| chr2:45029063 | SEQ ID NO:6 | 0.8465 | 9.60E-17 | 0.835 | 2.15E-06 |
| chr2:45029057,45029060 | SEQ ID NO:6 | 0.8186 | 4.38E-15 | 0.8065 | 0.00E+00 |
| chr2:45029057 | SEQ ID NO:6 | 0.833 | 9.57E-15 | 0.8167 | 1.05E-05 |
| chr2:45029128 | SEQ ID NO:6 | 0.8228 | 8.73E-13 | 0.8306 | 2.19E-05 |
| chr2:45029046,45029057 | SEQ ID NO:6 | 0.8335 | 5.11E-11 | 0.8165 | 0.00E+00 |

(continued)

| CpG sites and combinations | SEQ ID | Training set correlation | Training set p-value | Test set correlation | Test set p-value |
|---|---|---|---|---|---|
| chr2:71115978 | SEQ ID NO:7 | 0.8404 | 6.29E-37 | 0.8494 | 3.85E-19 |
| chr2:71115987 | SEQ ID NO:7 | 0.8316 | 1.60E-35 | 0.8498 | 3.56E-19 |
| chr2:71115981 | SEQ ID NO:7 | 0.8287 | 1.76E-27 | 0.8092 | 3.45E-16 |
| chr2:71116000 | SEQ ID NO:7 | 0.8342 | 1.99E-27 | 0.8302 | 2.02E-15 |
| chr2:71115968 | SEQ ID NO:7 | 0.8192 | 1.47E-26 | 0.8079 | 4.19E-16 |
| chr2:71115985 | SEQ ID NO:7 | 0.8387 | 1.21E-25 | 0.8282 | 3.39E-14 |
| chr2:71116022 | SEQ ID NO:7 | 0.8353 | 1.19E-22 | 0.8308 | 2.75E-11 |
| chr2:71115983 | SEQ ID NO:7 | 0.8264 | 1.19E-21 | 0.8056 | 5.85E-16 |
| chr2:71115968,71115978 | SEQ ID NO:7 | 0.8036 | 3.89E-21 | 0.8274 | 4.74E-12 |
| chr2:71115994 | SEQ ID NO:7 | 0.8139 | 5.07E-20 | 0.8238 | 3.45E-14 |
| chr2:73147584 | SEQ ID NO:8 | 0.835 | 2.51E-35 | 0.8334 | 0.00E+00 |
| chr2:73147582 | SEQ ID NO:8 | 0.8802 | 1.49E-44 | 0.9863 | 5.17E-51 |
| chr2:73147607 | SEQ ID NO:8 | 0.8538 | 3.08E-39 | 0.9223 | 1.07E-27 |
| chr2:73147607,73147613 | SEQ ID NO:8 | 0.8464 | 6.25E-38 | 0.9759 | 2.40E-43 |
| chr2:73147613 | SEQ ID NO:8 | 0.837 | 2.28E-36 | 0.925 | 3.61E-28 |

(continued)

| CpG sites and combinations | SEQ ID | Training set correlation | Training set p-value | Test set correlation | Test set p-value |
|---|---|---|---|---|---|
| chr2:73147620 | SEQ ID NO:8 | 0.8367 | 2.53E-36 | 0.905 | 4.60E-25 |
| chr2:73147595 | SEQ ID NO:8 | 0.8293 | 3.67E-35 | 0.9313 | 2.48E-29 |
| chr2:73147582,73147584 | SEQ ID NO:8 | 0.8279 | 5.81E-35 | 0.9879 | 1.04E-52 |
| chr2:73147598 | SEQ ID NO:8 | 0.8259 | 1.20E-34 | 0.9729 | 8.72E-42 |
| chr2:73147584,73147592 | SEQ ID NO:8 | 0.8138 | 6.48E-33 | 0.9861 | 8.76E-51 |
| chr2:74726651 | SEQ ID NO:9 | 0.9766 | 6.36E-90 | 0.9717 | 3.36E-41 |
| chr2:74726668 | SEQ ID NO:9 | 0.9534 | 1.56E-70 | 0.9149 | 1.67E-26 |
| chr2:74726672 | SEQ ID NO:9 | 0.9446 | 1.03E-65 | 0.954 | 1.12E-34 |
| chr2:74726649,74726651 | SEQ ID NO:9 | 0.9427 | 8.46E-65 | 0.9449 | 3.02E-32 |
| chr2:74726656 | SEQ ID NO:9 | 0.9413 | 3.94E-64 | 0.9444 | 3.98E-32 |
| chr2:74726651,74726656 | SEQ ID NO:9 | 0.9384 | 8.66E-63 | 0.9291 | 6.61E-29 |
| chr2:74726672,74726682 | SEQ ID NO:9 | 0.9377 | 1.90E-62 | 0.9338 | 8.09E-30 |
| chr2:74726649 | SEQ ID NO:9 | 0.9366 | 5.86E-62 | 0.954 | 1.13E-34 |
| chr2:74726642 | SEQ ID NO:9 | 0.9335 | 1.22E-60 | 0.9191 | 3.56E-27 |
| chr2:74726668,74726672 | SEQ ID NO:9 | 0.9314 | 8.48E-60 | 0.9108 | 6.77E-26 |

(continued)

| CpG sites and combinations | SEQ ID | Training set correlation | Training set p-value | Test set correlation | Test set p-value |
|---|---|---|---|---|---|
| chr2:74743 111 | SEQ ID NO: 10 | 0.8464 | 8.16E-3 5 | 0.8414 | 0.00E+0 0 |
| chr2:74743131 | SEQ ID NO: 10 | 0.8696 | 2.83E-42 | 0.9152 | 1.49E-26 |
| chr2:74743127,74743131 | SEQ ID NO: 10 | 0.8591 | 3.28E-40 | 0.9283 | 9.24E-29 |
| chr2:74743064 | SEQ ID NO: 10 | 0.8546 | 2.17E-39 | 0.9405 | 3.14E-31 |
| chr2:74743119 | SEQ ID NO: 10 | 0.8485 | 2.63E-38 | 0.9168 | 8.50E-27 |
| chr2:74743127 | SEQ ID NO: 10 | 0.8432 | 2.14E-37 | 0.9434 | 6.90E-32 |
| chr2:74743056 | SEQ ID NO: 10 | 0.8406 | 5.88E-37 | 0.947 | 8.94E-33 |
| chr2:74743061 | SEQ ID NO: 10 | 0.8371 | 2.19E-36 | 0.9509 | 8.50E-34 |
| chr2:74743059 | SEQ ID NO: 10 | 0.8276 | 6.58E-35 | 0.931 | 2.81E-29 |
| chr2:74743073 | SEQ ID NO: 10 | 0.8047 | 1.09E-31 | 0.9394 | 5.52E-31 |
| chr2:105480412 | SEQ ID NO: 11 | 0.8259 | 1.18E-34 | 0.8496 | 3.68E-19 |

(continued)

| CpG sites and combinations | SEQ ID | Training set correlation | Training set p-value | Test set correlation | Test set p-value |
|---|---|---|---|---|---|
| chr2:105480407 | SEQ ID NO: 11 | 0.8206 | 7.19E-34 | 0.8548 | 1.32E-19 |
| chr2:105480438 | SEQ ID NO: 11 | 0.8096 | 2.43E-32 | 0.854 | 1.56E-19 |
| chr2:105480429 | SEQ ID NO: 11 | 0.8089 | 3.02E-32 | 0.8686 | 6.99E-21 |
| chr2:105480426 | SEQ ID NO: 11 | 0.8068 | 5.75E-32 | 0.8546 | 1.38E-19 |
| chr2:105480424 | SEQ ID NO: 11 | 0.8033 | 1.38E-28 | 0.843 | 1.27E-18 |
| chr2:105480409 | SEQ ID NO: 11 | 0.8222 | 3.64E-27 | 0.8172 | 1.02E-16 |
| chr2:105480475 | SEQ ID NO: 11 | 0.8173 | 2.57E-25 | 0.8265 | 6.91E-15 |
| chr2:105480464 | SEQ ID NO: 11 | 0.8484 | 2.03E-23 | 0.829 | 1.50E-17 |
| chr2:105480433 | SEQ ID NO: 11 | 0.8371 | 9.95E-23 | 0.8155 | 1.32E-16 |
| chr2:105480407 | SEQ ID NO: 12 | 0.9695 | 1.64E-82 | 0.9917 | 6.89E-58 |
| chr2:105480409 | SEQ ID NO: 12 | 0.8362 | 3.06E-36 | 0.9529 | 2.31E-34 |

(continued)

| CpG sites and combinations | SEQ ID | Training set correlation | Training set p-value | Test set correlation | Test set p-value |
|---|---|---|---|---|---|
| chr2:105480407,105480409 | SEQ ID NO: 12 | 0.8451 | 5.10E-25 | 0.9287 | 7.84E-29 |
| chr2:105480412 | SEQ ID NO: 12 | 0.8338 | 6.49E-24 | 0.9375 | 1.39E-30 |
| chr2:105480438 | SEQ ID NO: 12 | 0.8264 | 4.70E-23 | 0.9062 | 3.13E-25 |
| chr2:105480429 | SEQ ID NO: 12 | 0.8311 | 2.11E-22 | 0.9062 | 3.14E-25 |
| chr2:105480426 | SEQ ID NO: 12 | 0.8272 | 1.48E-21 | 0.9188 | 3.94E-27 |
| chr2:105480424 | SEQ ID NO: 12 | 0.823 | 7.44E-20 | 0.9301 | 4.33E-29 |
| chr2:105480464 | SEQ ID NO: 12 | 0.8185 | 1.55E-17 | 0.8884 | 5.65E-23 |
| chr2:105480424,105480426 | SEQ ID NO: 12 | 0.8039 | 2.95E-17 | 0.8973 | 4.71E-24 |
| chr2:162280483 | SEQ ID N0: 13 | 0.8973 | 1.05E-48 | 0.9383 | 9.64E-31 |
| chr2:162280473,162280479 | SEQ ID N0: 13 | 0.8561 | 1.16E-39 | 0.8037 | 1.68E-15 |
| chr2:162280486 | SEQ ID N0: 13 | 0.8489 | 2.29E-3 8 | 0.9176 | 6.28E-27 |
| chr2:162280473 | SEQ ID N0: 13 | 0.835 | 4.74E-36 | 0.8071 | 4.72E-16 |
| chr2:162280489 | SEQ ID N0: 13 | 0.8065 | 6.42E-32 | 0.8075 | 1.28E-14 |

(continued)

| CpG sites and combinations | SEQ ID | Training set correlation | Training set p-value | Test set correlation | Test set p-value |
|---|---|---|---|---|---|
| chr2:162280470,162280473 | SEQ ID N0: 13 | 0.8033 | 1.68E-31 | 0.8084 | 3.88E-16 |
| chr2:162280466 | SEQ ID NO: 13 | 0.8026 | 2.07E-31 | 0.8181 | 2.21E-11 |
| chr2:162280479,162280483 | SEQ ID NO: 13 | 0.8018 | 1.07E-28 | 0.8532 | 1.83E-19 |
| chr2:162280466,162280470,162 280473 | SEQ ID NO: 13 | 0.8173 | 3.49E-28 | 0.8389 | 2.89E-13 |
| chr2:162280470,162280473,162 280479 | SEQ ID NO: 13 | 0.8496 | 1.50E-25 | 0.8185 | 2.60E-11 |
| chr2:176945351 | SEQ ID NO: 14 | 0.9438 | 2.53E-65 | 0.9569 | 1.54E-35 |
| chr2:176945378 | SEQ ID NO: 14 | 0.8655 | 1.83E-41 | 0.8682 | 7.63E-21 |
| chr2:176945345 | SEQ ID NO: 14 | 0.8107 | 1.74E-32 | 0.9234 | 6.82E-28 |
| chr2:176945417 | SEQ ID NO: 14 | 0.8075 | 4.68E-32 | 0.8774 | 9.21E-22 |
| chr2:176945384 | SEQ ID NO: 14 | 0.834 | 1.19E-29 | 0.8904 | 3.29E-23 |
| chr2:176945339 | SEQ ID NO: 14 | 0.8009 | 1.92E-27 | 0.926 | 2.36E-28 |
| chr2:176945387 | SEQ ID NO: 14 | 0.8458 | 1.67E-26 | 0.8907 | 2.99E-23 |

(continued)

| CpG sites and combinations | SEQ ID | Training set correlation | Training set p-value | Test set correlation | Test set p-value |
|---|---|---|---|---|---|
| chr2:176945347 | SEQ ID NO: 14 | 0.842 | 4.59E-23 | 0.8426 | 1.37E-18 |
| chr2:176945381 | SEQ ID NO: 14 | 0.8404 | 3.79E-21 | 0.8908 | 2.90E-23 |
| chr2:176945402 | SEQ ID NO: 14 | 0.8048 | 5.19E-21 | 0.81 | 3.05E-16 |
| chr2:176945570 | SEQ ID NO: 15 | 0.8219 | 4.70E-3 5 | 0.8147 | 0.00E+0 0 |
| chr2:176945570,176945580 | SEQ ID NO: 15 | 0.8746 | 2.54E-43 | 0.9319 | 1.93E-29 |
| chr2:176945580,176945582,176 945585 | SEQ ID NO: 15 | 0.8343 | 6.03E-36 | 0.8858 | 1.11E-22 |
| chr2:176945580,176945582 | SEQ ID NO: 15 | 0.828 | 5.62E-3 5 | 0.8715 | 3.61E-21 |
| chr2:176945570,176945580,176 945582 | SEQ ID NO: 15 | 0.827 | 8.07E-3 5 | 0.8764 | 1.15E-21 |
| chr2:176945580 | SEQ ID NO: 15 | 0.8167 | 2.52E-33 | 0.841 | 1.84E-18 |
| chr2:176945570,176945580,176 945582,176945585 | SEQ ID NO: 15 | 0.8466 | 7.91E-31 | 0.8447 | 9.25E-19 |
| chr2:176945582,176945585 | SEQ ID NO: 15 | 0.8346 | 1.98E-30 | 0.857 | 8.48E-20 |

(continued)

| CpG sites and combinations | SEQ ID | Training set correlation | Training set p-value | Test set correlation | Test set p-value |
|---|---|---|---|---|---|
| chr2:176945582 | SEQ ID NO: 15 | 0.8438 | 1.50E-23 | 0.8105 | 2.16E-14 |
| chr2:176945580,176945582,176945585,176945604 | SEQ ID NO: 15 | 0.8106 | 1.82E-18 | 0.8275 | 8.74E-14 |
| chr2:176964886 | SEQ ID NO: 16 | 0.8473 | 7.99E-30 | 0.8212 | 9.81E-05 |
| chr2:176964879 | SEQ ID NO: 16 | 0.8468 | 1.31E-21 | 0.8092 | 7.05E-04 |
| chr2:176964869 | SEQ ID NO: 16 | 0.8319 | 8.28E-17 | 0.8273 | 4.94E-05 |
| chr2:176964930 | SEQ ID NO: 16 | 0.8487 | 2.16E-15 | 0.8066 | 4.56E-04 |
| chr2:176964879,176964886 | SEQ ID NO: 16 | 0.8046 | 1.48E-14 | 0.8108 | 5.60E-04 |
| chr2:176964946 | SEQ ID NO: 16 | 0.8426 | 4.86E-13 | 0.8418 | 2.03E-07 |
| chr2:176964865,176964869 | SEQ ID NO: 16 | 0.844 | 1.32E-09 | 0.816 | 3.92E-05 |
| chr2:176964892 | SEQ ID NO: 16 | 0.8474 | 7.17E-09 | 0.8438 | 1.15E-04 |
| chr2:176964865 | SEQ ID NO: 16 | 0.8064 | 7.19E-09 | 0.8325 | 2.40E-04 |

(continued)

| CpG sites and combinations | SEQ ID | Training set correlation | Training set p-value | Test set correlation | Test set p-value |
|---|---|---|---|---|---|
| chr2:176964875 | SEQ ID NO: 16 | 0.8031 | 1.09E-08 | 0.8161 | 1.03E-04 |
| chr2:176994764 | SEQ ID NO: 17 | 0.8461 | 4.24E-3 5 | 0.8481 | 0.00E+0 0 |
| chr2:176994778 | SEQ ID NO: 17 | 0.9055 | 5.61E-51 | 0.9532 | 1.95E-34 |
| chr2:176994768 | SEQ ID NO: 17 | 0.885 | 1.17E-45 | 0.9502 | 1.34E-33 |
| chr2:176994773 | SEQ ID NO: 17 | 0.8747 | 2.36E-43 | 0.9378 | 1.20E-30 |
| chr2:176994764,176994768 | SEQ ID NO: 17 | 0.8639 | 3.94E-41 | 0.9608 | 8.57E-37 |
| chr2:176994783 | SEQ ID NO: 17 | 0.8617 | 1.01E-40 | 0.9402 | 3.57E-31 |
| chr2:176994773,176994778 | SEQ ID NO: 17 | 0.8396 | 8.64E-37 | 0.9483 | 4.10E-33 |
| chr2:176994801 | SEQ ID NO: 17 | 0.8386 | 1.26E-36 | 0.9378 | 1.21E-30 |
| chr2:176994753 | SEQ ID NO: 17 | 0.833 | 9.68E-36 | 0.9413 | 2.07E-31 |
| chr2:176994780 | SEQ ID NO: 17 | 0.8328 | 1.03E-35 | 0.9326 | 1.42E-29 |

(continued)

| CpG sites and combinations | SEQ ID | Training set correlation | Training set p-value | Test set correlation | Test set p-value |
|---|---|---|---|---|---|
| chr2:177017270 | SEQ ID NO: 18 | 0.8589 | 3.54E-40 | 0.8044 | 1.84E-15 |
| chr2:177017251 | SEQ ID NO: 18 | 0.8533 | 3.74E-39 | 0.8822 | 2.77E-22 |
| chr2:177017227 | SEQ ID NO: 18 | 0.8349 | 4.93E-36 | 0.8232 | 3.94E-17 |
| chr2:177017211 | SEQ ID NO: 18 | 0.8091 | 5.45E-30 | 0.8285 | 1.63E-17 |
| chr2:177017223 | SEQ ID NO: 18 | 0.8479 | 3.46E-28 | 0.8066 | 4.05E-15 |
| chr2:177017237 | SEQ ID NO: 18 | 0.8174 | 1.08E-23 | 0.825 | 6.17E-14 |
| chr2:177017182 | SEQ ID NO: 18 | 0.8304 | 1.85E-23 | 0.8294 | 1.41E-17 |
| chr2:177017267 | SEQ ID NO: 18 | 0.8091 | 2.43E-23 | 0.8159 | 1.24E-16 |
| chr2:177017225 | SEQ ID NO: 18 | 0.8122 | 3.51E-23 | 0.8229 | 1.82E-14 |
| chr2:177017193 | SEQ ID NO: 18 | 0.8108 | 3.95E-23 | 0.85 | 3.38E-19 |
| chr2:177024605 | SEQ ID NO: 19 | 0.9473 | 4.09E-67 | 0.977 | 5.05E-44 |

(continued)

| CpG sites and combinations | SEQ ID | Training set correlation | Training set p-value | Test set correlation | Test set p-value |
|---|---|---|---|---|---|
| chr2:177024616 | SEQ ID NO: 19 | 0.9265 | 7.10E-58 | 0.9782 | 1.07E-44 |
| chr2:177024616,177024619 | SEQ ID NO: 19 | 0.8312 | 1.85E-35 | 0.9392 | 5.92E-31 |
| chr2:177024619 | SEQ ID NO: 19 | 0.828 | 5.64E-3 5 | 0.9007 | 1.71E-24 |
| chr2:177024605,177024616 | SEQ ID NO: 19 | 0.8132 | 8.01E-33 | 0.9286 | 8.23E-29 |
| chr2:177024582 | SEQ ID NO: 19 | 0.8341 | 8.23E-27 | 0.8987 | 3.09E-24 |
| chr2:177024619,177024634 | SEQ ID NO: 19 | 0.8268 | 1.03E-26 | 0.8698 | 5.41E-21 |
| chr2:177024634 | SEQ ID NO: 19 | 0.8253 | 1.08E-26 | 0.8971 | 5.04E-24 |
| chr2:177024605,177024616,177 024619 | SEQ ID NO: 19 | 0.8129 | 1.47E-26 | 0.9082 | 1.64E-25 |
| chr2:177024616,177024619,177 024634 | SEQ ID NO: 19 | 0.8445 | 1.56E-24 | 0.8694 | 5.87E-21 |
| chr3:44063649 | SEQ ID NO: 20 | 0.8406 | 5.75E-37 | 0.9235 | 6.57E-28 |
| chr3:44063643 | SEQ ID NO: 20 | 0.8251 | 1.57E-34 | 0.915 | 1.61E-26 |

(continued)

| CpG sites and combinations | SEQ ID | Training set correlation | Training set p-value | Test set correlation | Test set p-value |
|---|---|---|---|---|---|
| chr3 :44063657 | SEQ ID NO: 20 | 0.8021 | 2.41E-31 | 0.9362 | 2.66E-30 |
| chr3 :44063649,44063657 | SEQ ID NO: 20 | 0.8289 | 4.32E-24 | 0.8761 | 1.25E-21 |
| chr3:44063620 | SEQ ID NO: 20 | 0.8081 | 6.73E-24 | 0.9039 | 6.44E-25 |
| chr3 :44063638 | SEQ ID NO: 20 | 0.8175 | 3.91E-23 | 0.8853 | 1.26E-22 |
| chr3:44063662 | SEQ ID NO: 20 | 0.8251 | 1.45E-21 | 0.8944 | 1.08E-23 |
| chr3:44063660 | SEQ ID NO: 20 | 0.819 | 4.27E-21 | 0.8988 | 3.02E-24 |
| chr3:44063633 | SEQ ID NO: 20 | 0.8085 | 4.95E-21 | 0.8829 | 2.33E-22 |
| chr3 :44063643,44063649 | SEQ ID NO: 20 | 0.8367 | 2.45E-17 | 0.8645 | 1.73E-20 |
| chr3:157812329 | SEQ ID NO: 21 | 0.8386 | 2.52E-18 | 0.8051 | 1.33E-10 |
| chr3:157812312 | SEQ ID NO: 21 | 0.8224 | 2.37E-15 | 0.8208 | 7.45E-10 |
| chr3:157812420 | SEQ ID NO: 21 | 0.839 | 8.24E-15 | 0.8032 | 1.63E-06 |

(continued)

| CpG sites and combinations | SEQ ID | Training set correlation | Training set p-value | Test set correlation | Test set p-value |
|---|---|---|---|---|---|
| chr3:157812302 | SEQ ID NO: 21 | 0.8398 | 4.06E-14 | 0.835 | 3.10E-10 |
| chr3:157812287 | SEQ ID NO: 21 | 0.8387 | 8.08E-14 | 0.8265 | 4.17E-07 |
| chr3:157812287,157812294 | SEQ ID NO: 21 | 0.8149 | 5.54E-13 | 0.8323 | 3.54E-07 |
| chr3:157812294 | SEQ ID NO: 21 | 0.8004 | 7.72E-13 | 0.8411 | 4.38E-08 |
| chr3:157812331 | SEQ ID NO: 21 | 0.8129 | 8.96E-13 | 0.8411 | 7.32E-05 |
| chr3:157812321 | SEQ ID NO: 21 | 0.8473 | 2.53E-12 | 0.8445 | 6.68E-07 |
| chr3:157812354 | SEQ ID NO: 21 | 0.813 | 1.71E-11 | 0.8432 | 1.49E-07 |
| chr4:9783277 | SEQ ID NO: 22 | 0.918 | 7.14E-55 | 0.9515 | 6.06E-34 |
| chr4:9783275 | SEQ ID NO: 22 | 0.8167 | 2.58E-33 | 0.8782 | 7.43E-22 |
| chr4:9783275,9783277 | SEQ ID NO: 22 | 0.8452 | 2.47E-22 | 0.8113 | 2.53E-16 |
| chr4:9783271 | SEQ ID NO: 22 | 0.805 | 1.04E-20 | 0.8335 | 3.92E-12 |

(continued)

| CpG sites and combinations | SEQ ID | Training set correlation | Training set p-value | Test set correlation | Test set p-value |
|---|---|---|---|---|---|
| chr4:9783196 | SEQ ID NO: 22 | 0.8424 | 2.49E-19 | 0.8129 | 3.06E-11 |
| chr4:9783198 | SEQ ID NO: 22 | 0.8422 | 1.49E-18 | 0.8218 | 5.58E-12 |
| chr4:9783196,9783198 | SEQ ID NO: 22 | 0.8345 | 2.59E-16 | 0.8348 | 5.24E-10 |
| chr4:9783192,9783196 | SEQ ID NO: 22 | 0.8171 | 4.38E-15 | 0.8197 | 2.27E-08 |
| chr4:9783192 | SEQ ID NO: 22 | 0.8408 | 5.23E-15 | 0.8473 | 2.81E-14 |
| chr4:9783271,9783275 | SEQ ID NO: 22 | 0.8386 | 1.59E-13 | 0.8269 | 2.31E-11 |
| chr4:39448528 | SEQ ID NO: 23 | 0.819 | 4.60E-3 5 | 0.8194 | 0.00E+0 0 |
| chr4:39448524,39448528 | SEQ ID NO: 23 | 0.9942 | 7.77E-130 | 0.9953 | 1.37E-65 |
| chr4:39448516,39448524,39448 528 | SEQ ID NO: 23 | 0.9929 | 7.90E-124 | 0.9936 | 2.40E-61 |
| chr4:39448503,39448516,39448 524,39448528 | SEQ ID NO: 23 | 0.9904 | 2.13E-115 | 0.991 | 8.31E-57 |
| chr4:39448528,39448549 | SEQ ID NO: 23 | 0.9881 | 4.27E-109 | 0.9889 | 7.25E-54 |

(continued)

| CpG sites and combinations | SEQ ID | Training set correlation | Training set p-value | Test set correlation | Test set p-value |
|---|---|---|---|---|---|
| chr4:39448524,39448528,39448 549 | SEQ ID NO: 23 | 0.9809 | 9.85E-96 | 0.9837 | 1.19E-48 |
| chr4:39448516,39448524,39448 528,39448549 | SEQ ID NO: 23 | 0.9795 | 1.07E-93 | 0.9825 | 1.10E-47 |
| chr4:39448503,39448516,39448 524,39448528,39448549 | SEQ ID NO: 23 | 0.9777 | 2.63E-91 | 0.9802 | 4.64E-46 |
| chr4:39448528,39448549,39448 551 | SEQ ID NO: 23 | 0.9759 | 3.87E-89 | 0.978 | 1.35E-44 |
| chr4:39448524,39448528,39448 549,39448551 | SEQ ID NO: 23 | 0.9705 | 1.95E-83 | 0.9736 | 3.87E-42 |
| chr4: 3 9448577,39448586,39448 593,39448613,39448625,394486 29 | SEQ ID NO: 24 | 0.8091 | 5.75E-35 | 0.8303 | 0.00E+0 0 |
| chr4:39448586,39448593,39448 613,39448625,39448629 | SEQ ID NO: 24 | 0.9808 | 1.40E-95 | 0.9986 | 4.17E-82 |
| chr4:39448577,39448586,39448 593,39448613,39448625,394486 29,39448633 | SEQ ID NO: 24 | 0.9747 | 9.17E-88 | 0.9863 | 5.57E-51 |
| chr4:39448593,39448613,39448 625,39448629 | SEQ ID NO: 24 | 0.9671 | 2.30E-80 | 0.9888 | 9.14E-54 |
| chr4:39448575,39448577,39448 586,39448593,39448613,394486 25,39448629 | SEQ ID NO: 24 | 0.962 | 2.83E-76 | 0.985 | 8.75E-50 |
| chr4:39448613,39448625,39448 629 | SEQ ID NO: 24 | 0.9589 | 4.52E-74 | 0.9857 | 2.12E-50 |

(continued)

| CpG sites and combinations | SEQ ID | Training set correlation | Training set p-value | Test set correlation | Test set p-value |
|---|---|---|---|---|---|
| chr4:39448586,39448593,39448 613,39448625,39448629,394486 33 | SEQ ID NO: 24 | 0.9542 | 5.15E-71 | 0.9864 | 4.30E-51 |
| chr4:39448577,39448586,39448 593,39448613,39448625 | SEQ ID NO: 24 | 0.9529 | 2.88E-70 | 0.9562 | 2.57E-35 |
| chr4:39448568,39448575,39448 577,39448586,39448593,394486 13,39448625,39448629 | SEQ ID NO: 24 | 0.9488 | 5.95E-68 | 0.9639 | 6.25E-38 |
| chr4:39448562,39448568,39448 575,39448577,39448586,394485 93,39448613,39448625,3944862 9 | SEQ ID NO: 24 | 0.948 | 1.71E-67 | 0.9605 | 1.03E-36 |
| chr4:57521377 | SEQ ID NO: 25 | 0.8304 | 1.06E-21 | 0.8178 | 5.25E-15 |
| chr4:57521426 | SEQ ID NO: 25 | 0.8238 | 2.07E-11 | 0.8105 | 1.27E-10 |
| chr4:57521397 | SEQ ID NO: 25 | 0.821 | 3.03E-08 | 0.8414 | 4.31E-10 |
| chr4:57521449 | SEQ ID NO: 25 | 0.8209 | 4.85E-08 | 0.8339 | 2.85E-07 |
| chr4:57521419 | SEQ ID NO: 25 | 0.8053 | 1.71E-06 | 0.8014 | 3.95E-06 |
| chr4:57521442 | SEQ ID NO: 25 | 0.8163 | 6.04E-06 | 0.8445 | 1.62E-06 |
| chr4:57521486 | SEQ ID NO: 25 | 0.8352 | 1.27E-05 | 0.8277 | 4.69E-10 |

(continued)

| CpG sites and combinations | SEQ ID | Training set correlation | Training set p-value | Test set correlation | Test set p-value |
|---|---|---|---|---|---|
| chr4:57521377,57521397 | SEQ ID NO: 25 | 0.8296 | 9.12E-04 | 0.8116 | 1.85E-05 |
| chr4:57521419,57521426 | SEQ ID NO: 25 | 0.8029 | 4.37E-03 | 0.8369 | 6.96E-05 |
| chr4:57521411 | SEQ ID NO: 25 | 0.8256 | 6.65E-03 | 0.8387 | 3.68E-07 |
| chr4:154709612 | SEQ ID NO: 26 | 0.9702 | 4.26E-83 | 0.9669 | 4.49E-39 |
| chr4:154709617 | SEQ ID NO: 26 | 0.8684 | 4.94E-42 | 0.9316 | 2.21E-29 |
| chr4:154709597 | SEQ ID NO: 26 | 0.8389 | 4.47E-26 | 0.8837 | 1.92E-22 |
| chr4:154709640 | SEQ ID NO: 26 | 0.8377 | 1.27E-22 | 0.9118 | 4.91E-26 |
| chr4:154709607,154709612 | SEQ ID NO: 26 | 0.8271 | 2.45E-19 | 0.8481 | 4.88E-19 |
| chr4:154709612,154709617 | SEQ ID NO: 26 | 0.8264 | 1.55E-18 | 0.8642 | 1.86E-20 |
| chr4:154709607 | SEQ ID NO: 26 | 0.8336 | 2.90E-18 | 0.8988 | 3.01E-24 |
| chr4:154709633 | SEQ ID NO: 26 | 0.8079 | 2.05E-17 | 0.9103 | 8.10E-26 |

(continued)

| CpG sites and combinations | SEQ ID | Training set correlation | Training set p-value | Test set correlation | Test set p-value |
|---|---|---|---|---|---|
| chr4:154709633,154709640 | SEQ ID NO: 26 | 0.8235 | 5.60E-14 | 0.8883 | 5.70E-23 |
| chr4:154709591,154709597 | SEQ ID NO: 26 | 0.801 | 2.27E-10 | 0.8369 | 3.84E-18 |
| chr5:1876386 | SEQ ID NO: 27 | 0.9552 | 1.11E-71 | 0.9455 | 2.17E-32 |
| chr5:1876395 | SEQ ID NO: 27 | 0.8444 | 1.33E-37 | 0.9291 | 6.54E-29 |
| chr5:1876403 | SEQ ID NO: 27 | 0.8408 | 5.41E-37 | 0.8748 | 1.70E-21 |
| chr5:1876386,1876395 | SEQ ID NO: 27 | 0.8019 | 2.56E-31 | 0.8487 | 4.38E-19 |
| chr5:1876374 | SEQ ID NO: 27 | 0.8469 | 3.85E-25 | 0.8666 | 1.10E-20 |
| chr5:1876399 | SEQ ID NO: 27 | 0.8148 | 9.64E-25 | 0.8672 | 9.67E-21 |
| chr5:1876399,1876403 | SEQ ID NO: 27 | 0.8277 | 1.74E-24 | 0.8288 | 1.55E-17 |
| chr5:1876395,1876397 | SEQ ID NO: 27 | 0.8413 | 1.84E-21 | 0.8434 | 1.19E-18 |
| chr5:1876374,1876386 | SEQ ID NO: 27 | 0.8343 | 3.60E-21 | 0.8243 | 3.27E-17 |

(continued)

| CpG sites and combinations | SEQ ID | Training set correlation | Training set p-value | Test set correlation | Test set p-value |
|---|---|---|---|---|---|
| chr5:1876397 | SEQ ID NO: 27 | 0.8216 | 1.15E-19 | 0.8662 | 1.19E-20 |
| chr6:85477166 | SEQ ID NO: 28 | 0.818 | 9.55E-35 | 0.801 | 0.00E+00 |
| chr6:85477153,85477166 | SEQ ID NO: 28 | 0.8241 | 3.01E-26 | 0.8431 | 1.25E-18 |
| chr6:85477166,85477175 | SEQ ID NO: 28 | 0.8143 | 1.54E-24 | 0.8607 | 3.91E-20 |
| chr6:85477175 | SEQ ID NO: 28 | 0.8053 | 2.32E-19 | 0.8404 | 3.85E-11 |
| chr6:85477151,85477153 | SEQ ID NO: 28 | 0.8257 | 1.25E-17 | 0.8003 | 1.77E-11 |
| chr6:85477151 | SEQ ID NO: 28 | 0.8356 | 7.34E-17 | 0.8122 | 5.81E-12 |
| chr6:85477153 | SEQ ID NO: 28 | 0.8421 | 1.05E-16 | 0.8234 | 3.78E-17 |
| chr6:85477166,85477175,85477 186 | SEQ ID NO: 28 | 0.8355 | 1.84E-13 | 0.8289 | 3.86E-11 |
| chr6:85477153,85477166,85477 175 | SEQ ID NO: 28 | 0.8479 | 4.38E-13 | 0.819 | 4.82E-14 |
| chr6:85477151,85477153,85477 166 | SEQ ID NO: 28 | 0.8462 | 5.49E-13 | 0.8205 | 5.98E-11 |

(continued)

| CpG sites and combinations | SEQ ID | Training set correlation | Training set p-value | Test set correlation | Test set p-value |
|---|---|---|---|---|---|
| chr6:137814749 | SEQ ID NO: 29 | 0.8498 | 1.02E-20 | 0.8182 | 1.26E-07 |
| chr6:137814707 | SEQ ID NO: 29 | 0.8464 | 5.21E-16 | 0.8261 | 4.89E-08 |
| chr6:137814723 | SEQ ID NO: 29 | 0.8293 | 2.38E-13 | 0.8341 | 1.21E-05 |
| chr6:137814695 | SEQ ID NO: 29 | 0.8242 | 3.32E-13 | 0.8046 | 1.70E-05 |
| chr6:137814710 | SEQ ID NO: 29 | 0.8243 | 1.42E-12 | 0.8299 | 2.58E-08 |
| chr6:137814744 | SEQ ID NO: 29 | 0.8373 | 2.38E-12 | 0.8052 | 6.23E-06 |
| chr6:137814695,137814707 | SEQ ID NO: 29 | 0.8218 | 5.53E-12 | 0.8083 | 1.35E-03 |
| chr6:137814728 | SEQ ID NO: 29 | 0.8448 | 3.24E-11 | 0.8007 | 1.11E-06 |
| chr6:137814746 | SEQ ID NO: 29 | 0.8054 | 3.79E-11 | 0.8071 | 8.99E-06 |
| chr6:137814768 | SEQ ID NO: 29 | 0.8003 | 1.62E-10 | 0.826 | 6.88E-07 |
| chr6:150285844 | SEQ ID NO: 30 | 0.8418 | 9.43E-35 | 0.8008 | 0.00E+00 |

(continued)

| CpG sites and combinations | SEQ ID | Training set correlation | Training set p-value | Test set correlation | Test set p-value |
|---|---|---|---|---|---|
| chr6:150285844,150285860 | SEQ ID NO: 30 | 0.8541 | 2.67E-39 | 0.9523 | 3.59E-34 |
| chr6:150285860 | SEQ ID NO: 30 | 0.8046 | 1.29E-30 | 0.9326 | 1.42E-29 |
| chr6:150285892,150285901 | SEQ ID NO: 30 | 0.8351 | 3.76E-24 | 0.9591 | 3.01E-36 |
| chr6:150285892 | SEQ ID NO: 30 | 0.8468 | 6.17E-24 | 0.8748 | 1.68E-21 |
| chr6:150285910 | SEQ ID NO: 30 | 0.8072 | 6.77E-22 | 0.843 | 1.29E-18 |
| chr6:150285901 | SEQ ID NO: 30 | 0.8314 | 3.71E-21 | 0.9015 | 1.33E-24 |
| chr6:150285890 | SEQ ID NO: 30 | 0.8153 | 5.49E-20 | 0.9506 | 1.06E-33 |
| chr6:150285901,150285908,150 285910 | SEQ ID NO: 30 | 0.8131 | 1.51E-19 | 0.9066 | 2.70E-25 |
| chr6:150285826 | SEQ ID NO: 30 | 0.8449 | 1.80E-18 | 0.8821 | 2.84E-22 |
| chr7:27244787 | SEQ ID NO: 31 | 0.9224 | 2.11E-56 | 0.8562 | 9.82E-20 |
| chr7:27244780 | SEQ ID NO: 31 | 0.8637 | 4.27E-41 | 0.8759 | 1.29E-21 |

(continued)

| CpG sites and combinations | SEQ ID | Training set correlation | Training set p-value | Test set correlation | Test set p-value |
|---|---|---|---|---|---|
| chr7:27244772 | SEQ ID NO: 31 | 0.8397 | 8.09E-37 | 0.8375 | 3.46E-18 |
| chr7:27244780,27244787 | SEQ ID NO: 31 | 0.8254 | 2.82E-26 | 0.8451 | 3.17E-12 |
| chr7:27244787,27244789 | SEQ ID NO: 31 | 0.8103 | 1.34E-20 | 0.8346 | 1.34E-07 |
| chr7:27244789 | SEQ ID NO: 31 | 0.8343 | 2.54E-20 | 0.8263 | 1.00E-08 |
| chr7:27244755 | SEQ ID NO: 31 | 0.8131 | 3.59E-18 | 0.8459 | 5.05E-10 |
| chr7:27244772,27244780 | SEQ ID NO: 31 | 0.8319 | 6.91E-18 | 0.8154 | 8.11E-10 |
| chr7:27244723,27244755 | SEQ ID NO: 31 | 0.8209 | 1.34E-17 | 0.8367 | 4.73E-07 |
| chr7:27244714,27244723,27244 755 | SEQ ID NO: 31 | 0.8066 | 1.27E-14 | 0.839 | 1.69E-07 |
| chr7:35293685 | SEQ ID NO: 32 | 0.9193 | 2.67E-55 | 0.909 | 1.23E-25 |
| chr7:35293700 | SEQ ID NO: 32 | 0.9182 | 6.30E-55 | 0.8654 | 1.42E-20 |
| chr7:35293692 | SEQ ID NO: 32 | 0.9172 | 1.33E-54 | 0.8831 | 2.24E-22 |

(continued)

| CpG sites and combinations | SEQ ID | Training set correlation | Training set p-value | Test set correlation | Test set p-value |
|---|---|---|---|---|---|
| chr7:35293690 | SEQ ID NO: 32 | 0.8708 | 1.59E-42 | 0.8339 | 6.50E-18 |
| chr7:35293676 | SEQ ID NO: 32 | 0.8694 | 3.00E-42 | 0.8183 | 8.57E-17 |
| chr7:35293687 | SEQ ID NO: 32 | 0.868 | 5.79E-42 | 0.8478 | 5.18E-19 |
| chr7:35293670 | SEQ ID NO: 32 | 0.8544 | 2.42E-39 | 0.8261 | 2.46E-17 |
| chr7:35293652 | SEQ ID NO: 32 | 0.8532 | 3.88E-39 | 0.8291 | 1.48E-17 |
| chr7:35293692,35293700 | SEQ ID NO: 32 | 0.8245 | 1.51E-30 | 0.814 | 1.72E-12 |
| chr7:35293656 | SEQ ID NO: 32 | 0.8233 | 2.27E-28 | 0.8216 | 5.62E-13 |
| chr7:50343850,50343853,50343 858,50343864,50343869,503438 72,50343883,50343890 | SEQ ID NO: 33 | 0.9899 | 5.41E-114 | 0.9882 | 4.23E-53 |
| chr7:50343853,50343858,50343 864,50343869,50343872,503438 83,50343890,50343897,5034390 7 | SEQ ID NO: 33 | 0.9899 | 5.41E-114 | 0.9361 | 2.80E-30 |
| chr7:50343853,50343858,50343 864,50343869,50343872,503438 83,50343890,50343897,5034390 7,50343909 | SEQ ID NO: 33 | 0.9899 | 5.41E-114 | 0.9361 | 2.80E-30 |
| chr7:50343858,50343864,50343 869,50343872,50343883,503438 90,50343897,50343907 | SEQ ID NO: 33 | 0.9899 | 5.41E-114 | 0.9361 | 2.80E-30 |

(continued)

| CpG sites and combinations | SEQ ID | Training set correlation | Training set p-value | Test set correlation | Test set p-value |
|---|---|---|---|---|---|
| chr7:50343858,50343864,50343 869,50343872,50343883,503438 90,50343897,50343907,5034390 9 | SEQ ID NO: 33 | 0.9899 | 5.41E-114 | 0.9361 | 2.80E-30 |
| chr7:50343869,50343872,50343 883,50343890,50343897,503439 07 | SEQ ID NO: 33 | 0.9899 | 5.41E-114 | 0.9361 | 2.80E-30 |
| chr7:50343869,50343872,50343 883,50343890,50343897,503439 07,50343909 | SEQ ID NO: 33 | 0.9899 | 5.41E-114 | 0.9361 | 2.80E-30 |
| chr7:50343872,50343883,50343 890,50343897,50343907 | SEQ ID NO: 33 | 0.9899 | 5.41E-114 | 0.9361 | 2.80E-30 |
| chr7:50343872,50343883,50343 890,50343897,50343907,503439 09 | SEQ ID NO: 33 | 0.9899 | 5.41E-114 | 0.9361 | 2.80E-30 |
| chr7: 50343 93 9, 50343 946, 50343 950,50343959,50343961,503439 63, 50343 969, 50343 974, 50343 98 0,50343990 | SEQ ID NO: 33 | 0.9899 | 5.41E-114 | 0.9906 | 3.61E-56 |
| chr7:155167562 | SEQ ID NO: 34 | 0.9155 | 4.98E-54 | 0.913 | 3.25E-26 |
| chr7:155167578 | SEQ ID NO: 34 | 0.8178 | 5.65E-29 | 0.831 | 1.07E-17 |
| chr7:155167568 | SEQ ID NO: 34 | 0.8486 | 6.59E-28 | 0.8121 | 3.50E-15 |
| chr7:155167552 | SEQ ID NO: 34 | 0.8411 | 2.64E-26 | 0.8395 | 2.42E-18 |
| chr7:155167507 | SEQ ID NO: 34 | 0.8073 | 4.70E-22 | 0.8226 | 4.32E-17 |

(continued)

| CpG sites and combinations | SEQ ID | Training set correlation | Training set p-value | Test set correlation | Test set p-value |
|---|---|---|---|---|---|
| chr7:155167555 | SEQ ID NO: 34 | 0.8074 | 3.80E-21 | 0.8482 | 4.84E-19 |
| chr7:155167552,155167555 | SEQ ID NO: 34 | 0.8302 | 1.49E-20 | 0.804 | 7.42E-16 |
| chr7:155167617 | SEQ ID NO: 34 | 0.8344 | 2.52E-20 | 0.8147 | 2.22E-15 |
| chr7:155167560,155167562 | SEQ ID NO: 34 | 0.8292 | 3.11E-20 | 0.8132 | 3.02E-11 |
| chr7:155167562,155167568 | SEQ ID NO: 34 | 0.8419 | 7.92E-18 | 0.8318 | 1.76E-11 |
| chr8:10588946 | SEQ ID NO: 35 | 0.9039 | 1.58E-50 | 0.8313 | 1.56E-13 |
| chr8:10588942 | SEQ ID NO: 35 | 0.8886 | 1.60E-46 | 0.8301 | 2.62E-09 |
| chr8:10588948 | SEQ ID NO: 35 | 0.8814 | 8.02E-45 | 0.8193 | 7.35E-17 |
| chr8:10588951 | SEQ ID NO: 35 | 0.8519 | 6.75E-39 | 0.8339 | 1.56E-13 |
| chr8:10588946,10588948 | SEQ ID NO: 35 | 0.834 | 6.87E-36 | 0.8265 | 2.40E-10 |
| chr8:10589003 | SEQ ID NO: 35 | 0.8154 | 3.90E-33 | 0.8456 | 7.86E-19 |

(continued)

| CpG sites and combinations | SEQ ID | Training set correlation | Training set p-value | Test set correlation | Test set p-value |
|---|---|---|---|---|---|
| chr8:10588948,10588951 | SEQ ID NO: 35 | 0.812 | 1.15E-32 | 0.8054 | 9.40E-09 |
| chr8:10588942,10588946 | SEQ ID NO: 35 | 0.8082 | 3.80E-32 | 0.8341 | 3.52E-06 |
| chr8:10589009 | SEQ ID NO: 35 | 0.8026 | 2.06E-31 | 0.8154 | 1.34E-16 |
| chr8:10588938 | SEQ ID NO: 35 | 0.8048 | 6.72E-31 | 0.8009 | 9.32E-10 |
| chr8:25907898,25907900 | SEQ ID NO: 36 | 0.8493 | 9.19E-36 | 0.8229 | 0.00E+00 |
| chr8:25907893,25907898,25907 900 | SEQ ID NO: 36 | 0.8652 | 2.16E-41 | 0.9881 | 6.76E-53 |
| chr8:25907898,25907900,25907 902 | SEQ ID NO: 36 | 0.8245 | 1.93E-34 | 0.9872 | 6.44E-52 |
| chr8:25907884,25907893,25907 898,25907900 | SEQ ID NO: 36 | 0.8134 | 7.35E-33 | 0.9849 | 9.69E-50 |
| chr8:25907893,25907898,25907 900,25907902 | SEQ ID NO: 36 | 0.8087 | 1.13E-28 | 0.9858 | 1.61E-50 |
| chr8:25907884,25907893,25907 898,25907900,25907902 | SEQ ID NO: 36 | 0.8259 | 4.37E-25 | 0.984 | 6.07E-49 |
| chr8:25907898,25907900,25907 902,25907906 | SEQ ID NO: 36 | 0.803 | 5.52E-24 | 0.8711 | 3.98E-21 |

(continued)

| CpG sites and combinations | SEQ ID | Training set correlation | Training set p-value | Test set correlation | Test set p-value |
|---|---|---|---|---|---|
| chr8:25907880,25907884,25907 893,25907898,25907900 | SEQ ID NO: 36 | 0.8162 | 1.92E-23 | 0.9834 | 2.15E-48 |
| chr8:25907874,25907880,25907 884,25907893,25907898,259079 00 | SEQ ID NO: 36 | 0.8225 | 5.77E-23 | 0.9818 | 3.93E-47 |
| chr8:25907898,25907900,25907 902,25907906,25907918 | SEQ ID NO: 36 | 0.8203 | 3.87E-22 | 0.8783 | 7.25E-22 |
| chr8:57069712 | SEQ ID NO: 37 | 0.8807 | 1.17E-44 | 0.9763 | 1.34E-43 |
| chr8:57069739 | SEQ ID NO: 37 | 0.8538 | 3.10E-39 | 0.9749 | 7.86E-43 |
| chr8:57069709 | SEQ ID NO: 37 | 0.8396 | 8.64E-37 | 0.9154 | 1.38E-26 |
| chr8:57069735 | SEQ ID NO: 37 | 0.832 | 1.38E-35 | 0.9811 | 1.12E-46 |
| chr8:57069722 | SEQ ID NO: 37 | 0.8296 | 3.22E-35 | 0.9777 | 2.08E-44 |
| chr8:57069709,57069712 | SEQ ID NO: 37 | 0.8092 | 2.81E-32 | 0.9043 | 5.58E-25 |
| chr8:57069755 | SEQ ID NO: 37 | 0.8442 | 8.32E-27 | 0.9036 | 7.03E-25 |
| chr8:57069735,57069739 | SEQ ID NO: 37 | 0.8297 | 9.83E-25 | 0.9796 | 1.32E-45 |

(continued)

| CpG sites and combinations | SEQ ID | Training set correlation | Training set p-value | Test set correlation | Test set p-value |
|---|---|---|---|---|---|
| chr8:57069712,57069722 | SEQ ID NO: 37 | 0.8002 | 2.43E-23 | 0.9872 | 6.40E-52 |
| chr8:57069709,57069712,57069 722 | SEQ ID NO: 37 | 0.8453 | 4.10E-21 | 0.9 | 2.12E-24 |
| chr10:28034654 | SEQ ID NO:3 8 | 0.9607 | 2.47E-75 | 0.993 | 3.18E-60 |
| chr10:28034658 | SEQ ID NO: 38 | 0.8399 | 1.07E-27 | 0.9904 | 8.14E-56 |
| chr10:28034669 | SEQ ID NO: 38 | 0.8453 | 8.40E-22 | 0.9783 | 8.82E-45 |
| chr10:28034682 | SEQ ID NO: 38 | 0.8393 | 1.43E-19 | 0.9821 | 2.06E-47 |
| chr10:28034697 | SEQ ID NO: 38 | 0.8054 | 1.83E-16 | 0.9695 | 3.32E-40 |
| chr10:28034727 | SEQ ID NO: 38 | 0.8065 | 4.37E-15 | 0.91 | 8.80E-26 |
| chr10:28034654,28034658 | SEQ ID NO: 38 | 0.81 | 1.88E-14 | 0.9758 | 2.59E-43 |
| chr10:28034757 | SEQ ID NO: 38 | 0.8363 | 1.97E-14 | 0.832 | 9.12E-18 |
| chr10:28034751 | SEQ ID NO: 38 | 0.8423 | 5.71E-13 | 0.8414 | 1.72E-18 |

(continued)

| CpG sites and combinations | SEQ ID | Training set correlation | Training set p-value | Test set correlation | Test set p-value |
|---|---|---|---|---|---|
| chr10:28034687 | SEQ ID NO: 38 | 0.8045 | 6.22E-13 | 0.9461 | 1.53E-32 |
| chr12:4919230 | SEQ ID NO: 39 | 0.8381 | 5.14E-21 | 0.9321 | 1.76E-29 |
| chr12:4919215 | SEQ ID NO: 39 | 0.8005 | 7.89E-21 | 0.9279 | 1.10E-28 |
| chr12:4919164 | SEQ ID NO: 39 | 0.8362 | 2.10E-20 | 0.9196 | 2.99E-27 |
| chr12:4919138 | SEQ ID NO: 39 | 0.8078 | 1.12E-18 | 0.919 | 3.69E-27 |
| chr12:4919147 | SEQ ID NO: 39 | 0.8387 | 1.00E-14 | 0.9204 | 2.18E-27 |
| chr12:4919191 | SEQ ID NO: 39 | 0.8386 | 2.39E-14 | 0.9409 | 2.54E-31 |
| chr12:4919239 | SEQ ID NO: 39 | 0.8216 | 4.99E-14 | 0.829 | 1.47E-15 |
| chr12:4919260 | SEQ ID NO: 39 | 0.8347 | 3.67E-12 | 0.8098 | 3.34E-08 |
| chr12:4919145 | SEQ ID NO: 39 | 0.8419 | 4.40E-11 | 0.92 | 2.57E-27 |
| chr12:4919184 | SEQ ID NO: 39 | 0.8292 | 4.50E-11 | 0.928 | 1.05E-28 |

(continued)

| CpG sites and combinations | SEQ ID | Training set correlation | Training set p-value | Test set correlation | Test set p-value |
|---|---|---|---|---|---|
| chr12:33592862 | SEQ ID NO: 40 | 0.8161 | 3.10E-33 | 0.9049 | 4.67E-25 |
| chr12:33592865 | SEQ ID NO: 40 | 0.8033 | 2.40E-27 | 0.8213 | 5.31E-17 |
| chr12:33592867 | SEQ ID NO: 40 | 0.8032 | 1.18E-21 | 0.8185 | 3.78E-13 |
| chr12:33592882 | SEQ ID NO: 40 | 0.8102 | 2.32E-13 | 0.8242 | 1.31E-07 |
| chr12:33592831 | SEQ ID NO: 40 | 0.8025 | 5.67E-13 | 0.8179 | 9.20E-10 |
| chr12:33592859 | SEQ ID NO: 40 | 0.8359 | 6.28E-13 | 0.8296 | 1.50E-11 |
| chr12:33592859,33592862 | SEQ ID NO: 40 | 0.813 | 9.00E-13 | 0.8367 | 7.52E-13 |
| chr12:33592867,33592875,3359 2882 | SEQ ID NO: 40 | 0.8111 | 1.90E-12 | 0.8007 | 1.32E-09 |
| chr12:33592862,33592865 | SEQ ID NO: 40 | 0.8486 | 1.72E-11 | 0.8452 | 2.62E-10 |
| chr12:33592875 | SEQ ID NO: 40 | 0.8194 | 2.10E-11 | 0.8473 | 1.64E-08 |
| chr12:58131345,58131348,5813 1384,58131390,58131404 | SEQ ID NO: 41 | 0.8258 | 3.76E-35 | 0.8243 | 0.00E+00 |

(continued)

| CpG sites and combinations | SEQ ID | Training set correlation | Training set p-value | Test set correlation | Test set p-value |
|---|---|---|---|---|---|
| chr12:58131348,58131384,5813 1390,58131404 | SEQ ID NO: 41 | 0.9623 | 1.64E-76 | 0.9669 | 4.61E-39 |
| chr12:58131384,58131390,5813 1404 | SEQ ID NO: 41 | 0.93 | 3.17E-59 | 0.9455 | 2.08E-32 |
| chr12:58131345,58131348,5813 1384,58131390,58131404,58131 412 | SEQ ID NO: 41 | 0.9134 | 2.31E-53 | 0.9433 | 7.04E-32 |
| chr12:58131345,58131348,5813 1384,58131390,58131404,58131 412,58131414 | SEQ ID NO: 41 | 0.9034 | 2.18E-50 | 0.9326 | 1.42E-29 |
| chr12:58131390,58131404 | SEQ ID NO: 41 | 0.9021 | 4.94E-50 | 0.9037 | 6.81E-25 |
| chr 12: 5 8131404 | SEQ ID NO: 41 | 0.8863 | 5.91E-46 | 0.8771 | 9.77E-22 |
| chr12:58131348,58131384,5813 1390,58131404,58131412 | SEQ ID NO: 41 | 0.8774 | 6.31E-44 | 0.9236 | 6.25E-28 |
| chr12:58131348,58131384,5813 1390,58131404,58131412,58131 414 | SEQ ID NO: 41 | 0.8728 | 6.07E-43 | 0.911 | 6.49E-26 |
| chr12:58131345,58131348,5813 1384,58131390,58131404,58131 412,58131414,58131426 | SEQ ID NO: 41 | 0.85 | 1.49E-38 | 0.8415 | 1.69E-18 |
| chr12:115125060 | SEQ ID NO: 42 | 0.8095 | 2.50E-32 | 0.8061 | 5.43E-16 |
| chr12:115125013 | SEQ ID NO: 42 | 0.8156 | 6.90E-31 | 0.8574 | 7.76E-20 |

(continued)

| CpG sites and combinations | SEQ ID | Training set correlation | Training set p-value | Test set correlation | Test set p-value |
|---|---|---|---|---|---|
| chr12:115125060,115125098 | SEQ ID NO: 42 | 0.8214 | 2.36E-27 | 0.8184 | 8.22E-13 |
| chr12:115125060,115125098,11 5125107 | SEQ ID NO: 42 | 0.8306 | 1.26E-26 | 0.8253 | 2.43E-12 |
| chr12:115125053,115125060,11 5125098,115125107 | SEQ ID NO: 42 | 0.8262 | 1.39E-25 | 0.8237 | 1.27E-11 |
| chr12:115125053,115125060,11 5125098 | SEQ ID NO: 42 | 0.8219 | 2.53E-25 | 0.8327 | 7.19E-12 |
| chr12:115125053,115125060 | SEQ ID NO: 42 | 0.8154 | 3.07E-25 | 0.828 | 3.44E-13 |
| chr12:115125098 | SEQ ID NO: 42 | 0.8173 | 5.71E-25 | 0.8288 | 1.66E-13 |
| chr12:115125013,115125034 | SEQ ID NO: 42 | 0.8021 | 1.01E-24 | 0.8317 | 3.79E-15 |
| chr12:115125053 | SEQ ID NO: 42 | 0.8152 | 1.07E-24 | 0.8028 | 4.53E-15 |
| chr13:37005694 | SEQ ID NO: 43 | 0.8012 | 6.85E-35 | 0.85 | 0.00E+00 |
| chr13:37005678 | SEQ ID NO: 43 | 0.8209 | 3.41E-25 | 0.9387 | 7.73E-31 |
| chr13:37005686 | SEQ ID NO: 43 | 0.8173 | 3.97E-20 | 0.9508 | 9.36E-34 |

(continued)

| CpG sites and combinations | SEQ ID | Training set correlation | Training set p-value | Test set correlation | Test set p-value |
|---|---|---|---|---|---|
| chr13:37005706 | SEQ ID NO: 43 | 0.8389 | 1.86E-19 | 0.9346 | 5.47E-30 |
| chr13:37005704 | SEQ ID NO: 43 | 0.8034 | 7.82E-16 | 0.9352 | 4.26E-30 |
| chr13:37005673 | SEQ ID NO: 43 | 0.835 | 9.88E-15 | 0.9261 | 2.28E-28 |
| chr13:37005686,37005694 | SEQ ID NO: 43 | 0.8426 | 4.34E-14 | 0.9375 | 1.39E-30 |
| chr13:37005721 | SEQ ID NO: 43 | 0.8205 | 5.95E-14 | 0.9365 | 2.23E-30 |
| chr13:37005694,37005704 | SEQ ID NO: 43 | 0.8362 | 2.00E-12 | 0.932 | 1.80E-29 |
| chr13:37005738 | SEQ ID NO: 43 | 0.846 | 1.13E-10 | 0.9278 | 1.15E-28 |
| chr13:100649745 | SEQ ID NO: 44 | 0.8958 | 2.46E-48 | 0.9142 | 2.15E-26 |
| chr13:100649734 | SEQ ID NO: 44 | 0.8443 | 1.85E-30 | 0.8101 | 3.02E-16 |
| chr13:100649740 | SEQ ID NO: 44 | 0.8092 | 1.22E-27 | 0.8495 | 4.11E-10 |
| chr13:100649740,100649745 | SEQ ID NO: 44 | 0.8086 | 8.73E-27 | 0.8194 | 1.87E-09 |

(continued)

| CpG sites and combinations | SEQ ID | Training set correlation | Training set p-value | Test set correlation | Test set p-value |
|---|---|---|---|---|---|
| chr13:100649734,100649738 | SEQ ID NO: 44 | 0.8412 | 1.60E-26 | 0.8369 | 3.18E-11 |
| chr13:100649738 | SEQ ID NO: 44 | 0.8169 | 3.45E-26 | 0.811 | 2.65E-16 |
| chr13:100649725 | SEQ ID NO: 44 | 0.8151 | 6.71E-26 | 0.8483 | 1.45E-11 |
| chr13:100649715 | SEQ ID NO: 44 | 0.8483 | 1.74E-25 | 0.8235 | 1.51E-07 |
| chr13:100649721 | SEQ ID NO: 44 | 0.8079 | 8.64E-25 | 0.8156 | 3.21E-05 |
| chr13:100649738,100649740 | SEQ ID NO: 44 | 0.8173 | 6.74E-24 | 0.8402 | 3.79E-06 |
| chr13:100649769 | SEQ ID NO: 45 | 0.8759 | 1.32E-43 | 0.9245 | 4.36E-28 |
| chr13:100649718 | SEQ ID NO: 45 | 0.804 | 2.09E-26 | 0.8276 | 1.13E-14 |
| chr13:100649718,100649721 | SEQ ID NO: 45 | 0.8208 | 2.87E-25 | 0.8164 | 4.87E-09 |
| chr13:100649745 | SEQ ID NO: 45 | 0.8065 | 4.52E-24 | 0.8162 | 1.12E-14 |
| chr13:100649731 | SEQ ID NO: 45 | 0.8004 | 8.65E-24 | 0.8352 | 5.21E-18 |

(continued)

| CpG sites and combinations | SEQ ID | Training set correlation | Training set p-value | Test set correlation | Test set p-value |
|---|---|---|---|---|---|
| chr13:100649725 | SEQ ID NO: 45 | 0.809 | 2.30E-23 | 0.8234 | 3.81E-17 |
| chr13: 100649731, 100649734 | SEQ ID NO: 45 | 0.8221 | 9.41E-23 | 0.8091 | 3.48E-16 |
| chr13:100649745,100649763 | SEQ ID NO: 45 | 0.848 | 1.03E-22 | 0.8069 | 1.44E-14 |
| chr13:100649701 | SEQ ID NO: 45 | 0.806 | 1.25E-22 | 0.8314 | 1.97E-14 |
| chr13:100649731,100649734,10 0649738 | SEQ ID NO: 45 | 0.8131 | 1.32E-22 | 0.8046 | 1.02E-12 |
| chr14:38724685 | SEQ ID NO: 46 | 0.8564 | 1.03E-39 | 0.9177 | 5.94E-27 |
| chr14:38724669 | SEQ ID NO: 46 | 0.8505 | 1.21E-38 | 0.9092 | 1.18E-25 |
| chr14:38724675 | SEQ ID NO: 46 | 0.8391 | 1.01E-36 | 0.9177 | 6.05E-27 |
| chr14:38724680 | SEQ ID NO: 46 | 0.8374 | 1.92E-36 | 0.9073 | 2.20E-25 |
| chr14:38724648,38724650 | SEQ ID NO: 46 | 0.8242 | 3.24E-27 | 0.8692 | 6.20E-21 |
| chr14:38724682 | SEQ ID NO: 46 | 0.8116 | 7.59E-27 | 0.8839 | 1.82E-22 |

(continued)

| CpG sites and combinations | SEQ ID | Training set correlation | Training set p-value | Test set correlation | Test set p-value |
|---|---|---|---|---|---|
| chr14:38724650 | SEQ ID NO: 46 | 0.8125 | 7.70E-27 | 0.9056 | 3.76E-25 |
| chr14:38724648 | SEQ ID NO: 46 | 0.8316 | 3.29E-25 | 0.9018 | 1.23E-24 |
| chr14:38724646 | SEQ ID NO: 46 | 0.8491 | 4.64E-25 | 0.8597 | 4.86E-20 |
| chr14:38724852 | SEQ ID NO: 46 | 0.8414 | 5.76E-21 | 0.8754 | 1.46E-21 |
| chr14:38724852 | SEQ ID NO: 47 | 0.975 | 4.13E-88 | 0.9744 | 1.57E-42 |
| chr14:38724858 | SEQ ID NO: 47 | 0.9422 | 1.57E-64 | 0.9341 | 7.13E-30 |
| chr14:38724864 | SEQ ID NO: 47 | 0.8644 | 3.12E-41 | 0.8856 | 1.16E-22 |
| chr14:38724852,38724858 | SEQ ID NO: 47 | 0.845 | 1.07E-37 | 0.8562 | 9.97E-20 |
| chr14:38724847 | SEQ ID NO: 47 | 0.8283 | 5.66E-29 | 0.8675 | 9.09E-21 |
| chr14:38724847,38724852 | SEQ ID NO: 47 | 0.848 | 2.20E-27 | 0.86 | 4.53E-20 |
| chr14:38724858,38724864 | SEQ ID NO: 47 | 0.8295 | 5.06E-26 | 0.8437 | 1.13E-18 |

(continued)

| CpG sites and combinations | SEQ ID | Training set correlation | Training set p-value | Test set correlation | Test set p-value |
|---|---|---|---|---|---|
| chr14:38724873 | SEQ ID NO: 47 | 0.8157 | 9.57E-26 | 0.8538 | 1.62E-19 |
| chr14:38724867 | SEQ ID NO: 47 | 0.8162 | 1.82E-17 | 0.843 | 1.29E-18 |
| chr14:38724852,38724858,3872 4864 | SEQ ID NO: 47 | 0.8257 | 2.15E-17 | 0.8234 | 3.78E-17 |
| chr14:57275896 | SEQ ID NO: 48 | 0.9371 | 3.32E-62 | 0.9721 | 2.16E-41 |
| chr14:57275885,57275896 | SEQ ID NO: 48 | 0.8145 | 3.81E-20 | 0.8418 | 1.60E-18 |
| chr14:57275908 | SEQ ID NO: 48 | 0.8462 | 1.04E-19 | 0.8144 | 6.12E-14 |
| chr14:57275885 | SEQ ID NO: 48 | 0.8364 | 1.35E-16 | 0.8732 | 2.48E-21 |
| chr14:57275852 | SEQ ID NO: 48 | 0.8157 | 7.06E-16 | 0.8229 | 2.30E-13 |
| chr14:57275924 | SEQ ID NO: 48 | 0.8176 | 1.32E-15 | 0.8333 | 7.24E-18 |
| chr14:57275823 | SEQ ID NO: 48 | 0.8084 | 3.03E-15 | 0.8257 | 2.59E-17 |
| chr14:57275831 | SEQ ID NO: 48 | 0.8191 | 3.97E-15 | 0.8427 | 1.20E-13 |

(continued)

| CpG sites and combinations | SEQ ID | Training set correlation | Training set p-value | Test set correlation | Test set p-value |
|---|---|---|---|---|---|
| chr14:57275896,57275908 | SEQ ID NO: 48 | 0.8163 | 1.11E-14 | 0.8165 | 1.37E-11 |
| chr14:57275827 | SEQ ID NO: 48 | 0.8241 | 6.71E-14 | 0.8054 | 1.26E-09 |
| chr14:60952634 | SEQ ID NO: 49 | 0.8105 | 1.02E-16 | 0.8491 | 1.91E-11 |
| chr14:60952658 | SEQ ID NO: 49 | 0.8332 | 5.40E-15 | 0.8152 | 3.97E-12 |
| chr14:60952762 | SEQ ID NO: 49 | 0.8056 | 2.10E-13 | 0.8151 | 4.09E-07 |
| chr14:60952658,60952683 | SEQ ID NO: 49 | 0.8164 | 3.87E-11 | 0.83 | 3.83E-09 |
| chr14:60952683 | SEQ ID NO: 49 | 0.8136 | 9.47E-11 | 0.8356 | 2.95E-12 |
| chr14:60952755 | SEQ ID NO: 49 | 0.8232 | 1.75E-08 | 0.8333 | 5.67E-07 |
| chr14:60952755,60952762 | SEQ ID NO: 49 | 0.8487 | 2.36E-08 | 0.8227 | 8.30E-06 |
| chr14:60952730 | SEQ ID NO: 49 | 0.8436 | 3.00E-08 | 0.8088 | 2.44E-05 |
| chr14:60952634,60952658 | SEQ ID NO: 49 | 0.8266 | 2.45E-07 | 0.8384 | 9.73E-08 |

(continued)

| CpG sites and combinations | SEQ ID | Training set correlation | Training set p-value | Test set correlation | Test set p-value |
|---|---|---|---|---|---|
| chr14:60952687 | SEQ ID NO: 49 | 0.8499 | 8.22E-07 | 0.8324 | 3.68E-09 |
| chr15:83952345 | SEQ ID NO: 50 | 0.9181 | 6.49E-55 | 0.9719 | 2.85E-41 |
| chr15:83952352 | SEQ ID NO: 50 | 0.8425 | 2.80E-37 | 0.9678 | 1.79E-39 |
| chr15:83952358 | SEQ ID NO: 50 | 0.8326 | 1.14E-35 | 0.8186 | 8.22E-17 |
| chr15:83952309 | SEQ ID NO: 50 | 0.8444 | 1.26E-20 | 0.9187 | 4.12E-27 |
| chr15:83952314 | SEQ ID NO: 50 | 0.8481 | 5.77E-20 | 0.9366 | 2.14E-30 |
| chr15:83952317 | SEQ ID NO: 50 | 0.8183 | 9.87E-20 | 0.9432 | 7.34E-32 |
| chr15:83952266 | SEQ ID NO: 50 | 0.8083 | 1.50E-18 | 0.9397 | 4.76E-31 |
| chr15:83952238 | SEQ ID NO: 50 | 0.8066 | 1.84E-17 | 0.8003 | 4.48E-11 |
| chr15:83952285 | SEQ ID NO: 50 | 0.832 | 2.97E-16 | 0.9194 | 3.21E-27 |
| chr15:83952291 | SEQ ID NO: 50 | 0.8437 | 5.75E-12 | 0.9231 | 7.68E-28 |

(continued)

| CpG sites and combinations | SEQ ID | Training set correlation | Training set p-value | Test set correlation | Test set p-value |
|---|---|---|---|---|---|
| chr16:31580246 | SEQ ID NO: 51 | 0.9502 | 1.09E-68 | 0.9505 | 1.10E-33 |
| chr16:31580254 | SEQ ID NO: 51 | 0.8073 | 5.03E-32 | 0.8026 | 3.43E-08 |
| chr16:31580246,31580254 | SEQ ID NO: 51 | 0.8453 | 9.24E-31 | 0.8212 | 3.61E-07 |
| chr16:31580287 | SEQ ID NO: 51 | 0.8461 | 4.65E-24 | 0.8005 | 7.15E-06 |
| chr16:31580296 | SEQ ID NO: 51 | 0.811 | 4.59E-19 | 0.8199 | 1.46E-04 |
| chr16:31580269 | SEQ ID NO: 51 | 0.8158 | 2.90E-16 | 0.8113 | 3.10E-05 |
| chr16:31580220,31580246 | SEQ ID NO: 51 | 0.8455 | 1.85E-15 | 0.8117 | 1.97E-08 |
| chr16:31580311 | SEQ ID NO: 51 | 0.8402 | 7.22E-15 | 0.8415 | 1.50E-05 |
| chr16:31580220 | SEQ ID NO: 51 | 0.8246 | 7.02E-14 | 0.8399 | 1.22E-08 |
| chr16:31580299 | SEQ ID NO: 51 | 0.8291 | 1.75E-11 | 0.8255 | 2.76E-03 |
| chr16:73097037 | SEQ ID NO: 52 | 0.8972 | 1.06E-48 | 0.9026 | 9.49E-25 |

(continued)

| CpG sites and combinations | SEQ ID | Training set correlation | Training set p-value | Test set correlation | Test set p-value |
|---|---|---|---|---|---|
| chr16:73097045 | SEQ ID NO: 52 | 0.8655 | 1.86E-41 | 0.8829 | 2.32E-22 |
| chr16:73097037,73097045 | SEQ ID NO: 52 | 0.8519 | 6.70E-39 | 0.8741 | 1.98E-21 |
| chr16:73097057 | SEQ ID NO: 52 | 0.8276 | 6.64E-35 | 0.8452 | 8.43E-19 |
| chr16:73097156 | SEQ ID NO: 52 | 0.8267 | 8.97E-35 | 0.8263 | 2.37E-17 |
| chr16:73097060 | SEQ ID NO: 52 | 0.8253 | 1.44E-34 | 0.8639 | 1.98E-20 |
| chr16:73097183 | SEQ ID NO: 52 | 0.8182 | 1.56E-33 | 0.8342 | 6.23E-18 |
| chr16:73097156,73097183 | SEQ ID NO: 52 | 0.8487 | 1.02E-28 | 0.845 | 4.04E-11 |
| chr16:73097045,73097057 | SEQ ID NO: 52 | 0.8379 | 2.37E-26 | 0.8024 | 9.27E-16 |
| chr16:73097069 | SEQ ID NO: 52 | 0.8254 | 3.06E-26 | 0.8235 | 3.74E-17 |
| chr17:35299974 | SEQ ID NO: 53 | 0.8088 | 1.73E-26 | 0.8385 | 5.26E-12 |
| chr17:35299990 | SEQ ID NO: 53 | 0.8187 | 1.24E-22 | 0.8457 | 2.24E-13 |

(continued)

| CpG sites and combinations | SEQ ID | Training set correlation | Training set p-value | Test set correlation | Test set p-value |
|---|---|---|---|---|---|
| chr17:35299972 | SEQ ID NO: 53 | 0.827 | 1.17E-21 | 0.836 | 4.20E-14 |
| chr17:35299963 | SEQ ID NO: 53 | 0.8257 | 6.51E-18 | 0.8491 | 7.55E-15 |
| chr17:35299974,35299990 | SEQ ID NO: 53 | 0.8031 | 4.20E-17 | 0.8069 | 1.57E-10 |
| chr17:35299972,35299974 | SEQ ID NO: 53 | 0.8311 | 4.71E-16 | 0.8085 | 7.48E-10 |
| chr17:35299966 | SEQ ID NO: 53 | 0.8024 | 3.37E-15 | 0.8044 | 9.71E-10 |
| chr17:35299944 | SEQ ID NO: 53 | 0.8473 | 1.72E-14 | 0.8554 | 1.16E-19 |
| chr17:35299972,35299974,3529 9990 | SEQ ID NO: 53 | 0.8034 | 1.01E-13 | 0.8111 | 1.71E-09 |
| chr17:35299966,35299972,3529 9974 | SEQ ID NO: 53 | 0.8497 | 2.00E-13 | 0.8103 | 6.11E-09 |
| chr17:76929873,76929926 | SEQ ID NO: 54 | 0.8482 | 4.29E-3 5 | 0.8276 | 0.00E+0 0 |
| chr17:76929873 | SEQ ID NO: 54 | 0.9043 | 1.26E-50 | 0.9472 | 7.95E-33 |
| chr17:76929926 | SEQ ID NO: 54 | 0.8066 | 1.47E-25 | 0.8052 | 6.13E-15 |

(continued)

| CpG sites and combinations | SEQ ID | Training set correlation | Training set p-value | Test set correlation | Test set p-value |
|---|---|---|---|---|---|
| chr17:76929829,76929873,7692 9926 | SEQ ID NO: 54 | 0.844 | 1.68E-06 | 0.8442 | 1.23E-03 |
| chr17:76929829,76929873 | SEQ ID NO: 54 | 0.8448 | 4.59E-05 | 0.842 | 7.49E-03 |
| chr17:76929829 | SEQ ID NO: 54 | 0.8126 | 2.78E-02 | 0.8195 | 0.00E+0 0 |
| chr17:76929769,76929829,7692 9873,76929926 | SEQ ID NO: 54 | 0.8054 | 3.80E-35 | 0.8495 | 0.00E+0 0 |
| chr17:76929769,76929829,7692 9873 | SEQ ID NO: 54 | 0.8313 | 6.64E-35 | 0.8271 | 0.00E+0 0 |
| chr17:76929769,76929829 | SEQ ID NO: 54 | 0.829 | 9.29E-3 5 | 0.8483 | 0.00E+0 0 |
| chr17:76929769 | SEQ ID NO: 54 | 0.8473 | 7.08E-35 | 0.8158 | 0.00E+0 0 |
| chr17:80846867,80846886,8084 6960 | SEQ ID NO: 55 | 0.8174 | 6.82E-35 | 0.8381 | 0.00E+0 0 |
| chr17:80846860,80846867,8084 6886,80846960 | SEQ ID NO: 55 | 0.9555 | 8.04E-72 | 0.9842 | 4.14E-49 |
| chr17:80846886,80846960 | SEQ ID NO: 55 | 0.9402 | 1.31E-63 | 0.9707 | 9.77E-41 |
| chr17:80846960 | SEQ ID NO: 55 | 0.916 | 3.26E-54 | 0.954 | 1.19E-34 |

(continued)

| CpG sites and combinations | SEQ ID | Training set correlation | Training set p-value | Test set correlation | Test set p-value |
|---|---|---|---|---|---|
| chr17:80846867,80846886,8084 6960,80846965 | SEQ ID NO: 55 | 0.8306 | 1.19E-29 | 0.8071 | 4.68E-16 |
| chr17:80846860,80846867,8084 6886,80846960,80846965 | SEQ ID NO: 55 | 0.8081 | 4.66E-27 | 0.8227 | 8.45E-14 |
| chr17:80846867,80846886 | SEQ ID NO: 55 | 0.8272 | 2.23E-26 | 0.8483 | 2.76E-12 |
| chr17:80846886,80846960,8084 6965 | SEQ ID NO: 55 | 0.8186 | 5.63E-26 | 0.8319 | 3.66E-14 |
| chr17:80846860,80846867,8084 6886 | SEQ ID NO: 55 | 0.8172 | 1.80E-25 | 0.8339 | 1.29E-12 |
| chr17:80846867 | SEQ ID NO: 55 | 0.8147 | 2.82E-23 | 0.8327 | 7.71E-12 |
| chr21:38081502 | SEQ ID NO: 56 | 0.8277 | 2.71E-18 | 0.8391 | 1.18E-10 |
| chr21:38081499 | SEQ ID NO: 56 | 0.8148 | 4.73E-15 | 0.8425 | 9.06E-14 |
| chr21:38081497 | SEQ ID NO: 56 | 0.8326 | 1.77E-09 | 0.8265 | 3.07E-07 |
| chr21:38081502,38081514 | SEQ ID NO: 56 | 0.8155 | 5.85E-08 | 0.8468 | 4.58E-04 |
| chr21:38081492,38081497 | SEQ ID NO: 56 | 0.809 | 3.51E-06 | 0.8023 | 6.89E-04 |

(continued)

| CpG sites and combinations | SEQ ID | Training set correlation | Training set p-value | Test set correlation | Test set p-value |
|---|---|---|---|---|---|
| chr21:38081492 | SEQ ID NO: 56 | 0.8203 | 4.12E-06 | 0.8348 | 7.80E-03 |
| chr21:38081514 | SEQ ID NO: 56 | 0.8438 | 3.78E-05 | 0.829 | 0.00E+00 |
| chr21:38081499,38081502 | SEQ ID NO: 56 | 0.8294 | 8.90E-05 | 0.8021 | 1.04E-03 |
| chr21:38081502,38081514,3808 1517 | SEQ ID NO: 56 | 0.8197 | 1.47E-04 | 0.8396 | 5.24E-03 |
| chr21:38081492,38081497,3808 1499 | SEQ ID NO: 56 | 0.8157 | 1.79E-04 | 0.8079 | 2.03E-03 |

## 1-2: Predictive performance of single methylation markers

In order to verify the differentiating performance of single methylation markers in patients with and without pancreatic cancer, the values of methylation levels of single methylation markers were used to verify the predictive performance of single markers.

First, the methylation level values of 56 methylation markers were used separately in the training set samples for training to determine the threshold, sensitivity and specificity for differentiating the presence and absence of pancreatic cancer, and then the threshold was used to statistically analyze the sensitivity and specificity of the samples in the test set. The results are shown in Table 1-4 below. It can be seen that a single marker can also achieve good differentiating performance.

**Table 1-4: Predictive performance of 56 methylation markers**

| Sequence | Group | AUC value | Sensitivity | Specificity | Threshold |
|---|---|---|---|---|---|
| SEQ ID NO:1 | Training set | 0.77572 | 0.793651 | 0.685185 | 0.833567 |
| SEQ ID NO:1 | Test set | 0.700993 | 0.677419 | 0.538462 | 0.833567 |
| SEQ ID NO:2 | Training set | 0.77866 | 0.825397 | 0.685185 | 0.623608 |
| SEQ ID NO:2 | Test set | 0.717122 | 0.774194 | 0.423077 | 0.623608 |
| SEQ ID NO:3 | Training set | 0.80776 | 0.698413 | 0.796296 | 0.519749 |
| SEQ ID NO:3 | Test set | 0.751861 | 0.677419 | 0.653846 | 0.519749 |
| SEQ ID NO:4 | Training set | 0.797178 | 0.698413 | 0.796296 | 0.916416 |
| SEQ ID NO:4 | Test set | 0.759305 | 0.645161 | 0.692308 | 0.916416 |
| SEQ ID NO:5 | Training set | 0.792916 | 0.730159 | 0.740741 | 0.856846 |
| SEQ ID NO:5 | Test set | 0.760546 | 0.774194 | 0.576923 | 0.856846 |
| SEQ ID NO:6 | Training set | 0.788948 | 0.68254 | 0.814815 | 0.502554 |

(continued)

| Sequence | Group | AUC value | Sensitivity | Specificity | Threshold |
|---|---|---|---|---|---|
| SEQ ID NO:6 | Test set | 0.718362 | 0.709677 | 0.538462 | 0.502554 |
| SEQ ID NO:7 | Training set | 0.798207 | 0.777778 | 0.685185 | 0.811377 |
| SEQ ID NO:7 | Test set | 0.792804 | 0.806452 | 0.576923 | 0.811377 |
| SEQ ID NO:8 | Training set | 0.786008 | 0.698413 | 0.796296 | 0.021244 |
| SEQ ID NO:8 | Test set | 0.837469 | 0.806452 | 0.692308 | 0.021244 |
| SEQ ID NO:9 | Training set | 0.788948 | 0.777778 | 0.685185 | 0.88238 |
| SEQ ID NO:9 | Test set | 0.771712 | 0.774194 | 0.576923 | 0.88238 |
| SEQ ID NO:10 | Training set | 0.781599 | 0.555556 | 0.944444 | 0.077874 |
| SEQ ID NO:10 | Test set | 0.789082 | 0.580645 | 0.807692 | 0.077874 |
| SEQ ID NO:11 | Training set | 0.793945 | 0.603175 | 0.888889 | 0.764823 |
| SEQ ID NO:11 | Test set | 0.764268 | 0.612903 | 0.730769 | 0.764823 |
| SEQ ID NO:12 | Training set | 0.781893 | 0.746032 | 0.777778 | 0.897736 |
| SEQ ID NO:12 | Test set | 0.784119 | 0.806452 | 0.576923 | 0.897736 |
| SEQ ID N0:13 | Training set | 0.770135 | 0.793651 | 0.611111 | 0.873318 |
| SEQ ID N0:13 | Test set | 0.771712 | 0.741935 | 0.653846 | 0.873318 |
| SEQ ID NO:14 | Training set | 0.78689 | 0.825397 | 0.62963 | 0.913279 |
| SEQ ID NO:14 | Test set | 0.78536 | 0.870968 | 0.538462 | 0.913279 |
| SEQ ID NO:15 | Training set | 0.798648 | 0.666667 | 0.814815 | 0.160867 |
| SEQ ID NO:15 | Test set | 0.705955 | 0.612903 | 0.692308 | 0.160867 |
| SEQ ID NO:16 | Training set | 0.797178 | 0.746032 | 0.796296 | 0.56295 |
| SEQ ID NO:16 | Test set | 0.616625 | 0.935484 | 0.192308 | 0.56295 |
| SEQ ID NO:17 | Training set | 0.782481 | 0.666667 | 0.777778 | 0.061143 |
| SEQ ID NO:17 | Test set | 0.76799 | 0.709677 | 0.692308 | 0.061143 |
| SEQ ID NO:18 | Training set | 0.762493 | 0.666667 | 0.777778 | 0.899668 |
| SEQ ID NO:18 | Test set | 0.759305 | 0.677419 | 0.653846 | 0.899668 |
| SEQ ID NO:19 | Training set | 0.751911 | 0.730159 | 0.666667 | 0.943553 |
| SEQ ID NO:19 | Test set | 0.745658 | 0.806452 | 0.461538 | 0.943553 |
| SEQ ID NO:20 | Training set | 0.779248 | 0.634921 | 0.833333 | 0.859903 |
| SEQ ID NO:20 | Test set | 0.801489 | 0.612903 | 0.807692 | 0.859903 |
| SEQ ID NO:21 | Training set | 0.771311 | 0.84127 | 0.62963 | 0.655087 |
| SEQ ID NO:21 | Test set | 0.647643 | 0.677419 | 0.5 | 0.655087 |
| SEQ ID NO:22 | Training set | 0.742504 | 0.698413 | 0.703704 | 0.922167 |
| SEQ ID NO:22 | Test set | 0.787841 | 0.741935 | 0.653846 | 0.922167 |
| SEQ ID NO:23 | Training set | 0.75485 | 0.698413 | 0.777778 | 0.248108 |
| SEQ ID NO:23 | Test set | 0.722084 | 0.548387 | 0.807692 | 0.248108 |
| SEQ ID NO:24 | Training set | 0.771311 | 0.634921 | 0.814815 | 0.157576 |
| SEQ ID NO:24 | Test set | 0.799007 | 0.709677 | 0.730769 | 0.157576 |
| SEQ ID NO:25 | Training set | 0.777778 | 0.730159 | 0.666667 | 0.911221 |

(continued)

| Sequence | Group | AUC value | Sensitivity | Specificity | Threshold |
|---|---|---|---|---|---|
| SEQ ID NO:25 | Test set | 0.69727 | 0.645161 | 0.576923 | 0.911221 |
| SEQ ID NO:26 | Training set | 0.765726 | 0.68254 | 0.759259 | 0.908358 |
| SEQ ID NO:26 | Test set | 0.776675 | 0.806452 | 0.576923 | 0.908358 |
| SEQ ID NO:27 | Test set | 0.764268 | 0.903226 | 0.346154 | 0.933709 |
| SEQ ID NO:27 | Training set | 0.767784 | 0.793651 | 0.611111 | 0.933709 |
| SEQ ID NO:28 | Training set | 0.783363 | 0.746032 | 0.703704 | 0.880336 |
| SEQ ID NO:28 | Test set | 0.781638 | 0.741935 | 0.692308 | 0.880336 |
| SEQ ID NO:29 | Training set | 0.768225 | 0.761905 | 0.666667 | 0.55838 |
| SEQ ID NO:29 | Test set | 0.734491 | 0.645161 | 0.615385 | 0.55838 |
| SEQ ID NO:30 | Training set | 0.780864 | 0.634921 | 0.87037 | 0.974684 |
| SEQ ID NO:30 | Test set | 0.756824 | 0.612903 | 0.769231 | 0.974684 |
| SEQ ID NO:31 | Training set | 0.782481 | 0.68254 | 0.740741 | 0.887647 |
| SEQ ID NO:31 | Test set | 0.728288 | 0.709677 | 0.615385 | 0.887647 |
| SEQ ID NO:32 | Training set | 0.800412 | 0.698413 | 0.740741 | 0.9042 |
| SEQ ID NO:32 | Test set | 0.832506 | 0.806452 | 0.576923 | 0.9042 |
| SEQ ID NO:33 | Training set | 0.751029 | 0.634921 | 0.796296 | 9.37E-06 |
| SEQ ID NO:33 | Test set | 0.859801 | 0.677419 | 0.884615 | 9.37E-06 |
| SEQ ID NO:34 | Training set | 0.771311 | 0.634921 | 0.777778 | 0.808219 |
| SEQ ID NO:34 | Test set | 0.744417 | 0.612903 | 0.807692 | 0.808219 |
| SEQ ID NO:35 | Training set | 0.771605 | 0.587302 | 0.851852 | 0.793764 |
| SEQ ID NO:35 | Test set | 0.751861 | 0.645161 | 0.692308 | 0.793764 |
| SEQ ID NO:36 | Training set | 0.751323 | 0.761905 | 0.703704 | 0.001854 |
| SEQ ID NO:36 | Test set | 0.668114 | 0.677419 | 0.538462 | 0.001854 |
| SEQ ID NO:37 | Test set | 0.812655 | 0.83871 | 0.576923 | 0.028402 |
| SEQ ID NO:37 | Training set | 0.786302 | 0.84127 | 0.62963 | 0.028402 |
| SEQ ID NO:38 | Training set | 0.758377 | 0.698413 | 0.703704 | 0.960583 |
| SEQ ID NO:38 | Test set | 0.677419 | 0.709677 | 0.423077 | 0.960583 |
| SEQ ID NO:39 | Training set | 0.789536 | 0.698413 | 0.796296 | 0.941044 |
| SEQ ID NO:39 | Test set | 0.681141 | 0.709677 | 0.576923 | 0.941044 |
| SEQ ID NO:40 | Training set | 0.777484 | 0.714286 | 0.777778 | 0.892282 |
| SEQ ID NO:40 | Test set | 0.815136 | 0.677419 | 0.730769 | 0.892282 |
| SEQ ID NO:41 | Training set | 0.783069 | 0.634921 | 0.777778 | 0.752404 |
| SEQ ID NO:41 | Test set | 0.764268 | 0.709677 | 0.807692 | 0.752404 |
| SEQ ID NO:42 | Training set | 0.759553 | 0.698413 | 0.703704 | 0.663212 |
| SEQ ID NO:42 | Test set | 0.739454 | 0.612903 | 0.692308 | 0.663212 |
| SEQ ID NO:43 | Training set | 0.781599 | 0.714286 | 0.740741 | 0.030791 |
| SEQ ID NO:43 | Test set | 0.764268 | 0.741935 | 0.653846 | 0.030791 |
| SEQ ID NO:44 | Training set | 0.751029 | 0.714286 | 0.722222 | 0.428244 |

(continued)

| Sequence | Group | AUC value | Sensitivity | Specificity | Threshold |
|---|---|---|---|---|---|
| SEQ ID NO:44 | Test set | 0.715881 | 0.741935 | 0.576923 | 0.428244 |
| SEQ ID NO:45 | Training set | 0.774544 | 0.809524 | 0.648148 | 0.818533 |
| SEQ ID NO:45 | Test set | 0.751861 | 0.741935 | 0.423077 | 0.818533 |
| SEQ ID NO:46 | Test set | 0.823821 | 0.870968 | 0.615385 | 0.873866 |
| SEQ ID NO:46 | Training set | 0.784245 | 0.888889 | 0.555556 | 0.873866 |
| SEQ ID NO:47 | Training set | 0.776602 | 0.666667 | 0.777778 | 0.939612 |
| SEQ ID NO:47 | Test set | 0.797767 | 0.806452 | 0.538462 | 0.939612 |
| SEQ ID NO:48 | Training set | 0.751617 | 0.587302 | 0.796296 | 0.833123 |
| SEQ ID NO:48 | Test set | 0.753102 | 0.741935 | 0.615385 | 0.833123 |
| SEQ ID NO:49 | Training set | 0.787625 | 0.825397 | 0.666667 | 0.915698 |
| SEQ ID NO:49 | Test set | 0.725806 | 0.774194 | 0.576923 | 0.915698 |
| SEQ ID NO:50 | Training set | 0.803645 | 0.777778 | 0.740741 | 0.964413 |
| SEQ ID NO:50 | Test set | 0.817618 | 0.83871 | 0.615385 | 0.964413 |
| SEQ ID NO:51 | Training set | 0.767784 | 0.68254 | 0.703704 | 0.759093 |
| SEQ ID NO:51 | Test set | 0.800248 | 0.806452 | 0.615385 | 0.759093 |
| SEQ ID NO:52 | Training set | 0.754556 | 0.650794 | 0.740741 | 0.203289 |
| SEQ ID NO:52 | Test set | 0.765509 | 0.677419 | 0.692308 | 0.203289 |
| SEQ ID NO:53 | Training set | 0.773075 | 0.698413 | 0.777778 | 0.866077 |
| SEQ ID NO:53 | Test set | 0.705955 | 0.741935 | 0.576923 | 0.866077 |
| SEQ ID NO:54 | Training set | 0.771899 | 0.84127 | 0.611111 | 0.780937 |
| SEQ ID NO:54 | Test set | 0.80273 | 0.903226 | 0.5 | 0.780937 |
| SEQ ID NO:55 | Training set | 0.749706 | 0.571429 | 0.87037 | 0.712991 |
| SEQ ID NO:55 | Test set | 0.631514 | 0.516129 | 0.730769 | 0.712991 |
| SEQ ID NO:56 | Training set | 0.786302 | 0.746032 | 0.722222 | 0.901679 |
| SEQ ID NO:56 | Test set | 0.630243 | 0.645161 | 0.607692 | 0.901679 |

**1-3: Prediction model for the combination of all markers**

In order to verify the potential ability of differentiating pancreatic cancer using methylation nucleic acid fragment markers, a support vector machine disease classification model was constructed based on 56 methylation nucleic acid fragment markers in the training group to verify the classification prediction effect of this cluster of methylation markers in the test group. The training group and the test group were divided according to the proportion, including 117 samples in the training group (samples 1-117) and 57 samples in the test group (samples 118-174).
The discovered methylation markers were used to construct a support vector machine model in the training set for both groups of samples.

1) The samples were pre-divided into 2 parts, 1 part was used for training the model and 1 part was used for model testing.
2) The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().

b) The sklearn software package (0.23.1) is used to input the methylation value data matrix to construct the SVM model, model.fit(x_train, y_train), where x_train represents the training set data matrix, and y_train represents the phenotypic information of the training set.

In the process of constructing the model, the pancreatic cancer sample type was coded as 1 and the pancreatic cancer-free sample type was coded as 0. In the process of constructing the model by the sklearn software package (0.23.1), the threshold was set as 0.895 by default. The constructed model finally distinguished samples with or without pancreatic cancer by 0.895. The prediction scores of the two models for the training set samples are shown in Table 1-5.

**Table 1-5: Model prediction scores of the training set**

| Sample | Type | Score | Sample | Type | Score |
|---|---|---|---|---|---|
| Sample 1 | Without pancreatic cancer | 0.893229976 | Sample 60 | Pancreatic cancer | 0.895863768 |
| Sample 2 | Without pancreatic cancer | 0.895013223 | Sample 61 | Pancreatic cancer | 0.9049507 |
| Sample 3 | Pancreatic cancer | 0.894882888 | Sample 62 | Pancreatic cancer | 0.898486446 |
| Sample 4 | Without pancreatic cancer | 0.893934677 | Sample 63 | Pancreatic cancer | 0.895516215 |
| Sample 5 | Without pancreatic cancer | 0.896841445 | Sample 64 | Pancreatic cancer | 0.899627853 |
| Sample 6 | Pancreatic cancer | 0.896054017 | Sample 65 | Pancreatic cancer | 0.894139084 |
| Sample 7 | Without pancreatic cancer | 0.893751222 | Sample 66 | Pancreatic cancer | 0.896066317 |
| Sample 8 | Pancreatic cancer | 0.895249143 | Sample 67 | Pancreatic cancer | 0.895653768 |
| Sample 9 | Pancreatic cancer | 0.895766138 | Sample 68 | Pancreatic cancer | 0.894574595 |
| Sample 10 | Without pancreatic cancer | 0.893661796 | Sample 69 | Pancreatic cancer | 0.899534971 |
| Sample 11 | Without pancreatic cancer | 0.894065433 | Sample 70 | Pancreatic cancer | 0.894752391 |
| Sample 12 | Without pancreatic cancer | 0.894278734 | Sample 71 | Pancreatic cancer | 0.899581479 |
| Sample 13 | Without pancreatic cancer | 0.8940632 | Sample 72 | Without pancreatic cancer | 0.895978159 |
| Sample 14 | Without pancreatic cancer | 0.893459631 | Sample 73 | Pancreatic cancer | 0.895617753 |
| Sample 15 | Without pancreatic cancer | 0.892932686 | Sample 74 | Pancreatic cancer | 0.894835698 |
| Sample 16 | Without pancreatic cancer | 0.893522949 | Sample 75 | Pancreatic cancer | 0.902355179 |
| Sample 17 | Without pancreatic cancer | 0.893741741 | Sample 76 | Pancreatic cancer | 0.895694906 |
| Sample 18 | Without pancreatic cancer | 0.894510469 | Sample 77 | Pancreatic cancer | 0.899999679 |
| Sample 19 | Without pancreatic cancer | 0.893866355 | Sample 78 | Pancreatic cancer | 0.9 |
| Sample 20 | Without pancreatic cancer | 0.895936638 | Sample 79 | Pancreatic cancer | 0.895848252 |

(continued)

| Sample | Type | Score | Sample | Type | Score |
|--------|------|-------|--------|------|-------|
| Sample 21 | Pancreatic cancer | 0.894688627 | Sample 80 | Pancreatic cancer | 0.897055645 |
| Sample 22 | Without pancreatic cancer | 0.894744381 | Sample 81 | Pancreatic cancer | 0.896997761 |
| Sample 23 | Pancreatic cancer | 0.899065574 | Sample 82 | Pancreatic cancer | 0.913242766 |
| Sample 24 | Pancreatic cancer | 0.894525057 | Sample 83 | Pancreatic cancer | 0.895900127 |
| Sample 25 | Pancreatic cancer | 0.894148842 | Sample 84 | Pancreatic cancer | 0.906476534 |
| Sample 26 | Pancreatic cancer | 0.894788972 | Sample 85 | Pancreatic cancer | 0.895385103 |
| Sample 27 | Without pancreatic cancer | 0.894274243 | Sample 86 | Without pancreatic cancer | 0.89468141 |
| Sample 28 | Without pancreatic cancer | 0.893406552 | Sample 87 | Without pancreatic cancer | 0.892735928 |
| Sample 29 | Pancreatic cancer | 0.895308274 | Sample 88 | Without pancreatic cancer | 0.893463424 |
| Sample 30 | Pancreatic cancer | 0.894795724 | Sample 89 | Without pancreatic cancer | 0.89251894 |
| Sample 31 | Without pancreatic cancer | 0.893519373 | Sample 90 | Without pancreatic cancer | 0.893331026 |
| Sample 32 | Pancreatic cancer | 0.895663331 | Sample 91 | Without pancreatic cancer | 0.893676574 |
| Sample 33 | Pancreatic cancer | 0.89616556 | Sample 92 | Without pancreatic cancer | 0.893355406 |
| Sample 34 | Pancreatic cancer | 0.894924496 | Sample 93 | Without pancreatic cancer | 0.892959544 |
| Sample 35 | Pancreatic cancer | 0.896503989 | Sample 94 | Without pancreatic cancer | 0.893132053 |
| Sample 36 | Pancreatic cancer | 0.899846218 | Sample 95 | Without pancreatic cancer | 0.893066687 |
| Sample 37 | Pancreatic cancer | 0.895594069 | Sample 96 | Without pancreatic cancer | 0.894354059 |
| Sample 38 | Pancreatic cancer | 0.912591937 | Sample 97 | Without pancreatic cancer | 0.892774769 |
| Sample 39 | Pancreatic cancer | 0.896002353 | Sample 98 | Without pancreatic cancer | 0.892266834 |
| Sample 40 | Pancreatic cancer | 0.908621377 | Sample 99 | Without pancreatic cancer | 0.893527234 |
| Sample 41 | Pancreatic cancer | 0.894850957 | Sample 100 | Without pancreatic cancer | 0.895184905 |
| Sample 42 | Pancreatic cancer | 0.894635011 | Sample 101 | Without pancreatic cancer | 0.893879752 |

(continued)

| Sample | Type | Score | Sample | Type | Score |
|---|---|---|---|---|---|
| Sample 43 | Pancreatic cancer | 0.897641236 | Sample 102 | Pancreatic cancer | 0.895086351 |
| Sample 44 | Pancreatic cancer | 0.895222579 | Sample 103 | Without pancreatic cancer | 0.896114863 |
| Sample 45 | Pancreatic cancer | 0.894991146 | Sample 104 | Without pancreatic cancer | 0.893436647 |
| Sample 46 | Without pancreatic cancer | 0.894120714 | Sample 105 | Without pancreatic cancer | 0.894703614 |
| Sample 47 | Pancreatic cancer | 0.902993927 | Sample 106 | Without pancreatic cancer | 0.893431172 |
| Sample 48 | Pancreatic cancer | 0.899321375 | Sample 107 | Without pancreatic cancer | 0.894666164 |
| Sample 49 | Pancreatic cancer | 0.897291974 | Sample 108 | Without pancreatic cancer | 0.893551029 |
| Sample 50 | Pancreatic cancer | 0.897914688 | Sample 109 | Without pancreatic cancer | 0.893621581 |
| Sample 51 | Pancreatic cancer | 0.896104384 | Sample 110 | Without pancreatic cancer | 0.893681846 |
| Sample 52 | Pancreatic cancer | 0.903706446 | Sample 111 | Without pancreatic cancer | 0.894345935 |
| Sample 53 | Pancreatic cancer | 0.895571142 | Sample 112 | Without pancreatic cancer | 0.89320714 |
| Sample 54 | Pancreatic cancer | 0.894370774 | Sample 113 | Without pancreatic cancer | 0.895288114 |
| Sample 55 | Pancreatic cancer | 0.899277534 | Sample 114 | Without pancreatic cancer | 0.893867075 |
| Sample 56 | Pancreatic cancer | 0.897717628 | Sample 115 | Without pancreatic cancer | 0.893701906 |
| Sample 57 | Without pancreatic cancer | 0.893134404 | Sample 116 | Without pancreatic cancer | 0.894679507 |
| Sample 58 | Pancreatic cancer | 0.894710346 | Sample 117 | Without pancreatic cancer | 0.893167765 |
| Sample 59 | Pancreatic cancer | 0.894246115 | | | |

Based on the methylation nucleic acid fragment marker cluster of the present application, it was predicted in the test set according to the model established by SVM in this example. The test set was predicted using the prediction function to output the prediction result (disease probability: the default score threshold is 0.895, and if the score is greater than 0.895, the subject is considered malignant). The test group included 57 samples (samples 118-174), and the calculation process is as follows:
Command Line:

$$test\_pred = model.predict(test\_df)$$

where test_pred represents the prediction score of the samples in the test set obtained by using the SVM prediction model constructed in this example, model represents the SVM prediction model constructed in this example, and test_df represents the test set data.

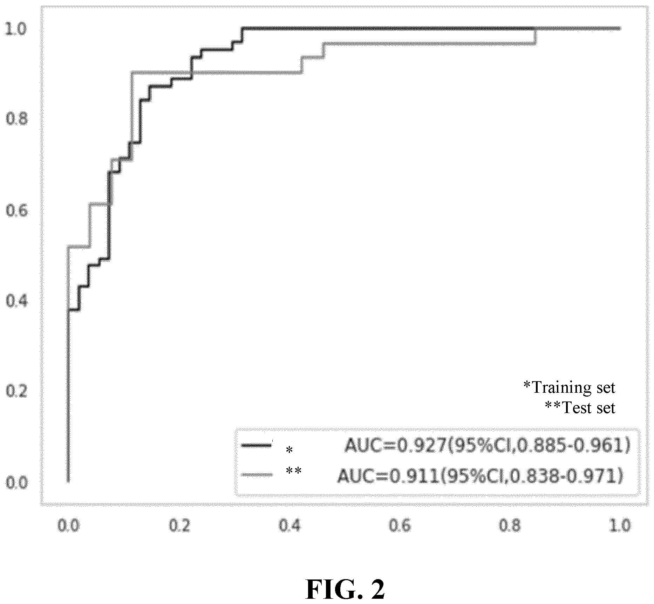

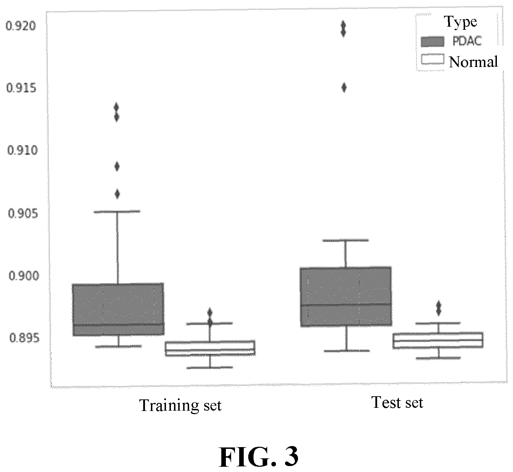

The prediction scores of the test group are shown in Table 1-6. The ROC curve is shown in Fig. 2. The prediction score distribution is shown in Fig. 3. The area under the overall AUC of the test group was 0.911. In the training set, the model's sensitivity could reach 71.4% when the specificity was 90.7%; in the test set, when the specificity was 88.5%, the sensitivity of the model could reach 83.9%. It can be seen that the differentiating effect of the SVM models established by the selected variables is good.

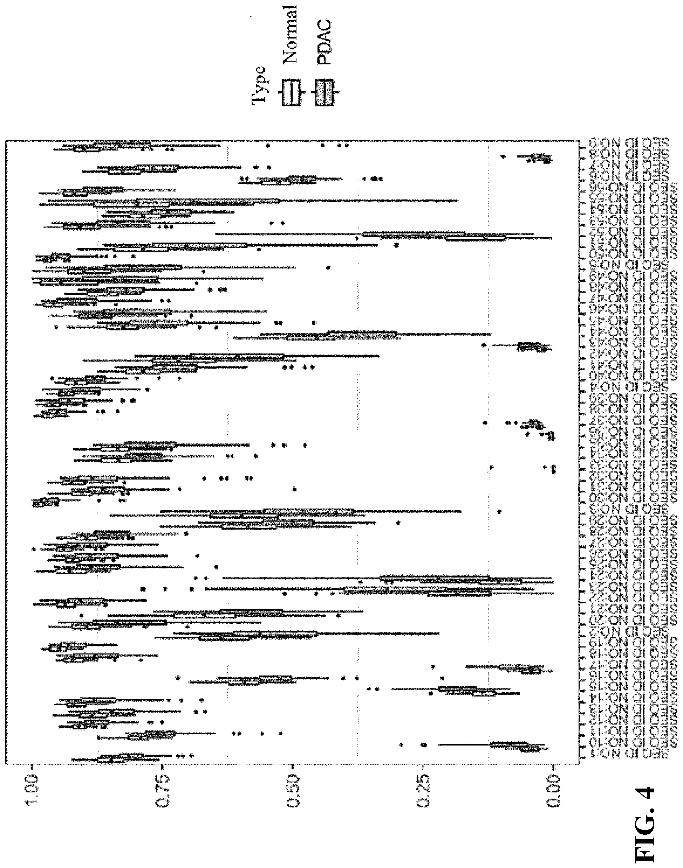

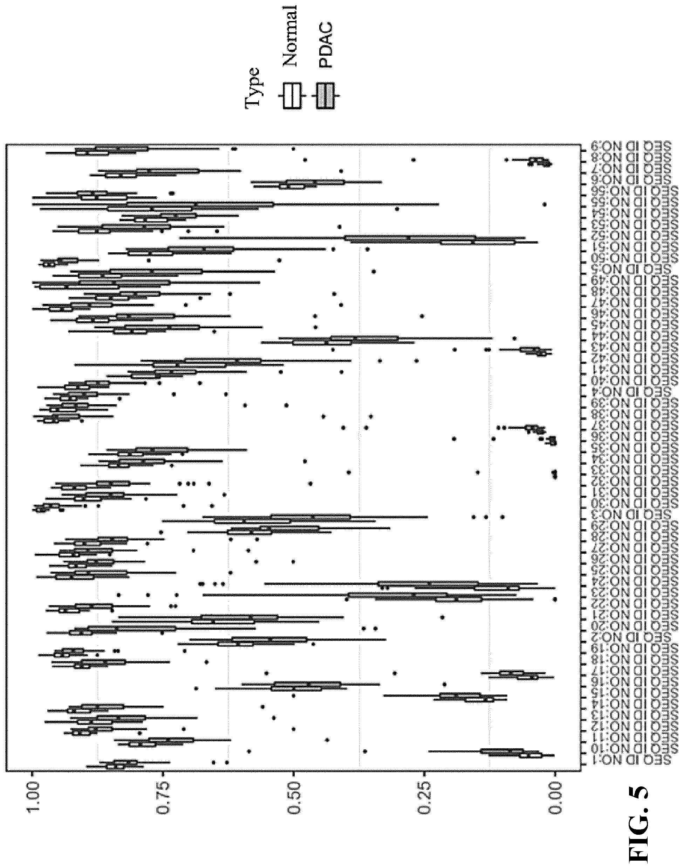

Figs. 4 and 5 show the distribution of the 56 methylation nucleic acid fragment markers in the training group and the test group respectively. It can be found that the difference of this cluster of methylation markers in the plasma of subjects without pancreatic cancer and the plasma of patients with pancreatic cancer was relatively stable.

**Table 1-6: Model prediction scores for test set samples**

| Sample | Type | Score | Sample | Type | Score |
|---|---|---|---|---|---|
| Sample 118 | Without pancreatic cancer | 0.892840415 | Sample 147 | Pancreatic cancer | 0.895445651 |
| Sample 119 | Without pancreatic cancer | 0.894808228 | Sample 148 | Pancreatic cancer | 0.896982419 |
| Sample 120 | Without pancreatic cancer | 0.893010572 | Sample 149 | Pancreatic cancer | 0.919640259 |
| Sample 121 | Without pancreatic cancer | 0.894819319 | Sample 150 | Pancreatic cancer | 0.902419155 |
| Sample 122 | Without pancreatic cancer | 0.896663158 | Sample 151 | Pancreatic cancer | 0.895090686 |
| Sample 123 | Without pancreatic cancer | 0.893419513 | Sample 152 | Pancreatic cancer | 0.897972041 |
| Sample 124 | Pancreatic cancer | 0.898460015 | Sample 153 | Pancreatic cancer | 0.897975186 |
| Sample 125 | Without pancreatic cancer | 0.894884278 | Sample 154 | Pancreatic cancer | 0.895608671 |
| Sample 126 | Pancreatic cancer | 0.895074685 | Sample 155 | Pancreatic cancer | 0.896923275 |
| Sample 127 | Without pancreatic cancer | 0.893856295 | Sample 156 | Pancreatic cancer | 0.919058207 |
| Sample 128 | Pancreatic cancer | 0.897375182 | Sample 157 | Pancreatic cancer | 0.914971841 |
| Sample 129 | Pancreatic cancer | 0.896724337 | Sample 158 | Pancreatic cancer | 0.89445029 |
| Sample 130 | Without pancreatic cancer | 0.895068998 | Sample 159 | Pancreatic cancer | 0.901561224 |
| Sample 131 | Without pancreatic cancer | 0.893616486 | Sample 160 | Pancreatic cancer | 0.894385595 |
| Sample 132 | Without pancreatic cancer | 0.894166762 | Sample 161 | Pancreatic cancer | 0.900253027 |
| Sample 133 | Without pancreatic cancer | 0.894683763 | Sample 162 | Pancreatic cancer | 0.895601176 |
| Sample 134 | Pancreatic cancer | 0.901640955 | Sample 163 | Without pancreatic cancer | 0.894637668 |

(continued)

| Sample | Type | Score | Sample | Type | Score |
|---|---|---|---|---|---|
| Sample 135 | Pancreatic cancer | 0.897357709 | Sample 164 | Without pancreatic cancer | 0.895669553 |
| Sample 136 | Pancreatic cancer | 0.893550856 | Sample 165 | Without pancreatic cancer | 0.894261195 |
| Sample 137 | Pancreatic cancer | 0.896530196 | Sample 166 | Without pancreatic cancer | 0.893549014 |
| Sample 138 | Without pancreatic cancer | 0.894001953 | Sample 167 | Without pancreatic cancer | 0.894968169 |
| Sample 139 | Pancreatic cancer | 0.897230848 | Sample 168 | Without pancreatic cancer | 0.897122587 |
| Sample 140 | Without pancreatic cancer | 0.893650349 | Sample 169 | Without pancreatic cancer | 0.894488706 |
| Sample 141 | Pancreatic cancer | 0.897730904 | Sample 170 | Without pancreatic cancer | 0.893611044 |
| Sample 142 | Pancreatic cancer | 0.895338332 | Sample 171 | Without pancreatic cancer | 0.894759854 |
| Sample 143 | Pancreatic cancer | 0.896436157 | Sample 172 | Without pancreatic cancer | 0.89405156 |
| Sample 144 | Pancreatic cancer | 0.90181511 | Sample 173 | Without pancreatic cancer | 0.894203576 |
| Sample 145 | Pancreatic cancer | 0.896206867 | Sample 174 | Without pancreatic cancer | 0.894115083 |
| Sample 146 | Pancreatic cancer | 0.900280003 | | | |

## 1-4: Tumor marker prediction comparison

Based on the methylation marker cluster of the present application, it was predicted in the test set according to the model established by SVM in Example 1-3. Pancreatic cancer was predicted based on the CA19-9 marker. There were 130 samples (Table 1-7). The calculation process is as follows:
Command Line:

$$\text{Combine\_scalar} = \text{RobustScaler}().\text{fit}(\text{combine\_train\_df})$$

$$\text{scaled\_combine\_train\_df} = \text{combine\_scalar}.\text{transform}(\text{combine\_train\_df})$$

$$\text{scaled\_combine\_test\_df} = \text{combine\_scalar}.\text{transform}(\text{combine\_test\_df})$$

$$\text{combine\_model} = \text{LogisticRegression}().\text{fit}(\text{scaled\_combine\_train\_df}, \text{train\_ca19\_pheno})$$

where combine_train_df represents the training set data matrix in which the prediction scores obtained by the SVM prediction model constructed in Example 1-3 of the test set samples are combined with CA19-9, and scaled_combine_train _df represents the training set data matrix after standardization. scaled_combine_test_df represents the standardized test set data matrix, and combine _model represents the logistic regression model fitted using the standardized training set data matrix.

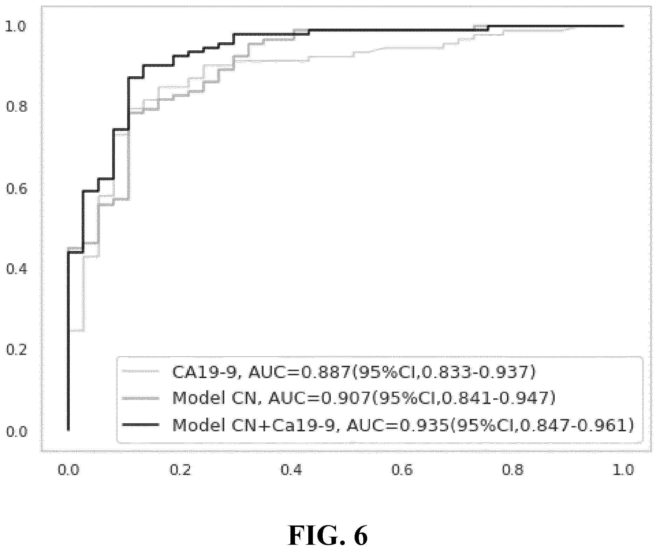

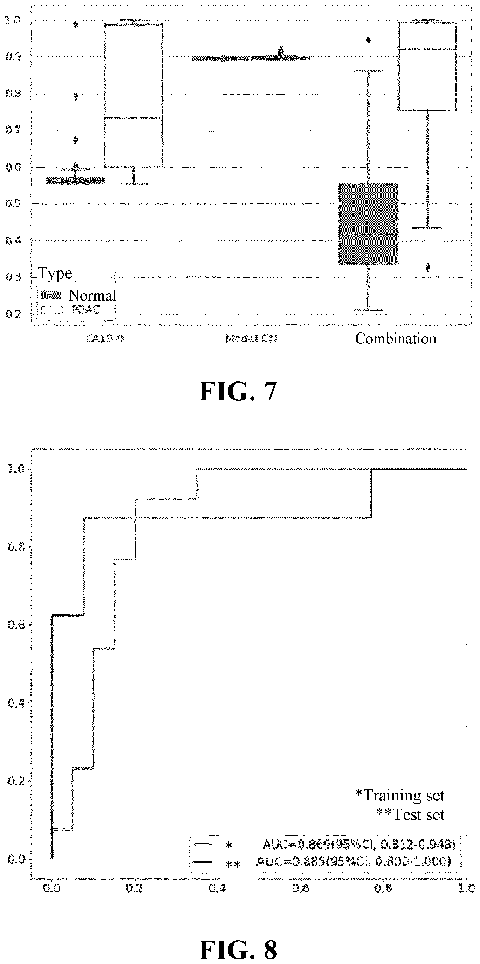

The prediction scores of the samples are shown in Table 1-7. The ROC curve is shown in Fig. 6. The prediction score distribution is shown in Fig. 7. The overall AUC of the test group is 0.935. It can be seen from the figure that the differentiating effect of the established logistic regression models is good.

Fig. 7 shows the distribution of classification prediction scores of the SVM model constructed using CA19-9 alone, using Example 3 alone, and the model constructed in Example 3 combined with CA19-9. It can be found that this method is more stably in the identification of pancreatic cancer.

**Table 1-7: Prediction scores of CA19-9 and prediction scores of the model combined with CA19-9**

| Sample | Type | CA19-9 measurement value | Model CN | Model CN combined with CA19-9 |
|---|---|---|---|---|
| Sample 1 | Without pancreatic cancer | 1 | 0.893229 976 | 0.26837584 |
| Sample 2 | Without pancreatic cancer | 1 | 0.895013 223 | 0.598167417 |
| Sample 3 | Without pancreatic cancer | 1 | 0.892840 415 | 0.212675448 |
| Sample 4 | Pancreatic cancer | 2 | 0.894882 888 | 0.573802169 |
| Sample 5 | Without pancreatic cancer | 2 | 0.893934 677 | 0.389973233 |
| Sample 6 | Without pancreatic cancer | 2.38 | 0.896841 445 | 0.862537633 |
| Sample 7 | Without pancreatic cancer | 2.6 | 0.894808 228 | 0.559686301 |
| Sample 8 | Without pancreatic cancer | 2.73 | 0.893010 572 | 0.236512984 |
| Sample 9 | Without pancreatic cancer | 3.09 | 0.894819 319 | 0.562063886 |
| Sample 10 | Pancreatic cancer | 3.17 | 0.896054 017 | 0.771981439 |
| Sample 11 | Without pancreatic cancer | 3.3 | 0.893751 222 | 0.356857798 |
| Sample 12 | Without pancreatic cancer | 3.65 | 0.896663 158 | 0.845394585 |
| Sample 13 | Pancreatic cancer | 3.8 | 0.895249 143 | 0.643027155 |
| Sample 14 | Without pancreatic cancer | 4.16 | 0.893419 513 | 0.299867684 |
| Sample 15 | Pancreatic cancer | 4.19 | 0.895766 138 | 0.730147078 |
| Sample 16 | Without pancreatic cancer | 4.41 | 0.893661 796 | 0.341382822 |
| Sample 17 | Pancreatic cancer | 4.61 | 0.898460 015 | 0.957392228 |
| Sample 18 | Without pancreatic cancer | 4.63 | 0.894065 433 | 0.415890987 |
| Sample 19 | Without pancreatic cancer | 4.8 | 0.894278 734 | 0.457156964 |
| Sample 20 | Without pancreatic cancer | 4.88 | 0.894884 278 | 0.575421664 |
| Sample 21 | Without pancreatic cancer | 6.4 | 0.894063 2 | 0.416291096 |

(continued)

| Sample | Type | CA19-9 measurement value | Model CN | Model CN combined with CA19-9 |
|---|---|---|---|---|
| Sample 22 | Without pancreatic cancer | 7 | 0.893459 631 | 0.307686129 |
| Sample 23 | Pancreatic cancer | 7 | 0.895074 685 | 0.612454757 |
| Sample 24 | Without pancreatic cancer | 7.15 | 0.893856 295 | 0.377752923 |
| Sample 25 | Pancreatic cancer | 7.41 | 0.897375 182 | 0.905973775 |
| Sample 26 | Without pancreatic cancer | 7.44 | 0.892932 686 | 0.227229577 |
| Sample 27 | Without pancreatic cancer | 8.6 | 0.893522 949 | 0.319048291 |
| Sample 28 | Without pancreatic cancer | 9.57 | 0.893741 741 | 0.357914549 |
| Sample 29 | Pancreatic cancer | 10.29 | 0.896724 337 | 0.853177242 |
| Sample 30 | Without pancreatic cancer | 11 | 0.895068 998 | 0.613218554 |
| Sample 31 | Without pancreatic cancer | 11.28 | 0.894510 469 | 0.505670555 |
| Sample 32 | Without pancreatic cancer | 12.78 | 0.893866 355 | 0.382163129 |
| Sample 33 | Without pancreatic cancer | 12.8 | 0.895936 638 | 0.758750029 |
| Sample 34 | Without pancreatic cancer | 13 | 0.893616 486 | 0.337104932 |
| Sample 35 | Pancreatic cancer | 14.05 | 0.894688 627 | 0.541888157 |
| Sample 36 | Without pancreatic cancer | 14.79 | 0.894166 762 | 0.440150986 |
| Sample 37 | Without pancreatic cancer | 15.65 | 0.894744 381 | 0.553498095 |
| Sample 38 | Pancreatic cancer | 18.14 | 0.899065 574 | 0.973758788 |
| Sample 39 | Pancreatic cancer | 18.47 | 0.894525 057 | 0.511987142 |
| Sample 40 | Pancreatic cancer | 20 | 0.894148 842 | 0.439149676 |
| Sample 41 | Without pancreatic cancer | 20.41 | 0.894683 763 | 0.543972765 |
| Sample 42 | Pancreatic cancer | 21 | 0.901640 955 | 0.996467645 |
| Sample 43 | Pancreatic cancer | 21.13 | 0.894788 972 | 0.56472723 |
| Sample 44 | Without pancreatic cancer | 22 | 0.894274 243 | 0.464492285 |
| Sample 45 | Without pancreatic cancer | 23.56 | 0.893406 552 | 0.305587252 |
| Sample 46 | Pancreatic cancer | 23.57 | 0.895308 274 | 0.66216627 |
| Sample 47 | Pancreatic cancer | 24.1 | 0.897357 709 | 0.907524955 |
| Sample 48 | Pancreatic cancer | 24.26 | 0.894795 724 | 0.567507228 |

(continued)

| Sample | Type | CA19-9 measurement value | Model CN | Model CN combined with CA19-9 |
|---|---|---|---|---|
| Sample 49 | Without pancreatic cancer | 24.67 | 0.893519 373 | 0.325177468 |
| Sample 50 | Pancreatic cancer | 24.78 | 0.893550 856 | 0.330674117 |
| Sample 51 | Pancreatic cancer | 30 | 0.896530 196 | 0.838230387 |
| Sample 52 | Without pancreatic cancer | 32.67 | 0.894001 953 | 0.416867288 |
| Sample 53 | Pancreatic cancer | 33.99 | 0.895663 331 | 0.72549358 |
| Sample 54 | Pancreatic cancer | 35 | 0.896165 56 | 0.79710724 |
| Sample 55 | Pancreatic cancer | 37.78 | 0.894924 496 | 0.598403217 |
| Sample 56 | Pancreatic cancer | 39.08 | 0.896503 989 | 0.837804472 |
| Sample 57 | Pancreatic cancer | 41.74 | 0.897230 848 | 0.901857032 |
| Sample 58 | Pancreatic cancer | 42.44 | 0.899846 218 | 0.986261372 |
| Sample 59 | Without pancreatic cancer | 46.07 | 0.893650 349 | 0.357535251 |
| Sample 60 | Pancreatic cancer | 52.11 | 0.895594 069 | 0.721575695 |
| Sample 61 | Pancreatic cancer | 52.64 | 0.897730 904 | 0.932877977 |
| Sample 62 | Pancreatic cancer | 54.62 | 0.912591 937 | 0.999999389 |
| Sample 63 | Pancreatic cancer | 55.9 | 0.895338 332 | 0.68107056 |
| Sample 64 | Pancreatic cancer | 59 | 0.896002 353 | 0.783508748 |
| Sample 65 | Pancreatic cancer | 63.8 | 0.896436 157 | 0.837017436 |
| Sample 66 | Pancreatic cancer | 66.68 | 0.901815 11 | 0.997176145 |
| Sample 67 | Pancreatic cancer | 67.3 | 0.908621 377 | 0.999986519 |
| Sample 68 | Pancreatic cancer | 72.52 | 0.894850 957 | 0.60056185 |
| Sample 69 | Pancreatic cancer | 86 | 0.896206 867 | 0.817388937 |
| Sample 70 | Pancreatic cancer | 91.9 | 0.894635 011 | 0.568423992 |
| Sample 71 | Pancreatic cancer | 93.7 | 0.897641 236 | 0.933406107 |
| Sample 72 | Pancreatic cancer | 101.1 | 0.895222 579 | 0.68018633 |
| Sample 73 | Pancreatic cancer | 106 | 0.894991 146 | 0.64158648 |
| Sample 74 | Without pancreatic cancer | 108.46 | 0.894120 714 | 0.475836853 |
| Sample 75 | Pancreatic cancer | 115.6 | 0.902993 927 | 0.998979834 |
| Sample 76 | Pancreatic cancer | 129.1 | 0.899321 375 | 0.982501294 |
| Sample 77 | pancreatic cancer | 130.68 | 0.897291 974 | 0.919601629 |
| Sample 78 | Pancreatic cancer | 135 | 0.900280 003 | 0.991774857 |
| Sample 79 | Pancreatic cancer | 137 | 0.897914 688 | 0.949703939 |
| Sample 80 | Pancreatic cancer | 143.77 | 0.896104 384 | 0.821898703 |
| Sample 81 | Pancreatic cancer | 144 | 0.903706 446 | 0.999447782 |
| Sample 82 | Pancreatic cancer | 168.47 | 0.895571 142 | 0.760946078 |

(continued)

| Sample | Type | CA19-9 measurement value | Model CN | Model CN combined with CA19-9 |
|--------|------|--------------------------|----------|-------------------------------|
| Sample 83 | Pancreatic cancer | 176 | 0.894370 774 | 0.557117459 |
| Sample 84 | Pancreatic cancer | 177.5 | 0.899277 534 | 0.983480246 |
| Sample 85 | Pancreatic cancer | 186 | 0.895445 651 | 0.748943699 |
| Sample 86 | Pancreatic cancer | 188.1 | 0.897717 628 | 0.946930642 |
| Sample 87 | Pancreatic cancer | 220.5 | 0.896982 419 | 0.914228079 |
| Sample 88 | Pancreatic cancer | 224 | 0.919640 259 | 0.999999998 |
| Sample 89 | Without pancreatic cancer | 240.42 | 0.893134 404 | 0.350260722 |
| Sample 90 | Pancreatic cancer | 262.77 | 0.894710 346 | 0.659918805 |
| Sample 91 | Pancreatic cancer | 336.99 | 0.894246 115 | 0.608474115 |
| Sample 92 | Pancreatic cancer | 343.9 | 0.902419 155 | 0.99896672 |
| Sample 93 | Pancreatic cancer | 373.2 | 0.895090 686 | 0.763845583 |
| Sample 94 | Pancreatic cancer | 440.56 | 0.895863 768 | 0.871081972 |
| Sample 95 | Pancreatic cancer | 482.61 | 0.904950 7 | 0.999891539 |
| Sample 96 | Pancreatic cancer | 488 | 0.898486 446 | 0.983073316 |
| Sample 97 | Pancreatic cancer | 535 | 0.895516 215 | 0.860450015 |
| Sample 98 | Pancreatic cancer | 612 | 0.899627 853 | 0.994495239 |
| Sample 99 | Pancreatic cancer | 614.32 | 0.894139 084 | 0.708835044 |
| Sample 100 | Pancreatic cancer | 670 | 0.896066 317 | 0.924877247 |
| Sample 101 | Pancreatic cancer | 683.78 | 0.895653 768 | 0.90140781 |
| Sample 102 | Pancreatic cancer | 685.45 | 0.894574 595 | 0.797137754 |
| Sample 103 | Pancreatic cancer | 768.08 | 0.897972 041 | 0.985166479 |
| Sample 104 | Pancreatic cancer | 771 | 0.899534 971 | 0.995632513 |
| Sample 105 | Pancreatic cancer | 836.06 | 0.894752 391 | 0.857851677 |
| Sample 106 | Pancreatic cancer | 849 | 0.899581 479 | 0.996372589 |
| Sample 107 | Without pancreatic cancer | 890 | 0.895978 159 | 0.946039423 |
| Sample 108 | Pancreatic cancer | 974 | 0.895617 753 | 0.939479671 |
| Sample 109 | Pancreatic cancer | 1149.48 | 0.894835 698 | 0.92166929 |
| Sample 110 | Pancreatic cancer | 1200 | 0.902355 179 | 0.99979012 |
| Sample 111 | Pancreatic cancer | 1200 | 0.895694 906 | 0.962211074 |
| Sample 112 | Pancreatic cancer | 1200 | 0.899999 679 | 0.99866642 |
| Sample 113 | Pancreatic cancer | 1200 | 0.9 | 0.998666756 |
| Sample 114 | Pancreatic cancer | 1200 | 0.895848 252 | 0.966355074 |
| Sample 115 | Pancreatic cancer | 1200 | 0.897055 645 | 0.986692867 |
| Sample 116 | Pancreatic cancer | 1200 | 0.896997 761 | 0.986082478 |
| Sample 117 | Pancreatic cancer | 1200 | 0.913242 766 | 0.999999959 |
| Sample 118 | Pancreatic cancer | 1200 | 0.895900 127 | 0.967655005 |

(continued)

| Sample | Type | CA19-9 measurement value | Model CN | Model CN combined with CA19-9 |
|---|---|---|---|---|
| Sample 119 | Pancreatic cancer | 1200 | 0.906476 534 | 0.999991756 |
| Sample 120 | Pancreatic cancer | 1200 | 0.895385 103 | 0.952296514 |
| Sample 121 | Pancreatic cancer | 1200 | 0.897975 186 | 0.993492974 |
| Sample 122 | Pancreatic cancer | 1200 | 0.895608 671 | 0.959669541 |
| Sample 123 | Pancreatic cancer | 1200 | 0.896923 275 | 0.985256265 |
| Sample 124 | Pancreatic cancer | 1200 | 0.919058 207 | 1 |
| Sample 125 | Pancreatic cancer | 1200 | 0.914971 841 | 0.99999999 |
| Sample 126 | Pancreatic cancer | 1200 | 0.894450 29 | 0.905474598 |
| Sample 127 | Pancreatic cancer | 1200 | 0.901561 224 | 0.999608496 |
| Sample 128 | Pancreatic cancer | 1200 | 0.894385 595 | 0.901034637 |
| Sample 129 | Pancreatic cancer | 1200 | 0.900253 027 | 0.998906803 |
| Sample 130 | Pancreatic cancer | 1200 | 0.895601 176 | 0.999999989 |

**1-5: Performance of classification prediction model in negative samples of traditional markers**

Based on the methylation marker cluster of the present application, the test was performed on samples that were negative for the traditional tumor marker CA19-9 (CA19-9 measurement value < 37) according to the model established by SVM in Example 1-3.

The CA19-9 measurements and model prediction values of relevant samples are shown in Table 1-8, and the ROC curve is shown in Fig. 8. Also using 0.895 as the scoring threshold, the AUC value in the test set reached 0.885. It can be seen that for patients who cannot be distinguished using CA19-9, the SVM model constructed in Example 3 can still achieve relatively good results.

**Table 1-8: CA19-9 measurements and prediction scores of SVM model**

| Sample | Type | CA19-9 measurement value | Model CN |
|---|---|---|---|
| Sample 1 | Without pancreatic cancer | 1 | 0.893229976 |
| Sample 2 | Without pancreatic cancer | 1 | 0.895013223 |
| Sample 3 | Without pancreatic cancer | 1 | 0.892840415 |
| Sample 4 | Pancreatic cancer | 2 | 0.894882888 |
| Sample 5 | Without pancreatic cancer | 2 | 0.893934677 |
| Sample 6 | Without pancreatic cancer | 2.38 | 0.896841445 |
| Sample 7 | Without pancreatic cancer | 2.6 | 0.894808228 |
| Sample 8 | Without pancreatic cancer | 2.73 | 0.893010572 |
| Sample 9 | Without pancreatic cancer | 3.09 | 0.894819319 |
| Sample 10 | Pancreatic cancer | 3.17 | 0.896054017 |
| Sample 11 | Without pancreatic cancer | 3.3 | 0.893751222 |
| Sample 12 | Without pancreatic cancer | 3.65 | 0.896663158 |
| Sample 13 | Pancreatic cancer | 3.8 | 0.895249143 |
| Sample 14 | Without pancreatic cancer | 4.16 | 0.893419513 |
| Sample 15 | Pancreatic cancer | 4.19 | 0.895766138 |

(continued)

| Sample | Type | CA19-9 measurement value | Model CN |
|---|---|---|---|
| Sample 16 | Without pancreatic cancer | 4.41 | 0.893661796 |
| Sample 17 | Pancreatic cancer | 4.61 | 0.898460015 |
| Sample 18 | Without pancreatic cancer | 4.63 | 0.894065433 |
| Sample 19 | Without pancreatic cancer | 4.8 | 0.894278734 |
| Sample 20 | Without pancreatic cancer | 4.88 | 0.894884278 |
| Sample 21 | Without pancreatic cancer | 6.4 | 0.8940632 |
| Sample 22 | Without pancreatic cancer | 7 | 0.893459631 |
| Sample 23 | Pancreatic cancer | 7 | 0.895074685 |
| Sample 24 | Without pancreatic cancer | 7.15 | 0.893856295 |
| Sample 25 | Pancreatic cancer | 7.41 | 0.897375182 |
| Sample 26 | Without pancreatic cancer | 7.44 | 0.892932686 |
| Sample 27 | Without pancreatic cancer | 8.6 | 0.893522949 |
| Sample 28 | Without pancreatic cancer | 9.57 | 0.893741741 |
| Sample 29 | Pancreatic cancer | 10.29 | 0.896724337 |
| Sample 30 | Without pancreatic cancer | 11 | 0.895068998 |
| Sample 31 | Without pancreatic cancer | 11.28 | 0.894510469 |
| Sample 32 | Without pancreatic cancer | 12.78 | 0.893866355 |
| Sample 33 | Without pancreatic cancer | 12.8 | 0.895936638 |
| Sample 34 | Without pancreatic cancer | 13 | 0.893616486 |
| Sample 35 | Pancreatic cancer | 14.05 | 0.894688627 |
| Sample 36 | Without pancreatic cancer | 14.79 | 0.894166762 |
| Sample 37 | Without pancreatic cancer | 15.65 | 0.894744381 |
| Sample 38 | Pancreatic cancer | 18.14 | 0.899065574 |
| Sample 39 | Pancreatic cancer | 18.47 | 0.894525057 |
| Sample 40 | Pancreatic cancer | 20 | 0.894148842 |
| Sample 41 | Without pancreatic cancer | 20.41 | 0.894683763 |
| Sample 42 | Pancreatic cancer | 21 | 0.901640955 |
| Sample 43 | Pancreatic cancer | 21.13 | 0.894788972 |
| Sample 44 | Without pancreatic cancer | 22 | 0.894274243 |
| Sample 45 | Without pancreatic cancer | 23.56 | 0.893406552 |
| Sample 46 | Pancreatic cancer | 23.57 | 0.895308274 |
| Sample 47 | Pancreatic cancer | 24.1 | 0.897357709 |
| Sample 48 | Pancreatic cancer | 24.26 | 0.894795724 |
| Sample 49 | Without pancreatic cancer | 24.67 | 0.893519373 |
| Sample 50 | Pancreatic cancer | 24.78 | 0.893550856 |
| Sample 51 | Pancreatic cancer | 30 | 0.896530196 |
| Sample 52 | Without pancreatic cancer | 32.67 | 0.894001953 |
| Sample 53 | Pancreatic cancer | 33.99 | 0.895663331 |

(continued)

| Sample | Type | CA19-9 measurement value | Model CN |
|---|---|---|---|
| Sample 54 | Pancreatic cancer | 35 | 0.89616556 |

**1-6: Model construction and performance evaluation of the combination of 7 markers SEQ ID NOs: 9, 14, 13, 26, 40, 43, 52**

In order to verify the prediction performance of the combination of different markers, based on the cluster of 56 methylation markers in the present application, 7 markers SEQ ID NOs: 9, 14, 13, 26, 40, 43, 52 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.
2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().
b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

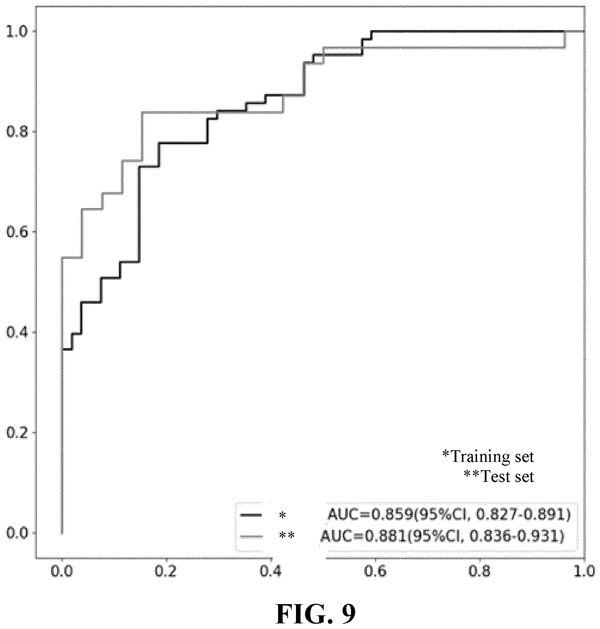

The ROC curve of this 7-marker combination model is shown in Fig. 9. The AUC of the constructed model was 0.881. In the test set, when the specificity was 0.846, the sensitivity could reach 0.774 (Table 1-9), achieving a good differentiating effect for patients with pancreatic cancer and healthy people.

**Table 1-9: Performance of the 7-marker combination model**

| Group | AUC value | Sensitivity | Specificity | Threshold |
|---|---|---|---|---|
| Training set | 0.8586 | 0.7302 | 0.8519 | 0.5786 |
| Test set | 0.8809 | 0.7742 | 0.8462 | 0.5786 |

**1-7: Model construction and performance evaluation of the combination of 7 markers SEQ ID NOs: 5, 18, 34, 40, 43, 45, 46**

In order to verify the prediction performance of the combination of different markers, based on the cluster of 56 methylation markers in the present application, 7 markers SEQ ID NOs: 5, 18, 34, 40, 43, 45, 46 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.
2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().

b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

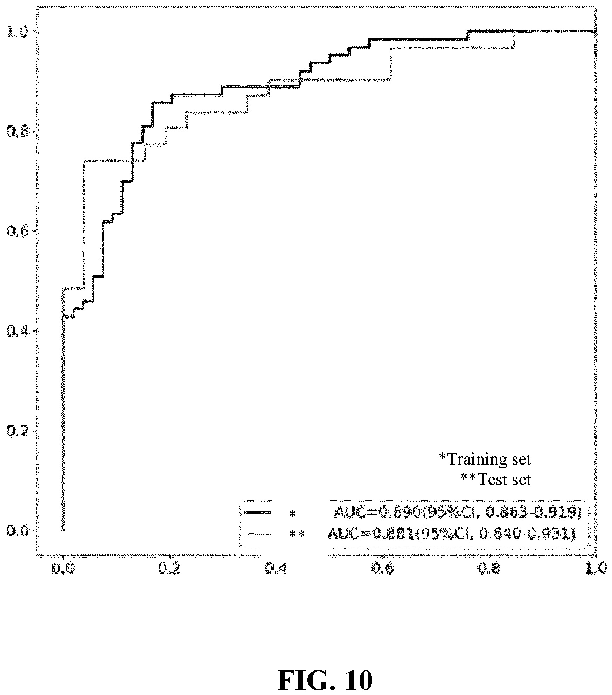

The ROC curve of this 7-marker combination model is shown in Fig. 10. The AUC of the constructed model was 0.881. In the test set, when the specificity was 0.692, the sensitivity could reach 0.839 (Table 1-10), achieving a good differentiating effect for patients with pancreatic cancer and healthy people.

**Table 1-10: Performance of the 7-marker combination model**

| Group | AUC value | Sensitivity | Specificity | Threshold |
|---|---|---|---|---|
| Training set | 0.8898 | 0.8095 | 0.8519 | 0.4179 |
| Test set | 0.8809 | 0.8387 | 0.6923 | 0.4179 |

**1-8: Model construction and performance evaluation of the combination of 7 markers SEQ ID NOs: 8, 11, 20, 44, 48, 51, 54**

In order to verify the prediction performance of the combination of different markers, based on the cluster of 56 methylation markers in the present application, 7 markers SEQ ID NOs: 8, 11, 20, 44, 48, 51, 54 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.

2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().

b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

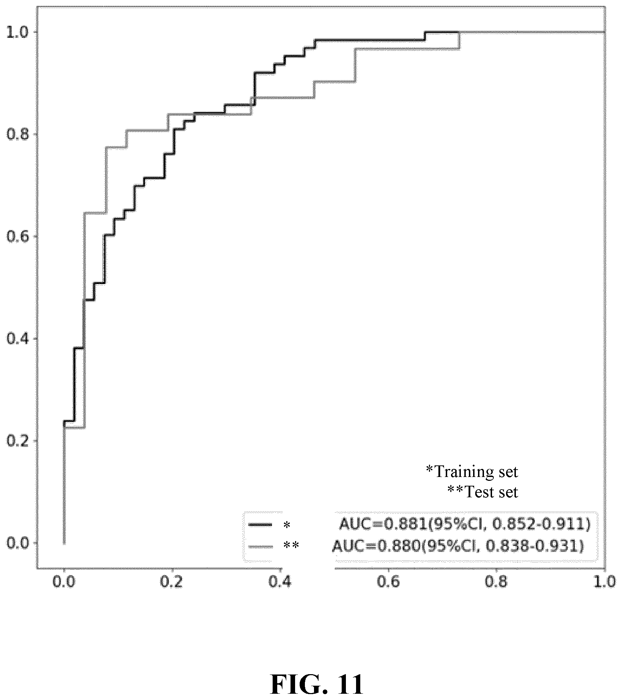

The ROC curve of this 7-marker combination model is shown in Fig. 11. The AUC of the constructed model was 0.880. In the test set, when the specificity was 0.769, the sensitivity could reach 0.839 (Table 1-11), achieving a good differentiating effect for patients with pancreatic cancer and healthy people.

**Table 1-11: Performance of the 7-marker combination model**

| Group | AUC value | Sensitivity | Specificity | Threshold |
|---|---|---|---|---|
| Training set | 0.8812 | 0.7143 | 0.8519 | 0.4434 |
| Test set | 0.8797 | 0.8387 | 0.7692 | 0.4434 |

**1-9: Model construction and performance evaluation of the combination of 7 markers SEQ ID NOs: 8, 14, 26, 24, 31, 40, 46**

In order to verify the prediction performance of the combination of different markers, based on the cluster of 56 methylation markers in the present application, 7 markers SEQ ID NOs: 8, 14, 26, 24, 31, 40, 46 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.
2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().
b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

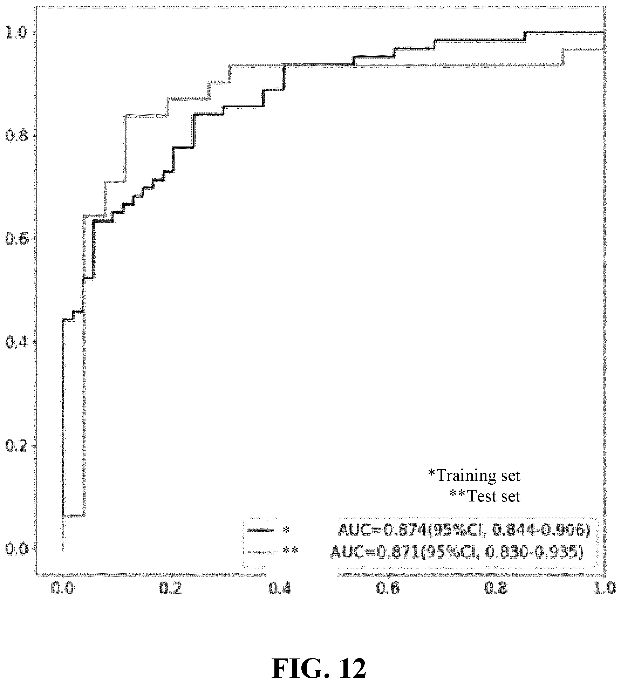

The ROC curve of this 7-marker combination model is shown in Fig. 12. The AUC of the constructed model was 0.871. In the test set, when the specificity was 0.885, the sensitivity could reach 0.710 (Table 1-12), achieving a good differentiating effect for patients with pancreatic cancer and healthy people.

**Table 1-12: Performance of the 7-marker combination model**

| Group | AUC value | Sensitivity | Specificity | Threshold |
|---|---|---|---|---|
| Training set | 0.8745 | 0.6984 | 0.8519 | 0.5380 |
| Test set | 0.8710 | 0.7097 | 0.8846 | 0.5380 |

**1-10: Model construction and performance evaluation of the combination of 7 markers SEQ ID NOs: 3, 9, 8, 29, 42, 40, 41**

In order to verify the prediction performance of the combination of different markers, based on the cluster of 56 methylation markers in the present application, 7 markers SEQ ID NOs: 3, 9, 8, 29, 42, 40, 41 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.
2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().
b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

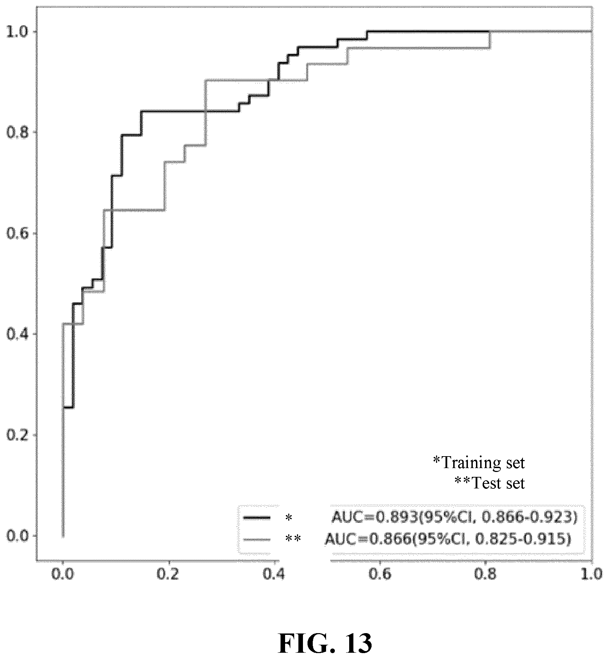

The ROC curve of this 7-marker combination model is shown in Fig. 13. The AUC of the constructed model was 0.866. In the test set, when the specificity was 0.538, the sensitivity could reach 0.903 (Table 1-13), achieving a good differentiating effect for patients with pancreatic cancer and healthy people.

**Table 1-13: Performance of the 7-marker combination model**

| Group | AUC value | Sensitivity | Specificity | Threshold |
|---|---|---|---|---|
| Training set | 0.8930 | 0.8413 | 0.8519 | 0.4014 |
| Test set | 0.8660 | 0.9032 | 0.5385 | 0.4014 |

**1-11: Model construction and performance evaluation of the combination of 7 markers SEQ ID NOs: 5, 8, 19, 7, 44, 47, 53**

In order to verify the prediction performance of the combination of different markers, based on the cluster of 56 methylation markers in the present application, 7 markers SEQ ID NOs: 5, 8, 19, 7, 44, 47, 53 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.
2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().
b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

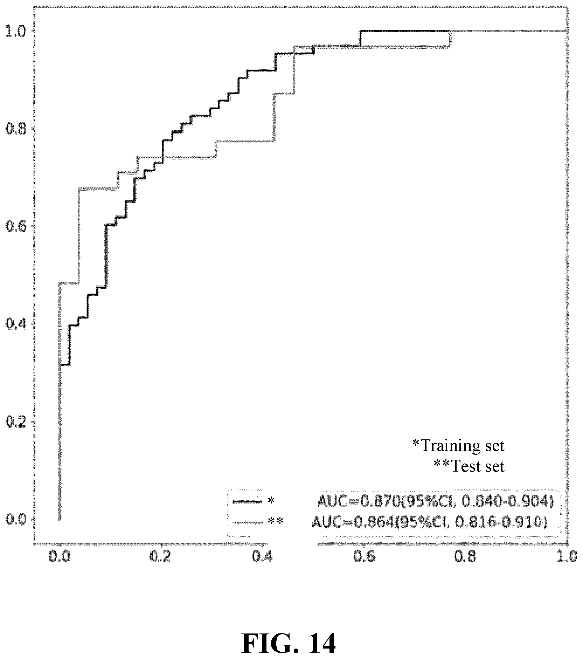

The ROC curve of this 7-marker combination model is shown in Fig. 14. The AUC of the constructed model was 0.864. In the test set, when the specificity was 0.577, the sensitivity could reach 0.774 (Table 1-14), achieving a good differentiating effect for patients with pancreatic cancer and healthy people.

**Table 1-14: Performance of the 7-marker combination model**

| Group | AUC value | Sensitivity | Specificity | Threshold |
|---|---|---|---|---|
| Training set | 0.8704 | 0.6984 | 0.8519 | 0.4803 |
| Test set | 0.8635 | 0.7742 | 0.5769 | 0.4803 |

**1-12: Model construction and performance evaluation of the combination of 7 markers SEQ ID NOs: 12, 17, 24, 28, 40, 42, 47**

In order to verify the prediction performance of the combination of different markers, based on the cluster of 56 methylation markers in the present application, 7 markers SEQ ID NOs: 12, 17, 24, 28, 40, 42, 47 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.

2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().

b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

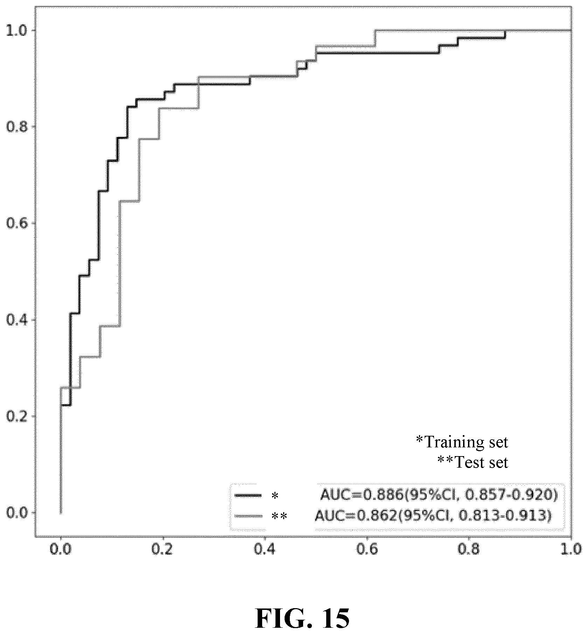

The ROC curve of this 7-marker combination model is shown in Fig. 15. The AUC of the constructed model was 0.862. In the test set, when the specificity was 0.731, the sensitivity could reach 0.871 (Table 1-15), achieving a good differentiating effect for patients with pancreatic cancer and healthy people.

**Table 1-15: Performance of the 7-marker combination model**

| Group | AUC value | Sensitivity | Specificity | Threshold |
|---|---|---|---|---|
| Training set | 0.8859 | 0.8571 | 0.8519 | 0.4514 |
| Test set | 0.8623 | 0.8710 | 0.7308 | 0.4514 |

**1-13: Model construction and performance evaluation of the combination of 7 markers SEQ ID NOs: 5, 18, 14, 10, 8, 19, 27**

In order to verify the prediction performance of the combination of different markers, based on the cluster of 56 methylation markers in the present application, 7 markers SEQ ID NOs: 5, 18, 14, 10, 8, 19, 27 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.

2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().

b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

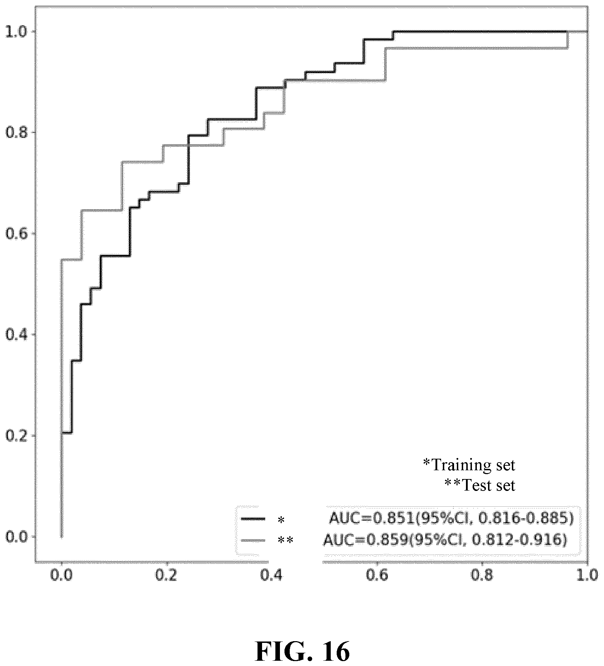

The ROC curve of this 7-marker combination model is shown in Fig. 16. The AUC of the constructed model was 0.859. In the test set, when the specificity was 0.615, the sensitivity could reach 0.839 (Table 1-16), achieving a good differentiating effect for patients with pancreatic cancer and healthy people.

**Table 1-16: Performance of the 7-marker combination model**

| Group | AUC value | Sensitivity | Specificity | Threshold |
|---|---|---|---|---|
| Training set | 0.8510 | 0.6667 | 0.8519 | 0.4124 |
| Test set | 0.8586 | 0.8387 | 0.6154 | 0.4124 |

**1-14: Model construction and performance evaluation of the combination of 7 markers SEQ ID NOs: 6, 12, 20, 26, 24, 47, 50**

In order to verify the prediction performance of the combination of different markers, based on the cluster of 56 methylation markers in the present application, 7 markers SEQ ID NOs: 6, 12, 20, 26, 24, 47, 50 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.
2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().
b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

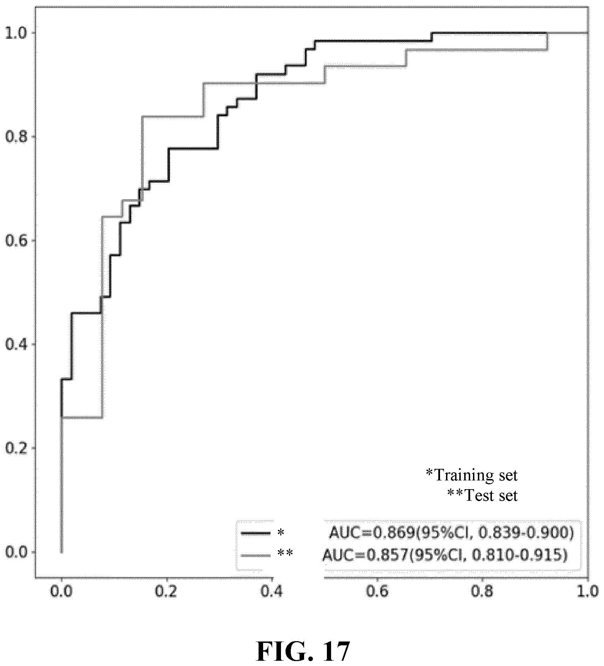

The ROC curve of this 7-marker combination model is shown in Fig. 17. The AUC of the constructed model was 0.857. In the test set, when the specificity was 0.846, the sensitivity could reach 0.774 (Table 1-17), achieving a good differentiating effect for patients with pancreatic cancer and healthy people.

**Table 1-17: Performance of the 7-marker combination model**

| Group | AUC value | Sensitivity | Specificity | Threshold |
|---|---|---|---|---|
| Training set | 0.8695 | 0.6984 | 0.8519 | 0.5177 |
| Test set | 0.8573 | 0.7742 | 0.8462 | 0.5177 |

**1-15: Model construction and performance evaluation of the combination of 7 markers SEQ ID NOs: 1, 19, 27, 34, 37, 46, 47**

In order to verify the prediction performance of the combination of different markers, based on the cluster of 56 methylation markers in the present application, 7 markers SEQ ID NOs: 1, 19, 27, 34, 37, 46, 47 were selected for model construction and performance testing. The training group and the test group were divided, including 117 samples in the training group (samples 1-117) and 57 samples in the test group (samples 118-174).

The 7 methylation markers were used to construct a support vector machine model in the training set for both groups of samples:

1. The samples were pre-divided into 2 parts, 1 part was used for training the model and 1 part was used for model testing.

2. The SVM model was trained using methylation marker levels in the training set. The specific training process is as follows:

a) The sklearn software package (0.23.1) of python software (v3.6.9) is used to construct the training model and cross-validate the training mode of the training model, command line: model = SVR().

b) The sklearn software package (0.23.1) is used to input the methylation value data matrix to construct the SVM model, model.fit(x_train, y_train), where x_train represents the training set data matrix, and y_train represents the phenotypic information of the training set.

3. Test was carried out using the test set data: the above model was brought into the test set for testing, command line: test_pred = model.predict(test_df), where test_pred represents the prediction score obtained by the SVM prediction model constructed in this example for the test set samples, model represents the SVM prediction model constructed in this example, and test_df represents the test set data.

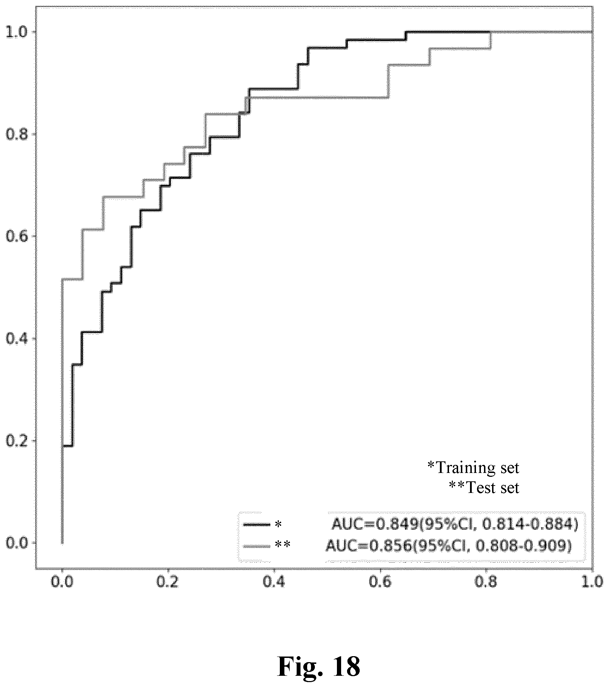

The ROC curve of this 7-marker combination model is shown in Fig. 18. The AUC of the constructed model was 0.856. In the test set, when the specificity was 0.808, the sensitivity could reach 0.742 (Table 1-18), achieving a good differentiating effect for patients with pancreatic cancer and healthy people.

**Table 1-18: Performance of the 7-marker combination model**

| Group | AUC value | Sensitivity | Specificity | Threshold |
|---|---|---|---|---|
| Training set | 0.8492 | 0.6508 | 0.8519 | 0.5503 |
| Test set | 0.8561 | 0.7419 | 0.8077 | 0.5503 |

This study used the methylation levels of related genes in plasma cfDNA to study the differences between the plasma of subjects without pancreatic cancer and the plasma of those with pancreatic cancer, and screened out 56 methylation nucleic acid fragments with significant differences. Based on the above methylation nucleic acid fragment marker cluster, a pancreatic cancer risk prediction model was established through the support vector machine method, which can effectively identify pancreatic cancer with high sensitivity and specificity, and is suitable for screening and diagnosis of pancreatic cancer.

**Example 2**

**2-1: Screening of differentially methylated sites for pancreatic cancer by targeted methylation sequencing**

The inventor collected blood samples from 94 patients with pancreatic cancer and 25 patients with chronic pancreatitis in total, and all the patients signed informed consent forms. The patients with pancreatic cancer had a previous diagnosis of pancreatitis. See the table below for sample information.

| | Training set | Test set |
|---|---|---|
| Sample type | | |
| Pancreatic cancer | 63 | 31 |
| Chronic pancreatitis | 17 | 8 |
| Age | | |
| | 62 (25-80) | 62 (40-79) |
| Gender | | |
| Male | 52 | 23 |
| Female | 28 | 16 |
| Pathological stage | | |
| Chronic pancreatitis | 17 | 8 |

(continued)

| Pathological stage | | |
|---|---|---|
| I | 18 | 7 |
| II | 30 | 14 |

| | | |
|---|---|---|
| III or IV | 14 | 9 |
| Unknown | 1 | 1 |
| CA19-9 | | |
| Distribution (mean, maximum and minimum) | 133.84(1-1200) | 86.0(1-1200) |
| >37 | 51 | 23 |
| ≤37 | 21 | 12 |
| NA | 8 | 4 |

The methylation sequencing data of plasma DNA were obtained by the MethylTitan assay to identify DNA methylation classification markers therein. The process is as follows:

1. Extraction of plasma cfDNA samples

A 2 ml whole blood sample was collected from the patient using a Streck blood collection tube, the plasma was separated by centrifugation timely (within 3 days), transported to the laboratory, and then cfDNA was extracted using the QIAGEN QIAamp Circulating Nucleic Acid Kit according to the instructions.

2. Sequencing and data pre-processing

1) The library was paired-end sequenced using an Illumina Nextseq 500 sequencer.
2) Pear (v0.6.0) software combined the paired-end sequencing data of the same paired-end 150bp sequenced fragment from the Illumina Hiseq X10/ Nextseq 500/Nova seq sequener into one sequence, with the shortest overlapping length of 20 bp and the shortest length of 30bp after combination.
3) Trim_galore v 0.6.0 and cutadapt v1.8.1 software were used to perform adapter removal on the combined sequencing data. The adapter sequence "AGATCGGAAGAGCAC" was removed from the 5' end of the sequence, and bases with sequencing quality value lower than 20 at both ends were removed.

3. Sequencing data alignment

The reference genome data used herein were from the UCSC database (UCSC: HG19, hgdownload.soe.ucsc.edu/goldenPath/hg19/bigZips/hg19.fa.gz).

1) First, HG19 was subjected to conversion from cytosine to thymine (CT) and adenine to guanine (GA) using Bismark software, and an index for the converted genome was constructed using Bowtie2 software.
2) The pre-processed data were also subjected to conversions of CT and GA.
3) The converted sequences were aligned to the converted HG19 reference genome using Bowtie2 software. The minimum seed sequence length was 20, and no mismatching was allowed in the seed sequence.

4. Calculation of MHF

For the CpG sites in each target region HG19, the methylation status corresponding to each site was obtained based on the above alignment results. The nucleotide numbering of sites herein corresponds to the nucleotide position numbering of HG19. One target methylated region may have multiple methylated haplotypes. This value needs to be calculated for each methylated haplotype in the target region. An example of the MHF calculation formula is as follows:

$$MHF_{i,h} = \frac{N_{i,h}}{N_i}$$

where i represents the target methylated region, h represents the target methylated haplotype, $N_i$ represents the number of reads located in the target methylated region, and $N_{i,h}$ represents the number of reads containing the target methylated haplotype.

5. Methylation data matrix

1) The methylation sequencing data of each sample in the training set and the test set were combined into a data matrix, and each site with a depth less than 200 was taken as a missing value.
2) Sites with a missing value proportion higher than 10% were removed.
3) For missing values in the data matrix, the KNN algorithm was used to interpolate the missing data.

6. Discovering feature methylated segments based on training set sample group

1) A logistic regression model was constructed for each methylated segment with regard to the phenotype, and the methylated segment with the most significant regression coefficient was screened out for each amplified target region to form candidate methylated segments.
2) The training set was randomly divided into ten parts for ten-fold cross-validation incremental feature selection.
3) The candidate methylated segments in each region were ranked in descending order according to the significance of the regression coefficient, and the data of one methylated segment was added each time to predict the test data.
4) In step 3), 10 copies of data generated in step 2) were used. For each copy of data, 10 times of calculation were conducted, and the final AUC was the average of 10 calculations. If the AUC of the training data increases, the candidate methylated segment is retained as the feature methylated segment, otherwise it is discarded.
5) The feature combination corresponding to the average AUC median under different number of features in the training set was taken as the final combination of feature methylated segments.

The distribution of the selected characteristic methylation markers in HG19 is as follows: SEQ ID NO: 57 in the SIX3 gene region, SEQ ID NO: 58 in the TLX2 gene region, and SEQ ID NO: 59 in the CILP2 gene region. The levels of the above methylation markers increased or decreased in cfDNA of the patients with pancreatic cancer (Table 2-1). The sequences of the above 3 marker regions are set forth in SEQ ID NOs: 57-59. The methylation levels of all CpG sites in each marker region can be obtained by MethylTitan sequencing. The average methylation level of all CpG sites in each region, as well as the methylation status of a single CpG site, can both be used as a marker for the diagnosis of pancreatic cancer.

**Table 2-1: Methylation levels of DNA methylation markers in the training set**

| Sequence | Marker | Pancreatic cancer | Chronic pancreatitis |
|---|---|---|---|
| SEQ ID NO:57 | chr2:45028785-45029307 | 0.843731054 | 0.909570522 |
| SEQ ID NO:58 | chr2:74742834-74743351 | 0.953274962 | 0.978544302 |
| SEQ ID NO:59 | chr19:19650745-19651270 | 0.408843665 | 0.514101315 |

The methylation levels of methylation markers of people with pancreatic cancer and those with chronic pancreatitis in the test set are shown in Table 2-2. As can be seen from the table, the distribution of methylation level of methylation markers was significantly different between people with pancreatic cancer and those with chronic pancreatitis, achieving good differentiating effects.

**Table 2-2: Methylation levels of DNA methylation markers in the test set**

| Sequence | Marker | Pancreatic cancer | Chronic pancreatitis |
|---|---|---|---|
| SEQ ID NO:57 | chr2:45028785-45029307 | 0.843896661 | 0.86791556 |
| SEQ ID NO:58 | chr2:74742834-74743351 | 0.926459851 | 0.954493044 |
| SEQ ID NO:59 | chr19:19650745-19651270 | 0.399831579 | 0.44918572 |

Table 2-3 lists the correlation (Pearson correlation coefficient) between the methylation levels of 10 random CpG sites or combinations thereof and the methylation level of the entire marker in each selected marker, as well as the corresponding significance p value. It can be seen that the methylation status or level of a single CpG site or a combination of multiple CpG sites within the marker had a significant correlation with the methylation level of the entire region ($p < 0.05$), and the correlation coefficients were all above 0.8. This strong or extremely strong correlation indicates that a single CpG site or a combination of multiple CpG sites within the marker has the same good differentiating effect as the entire marker.

**Table 2-3: Correlation between the methylation level of random CpG sites or combinations of multiple sites and the methylation level of the entire marker in 3 markers**

| CpG sites or combinations | SEQ ID | Training set correlation | Training set p-value | Test set correlation | Test set p-value |
|---|---|---|---|---|---|
| chr2:45029035 | SEQ ID NO:57 | 0.8383 | 6.6E-09 | 0.8471 | 0.000000 135 |
| chr2:45029063 | SEQ ID NO:57 | 0.8484 | 1.27E-09 | 0.826 | 0.000060 8 |
| chr2:45029065 | SEQ ID NO:57 | 0.8054 | 3.46E-10 | 0.8369 | 0.000047 8 |
| chr2:45029046,45029057,45029060 | SEQ ID NO:57 | 0.841 | 8.33E-11 | 0.8126 | 0.00899 |
| chr2:45029060 | SEQ ID NO:57 | 0.8241 | 5.78E-11 | 0.8165 | 2.35E-10 |
| chr2:45029117 | SEQ ID NO:57 | 0.8356 | 8.54E-12 | 0.807 | 0.000834 |
| chr2:45029057,45029060 | SEQ ID NO:57 | 0.8333 | 6.19E-13 | 0.8267 | 0.00138 |
| chr2:45029046,45029057 | SEQ ID NO:57 | 0.808 | 2.16E-16 | 0.8315 | 0.00114 |
| chr2:45029057 | SEQ ID NO:57 | 0.802 | 3.89E-19 | 0.8436 | 0.000000 177 |
| chr2:45029046 | SEQ ID NO:57 | 0.846 | 5.23E-23 | 0.835 | 3.86E-11 |
| chr2:74743119,74743121 | SEQ ID NO:58 | 0.8015 | 3.49E-18 | 0.9822 | 1.82E-28 |
| chr2:74743108,74743111 | SEQ ID NO:58 | 0.8043 | 1.52E-18 | 0.9864 | 1.32E-30 |
| chr2:74743111,74743119 | SEQ ID NO:58 | 0.8204 | 8.06E-19 | 0.9827 | 1.02E-28 |
| chr2:74743 082 | SEQ ID NO:58 | 0.8363 | 5.84E-19 | 0.981 | 6.15E-28 |
| chr2:74743073 | SEQ ID NO:58 | 0.8064 | 1.77E-19 | 0.9843 | 1.69E-29 |

(continued)

| CpG sites or combinations | SEQ ID | Training set correlation | Training set p-value | Test set correlation | Test set p-value |
|---|---|---|---|---|---|
| chr2:74743119 | SEQ ID NO:58 | 0.814 | 4.38E-20 | 0.9806 | 8.97E-28 |
| chr2:74743111 | SEQ ID NO:58 | 0.8145 | 3.96E-20 | 0.9465 | 9.07E-20 |
| chr2:74743056 | SEQ ID NO:58 | 0.8277 | 2.91E-21 | 0.9769 | 2.04E-26 |
| chr2:74743084 | SEQ ID NO:58 | 0.8488 | 2.74E-23 | 0.9796 | 2.09E-27 |
| chr2:74743101 | SEQ ID NO:58 | 0.8695 | 1.31E-25 | 0.9954 | 2.39E-39 |
| chr19:19650995,19650 997,19651001 | SEQ ID NO:59 | 0.8255 | 7.66E-11 | 0.8212 | 0.00244 |
| chr19:19650981,19650 995 | SEQ ID NO:59 | 0.8171 | 5.11E-11 | 0.8408 | 0.000051 8 |
| chr19:19650997,19651 001,19651008 | SEQ ID NO:59 | 0.8171 | 2.2E-11 | 0.8359 | 0 |
| chr19:19650995,19650 997 | SEQ ID NO:59 | 0.8072 | 3.37E-12 | 0.8039 | 0.000033 7 |
| chr19:19651008 | SEQ ID NO:59 | 0.8159 | 1.73E-13 | 0.841 | 0.000008 24 |
| chr19:19651001,19651 008 | SEQ ID NO:59 | 0.8437 | 5.21E-14 | 0.8282 | 0.00422 |
| chr19:19650997,19651 001 | SEQ ID NO:59 | 0.8378 | 1.5E-14 | 0.8279 | 0.00205 |
| chr19:19650997 | SEQ ID NO:59 | 0.8195 | 4.64E-16 | 0.8127 | 2.29E-08 |
| chr19:19650995 | SEQ ID NO:59 | 0.8211 | 3.26E-16 | 0.807 | 0.000000 707 |
| chr19:19651001 | SEQ ID NO:59 | 0.8342 | 4.93E-17 | 0.8118 | 2.58E-09 |

**2-2: Predictive performance of single methylation markers**

In order to verify the ability of a single methylation marker to differentiate between pancreatitis and pancreatic cancer, the values of methylation levels of single methylation markers were used to verify the predictive performance of single markers.

First, the methylation level values of 3 methylation markers were used separately in the training set samples for training to determine the threshold, sensitivity and specificity for differentiating between pancreatic cancer and pancreatitis, and then the threshold was used to statistically analyze the sensitivity and specificity of the samples in the test set. The results are shown in Table 2-4 below. It can be seen that a single marker can also achieve good differentiating performance.

**Table 2-4: Predictive performance of 56 single methylation markers**

| Marker | Group | AUC value | Sensitivity | Specificity | Threshold |
|---|---|---|---|---|---|
| SEQ ID NO:57 | Training set | 0.8870 | 0.7937 | 0.8824 | 0.8850 |

(continued)

| Marker | Group | AUC value | Sensitivity | Specificity | Threshold |
|--------|-------|-----------|-------------|-------------|-----------|
| SEQ ID NO:57 | Test set | 0.6532 | 0.7742 | 0.3750 | 0.8850 |
| SEQ ID NO:58 | Training set | 0.8497 | 0.6508 | 0.8824 | 0.9653 |
| SEQ ID NO:58 | Test set | 0.6210 | 0.8065 | 0.5000 | 0.9653 |
| SEQ ID NO:59 | Training set | 0.8301 | 0.4286 | 0.8824 | 0.3984 |
| SEQ ID NO:59 | Test set | 0.6694 | 0.5806 | 0.6250 | 0.3984 |

### 2-3: Construction of classification prediction model

In order to verify the potential ability of classifying patients with pancreatic cancer and patients with chronic pancreatitis using marker DNA methylation levels (such as methylated haplotype fraction), in the training group, a support vector machine disease classification model was constructed based on the combination of 3 DNA methylation markers to verify the classification prediction effect of this cluster of DNA methylation markers in the test group. The training group and the test group were divided according to the proportion, including 80 samples in the training group (samples 1-80) and 39 samples in the test group (samples 80-119).

A support vector machine model was constructed in the training set for both groups of samples using the discovered DNA methylation markers.

1) The samples were pre-divided into 2 parts, 1 part was used for training the model and 1 part was used for model testing.

2) To exploit the potential of identifying pancreatic cancer using methylation markers, a disease classification system was developed based on genetic markers. The SVM model was trained using methylation marker levels in the training set. The specific training process is as follows:

a) Using the sklearn software package (v0.23.1) of python software (v3.6.9) to construct the training model and cross-validate the training mode of the training model, command line: model = SVR().

b) Using the sklearn software package (v0.23.1) to input the methylation value data matrix to construct the SVM model, model.fit(x_train, y_train), where x_train represents the training set data matrix, and y_train represents the phenotypic information of the training set.

In the process of constructing the model, the pancreatic cancer type was coded as 1 and the chronic pancreatitis type was coded as 0. In the process of constructing the model by the sklearn software package (v0.23.1), the threshold was set as 0.897 by default. Finally, the constructed model used 0.897 as the score threshold to differentiate between pancreatic cancer and pancreatitis. The prediction scores of the two models for the training set samples are shown in Table 2-5.

**Table 2-5: Prediction scores of the models in the training set**

| Sample | Type | Score | Sample | Type | Score |
|--------|------|-------|--------|------|-------|
| Sample 1 | Pancreatic cancer | 0.906363896 | Sample 41 | Pancreatic cancer | 0.895671254 |
| Sample 2 | Pancreatic cancer | 0.898088428 | Sample 42 | Pancreatic cancer | 0.917370358 |
| Sample 3 | Pancreatic cancer | 0.96514133 | Sample 43 | Pancreatic cancer | 0.899939907 |
| Sample 4 | Pancreatic cancer | 0.947218787 | Sample 44 | Chronic pancreatitis | 0.819877173 |
| Sample 5 | Chronic pancreatitis | 0.814559896 | Sample 45 | Pancreatic cancer | 0.864307914 |
| Sample 6 | Pancreatic cancer | 0.899770509 | Sample 46 | Pancreatic cancer | 0.97794434 |
| Sample 7 | Pancreatic cancer | 1.171999028 | Sample 47 | Chronic pancreatitis | 0.786462108 |
| Sample 8 | Pancreatic cancer | 0.896938646 | Sample 48 | Chronic pancreatitis | 0.646721483 |
| Sample 9 | Chronic pancreatitis | 0.760177073 | Sample 49 | Pancreatic cancer | 0.911479846 |
| Sample 10 | Chronic pancreatitis | 0.887726067 | Sample 50 | Pancreatic cancer | 0.899897548 |

(continued)

| Sample | Type | Score | Sample | Type | Score |
|--------|------|-------|--------|------|-------|
| Sample 11 | Pancreatic cancer | 0.531337905 | Sample 51 | Pancreatic cancer | 0.824992525 |
| Sample 12 | Pancreatic cancer | 0.90484915 | Sample 52 | Chronic pancreatitis | 0.245182024 |
| Sample 13 | Chronic pancreatitis | 0.898855566 | Sample 53 | Pancreatic cancer | 0.924471595 |
| Sample 14 | Pancreatic cancer | 0.972688399 | Sample 54 | Pancreatic cancer | 1.034876438 |
| Sample 15 | Pancreatic cancer | 0.898868258 | Sample 55 | Pancreatic cancer | 1.099788336 |
| Sample 16 | Chronic pancreatitis | 0.898883166 | Sample 56 | Pancreatic cancer | 0.89944059 |
| Sample 17 | Pancreatic cancer | 0.899875594 | Sample 57 | Chronic pancreatitis | 0.211506728 |
| Sample 18 | Pancreatic cancer | 0.902123447 | Sample 58 | Pancreatic cancer | 0.899895698 |
| Sample 19 | Pancreatic cancer | 0.898527925 | Sample 59 | Pancreatic cancer | 0.91285525 |
| Sample 20 | Pancreatic cancer | 0.992521216 | Sample 60 | Pancreatic cancer | 0.893568369 |
| Sample 21 | Chronic pancreatitis | 0.678536161 | Sample 61 | Pancreatic cancer | 0.929428735 |
| Sample 22 | Pancreatic cancer | 0.943101949 | Sample 62 | Pancreatic cancer | 0.865378859 |
| Sample 23 | Pancreatic cancer | 0.893582535 | Sample 63 | Chronic pancreatitis | 0.23424179 |
| Sample 24 | Pancreatic cancer | 0.846727508 | Sample 64 | Pancreatic cancer | 1.03871855 |
| Sample 25 | Pancreatic cancer | 0.993891187 | Sample 65 | Pancreatic cancer | 1.001209954 |
| Sample 26 | Pancreatic cancer | 1.09987453 | Sample 66 | Pancreatic cancer | 0.981189452 |
| Sample 27 | Pancreatic cancer | 0.900023617 | Sample 67 | Chronic pancreatitis | 0.593205453 |
| Sample 28 | Pancreatic cancer | 0.919070531 | Sample 68 | Pancreatic cancer | 0.905930493 |
| Sample 29 | Pancreatic cancer | 0.910053964 | Sample 69 | Pancreatic cancer | 1.100033741 |
| Sample 30 | Pancreatic cancer | 0.886760785 | Sample 70 | Pancreatic cancer | 1.100772446 |
| Sample 31 | Pancreatic cancer | 0.91917744 | Sample 71 | Pancreatic cancer | 0.898821581 |
| Sample 32 | Pancreatic cancer | 0.975091185 | Sample 72 | Chronic pancreatitis | 0.869308711 |
| Sample 33 | Pancreatic cancer | 0.900548389 | Sample 73 | Pancreatic cancer | 0.6730075 |
| Sample 34 | Pancreatic cancer | 0.8981704 | Sample 74 | Pancreatic cancer | 1.037048136 |
| Sample 35 | Pancreatic cancer | 1.009222108 | Sample 75 | Pancreatic cancer | 0.972542948 |
| Sample 36 | Pancreatic cancer | 1.322966423 | Sample 76 | Pancreatic cancer | 0.933799461 |
| Sample 37 | Chronic pancreatitis | 0.874263052 | Sample 77 | Pancreatic cancer | 1.016413808 |
| Sample 38 | Chronic pancreatitis | 0.706851745 | Sample 78 | Pancreatic cancer | 1.243523664 |
| Sample 39 | Chronic pancreatitis | 0.762970982 | Sample 79 | Pancreatic cancer | 0.899887112 |
| Sample 40 | Pancreatic cancer | 0.950107015 | Sample 80 | Pancreatic cancer | 0.892289956 |

## 2-4: Classification prediction model test

MethylTitan sequencing was performed using the blood samples of the aforementioned pancreatic cancer and pancreatitis subjects, and classification analysis such as PCA and clustering was performed based on the characteristic methylation marker signals in the sequencing results.

Based on the methylation marker cluster of the present application, it was predicted in the test set according to the model established by SVM in Example 2-3. The test set was predicted using the prediction function to output the prediction result (disease probability: the default score threshold is 0.897, and if the score is greater than 0.897, the subject is considered as a patient with pancreatic acid, otherwise the subject is a patient with chronic pancreatitis). The test group had 57 samples (samples 118-174), and the calculation process is as follows:
Command Line:

$$test\_pred = model.predict(test\_df)$$

where test_pred represents the prediction score of the samples in the test set obtained by using the SVM prediction model constructed in Example 2-3, model represents the SVM prediction model constructed in Example 2-3, and test_df represents the test set data.

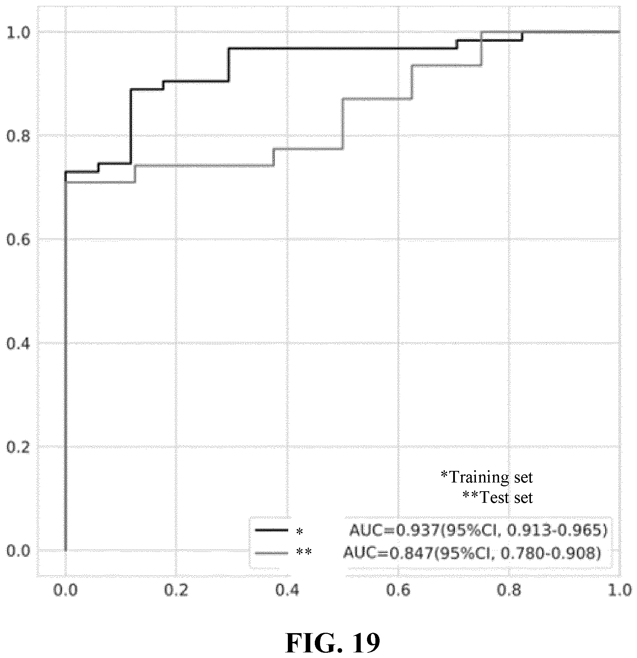

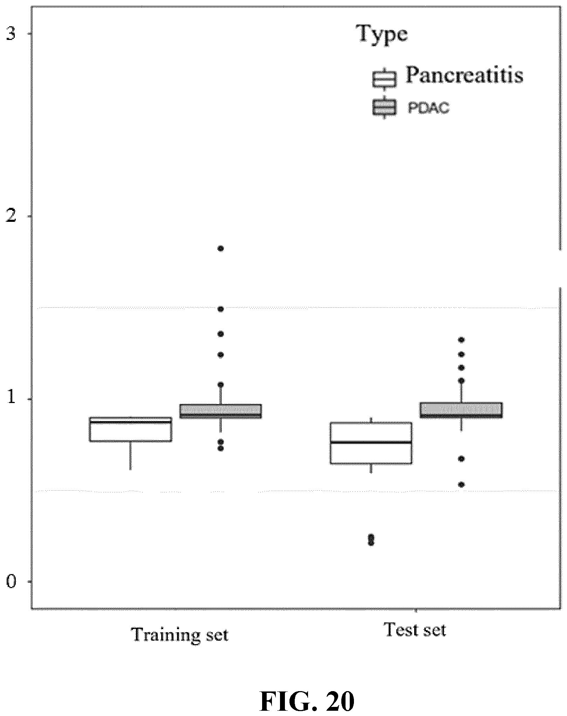

The prediction scores of the test group are shown in Table 2-6. The ROC curve is shown in Fig. 19. The prediction score distribution is shown in Fig. 20. The area under the overall AUC of the test group was 0.847. In the training set, when the specificity was 88.2%, the sensitivity of this model could reach 88.9%; in the test set, when the specificity was 87.5%, the sensitivity could reach 74.2%. It can be seen that the differentiating effect of the SVM models established by the selected variables is good.

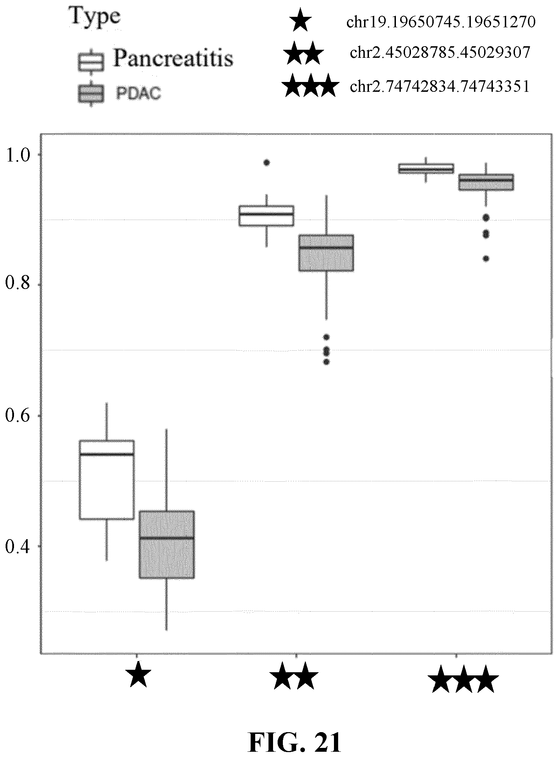

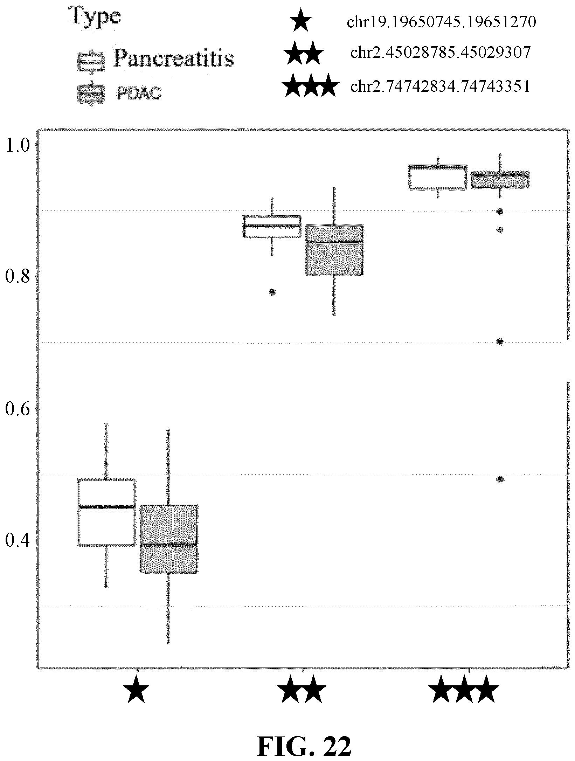

Figs. 21 and 22 show the distribution of the 3 methylation markers in the training group and the test group respectively. It can be found that the difference of this cluster of methylation markers in the plasma of the patient with pancreatitis and the plasma of the patients with pancreatic cancer was relatively stable.

**Table 2-6: Model prediction scores for test set samples**

| Sample ID | Type | Score | Sample ID | Type | Score |
|---|---|---|---|---|---|
| Sample 81 | Chronic pancreatitis | 0.610488911 | Sample 101 | Pancreatic cancer | 15.62766141 |
| Sample 82 | Pancreatic cancer | 0.912018264 | Sample 102 | Pancreatic cancer | 0.909976179 |
| Sample 83 | Pancreatic cancer | 0.870225426 | Sample 103 | Pancreatic cancer | 0.92289051 |
| Sample 84 | Pancreatic cancer | 0.897368929 | Sample 104 | Pancreatic cancer | 1.823319531 |
| Sample 85 | Pancreatic cancer | 1.491556374 | Sample 105 | Pancreatic cancer | 0.913625979 |
| Sample 86 | Pancreatic cancer | 0.99785215 | Sample 106 | Pancreatic cancer | 0.730447081 |
| Sample 87 | Pancreatic cancer | 0.909901733 | Sample 107 | Pancreatic cancer | 0.900701224 |
| Sample 88 | Pancreatic cancer | 0.955726751 | Sample 108 | Chronic pancreatitis | 0.893221308 |
| Sample 89 | Pancreatic cancer | 0.96582068 | Sample 109 | Chronic pancreatitis | 0.899073184 |
| Sample 90 | Pancreatic cancer | 0.910414113 | Sample 110 | Chronic pancreatitis | 0.783284566 |
| Sample 91 | Pancreatic cancer | 0.850903621 | Sample 111 | Chronic pancreatitis | 0.725251615 |
| Sample 92 | Pancreatic cancer | 0.916651697 | Sample 112 | Pancreatic cancer | 0.893141436 |
| Sample 93 | Chronic pancreatitis | 0.904231501 | Sample 113 | Pancreatic cancer | 1.354991317 |
| Sample 94 | Pancreatic cancer | 0.764872522 | Sample 114 | Pancreatic cancer | 0.817727331 |
| Sample 95 | Pancreatic cancer | 1.241367038 | Sample 115 | Pancreatic cancer | 1.079401681 |
| Sample 96 | Chronic pancreatitis | 0.897789105 | Sample 116 | Pancreatic cancer | 0.969607597 |
| Sample 97 | Chronic pancreatitis | 0.852404121 | Sample 117 | Pancreatic cancer | 0.878877727 |
| Sample 98 | Pancreatic cancer | 1.068601129 | Sample 118 | Pancreatic cancer | 0.911801452 |
| Sample 99 | Pancreatic cancer | 3.715591125 | Sample 119 | Pancreatic cancer | 0.934497862 |
| Sample 100 | Pancreatic cancer | 0.920532374 | | | |

### 2-5: Predictive effect for patients that are tumor marker negative

Based on the methylation marker cluster of the present application, patients that were negative for the tumor marker CA19-9 (< 37) were distinguished according to the model established by SVM in Example 2-3.

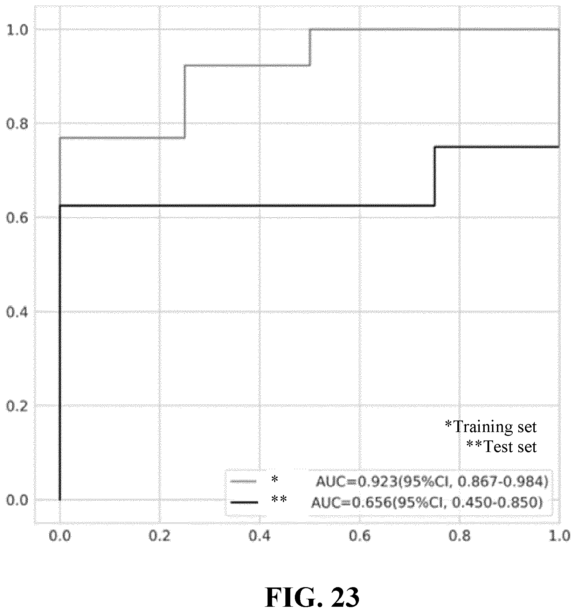

The prediction scores of the test group are shown in Table 2-7, and the ROC curve is shown in Fig. 23. It can be seen that for patients who cannot be distinguished by the traditional tumor marker CA19-9, the constructed SVM model can also achieve good results.

**Table 2-7: CA19-9 measurements and prediction scores of SVM model**

| Sample | CA19-9 | Model score | Type |
|---|---|---|---|
| Sample 1 | 30.3 | 0.21151 | Chronic pancreatitis |
| Sample 2 | 28.35 | 0.23424 | Chronic pancreatitis |
| Sample 3 | 26.21 | 0.87426 | Chronic pancreatitis |
| Sample 4 | 4.19 | 0.97794 | Pancreatic cancer |
| Sample 5 | 18.47 | 0.67301 | Pancreatic cancer |
| Sample 6 | 3.17 | 0.91286 | Pancreatic cancer |
| Sample 7 | 1 | 0.59321 | Chronic pancreatitis |
| Sample 8 | 2.61 | 0.81456 | Chronic pancreatitis |
| Sample 9 | 2 | 0.91148 | Pancreatic cancer |
| Sample 10 | 2.57 | 0.67854 | Chronic pancreatitis |
| Sample 11 | 24.26 | 0.84673 | Pancreatic cancer |
| Sample 12 | 5 | 0.24518 | Chronic pancreatitis |
| Sample 13 | 33.99 | 0.89817 | Pancreatic cancer |
| Sample 14 | 7 | 0.86931 | Chronic pancreatitis |
| Sample 15 | 21.13 | 0.86431 | Pancreatic cancer |
| Sample 16 | 3.8 | 0.92447 | Pancreatic cancer |
| Sample 17 | 23.57 | 0.97269 | Pancreatic cancer |
| Sample 18 | 20 | 0.89357 | Pancreatic cancer |
| Sample 19 | 18.14 | 0.91737 | Pancreatic cancer |
| Sample 20 | 14.05 | 1.00922 | Pancreatic cancer |
| Sample 21 | 35 | 1.172 | Pancreatic cancer |
| Sample 22 | 6 | 0.89322 | Chronic pancreatitis |
| Sample 23 | 2.42 | 0.90423 | Chronic pancreatitis |
| Sample 24 | 10.29 | 1.0794 | Pancreatic cancer |
| Sample 25 | 4.61 | 0.8509 | Pancreatic cancer |
| Sample 26 | 5.56 | 0.89907 | Chronic pancreatitis |
| Sample 27 | 24.78 | 0.87888 | Pancreatic cancer |
| Sample 28 | 7.41 | 1.0686 | Pancreatic cancer |
| Sample 29 | 24.1 | 1.82332 | Pancreatic cancer |
| Sample 30 | 7 | 0.73045 | Pancreatic cancer |
| Sample 31 | 1 | 0.8524 | Chronic pancreatitis |
| Sample 32 | 30 | 0.91363 | Pancreatic cancer |
| Sample 33 | 21 | 0.9345 | Pancreatic cancer |

This study used the methylation levels of methylation markers in plasma cfDNA to study the differences between the plasma of subjects with chronic pancreatitis and the plasma of those with pancreatic cancer, and screened out 3 DNA methylation markers with significant differences. Based on the above DNA methylation marker cluster, a malignant pancreatic cancer risk prediction model was established through the support vector machine method, which can effectively differentiate between patients with pancreatic cancer and those with chronic pancreatitis with high sensitivity and specificity, and is suitable for screening and diagnosis of pancreatic cancer in patients with chronic pancreatitis.

**Example 3**

**3-1: Screening of pancreatic cancer-specific methylation sites by targeted methylation sequencing**

A total of 110 pancreatic cancer blood samples and 110 samples without pancreatic cancer with matched age and gender were collected. All enrolled patients signed informed consent forms. The sample information is shown in Table 3-1.

Table 3-1

|  | Training set | Test set |
|---|---|---|
| Sample type |  |  |
| Pancreatic cancer | 69 | 41 |
| Without pancreatic cancer | 63 | 47 |
| Age |  |  |
|  | 64 (33-89) | 65 (43-81) |
| Gender |  |  |
| Male | 80 | 52 |
| Female | 52 | 36 |
| Pathological stage |  |  |
| I | 17 | 10 |
| II | 24 | 7 |
| III or IV | 15 | 18 |
| NA | 13 | 6 |

The present application provides a cluster of DNA methylation markers. By detecting the methylation level of DNA methylation markers in patient's plasma samples, the detected methylation level data are used to predict scores according to the diagnostic model to differentiate between patients with pancreatic cancer and healthy people to achieve the purpose of early diagnosis of pancreatic cancer with higher accuracy and lower cost during early screening.

1. Sample cfDNA extraction

All blood samples were collected in Streck tubes, and to extract plasma, the blood samples were first centrifuged at 1600g at 4°C for 10 min. In order to prevent damage to the buffy coat layer, smooth braking mode needed to be set. The supernatant was then transferred to a new 1.5 ml conical tube and centrifuged at 16000g at 4°C for 10 min. The supernatant was again transferred to a new 1.5 ml conical tube and store at -80°C.

To extract circulating cell-free DNA (cfDNA), plasma aliquots were thawed and processed immediately using the QIAamp Circulating Nucleic Acid Extraction Kit (Qiagen 55114) according to the manufacturer's instructions. The extracted cfDNA concentration was quantified using qubit3.0.

2. Bisulfite conversion and library preparation

Sodium bisulfite conversion of cytosine bases was performed using a bisulfite conversion kit (ThermoFisher, MECOV50). According to the manufacturer's instructions, 20 ng of genomic DNA or ctDNA was converted and purified for downstream applications.

Extraction of sample DNA, quality inspection, and conversion of unmethylated cytosine on DNA into bases that do not bind to guanine were carried out. In one or more embodiments, the conversion is performed using enzymatic methods, preferably treatment with deaminase, or the conversion is performed using non-enzymatic methods, preferably treatment with bisulfite or bisulfate, more preferably treatment with calcium bisulfite, sodium bisulfite, potassium bisulfite, ammonium bisulfite, sodium bisulfate, potassium bisulfate and ammonium bisulfate.

The library was constructed using the MethylTitan (Patent No.: CN201910515830) method. The MethylTitan method is as follows. The DNA converted by bisulfite was dephosphorylated and then ligated to a universal Illumina sequencing adapter with a molecular tag (UMI). After second-strand synthesis and purification, the converted DNA was subjected to a semi-targeted PCR reaction for targeted amplification of the required target region. After purification again, sample-specific barcodes and full-length Illumina sequencing adapters were added to the target DNA molecules through a PCR reaction. The final library was then quantified using Illumina's KAPA library quantification kit (KK4844) and sequenced on an Illumina sequencer. The MethylTitan library construction method can effectively enrich the required target fragment with a smaller amount of DNA, especially cfDNA, while this method can well preserve the methylation status of the original DNA, and ultimately by analyzing adjacent CpG methylated cytosine (a given target may have several to dozens of CpGs, depending on the given region), the entire methylation pattern of that particular region can serve as a unique marker, rather than comparing the status of individual bases.

3. Sequencing and data pre-processing

1) Paired-end sequencing was performed using the Illumina Hiseq 2500 sequencer. The sequencing volume was 25-35M per sample. The paired-end 150bp sequencing data from the Illumina Hiseq 2500 sequencer was subjected to adapter removal using Trim_galore v 0.6.0 and cutadapt v2.1 software. The adapter sequence "AGATCGGAAGAGCACACGTCTGAACTCCAGTC" at the 3' end of Read 1 was removed, the adapter sequence "AGATCGGAAGAGCGTCGTGTA GGGAAAGAGTGT" at the 3' end of Read 2 was removed, and bases whose sequencing quality was less than 20 were removed at both ends. If there is a 3 bp adapter sequence at the 5' end, the entire read will be removed. Reads shorter than 30 bases were also removed after adapter removal.
2) Paired-end sequences were combined into single-end sequences using Pear v0.9.6 software. Reads from both ends that overlap by at least 20 bases were combined, and discarded if the combined reads are shorter than 30 bases.

4. Sequencing data comparison

The reference genome data used in the present application were from the UCSC database (UCSC: hg19, hgdownload.soe.ucsc.edu/goldenPath/hg19/bigZips/hg19.fa.gz).

1) First, hg19 was subjected to conversion from cytosine to thymine (CT) and adenine to guanine (GA) using Bismark software, and an index for the converted genome was constructed using Bowtie2 software.
2) The pre-processed data were also subjected to CT and GA conversion.
3) The converted sequences were aligned to the converted HG19 reference genome by using Bowtie2 software. The minimum seed sequence length was 20, and no mismatching was allowed in the seed sequence.

5. Extraction of methylation information

For the CpG sites in each target region hg19, the methylation level corresponding to each site was obtained based on the above alignment results. The nucleotide numbering of sites involved in the present invention corresponds to the nucleotide position numbering of hg19.

1) To calculate the methylated haplotype fraction (MHF), for the CpG sites in each target region hg19, based on the above comparison results, the base sequence corresponding to each site in the reads was obtained, where C indicates that methylation occurs at this site, T indicates the unmethylated state of this site. The nucleotide numbering of sites herein corresponds to the nucleotide position numbering of HG19. One target methylated region may have multiple methylated haplotypes. This value needs to be calculated for each methylated haplotype in the target region. An example of the MHF calculation formula is as follows:

$$MHF_{i,h}=(N_{i,h})/N_i$$

where i represents the target methylated region, h represents the target methylated haplotype, $N_i$ represents the number of reads located in the target methylated region, and $N_{i,h}$ represents the number of reads containing the target methylated haplotype.
2) With regard to calculation of average methylation level (AMF), for each target region, the average level of methylation within this region is calculated. The formula is as follows:

$$AMF = \frac{\sum_i^m N_{C,i}}{\sum_i^m (N_{C,i} + N_{T,i})}$$

where m is the total number of CpG sites in the target, i is each CpG site in the region, $N_{C,i}$ is the number of reads at the CpG site whose base is T (that is, the number of reads that are methylated at this site), $N_{T,i}$ is the number of reads at the CpG site whose base is T (that is, the number of sequencing reads that are unmethylated at this site)

6. Construction of feature matrix

1) The data of methylated haplotype fraction (MHF) and average methylation fraction (AMF) of the samples in the training set and the test set were combined into a data matrix respectively, and each site with a depth less than 200 was taken as a missing value.
2) Sites with a missing value proportion higher than 10% were removed.
3) For the missing values in the data matrix, the KNN algorithm was used to interpolate the missing data. First, the interpolator was trained using the training set by the KNN algorithm, and then the training set matrix and the test set matrix were interpolated respectively.

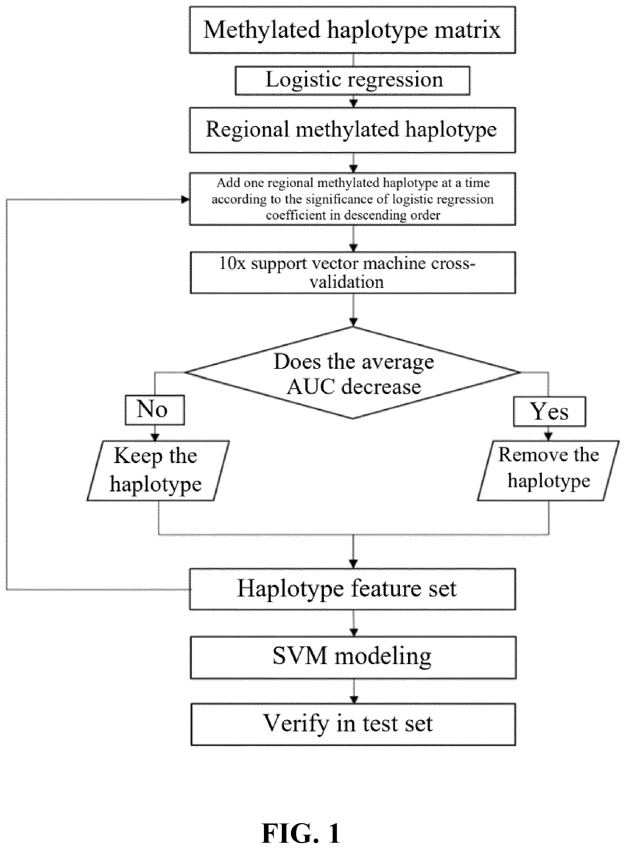

7. Screening methylation markers according to the feature matrix (Fig. 1)

1) The training set was randomly divided into 3 folds, a logistic regression model was built, the average AUC of each target area was calculated, the feature with the largest AUC for each target area was selected as the representative feature of the area, and ranked according to AUC in descending order.
2) The training set was randomly divided into ten parts for ten-fold cross-validation incremental feature selection. The specific process comprised: setting aside a portion of the data in the training set as test data, and the remaining data in the training set as training data. According to the above order, the representative feature of each region was incorporated into the feature combination, and a logistic regression model was constructed using 9 pieces of training data to predict the test data. After repeating 10 times, the average AUC of the test data was calculated.
3) If the AUC of the training data increases, the methylation marker is kept, otherwise its is removed. After the cycle, the obtained feature combination was used as the methylation marker combination, all the training set data were used to train a new model, and it was verified using the test set data.

A total of 101 methylation markers were screened out. The GREAT tool (great.stanford.edu/great/public-3.0.0/html/index.php) was used for gene annotation (see Table 3-2). In GREAT analysis, the marker region was correlated with adjacent genes, and the region with adjacent genes was annotated. The correlation was divided into two processes. First, the regulatory domain of each gene was found, and then the genes covering the regulatory domain of this region were correlated with this region.
For example, ARHGEF16 (-60,185) and PRDM16 (+325,030) represent markers that are 60,185 bp upstream from the transcription start site (TSS) of the ARHGEF16 gene and 325,030 bp downstream from the transcription start site (TSS) of the PRDM16 gene.

Table 3-2 Methylation marker genes and locations

| Serial No. | Chromoso me | Starting position | Ending position | Gene annotation |
|---|---|---|---|---|
| SEQ ID NO: 60 | chr1 | 3310705 | 3310905 | ARHGEF16 (-60,185), PRDM16 (+325,030) |
| SEQ ID NO: 61 | chr1 | 61520321 | 61520632 | NFIA (-27,057) |
| SEQ ID NO: 62 | chr1 | 77333096 | 77333296 | ST6GALNAC5 (+70) |
| SEQ ID NO: 63 | chr1 | 170630461 | 170630661 | PRRX1 (-2,486) |
| SEQ ID NO: 64 | chr1 | 180202481 | 180202846 | LHX4 (+3,243), ACBD6 (+269,425) |
| SEQ ID NO: 65 | chr1 | 240161230 | 240161455 | FMN2 (-93,837), CHRM3 (+368,970) |

(continued)

| Serial No. | Chromoso me | Starting position | Ending position | Gene annotation |
|---|---|---|---|---|
| SEQ ID NO: 66 | chr2 | 468096 | 468607 | FAM150B (-180,056), TMEM18 (+209,087) |
| SEQ ID NO: 67 | chr2 | 469568 | 469933 | FAM150B (-181,455), TMEM18 (+207,688) |
| SEQ ID NO: 68 | chr2 | 45155938 | 45156214 | SIX3 (-12,826), CAMKMT (+566,973) |
| SEQ ID NO: 69 | chr2 | 63285937 | 63286137 | OTX1 (+8,100), WDPCP (+529,896) |
| SEQ ID NO: 70 | chr2 | 63286154 | 63286354 | OTX1 (+8,317), WDPCP (+529,679) |
| SEQ ID NO: 71 | chr2 | 72371208 | 72371433 | CYP26B1 (+3,846), DYSF (+677,489) |
| SEQ ID NO: 72 | chr2 | 177043062 | 177043477 | HOXD1 (-10,037), HOXD4 (+27,320) |
| SEQ ID NO: 73 | chr2 | 238864855 | 238865085 | UBE2F (-10,627), RAMP1 (+96,783) |
| SEQ ID NO: 74 | chr3 | 49459532 | 49459732 | AMT (+554) |
| SEQ ID NO: 75 | chr3 | 147109862 | 147110062 | PLSCR5 (-785,959), ZIC4 (+12,109) |
| SEQ ID NO: 76 | chr3 | 179754913 | 179755264 | PEX5L (-371) |
| SEQ ID NO: 77 | chr3 | 185973717 | 185973917 | ETV5 (-146,916), DGKG (+106,209) |
| SEQ ID NO: 78 | chr3 | 192126117 | 192126324 | FGF12 (+617) |
| SEQ ID NO: 79 | chr4 | 1015773 | 1015973 | FGFRL1 (+12,106), RNF212 (+91,441) |
| SEQ ID NO: 80 | chr4 | 3447856 | 3448097 | DOK7 (-17,061), HGFAC (+4,363) |
| SEQ ID NO: 81 | chr4 | 5710006 | 5710312 | EVC (-2,765), EVC2 (+135) |
| SEQ ID NO: 82 | chr4 | 8859842 | 8860042 | HMX1 (+13,601), CPZ (+265,555) |
| SEQ ID NO: 83 | chr5 | 3596560 | 3596842 | IRX1 (+533) |
| SEQ ID NO: 84 | chr5 | 3599720 | 3599934 | IRX1 (+3,659) |
| SEQ ID NO: 85 | chr5 | 37840176 | 37840376 | GDNF (-4,347) |
| SEQ ID NO: 86 | chr5 | 76249591 | 76249791 | AGGF1 (-76,519), CRHBP (+1,153) |
| SEQ ID NO: 87 | chr5 | 134364359 | 134364559 | PITX1 (+5,529), CATSPER3 (+60,863) |
| SEQ ID NO: 88 | chr5 | 134870613 | 134870990 | NEUROG1 (+837) |
| SEQ ID NO: 89 | chr5 | 170742525 | 170742728 | NPM1 (-72,025), TLX3 (+6,339) |
| SEQ ID NO: 90 | chr5 | 172659554 | 172659918 | NKX2-5 (+2,624), BNIP1 (+88,291) |
| SEQ ID NO: 91 | chr5 | 177411431 | 177411827 | PROP1 (+11,614), B4GALT7 (+384,528) |
| SEQ ID NO: 92 | chr6 | 391439 | 391639 | IRF4 (-200) |
| SEQ ID NO: 93 | chr6 | 1378941 | 1379141 | FOXF2 (-11,028), FOXQ1 (+66,366) |
| SEQ ID NO: 94 | chr6 | 1625294 | 1625494 | FOXC1 (+14,713), GMDS (+620,532) |
| SEQ ID NO: 95 | chr6 | 40308768 | 40308968 | MOCS1 (-413,413), LRFN2 (+246,336) |
| SEQ ID NO: 96 | chr6 | 99291616 | 99291816 | POU3F2 (+9,136), FBXL4 (+104,086) |
| SEQ ID NO: 97 | chr6 | 167544878 | 167545117 | CCR6 (+8,741), GPR31 (+26,819) |
| SEQ ID NO: 98 | chr7 | 35297370 | 35297570 | TBX20 (-3,712) |
| SEQ ID NO: 99 | chr7 | 35301095 | 35301411 | TBX20 (-7,495), HERPUD2 (+433,492) |
| SEQ ID NO: 100 | chr7 | 158937005 | 158937205 | VIPR2 (+544) |

(continued)

| Serial No. | Chromoso me | Starting position | Ending position | Gene annotation |
|---|---|---|---|---|
| SEQ ID NO: 101 | chr8 | 20375580 | 20375780 | LZTS1 (-214,206) |
| SEQ ID NO: 102 | chr8 | 23564023 | 23564306 | NKX2-6 (-54) |
| SEQ ID NO: 103 | chr8 | 23564051 | 23564251 | NKX2-6 (-40) |
| SEQ ID NO: 104 | chr8 | 57358434 | 57358672 | PENK (+36) |
| SEQ ID NO: 105 | chr8 | 70983528 | 70983793 | PRDM14 (-99) |
| SEQ ID NO: 106 | chr8 | 99986831 | 99987031 | VPS13B (-38,563), OSR2 (+30,261) |
| SEQ ID NO: 107 | chr9 | 126778194 | 126778644 | NEK6 (-241,823), LHX2 (+4,530) |
| SEQ ID NO: 108 | chr10 | 74069147 | 74069510 | DDIT4 (+35,651), DNAJB12 (+45,578) |
| SEQ ID NO: 109 | chr10 | 99790636 | 99790963 | CRTAC1 (-215) |
| SEQ ID NO: 110 | chr10 | 102497304 | 102497504 | PAX2 (-8,064), HIF1AN (+201,788) |
| SEQ ID NO: 111 | chr10 | 103986463 | 103986663 | ELOVL3 (+478) |
| SEQ ID NO: 112 | chr10 | 105036590 | 105036794 | INA (-228) |
| SEQ ID NO: 113 | chr10 | 124896740 | 124897020 | HMX2 (-10,758), HMX3 (+1,402) |
| SEQ ID NO: 114 | chr10 | 124905504 | 124905704 | HMX2 (-2,034) |
| SEQ ID NO: 115 | chr10 | 130084908 | 130085108 | MKI67 (-160,359) |
| SEQ ID NO: 116 | chr10 | 134016194 | 134016408 | DPYSL4 (+15,897), STK32C (+105,143) |
| SEQ ID NO: 117 | chr11 | 2181981 | 2182295 | INS (+296), INS-IGF2 (+301) |
| SEQ ID NO: 118 | chr11 | 2292332 | 2292651 | ASCL2 (-310) |
| SEQ ID NO: 119 | chr11 | 31839396 | 31839726 | PAX6 (-52) |
| SEQ ID NO: 120 | chr11 | 73099779 | 73099979 | RELT (+12,570), FAM168A (+209,349) |
| SEQ ID NO: 121 | chr11 | 132813724 | 132813924 | OPCML (-258) |
| SEQ ID NO: 122 | chr12 | 52311647 | 52311991 | ACVR1B (-33,666), ACVRL1 (+10,617) |

(continued)

| Serial No. | Chromoso me | Starting position | Ending position | Gene annotation |
|---|---|---|---|---|
| SEQ ID NO: 123 | chr12 | 63544037 | 63544348 | AVPR1A (+529) |
| SEQ ID NO: 124 | chr12 | 113902107 | 113902307 | LHX5 (+7,670), SDSL (+42,165) |
| SEQ ID NO: 125 | chr13 | 111186630 | 111186830 | RAB20 (+27,350), COL4A2 (+227,116) |
| SEQ ID NO: 126 | chr13 | 111277395 | 111277690 | CARKD (+9,535), CARS2 (+80,961) |
| SEQ ID NO: 127 | chr13 | 112711391 | 112711603 | SOX1 (-10,416), TEX29 (+738,482) |
| SEQ ID NO: 128 | chr13 | 112758741 | 112758954 | SPACA7 (-271,785), SOX1 (+36,935) |
| SEQ ID NO: 129 | chr13 | 112759950 | 112760185 | SPACA7 (-270,565), SOX1 (+38,155) |
| SEQ ID NO: 130 | chr14 | 36986598 | 36986864 | SFTA3 (-3,697) |
| SEQ ID NO: 131 | chr14 | 60976665 | 60976952 | SIX6 (+1,140), SIX1 (+139,371) |
| SEQ ID NO: 132 | chr14 | 105102449 | 105102649 | INF2 (-53,425), TMEM179 (-30,565) |
| SEQ ID NO: 133 | chr14 | 105933655 | 105933855 | CRIP2 (-5,544), MTA1 (+47,596) |
| SEQ ID NO: 134 | chr15 | 68114350 | 68114550 | PIAS1 (-232,067), SKOR1 (+2,408) |
| SEQ ID NO: 135 | chr15 | 68121381 | 68121679 | PIAS1 (-224,987), SKOR1 (+9,488) |
| SEQ ID NO: 136 | chr15 | 68121923 | 68122316 | PIAS1 (-224,397), SKOR1 (+10,078) |
| SEQ ID NO: 137 | chr15 | 76635120 | 76635744 | ISL2 (+6,367), SCAPER (+562,244) |
| SEQ ID NO: 138 | chr15 | 89952386 | 89952646 | POLG (-74,438), RHCG (+87,328) |
| SEQ ID NO: 139 | chr15 | 96856960 | 96857162 | NR2F2 (-16,885) |
| SEQ ID NO: 140 | chr16 | 630128 | 630451 | RAB40C (-9,067), PIGQ (+10,272) |
| SEQ ID NO: 141 | chr16 | 57025884 | 57026193 | CPNE2 (-100,480), NLRC5 (+2,629) |
| SEQ ID NO: 142 | chr16 | 67919979 | 67920237 | PSKH1 (-7,067), NRN1L (+1,400) |
| SEQ ID NO: 143 | chr17 | 2092044 | 2092244 | SRR (-114,854), HIC1 (+132,540) |
| SEQ ID NO: 144 | chr17 | 46796653 | 46796853 | HOXB9 (-92,914), PRAC1 (+3,131) |

(continued)

| Serial No. | Chromoso me | Starting position | Ending position | Gene annotation |
|---|---|---|---|---|
| SEQ ID NO: 145 | chr17 | 73607909 | 73608115 | SMIMS (-24,663), MYO15B (+9,414) |
| SEQ ID NO: 146 | chr17 | 75369368 | 75370149 | TNRC6C (-631,378), SEPT9 (+92,267) |
| SEQ ID NO: 147 | chr17 | 80745056 | 80745446 | TBCD (+35,311), ZNF750 (+53,203) |
| SEQ ID NO: 148 | chr18 | 24130835 | 24131035 | KCTD1 (-1,536) |
| SEQ ID NO: 149 | chr18 | 76739171 | 76739371 | SALL3 (-1,004) |
| SEQ ID NO: 150 | chr18 | 77256428 | 77256628 | CTDP1 (-183,273), NFATC1 (+96,192) |
| SEQ ID NO: 151 | chr19 | 2800642 | 2800863 | ZNF554 (-19,119), THOP1 (+15,295) |
| SEQ ID NO: 152 | chr19 | 3688030 | 3688230 | CACTIN (-61,317), PIP5K1C (+12,347) |
| SEQ ID NO: 153 | chr19 | 4912069 | 4912269 | KDM4B (-56,963), PLIN3 (-44,389) |
| SEQ ID NO: 154 | chr19 | 16511819 | 16512143 | EPS15L1 (+70,842), KLF2 (+76,353) |
| SEQ ID NO: 155 | chr19 | 55593132 | 55593428 | EPS8L1 (+6,011), PPP1R12C (+35,647) |
| SEQ ID NO: 156 | chr20 | 21492735 | 21492935 | NKX2-4 (-114,169), NKX2-2 (+1,829) |
| SEQ ID NO: 157 | chr20 | 55202107 | 55202685 | TFAP2C (-1,962) |
| SEQ ID NO: 158 | chr20 | 55925328 | 55925530 | RAE1 (-637) |
| SEQ ID NO: 159 | chr20 | 62330559 | 62330808 | TNFRSF6B (+2,663), ARFRP1 (+8,326) |
| SEQ ID NO: 160 | chr22 | 36861325 | 36861709 | MYH9 (-77,454), TXN2 (+16,560) |

The methylation level of the methylation marker region increased or decreased in pancreatic cancer cfDNA (see Table 3-3). The sequences of the obtained 101 methylation markers are as set forth in SEQ ID NOs: 60-160. The methylation levels of all CpG sites of each methylation marker can be obtained by MethylTitan methylation sequencing. The average methylation level of all CpG sites in each region, as well as the methylation level of a single CpG site, can both be used as a marker for pancreatic cancer.

Table 3-3 Methylation levels of methylation markers in pancreatic cancer in the training set and the test set

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 60 | 0.82373067 | 0.85751849 | 1.09E-06 | 0.81966101 | 0.86497135 | 1.85E-06 |
| SEQ ID NO: 61 | 0.00422647 | 0.00338352 | 2.31E-06 | 0.00448467 | 0.0034 | 3.39E-06 |
| SEQ ID NO: 62 | 0.02252656 | 0.01623844 | 895E-09 | 0.02307998 | 0.01837146 | 5.91E-05 |
| SEQ ID NO: 63 | 0.00275101 | 0.0008819 | 1.78E-07 | 0.00218178 | 0.00098158 | 3.84E-05 |
| SEQ ID NO: 64 | 0.00900877 | 0.00363731 | 1.06E-06 | 0.00829831 | 0.0033292 | 2.57E-05 |
| SEQ ID NO: 65 | 0.00435137 | 0.00069153 | 2.39E-07 | 0.00448689 | 0.00093841 | 2.69E-06 |
| SEQ ID NO: 66 | 0.003317 | 0.00098353 | 2.17E-07 | 0.00499834 | 0.00131321 | 7.90E-06 |
| SEQ ID NO: 67 | 0.23967459 | 0.1789925 | 2.69E-15 | 0.22905332 | 0.18176365 | 8.82E-12 |
| SEQ ID NO: 68 | 0.00551876 | 0.00120337 | 2.26E-08 | 0.00615114 | 0.00199402 | 1.35E-05 |
| SEQ ID NO: 69 | 0.0028249 | 0.00014991 | 4.26E-07 | 0.00161653 | 0.00019708 | 0.00014527 |
| SEQ ID NO: 70 | 0.00215817 | 0.00022747 | 2.64E-06 | 0.00336076 | 0.00016595 | 2.57E-06 |

(continued)

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 71 | 0.01125176 | 0.00552721 | 1.96E-07 | 0.01066098 | 0.00614414 | 0.0001233 |
| SEQ ID NO: 72 | 0.00178729 | 0.00068784 | 6.68E-07 | 0.00204761 | 0.00076546 | 8.65E-05 |
| SEQ ID NO: 73 | 0.02428677 | 0.01554514 | 4.13E-08 | 0.02244006 | 0.01573139 | 2.99E-07 |
| SEQ ID NO: 74 | 0.15087918 | 0.18430182 | 2.56E-05 | 0.1401783 | 0.19419159 | 7.91E-08 |
| SEQ ID NO: 75 | 0.01181004 | 0.00330796 | 4.57E-07 | 0.01300735 | 0.00486442 | 2.09E-05 |
| SEQ ID NO: 76 | 0.00385356 | 0.00115473 | 6.70E-07 | 0.00401929 | 0 | 7 2.85E-05 |
| SEQ ID NO: 77 | 0.31717172 | 0.4071511 | 7.06E-11 | 0.32853186 | 0.40697674 | 5.15E-11 |
| SEQ ID NO: 78 | 0.06244796 | 0.0430622 | 1.12E-08 | 0.06029757 | 0.0443996 | 5.91E-05 |
| SEQ ID NO: 79 | 0.00658467 | 0.00397489 | 2.47E-09 | 0.00594278 | 0.0042785 | 0.00106348 |
| SEQ ID NO: 80 | 0.00252685 | 0.00165901 | 2.68E-09 | 0.002439 | 0.00163347 | 1.06E-08 |
| SEQ ID NO: 81 | 0.01846223 | 0.01303351 | 6.52E-07 | 0.01987061 | 0.01217915 | 6.07E-06 |

(continued)

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 82 | 0.02265101 | 0.01278805 | 5.96E-09 | 0.02482182 | 0.01380227 | 3.83E-08 |
| SEQ ID NO: 83 | 0.01178647 | 0.0018438 | 1.08E-08 | 0.0063001 | 0.00202986 | 2.79E-05 |
| SEQ ID NO: 84 | 0.02212389 | 0.00787402 | 1.33E-06 | 0.02136752 | 0.00584795 | 4.18E-05 |
| SEQ ID NO: 85 | 0.03535918 | 0.02680765 | 2.54E-09 | 0.0324843 | 0.02897168 | 0.00816849 |
| SEQ ID NO: 86 | 0.01393244 | 0.01099045 | 4.80E-07 | 0.01403699 | 0.01061595 | 8.33E-05 |
| SEQ ID NO: 87 | 0.01704967 | 0.0071599 | 1.43E-06 | 0.01854305 | 0.00815047 | 1.85E-7 06 |
| SEQ ID NO: 88 | 0.00498337 | 0.00174847 | 2.92E-09 | 0.00454174 | 0.00201865 | 2.31E-07 |
| SEQ ID NO: 89 | 0.00499213 | 0.0027002 | 1.31E-06 | 0.0062411 | 0.00252838 | 4.54E-09 |
| SEQ ID NO: 90 | 0.00719424 | 0.00204499 | 1.91E-08 | 0.00791139 | 0.00298211 | 0.00059236 |
| SEQ ID NO: 91 | 0.02641691 | 0.02068176 | 1.89E-08 | 0.02458021 | 0.02120684 | 0.00201115 |
| SEQ ID NO: 92 | 0.19890261 | 0.16853385 | 3.96E-07 | 0.2186405 | 0.17086591 | 6.17E-09 |

(continued)

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 93 | 0.0192147 | 0.00066711 | 2.57E-08 | 0.01620746 | 0.00132275 | 1.48E-05 |
| SEQ ID NO: 94 | 0.00049287 | 1.86E-05 | 2.01E-07 | 0.00054266 | 1.56E-05 | 4.36E-10 |
| SEQ ID NO: 95 | 0.03361345 | 0.01538462 | 2.03E-05 | 0.04918033 | 0.01709402 | 1.67E-08 |
| SEQ ID NO: 96 | 0.00476161 | 0.00130935 | 7.06E-11 | 0.00471794 | 0.00146201 | 3.24E-06 |
| SEQ ID NO: 97 | 0.97061224 | 0.98041834 | 1.09E-08 | 0.97198599 | 0.9787234 | 0.00019375 |
| SEQ ID NO: 98 | 0.0052702 | 0.00166204 | 9.26E-07 | 0.00514466 | 0.00189901 | 9.81E-06 |
| SEQ ID NO: 99 | 0.00521032 | 0.00145114 | 1.99E-08 | 0.00409251 | 0.00165181 | 0.00014007 |
| SEQ ID NO: 100 | 0.02294348 | 0.01429529 | 8.26E-09 | 0.02465555 | 0.01431193 | 1.70E-05 |
| SEQ ID NO: 101 | 0.09486781 | 0.19602978 | 1.48E-11 | 0.09484536 | 0.18716578 | 6.10E-11 |
| SEQ ID NO: 102 | 0.02619601 | 0.0163879 | 9.09E-08 | 0.03325942 | 0.0169506 | 1.35E-08 |
| SEQ ID NO: 103 | 0.02634016 | 0.01619835 | 9.09E-08 | 0.0331343 | 0.01694769 | 1.71E-08 |

(continued)

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 104 | 0.00997314 | 0.00283686 | 3.43E-07 | 0.01249569 | 0.00342328 | 0.00010828 |
| SEQ ID NO: 105 | 0.00252237 | 0.00045651 | 6.68E-07 | 0.00282189 | 0.00059216 | 2.09E-05 |
| SEQ ID NO: 106 | 0.00114108 | 4.26E-05 | 5.40E-07 | 0.0015606 | 5.32E-05 | 5.47E-05 |
| SEQ ID NO: 107 | 0.00856073 | 0.00256246 | 3.42E-07 | 0.00990099 | 0.003861 | 1.71E-05 |
| SEQ ID NO: 108 | 0.28023407 | 0.21170732 | 5.36E-11 | 0.29900839 | 0.22271147 | 2.42E-09 |
| SEQ ID NO: 109 | 0.0424092 | 0.02860803 | 1.14E-08 | 0.0439036 | 0.02844689 | 1.16E-07 |
| SEQ ID NO: 110 | 0.00064526 | 0.00031037 | 1.01E-07 | 0.00060562 | 0.00032366 | 2.37E-05 |
| SEQ ID NO: 111 | 0.10916922 | 0.24085613 | 1.15E-09 | 0.11234316 | 0.22166523 | 0.00016195 |
| SEQ ID NO: 112 | 0.01485662 | 0.01099437 | 3.27E-07 | 0.01536 | 0.01093863 | 4.68E-05 |
| SEQ ID NO: 113 | 0.02176625 | 0.00244362 | 1.71E-09 | 0.02520301 | 0.00399935 | 1.61E-08 |
| SEQ ID NO: 114 | 0.00831202 | 0.00121359 | 8.87E-08 | 0.00878906 | 0.0032 | 6.71E-05 |

(continued)

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 115 | 0.02676277 | 0.0191044 | 6.89E-10 | 0.02404265 | 0.01881775 | 1.32E-05 |
| SEQ ID NO: 116 | 0.25073206 | 0.21964051 | 2.33E-08 | 0.24941397 | 0.21802935 | 2.45E-06 |
| SEQ ID NO: 117 | 0.00134224 | 0.00040418 | 2.52E-08 | 0.00091536 | 0.00034119 | 0.00019375 |
| SEQ ID NO: 118 | 0.00458594 | 0.00015011 | 1.34E-06 | 0.00552597 | 0.00010777 | 6.39E-07 |
| SEQ ID NO: 119 | 0.00336652 | 0.00180542 | 2.33E-08 | 0.00334388 | 0.0018575 | 0.00044407 |
| SEQ ID NO: 120 | 0.2578125 | 0.52083333 | 19-11-13 | 0.27027027 | 0.49545455 | 6.27E-09 |
| SEQ ID NO: 121 | 0.01818182 | 0 | 8.02E-08 | 0.01290323 | 0.00346021 | 7.04E-05 |
| SEQ ID NO: 122 | 0.15543203 | 0.25349825 | 1.01E-07 | 0.1346129 | 0.2294904 | 3.67E-07 |
| SEQ ID NO: 123 | 0.01204819 | 0.00274725 | 1.07E-06 | 0.02216066 | 0.00373134 | 1.83E-06 |
| SEQ ID NO: 124 | 0.03231732 | 0.02511309 | 2.63E-10 | 0.03114808 | 0.0260203 | 1.21E-06 |
| SEQ ID NO: 125 | 0.00566397 | 0.00307994 | 7.41E-09 | 0.0050168 | 0.00365739 | 0.00445114 |

(continued)

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 126 | 0.94678614 | 0.9583787 | 2.68E-14 | 0.94469098 | 0.95835066 | 5.12E-13 |
| SEQ ID NO: 127 | 0.04160247 | 0.01156069 | 2.83E-07 | 0.03602058 | 0.01886792 | 0.00011515 |
| SEQ ID NO: 128 | 0.01030928 | 0.00208189 | 8.11E-08 | 0.00888395 | 0.00349895 | 3.53E-05 |
| SEQ ID NO: 129 | 0.00392456 | 0.00169606 | 3.72E-08 | 0.00359362 | 0.00217744 | 0.00028516 |
| SEQ ID NO: 130 | 0.01060305 | 0.00228571 | 3.80E-08 | 0.00975434 | 0.00317209 | 4.28E-06 |
| SEQ ID NO: 131 | 0.00224463 | 0.00128461 | 6.61E-06 | 0.00256043 | 0.00115094 | 1.29E-07 |
| SEQ ID NO: 132 | 0.01117031 | 0.00897862 | 2.83E-07 | 0.01085661 | 0.00884113 | 1.63E-05 |
| SEQ ID NO: 133 | 0.93196174 | 0.94088746 | 5.34E-08 | 0.93135784 | 0.94047703 | 7.88E-09 |
| SEQ ID NO: 134 | 0.00669344 | 0 | 1.54E-09 | 0.00437158 | 0 | 2.48E-05 |
| SEQ ID NO: 135 | 0.00465319 | 0.00065683 | 7.05E-06 | 0.00613092 | 0.0008653 | 1.36E-07 |
| SEQ ID NO: 136 | 0.00909091 | 0.00067705 | 1.32E-09 | 0.00813008 | 0.00148588 | 7.00E-07 |

(continued)

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 137 | 0.02396804 | 0.00646552 | 9.40E-10 | 0.02583026 | 0.01020408 | 3.88E-06 |
| SEQ ID NO: 138 | 0.0003891 | 8.64E-05 | 1.61E-06 | 0.00055372 | 0.00011055 | 1.02E-05 |
| SEQ ID NO: 139 | 0.1598513 | 0.21118012 | 7.25E-07 | 0.17195767 | 0.21818182 | 3.02E-05 |
| SEQ ID NO: 140 | 0.00018254 | 0.00012983 | 3.96E-07 | 0.00016045 | 0.00012115 | 4.32E-05 |
| SEQ ID NO: 141 | 0.85239931 | 0.78224274 | 5.48E-08 | 0.85606061 | 0.78532749 | 9.13E-10 |
| SEQ ID NO: 142 | 0.15508329 | 0.12669039 | 5.94E-06 | 0.15310078 | 0.11932203 | 1.27E-06 |
| SEQ ID NO: 143 | 0.90582192 | 0.8245614 | 1.07E-08 | 0.90669371 | 0.84391081 | 2.69E-06 |
| SEQ ID NO: 144 | 0.01746725 | 0.00883002 | 1.54E-05 | 0.01495163 | 0.0077821 | 1.15E-06 |
| SEQ ID NO: 145 | 0.94989748 | 0.96148844 | 1.14E-11 | 0.94640006 | 0.9597437 | 3.83E-08 |
| SEQ ID NO: 146 | 0.08468312 | 0.07302075 | 6.89E-08 | 0.08874743 | 0.07260726 | 9.95E-07 |
| SEQ ID NO: 147 | 0.00556635 | 0.00395993 | 6.89E-10 | 0.00538181 | 0.00373748 | 2.04E-08 |

(continued)

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 148 | 0.0032219 | 0.00235948 | 1.06E-06 | 0.0034959 | 0.00232258 | 9.00E-06 |
| SEQ ID NO: 149 | 0.02113182 | 0.0146704 | 3.78E-07 | 0.02319849 | 0.01422394 | 1.44E-05 |
| SEQ ID NO: 150 | 0.0104712 | 0.00263158 | 4.49E-06 | 0.00712589 | 0 | 3.73E-05 |
| SEQ ID NO: 151 | 0.00013792 | 9.91E-05 | 1.57E-05 | 0.00015358 | 9.98E-05 | 8.18E-07 |
| SEQ ID NO: 152 | 0.31430901 | 0.40820734 | 1.42E-07 | 0.30192235 | 0.39311682 | 3.49E-07 |
| SEQ ID NO: 153 | 0.48933144 | 0.56835938 | 1.93E-10 | 0.48435814 | 0.5465995 | 1.98E-06 |
| SEQ ID NO: 154 | 0.00983359 | 0.00367309 | 3.02E-08 | 0.00848896 | 0.00466744 | 0.00036008 |
| SEQ ID NO: 155 | 0.01250085 | 0.00589491 | 2.52E-08 | 0.01422469 | 0.00643813 | 3.54E-06 |
| SEQ ID NO: 156 | 0.01501761 | 0.00269123 | 6.32E-10 | 0.01048249 | 0.00233003 | 0.00014007 |
| SEQ ID NO: 157 | 0.00539084 | 0.00120337 | 1.61E-06 | 0.00624025 | 0.00116279 | 1.19E-06 |
| SEQ ID NO: 158 | 0.10661269 | 0.07042254 | 2.76E-09 | 0.11753731 | 0.08276798 | 6.72E-07 |

(continued)

| Serial No. | Pancreatic cancer methylation levels in training set | Non-pancreatic cancer methylation levels in training set | Training set P value | Pancreatic cancer methylation levels in test set | Non-pancreatic cancer methylation levels in test set | Test set P value |
|---|---|---|---|---|---|---|
| SEQ ID NO: 159 | 0.85753138 | 0.8999533 | 2.88E-10 | 0.87342162 | 0.8933043 | 2.19E-07 |
| SEQ ID NO: 160 | 0.1625 | 0.142068-16 | 5.53E-07 | 0.16257769 | 0.14026885 | 2.24E-06 |

As can be seen from Table 3-3, the distribution of average methylation levels in the methylation marker region is significantly different between people with pancreatic cancer and those without pancreatic cancer, with good differentiating effect and significant difference (P < 0.01), so that it is a good methylation marker for pancreatic cancer.

3-2: Differentiating ability of single methylation markers

In order to verify the ability of a single methylation marker to differentiating pancreatic cancer from the absence of pancreatic cancer, the methylation level data of a single marker was used to train the model in the training set data of Example 3-1, and the test set samples were used to verify the performance of the model.

The logistic regression model in the sklearn (V1.0.1) package in python (V3.9.7) was used: model=LogisticRegression(). The formula of the model is as follows, where x is the methylation level value of the sample target marker, and w is the coefficient of different markers, b is the intercept value, and y is the model prediction score:

$$y = \frac{1}{1 + e^{(-w^T x + b)}}$$

Training was conducted using samples from the training set: model.fit (Traindata, TrainPheno), where TrainData is the data of the target methylation site in the training set samples, and TrainPheno is the trait of the training set samples (1 for pancreatic cancer, 0 for absence of pancreatic cancer). The relevant threshold of the model was determined based on the samples of the training set.

Testing was conducted using the samples of the test set: TestPred = model.predict_proba(TestData)[:, 1], where TestData is the data of the target methylation site in the test set samples, and TestPred is the model prediction score. Whether the sample is pancreatic cancer or not was determined using this prediction score based on the above threshold.

The effect of the logistic regression model of single methylation markers in this example is shown in Table 3-4. From this table, it can be seen that the AUC values of all methylation markers can reach more than 0.55 in both the test set and the training set, and they are all good markers of pancreatic cancer.

Each single methylation marker in this patent can be used as a pancreatic cancer marker. Logistic regression modeling is used to set a threshold according to the training set. If the score is greater than the threshold, it is predicted to be pancreatic cancer, and vice versa, it is predicted to be absence of pancreatic cancer, the training set and the test set can achieve very good accuracy, specificity and sensitivity, and other machine learning models can also achieve similar results.

Table 3-4. Performance of logistic regression models for single methylation markers

| Serial No. | Training set AUC | Test set AUC | Thres hold | Training set accuracy | Training set specificity | Training set sensitivity | Test set accuracy | Test set specificity | Test set sensitivity |
|---|---|---|---|---|---|---|---|---|---|
| SEQ ID NO: 126 | 0.885 | 0.907 | 0.522 | 0.833 | 0.873 | 0.797 | 0.875 | 0.915 | 0.829 |
| SEQ ID NO: 101 | 0.841 | 0.906 | 0.531 | 0.803 | 0.810 | 0.826 | 0.841 | 0.830 | 0.854 |
| SEQ ID NO: 67 | 0.899 | 0.889 | 0.524 | 0.841 | 0.952 | 0.754 | 0.784 | 0.872 | 0.683 |
| SEQ ID NO: 77 | 0.829 | 0.878 | 0.517 | 0.788 | 0.841 | 0.783 | 0.761 | 0.787 | 0.732 |
| SEQ ID NO: 94 | 0.763 | 0.862 | 0.514 | 0.727 | 0.841 | 0.623 | 0.773 | 0.915 | 0.610 |
| SEQ ID NO: 120 | 0.871 | 0.861 | 0.530 | 0.833 | 0.873 | 0.797 | 0.784 | 0.830 | 0.732 |
| SEQ ID NO: 141 | 0.775 | 0.856 | 0.531 | 0.765 | 0.825 | 0.710 | 0.773 | 0.809 | 0.732 |
| SEQ ID NO: 95 | 0.715 | 0.850 | 0.522 | 0.682 | 0.794 | 0.609 | 0.784 | 0.787 | 0.780 |
| SEQ ID NO: 108 | 0.831 | 0.848 | 0.519 | 0.795 | 0.841 | 0.754 | 0.727 | 0.681 | 0.780 |
| SEQ ID NO: 89 | 0.744 | 0.843 | 0.520 | 0.720 | 0.873 | 0.580 | 0.739 | 0.851 | 0.610 |
| SEQ ID NO: 92 | 0.756 | 0.841 | 0.519 | 0.735 | 0.667 | 0.797 | 0.705 | 0.574 | 0.854 |
| SEQ ID NO: 133 | 0.775 | 0.839 | 0.521 | 0.735 | 0.746 | 0.725 | 0.716 | 0.638 | 0.805 |
| SEQ ID NO: 80 | 0.801 | 0.836 | 0.522 | 0.758 | 0.651 | 0.870 | 0.727 | 0.574 | 0.902 |
| SEQ ID NO: 102 | 0.770 | 0.834 | 0.516 | 0.705 | 0.714 | 0.739 | 0.693 | 0.553 | 0.854 |

EP 4 372 103 A1

| Serial No. | Training set AUC | Test set AUC | Thres hold | Training set accuracy | Training set specificity | Training set sensitivity | Test set accuracy | Test set specificity | Test set sensitivity |
|---|---|---|---|---|---|---|---|---|---|
| SEQ ID NO: 113 | 0.804 | 0.832 | 0.511 | 0.712 | 0.746 | 0.739 | 0.739 | 0.660 | 0.829 |
| SEQ ID NO: 103 | 0.770 | 0.832 | 0.516 | 0.720 | 0.714 | 0.725 | 0.682 | 0.553 | 0.829 |
| SEQ ID NO: 147 | 0.812 | 0.830 | 0.522 | 0.758 | 0.889 | 0.667 | 0.739 | 0.745 | 0.732 |
| SEQ ID NO: 145 | 0.843 | 0.825 | 0.519 | 0.765 | 0.937 | 0.696 | 0.750 | 0.809 | 0.683 |
| SEQ ID NO: 82 | 0.794 | 0.825 | 0.513 | 0.773 | 0.857 | 0.710 | 0.705 | 0.702 | 0.707 |
| SEQ ID NO: 74 | 0.713 | 0.818 | 0.524 | 0.705 | 0.730 | 0.681 | 0.773 | 0.787 | 0.756 |
| SEQ ID NO: 109 | 0.788 | 0.814 | 0.511 | 0.750 | 0.698 | 0.797 | 0.739 | 0.702 | 0.780 |
| SEQ ID NO: 131 | 0.728 | 0.813 | 0.522 | 0.697 | 0.825 | 0.594 | 0.716 | 0.830 | 0.585 |
| SEQ ID NO: 135 | 0.727 | 0.813 | 0.517 | 0.682 | 0.857 | 0.522 | 0.750 | 0.894 | 0.585 |
| SEQ ID NO: 159 | 0.818 | 0.808 | 0.514 | 0.773 | 0.794 | 0.754 | 0.784 | 0.830 | 0.732 |
| SEQ ID NO: 88 | 0.800 | 0.807 | 0.520 | 0.758 | 0.794 | 0.725 | 0.705 | 0.681 | 0.732 |
| SEQ ID NO: 136 | 0.801 | 0.807 | 0.516 | 0.780 | 0.905 | 0.681 | 0.727 | 0.787 | 0.659 |
| SEQ ID NO: 73 | 0.777 | 0.805 | 0.515 | 0.727 | 0.778 | 0.681 | 0.716 | 0.702 | 0.732 |
| SEQ ID NO: 152 | 0.766 | 0.803 | 0.521 | 0.742 | 0.778 | 0.710 | 0.693 | 0.617 | 0.780 |

| Serial No. | Training set AUC | Test set AUC | Thres hold | Training set accuracy | Training set specificity | Training set sensitivity | Test set accuracy | Test set specificity | Test set sensitivity |
|---|---|---|---|---|---|---|---|---|---|
| SEQ ID NO: 122 | 0.769 | 0.803 | 0.511 | 0.750 | 0.651 | 0.841 | 0.693 | 0.574 | 0.829 |
| SEQ ID NO: 157 | 0.740 | 0.801 | 0.518 | 0.705 | 0.778 | 0.638 | 0.716 | 0.745 | 0.683 |
| SEQ ID NO: 118 | 0.744 | 0.797 | 0.512 | 0.720 | 0.762 | 0.696 | 0.727 | 0.745 | 0.707 |
| SEQ ID NO: 158 | 0.800 | 0.797 | 0.522 | 0.750 | 0.841 | 0.696 | 0.727 | 0.702 | 0.756 |
| SEQ ID NO: 153 | 0.822 | 0.795 | 0.512 | 0.727 | 0.778 | 0.725 | 0.682 | 0.574 | 0.805 |
| SEQ ID NO: 151 | 0.718 | 0.794 | 0.523 | 0.667 | 0.714 | 0.652 | 0.727 | 0.723 | 0.732 |
| SEQ ID NO: 123 | 0.744 | 0.794 | 0.510 | 0.720 | 0.698 | 0.739 | 0.693 | 0.574 | 0.829 |
| SEQ ID NO: 146 | 0.772 | 0.792 | 0.522 | 0.720 | 0.730 | 0.710 | 0.705 | 0.617 | 0.805 |
| SEQ ID NO: 144 | 0.718 | 0.791 | 0.515 | 0.697 | 0.746 | 0.652 | 0.716 | 0.787 | 0.634 |
| SEQ ID NO: 124 | 0.819 | 0.790 | 0.518 | 0.773 | 0.746 | 0.797 | 0.739 | 0.660 | 0.829 |
| SEQ ID NO: 142 | 0.729 | 0.790 | 0.521 | 0.727 | 0.667 | 0.783 | 0.727 | 0.681 | 0.780 |
| SEQ ID NO: 60 | 0.746 | 0.786 | 0.515 | 0.705 | 0.762 | 0.667 | 0.716 | 0.723 | 0.707 |
| SEQ ID NO: 87 | 0.744 | 0.786 | 0.514 | 0.697 | 0.571 | 0.826 | 0.670 | 0.511 | 0.854 |
| SEQ ID NO: 130 | 0.777 | 0.785 | 0.516 | 0.735 | 0.841 | 0.652 | 0.773 | 0.809 | 0.732 |

(continued)

| Serial No. | Training set AUC | Test set AUC | Thres hold | Training set accuracy | Training set specificity | Training set sensitivity | Test set accuracy | Test set specificity | Test set sensitivity |
|---|---|---|---|---|---|---|---|---|---|
| SEQ ID NO: 160 | 0.753 | 0.784 | 0.519 | 0.705 | 0.683 | 0.768 | 0.727 | 0.702 | 0.756 |
| SEQ ID NO: 116 | 0.782 | 0.783 | 0.523 | 0.742 | 0.841 | 0.667 | 0.716 | 0.766 | 0.659 |
| SEQ ID NO: 70 | 0.737 | 0.782 | 0.513 | 0.712 | 0.714 | 0.725 | 0.716 | 0.723 | 0.707 |
| SEQ ID NO: 143 | 0.789 | 0.782 | 0.538 | 0.735 | 0.825 | 0.667 | 0.761 | 0.830 | 0.683 |
| SEQ ID NO: 65 | 0.761 | 0.782 | 0.522 | 0.720 | 0.857 | 0.609 | 0.727 | 0.830 | 0.610 |
| SEQ ID NO: 96 | 0.829 | 0.779 | 0.521 | 0.811 | 0.905 | 0.725 | 0.750 | 0.851 | 0.634 |
| SEQ ID NO: 61 | 0.739 | 0.779 | 0.523 | 0.667 | 0.524 | 0.855 | 0.693 | 0.468 | 0.951 |
| SEQ ID NO: 155 | 0.781 | 0.778 | 0.519 | 0.742 | 0.698 | 0.783 | 0.727 | 0.766 | 0.683 |
| SEQ ID NO: 137 | 0.809 | 0.777 | 0.508 | 0.750 | 0.794 | 0.710 | 0.670 | 0.660 | 0.683 |
| SEQ ID NO: 81 | 0.751 | 0.772 | 0.517 | 0.682 | 0.794 | 0.623 | 0.682 | 0.766 | 0.585 |
| SEQ ID NO: 68 | 0.782 | 0.770 | 0.517 | 0.750 | 0.746 | 0.768 | 0.648 | 0.617 | 0.683 |
| SEQ ID NO: 66 | 0.762 | 0.769 | 0.519 | 0.705 | 0.762 | 0.652 | 0.705 | 0.702 | 0.707 |
| SEQ ID NO: 148 | 0.746 | 0.768 | 0.522 | 0.659 | 0.698 | 0.652 | 0.682 | 0.638 | 0.732 |
| SEQ ID NO: 107 | 0.758 | 0.767 | 0.520 | 0.705 | 0.651 | 0.754 | 0.648 | 0.447 | 0.878 |

| Serial No. | Training set AUC | Test set AUC | Thres hold | Training set accuracy | Training set specificity | Training set sensitivity | Test set accuracy | Test set specificity | Test set sensitivity |
|---|---|---|---|---|---|---|---|---|---|
| SEQ ID NO: 98 | 0.748 | 0.766 | 0.520 | 0.705 | 0.810 | 0.609 | 0.727 | 0.809 | 0.634 |
| SEQ ID NO: 93 | 0.779 | 0.766 | 0.507 | 0.720 | 0.651 | 0.783 | 0.670 | 0.574 | 0.780 |
| SEQ ID NO: 138 | 0.742 | 0.766 | 0.522 | 0.674 | 0.683 | 0.696 | 0.636 | 0.532 | 0.756 |
| SEQ ID NO: 115 | 0.812 | 0.763 | 0.519 | 0.735 | 0.841 | 0.667 | 0.670 | 0.766 | 0.561 |
| SEQ ID NO: 149 | 0.757 | 0.762 | 0.516 | 0.705 | 0.762 | 0.681 | 0.670 | 0.660 | 0.683 |
| SEQ ID NO: 132 | 0.759 | 0.760 | 0.522 | 0.705 | 0.698 | 0.725 | 0.693 | 0.660 | 0.732 |
| SEQ ID NO: 100 | 0.791 | 0.760 | 0.514 | 0.689 | 0.730 | 0.739 | 0.670 | 0.596 | 0.756 |
| SEQ ID NO: 75 | 0.755 | 0.757 | 0.515 | 0.697 | 0.698 | 0.725 | 0.670 | 0.574 | 0.780 |
| SEQ ID NO: 105 | 0.751 | 0.757 | 0.516 | 0.712 | 0.762 | 0.681 | 0.750 | 0.702 | 0.805 |
| SEQ ID NO: 128 | 0.771 | 0.757 | 0.518 | 0.720 | 0.825 | 0.623 | 0.682 | 0.766 | 0.585 |
| SEQ ID NO: 110 | 0.769 | 0.756 | 0.523 | 0.735 | 0.794 | 0.681 | 0.693 | 0.681 | 0.707 |
| SEQ ID NO: 64 | 0.746 | 0.755 | 0.519 | 0.742 | 0.794 | 0.696 | 0.693 | 0.723 | 0.659 |
| SEQ ID NO: 83 | 0.789 | 0.754 | 0.518 | 0.742 | 0.762 | 0.739 | 0.659 | 0.660 | 0.659 |
| SEQ ID NO: 76 | 0.749 | 0.753 | 0.515 | 0.705 | 0.603 | 0.812 | 0.670 | 0.638 | 0.707 |

(continued)

| Serial No. | Training set AUC | Test set AUC | Thres hold | Training set accuracy | Training set specificity | Training set sensitivity | Test set accuracy | Test set specificity | Test set sensitivity |
|---|---|---|---|---|---|---|---|---|---|
| SEQ ID NO: 139 | 0.750 | 0.752 | 0.525 | 0.705 | 0.746 | 0.696 | 0.693 | 0.787 | 0.585 |
| SEQ ID NO: 84 | 0.744 | 0.752 | 0.517 | 0.712 | 0.873 | 0.580 | 0.682 | 0.787 | 0.561 |
| SEQ ID NO: 134 | 0.787 | 0.752 | 0.516 | 0.765 | 0.825 | 0.725 | 0.716 | 0.681 | 0.756 |
| SEQ ID NO: 150 | 0.730 | 0.750 | 0.522 | 0.727 | 0.778 | 0.681 | 0.716 | 0.894 | 0.512 |
| SEQ ID NO: 63 | 0.764 | 0.749 | 0.520 | 0.705 | 0.587 | 0.812 | 0.693 | 0.574 | 0.829 |
| SEQ ID NO: 140 | 0.756 | 0.748 | 0.523 | 0.674 | 0.746 | 0.652 | 0.682 | 0.766 | 0.585 |
| SEQ ID NO: 114 | 0.769 | 0.748 | 0.518 | 0.697 | 0.698 | 0.725 | 0.648 | 0.489 | 0.829 |
| SEQ ID NO: 112 | 0.758 | 0.747 | 0.522 | 0.705 | 0.825 | 0.623 | 0.705 | 0.766 | 0.634 |
| SEQ ID NO: 106 | 0.753 | 0.745 | 0.521 | 0.720 | 0.857 | 0.594 | 0.716 | 0.809 | 0.610 |
| SEQ ID NO: 62 | 0.790 | 0.744 | 0.521 | 0.742 | 0.714 | 0.768 | 0.648 | 0.553 | 0.756 |
| SEQ ID NO: 78 | 0.788 | 0.744 | 0.518 | 0.720 | 0.746 | 0.696 | 0.659 | 0.681 | 0.634 |
| SEQ ID NO: 121 | 0.763 | 0.740 | 0.511 | 0.727 | 0.762 | 0.696 | 0.705 | 0.723 | 0.683 |
| SEQ ID NO: 127 | 0.759 | 0.739 | 0.504 | 0.689 | 0.619 | 0.783 | 0.614 | 0.362 | 0.902 |
| SEQ ID NO: 86 | 0.754 | 0.739 | 0.520 | 0.682 | 0.714 | 0.681 | 0.670 | 0.596 | 0.756 |

(continued)

| Serial No. | Training set AUC | Test set AUC | Threshold | Training set accuracy | Training set specificity | Training set sensitivity | Test set accuracy | Test set specificity | Test set sensitivity |
|---|---|---|---|---|---|---|---|---|---|
| SEQ ID NO: 71 | 0.763 | 0.738 | 0.519 | 0.689 | 0.730 | 0.681 | 0.682 | 0.681 | 0.683 |
| SEQ ID NO: 72 | 0.751 | 0.738 | 0.522 | 0.720 | 0.857 | 0.594 | 0.670 | 0.787 | 0.537 |
| SEQ ID NO: 104 | 0.758 | 0.735 | 0.519 | 0.697 | 0.762 | 0.652 | 0.716 | 0.787 | 0.634 |
| SEQ ID NO: 156 | 0.812 | 0.732 | 0.513 | 0.780 | 0.714 | 0.855 | 0.648 | 0.574 | 0.732 |
| SEQ ID NO: 99 | 0.784 | 0.732 | 0.521 | 0.712 | 0.571 | 0.841 | 0.614 | 0.511 | 0.732 |
| SEQ ID NO: 69 | 0.755 | 0.731 | 0.511 | 0.727 | 0.778 | 0.696 | 0.739 | 0.809 | 0.659 |
| SEQ ID NO: 111 | 0.807 | 0.730 | 0.531 | 0.765 | 0.714 | 0.812 | 0.670 | 0.638 | 0.707 |
| SEQ ID NO: 97 | 0.789 | 0.727 | 0.521 | 0.727 | 0.778 | 0.696 | 0.648 | 0.702 | 0.585 |
| SEQ ID NO: 117 | 0.781 | 0.727 | 0.519 | 0.765 | 0.778 | 0.754 | 0.636 | 0.638 | 0.634 |
| SEQ ID NO: 154 | 0.780 | 0.722 | 0.521 | 0.697 | 0.873 | 0.565 | 0.670 | 0.851 | 0.463 |
| SEQ ID NO: 129 | 0.778 | 0.721 | 0.522 | 0.705 | 0.762 | 0.681 | 0.670 | 0.596 | 0.756 |
| SEQ ID NO: 119 | 0.782 | 0.715 | 0.521 | 0.697 | 0.714 | 0.725 | 0.648 | 0.596 | 0.707 |
| SEQ ID NO: 90 | 0.783 | 0.713 | 0.516 | 0.742 | 0.794 | 0.696 | 0.614 | 0.617 | 0.610 |
| SEQ ID NO: 79 | 0.801 | 0.701 | 0.521 | 0.795 | 0.905 | 0.696 | 0.636 | 0.702 | 0.561 |

(continued)

| Serial No. | Training set AUC | Test set AUC | Thres hold | Training set accuracy | Training set specificity | Training set sensitivity | Test set accuracy | Test set specificity | Test set sensitivity |
|---|---|---|---|---|---|---|---|---|---|
| SEQ ID NO: 91 | 0.784 | 0.690 | 0.519 | 0.750 | 0.714 | 0.812 | 0.591 | 0.553 | 0.634 |
| SEQ ID NO: 125 | 0.792 | 0.675 | 0.522 | 0.735 | 0.857 | 0.623 | 0.614 | 0.681 | 0.537 |
| SEQ ID NO: 85 | 0.801 | 0.663 | 0.522 | 0.727 | 0.683 | 0.797 | 0.614 | 0.553 | 0.683 |

3-3: Machine learning model for all target methylation markers

This example uses the methylation levels of all the 101 methylation markers to construct a logistic regression machine learning model MODEL1, which can accurately distinguish samples with pancreatic cancer and those without pancreatic cancer in the data. The specific steps are basically the same as Example 3-2, except that the data input model of the combination of all the 101 target methylation markers (SEQ ID NOs: 60-160) is used.

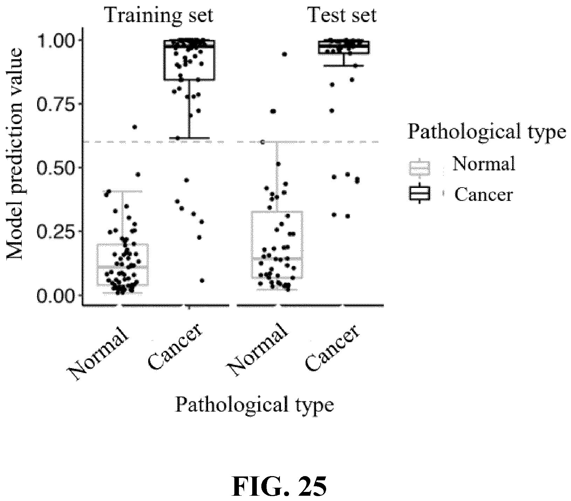

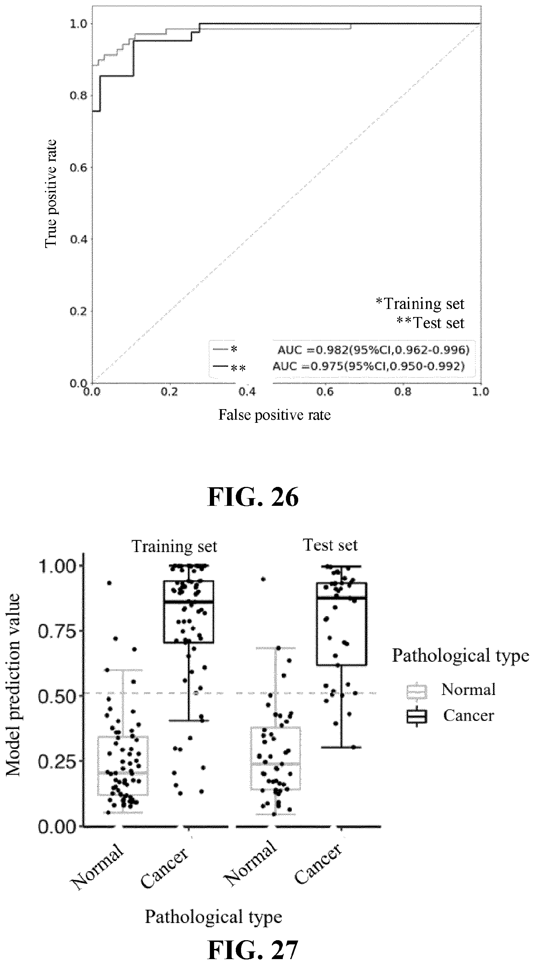

The distribution of model prediction scores in the training set and the test set is shown in Fig. 25. The ROC curve is shown in Fig. 26. In the training set, the AUC for differentiating samples with pancreatic cancer and those without pancreatic cancer samples reached 0.982. In the test set, the AUC for differentiating samples with pancreatic cancer and those without pancreatic cancer samples reached 0.975. The threshold was set to be 0.600, if the score is greater than this value, it is predicted as pancreatic cancer, otherwise it is predicted as absence of pancreatic cancer. Under this threshold, the training set accuracy is 0.939, the training set specificity is 0.984, the training set sensitivity is 0.899, the test set accuracy is 0.886, and the test set specificity is 0.915 , the test set sensitivity is 0.854, and the model can differentiate samples with pancreatic cancer and those without pancreatic cancer.

3-4: Machine learning model of methylation marker combination 1

In order to verify the effect of the relevant marker combination, in this example, a total of 6 methylation markers including SEQ ID NO: 113, SEQ ID NO: 124, SEQ ID NO: 67, SEQ ID NO: 77, SEQ ID NO: 80, SEQ ID NO: 96 were selected from all the 101 methylation markers based on methylation level to construct a logistic regression machine learning model.

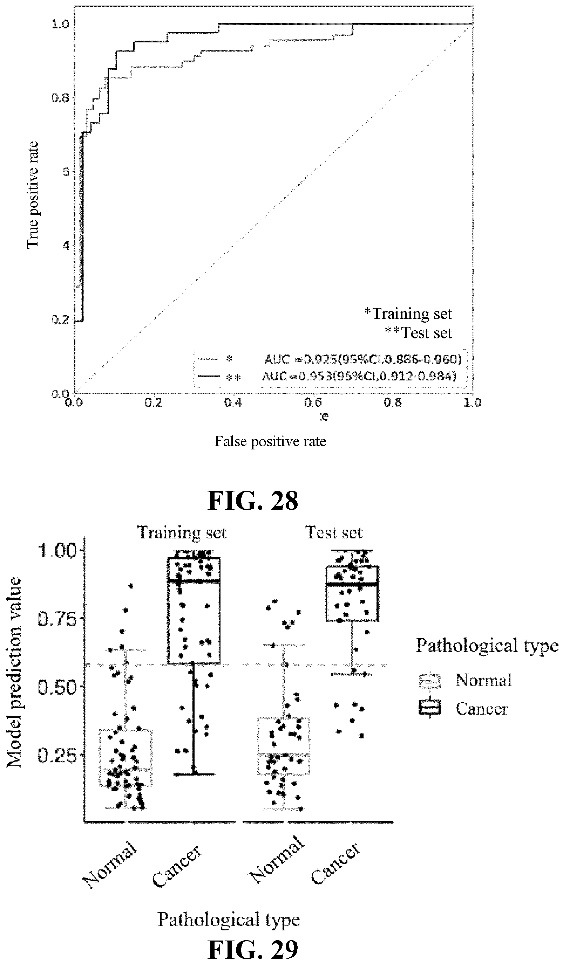

The method of constructing the machine learning model is also consistent with Example 3-2, but the relevant samples only use the data of the above 6 markers in that example. The model scores of the model in the training set and the test set are shown in Fig. 27. The ROC curve of the model is shown in Fig. 28. It can be seen that in the training set and the test set of this model, the scores of samples with pancreatic cancer and those without pancreatic cancer are significantly different from those of other cancer species. In the training set of this model, the AUC for differentiating samples with pancreatic cancer and those without pancreatic cancer samples reached 0.925. In the test set, the AUC for differentiating samples with pancreatic cancer and those without pancreatic cancer samples reached 0.953. The threshold was set to be 0.511, if the score is greater than this value, it is predicted as pancreatic cancer, otherwise it is predicted as absence of pancreatic cancer. Under this threshold, the training set accuracy is 0.886, the training set specificity is 0.921, the training set sensitivity is 0.855, the test set accuracy is 0.886, and the test set specificity is 0.915 , the test set sensitivity is 0.854, which indicates the good performance of this combination model.

3-5: Machine learning model of methylation marker combination 2

In order to verify the effect of the relevant marker combination, in this example, a total of 7 methylation markers including SEQ ID NO: 108, SEQ ID NO: 126, SEQ ID NO: 136, SEQ ID NO: 141, SEQ ID NO: 153, SEQ ID NO: 159, SEQ ID NO: 82 were selected from all the 101 methylation markers based on methylation level to construct a logistic regression machine learning model.

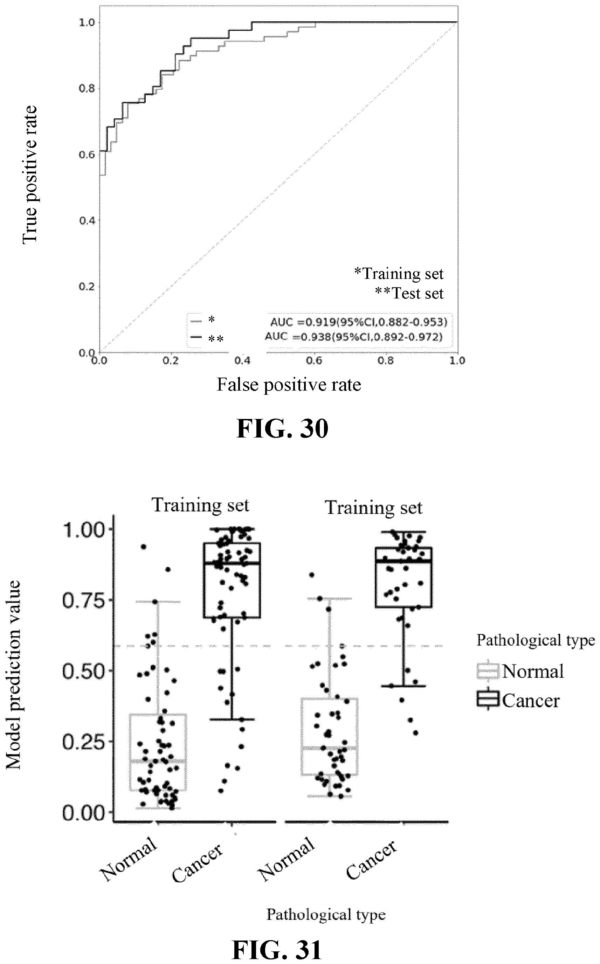

The method of constructing the machine learning model is also consistent with Example 3-2, but the relevant samples only use the data of the above 7 markers in that example. The model scores of the model in the training set and the test set are shown in Fig. 29. The ROC curve of the model is shown in Fig. 30. It can be seen that in the training set and the test set of this model, the scores of samples with pancreatic cancer and those without pancreatic cancer are significantly different from those of other cancer species. In the training set of this model, the AUC for differentiating samples with pancreatic cancer and those without pancreatic cancer samples reached 0.919. In the test set, the AUC for differentiating samples with pancreatic cancer and those without pancreatic cancer samples reached 0.938. The threshold was set to be 0.581, if the score is greater than this value, it is predicted as pancreatic cancer, otherwise it is predicted as absence of pancreatic cancer. Under this threshold, the training set accuracy is 0.826, the training set specificity is 0.921, the training set sensitivity is 0.754, the test set accuracy is 0.818, and the test set specificity is 0.830, the test set sensitivity is 0.805, which indicates the good performance of this combination model.

3-6: Machine learning model of methylation marker combination 3

In order to verify the effect of the relevant marker combination, in this example, a total of 10 methylation markers including SEQ ID NO: 115, SEQ ID NO: 109, SEQ ID NO: 120, SEQ ID NO: 137, SEQ ID NO: 145, SEQ ID NO: 147, SEQ ID NO: 158, SEQ ID NO: 88, SEQ ID NO: 94, SEQ ID NO: 101 were selected from all the 101 methylation markers based on methylation level to construct a logistic regression machine learning model.

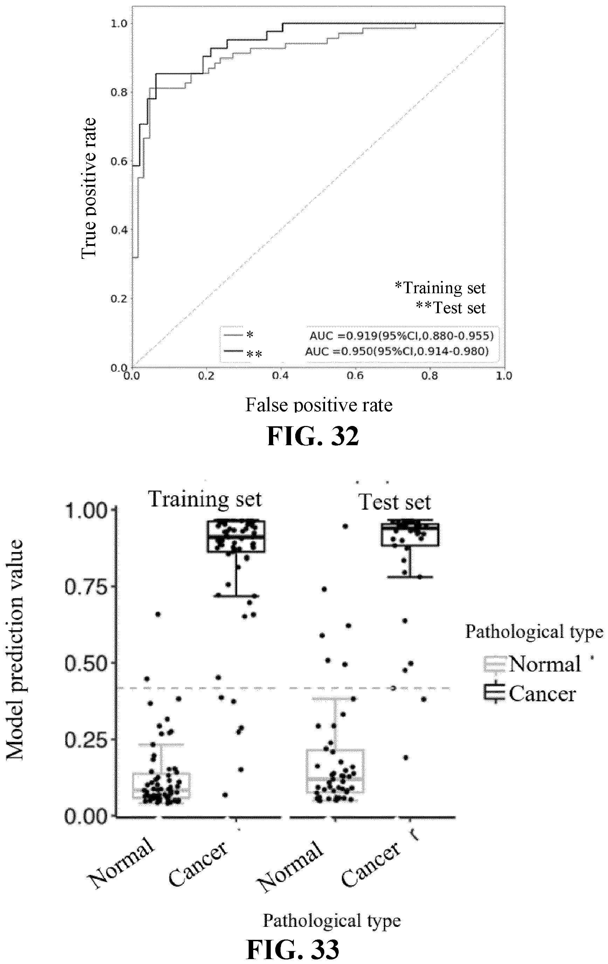

The method of constructing the machine learning model is also consistent with Example 3-2, but the relevant samples only use the data of the above 10 markers in that example. The model scores of the model in the training set and the test set are shown in Fig. 31. The ROC curve of the model is shown in Fig. 32. It can be seen that in the training set and the test set of this model, the scores of samples with pancreatic cancer and those without pancreatic cancer are significantly different from those of other cancer species. In the training set of this model, the AUC for differentiating samples with pancreatic cancer and those without pancreatic cancer samples reached 0.919. In the test set, the AUC for differentiating samples with pancreatic cancer and those without pancreatic cancer samples reached 0.950. The threshold was set to be 0.587, if the score is greater than this value, it is predicted as pancreatic cancer, otherwise it is predicted as absence of pancreatic cancer. Under this threshold, the training set accuracy is 0.848, the training set specificity is 0.952, the training set sensitivity is 0.812, the test set accuracy is 0.886, and the test set specificity is 0.915, the test set sensitivity is 0.854, which indicates the good performance of this combination model.

3-7: The prediction effect of the fusion model of the model of all target methylation markers MODEL1 and other patented prediction models

In the previous patent (Patent No.: CN2021106792818), we provided 56 methylation markers. We used the 56 methylation markers in the previous patent to construct the logistic regression model MODEL2, and used the prediction values of the model MODEL1 in Example 3-3 and the MODEL2 for machine learning modeling (see Table 3-5 for prediction values) to construct a fusion model DUALMODEL.

Table 3-5

| Sample No. | Age | Gender | Sample type | Group | MODEL1 | MODEL2 |
|---|---|---|---|---|---|---|
| Sample 1 | 68 | Male | Without pancreatic cancer | Training set | 0.25078081 | 0.65174889 |
| Sample 2 | 43 | Male | Pancreatic cancer | Training set | 0.84424996 | 0.73201041 |
| Sample 3 | 58 | Female | Pancreatic cancer | Training set | 0.99186158 | 0.91326099 |
| Sample 4 | 70 | Male | Without pancreatic cancer | Training set | 0.08510601 | 0.4047784 |
| Sample 5 | 68 | Male | Without pancreatic cancer | Training set | 0.40610013 | 0.25761509 |
| Sample 6 | 63 | Male | Without pancreatic cancer | Training set | 0.01067555 | 0.13177619 |
| Sample 7 | 53 | Female | Pancreatic cancer | Training set | 0.99469338 | 0.39029108 |
| Sample 8 | 73 | Female | Pancreatic cancer | Training set | 0.9040018 | 0.56356383 |
| Sample 9 | 78 | Female | Without pancreatic cancer | Training set | 0.15905093 | 0.05194212 |
| Sample 10 | 52 | Female | Pancreatic cancer | Training set | 0.99217081 | 0.4976904 |
| Sample 11 | 65 | Female | Pancreatic cancer | Training set | 0.99950316 | 0.95377297 |
| Sample 12 | 64 | Female | Without pancreatic cancer | Training set | 0.03258942 | 0.05961452 |
| Sample 13 | 70 | Female | Without pancreatic cancer | Training set | 0.2179057 | 0.15433055 |
| Sample 14 | 75 | Female | Pancreatic cancer | Training set | 0.9875618 | 0.61078338 |
| Sample 15 | 52 | Male | Pancreatic cancer | Training set | 0.05775145 | 0.25424531 |
| Sample 16 | 55 | Male | Without pancreatic cancer | Training set | 0.00966501 | 0.18725982 |
| Sample 17 | 67 | Male | Pancreatic cancer | Training set | 0.9975897 | 0.94281288 |
| Sample 18 | 68 | Male | Pancreatic cancer | Training set | 0.98029326 | 0.29507811 |
| Sample 19 | 50 | Male | Pancreatic cancer | Training set | 0.99478232 | 0.73780851 |
| Sample 20 | 61 | Female | Without pancreatic cancer | Training set | 0.02333566 | 0.11459015 |
| Sample 21 | 61 | Female | Without pancreatic cancer | Training set | 0.04236396 | 0.26461884 |
| Sample 22 | 75 | Female | Without pancreatic cancer | Training set | 0.12382218 | 0.31538719 |
| Sample 23 | 68 | Male | Pancreatic cancer | Training set | 1 | 0.99999982 |
| Sample 24 | 68 | Female | Pancreatic cancer | Training set | 0.99901289 | 0.96324118 |
| Sample 25 | 63 | Male | Pancreatic cancer | Training set | 0.99090999 | 0.95328414 |

(continued)

| Sample No. | Age | Gender | Sample type | Group | MODEL1 | MODEL2 |
|---|---|---|---|---|---|---|
| Sample 26 | 46 | Male | Pancreatic cancer | Training set | 0.99904043 | 0.99826612 |
| Sample 27 | 61 | Male | Pancreatic cancer | Training set | 0.99999651 | 0.98861223 |
| Sample 28 | 81 | Male | Pancreatic cancer | Training set | 0.9931298 | 0.7917371 |
| Sample 29 | 51 | Female | Without pancreatic cancer | Training set | 0.05085159 | 0.27894715 |
| Sample 30 | 71 | Male | Without pancreatic cancer | Training set | 0.22087186 | 0.21463958 |
| Sample 31 | 66 | Female | Without pancreatic cancer | Training set | 0.05196845 | 0.26969563 |
| Sample 32 | 74 | Male | Without pancreatic cancer | Training set | 0.0222437 | 0.28885596 |
| Sample 33 | 61 | Female | Pancreatic cancer | Training set | 0.95430773 | 0.50709414 |
| Sample 34 | 64 | Male | Without pancreatic cancer | Training set | 0.19472334 | 0.08202203 |
| Sample 35 | 60 | Male | Pancreatic cancer | Training set | 0.78608474 | 0.80666115 |
| Sample 36 | 59 | Male | Without pancreatic cancer | Training set | 0.17703564 | 0.28204181 |
| Sample 37 | 59 | Male | Pancreatic cancer | Training set | 0.90702933 | 0.54538408 |
| Sample 38 | 58 | Male | Without pancreatic cancer | Training set | 0.12213927 | 0.22721625 |
| Sample 39 | 70 | Female | Without pancreatic cancer | Training set | 0.02897606 | 0.15557722 |
| Sample 40 | 63 | Male | Pancreatic cancer | Training set | 0.97500758 | 0.5401742 |
| Sample 41 | 65 | Male | Pancreatic cancer | Training set | 0.96889354 | 0.38259646 |
| Sample 42 | 65 | Male | Pancreatic cancer | Training set | 0.72260556 | 0.41643945 |
| Sample 43 | 68 | Male | Without pancreatic cancer | Training set | 0.39268897 | 0.49625219 |
| Sample 44 | 73 | Male | Without pancreatic cancer | Training set | 0.30300244 | 0.14519084 |
| Sample 45 | 33 | Male | Without pancreatic cancer | Training set | 0.11876943 | 0.51680364 |
| Sample 46 | 72 | Male | Pancreatic cancer | Training set | 0.99998994 | 0.99205528 |
| Sample 47 | 61 | Male | Without pancreatic cancer | Training set | 0.02970681 | 0.14617613 |
| Sample 48 | 65 | Male | Without pancreatic cancer | Training set | 0.65896252 | 0.47554232 |
| Sample 49 | 62 | Male | Without pancreatic cancer | Training set | 0.08777733 | 0.28046503 |
| Sample 50 | 59 | Male | Without pancreatic cancer | Training set | 0.25340248 | 0.35851029 |
| Sample 51 | 58 | Female | Pancreatic cancer | Training set | 0.6152768 | 0.55662049 |
| Sample 52 | 52 | Female | Without pancreatic cancer | Training set | 0.1617307 | 0.30088731 |
| Sample 53 | 63 | Female | Without pancreatic cancer | Training set | 0.16210091 | 0.12832645 |
| Sample 54 | 66 | Female | Pancreatic cancer | Training set | 0.84346289 | 0.79803863 |
| Sample 55 | 48 | Male | Without pancreatic cancer | Training set | 0.14509109 | 0.48815487 |
| Sample 56 | 52 | Male | Pancreatic cancer | Training set | 0.31792133 | 0.69977184 |
| Sample 57 | 63 | Female | Pancreatic cancer | Training set | 0.99971764 | 0.99709014 |
| Sample 58 | 66 | Female | Pancreatic cancer | Training set | 0.999994 | 0.99962091 |
| Sample 59 | 65 | Female | Without pancreatic cancer | Training set | 0.02202481 | 0.26699534 |
| Sample 60 | 64 | Male | Pancreatic cancer | Training set | 0.90270247 | 0.61235916 |
| Sample 61 | 48 | Male | Pancreatic cancer | Training set | 0.99978206 | 0.98503998 |
| Sample 62 | 51 | Female | Without pancreatic cancer | Training set | 0.24623557 | 0.41186833 |
| Sample 63 | 60 | Male | Without pancreatic cancer | Training set | 0.08294895 | 0.44268466 |

(continued)

| Sample No. | Age | Gender | Sample type | Group | MODEL1 | MODEL2 |
|---|---|---|---|---|---|---|
| Sample 64 | 56 | Male | Without pancreatic cancer | Training set | 0.47217743 | 0.21183073 |
| Sample 65 | 64 | Female | Pancreatic cancer | Training set | 0.77824052 | 0.59294107 |
| Sample 66 | 57 | Female | Pancreatic cancer | Training set | 0.9974722 | 0.31385624 |
| Sample 67 | 54 | Male | Without pancreatic cancer | Training set | 0.11018546 | 0.20134804 |
| Sample 68 | 58 | Male | Without pancreatic cancer | Training set | 0.16540707 | 0.15323002 |
| Sample 69 | 50 | Male | Without pancreatic cancer | Training set | 0.25309582 | 0.49754535 |
| Sample 70 | 67 | Male | Pancreatic cancer | Training set | 0.99677626 | 0.93696315 |
| Sample 71 | 69 | Female | Without pancreatic cancer | Training set | 0.16044136 | 0.41599393 |
| Sample 72 | 65 | Male | Pancreatic cancer | Training set | 0.970308 | 0.469277 |
| Sample 73 | 71 | Male | Pancreatic cancer | Training set | 0.9157059 | 0.87305787 |
| Sample 74 | 51 | Male | Pancreatic cancer | Training set | 0.9901979 | 0.79482221 |
| Sample 75 | 63 | Female | Pancreatic cancer | Training set | 0.89611651 | 0.42558101 |
| Sample 76 | 50 | Male | Pancreatic cancer | Training set | 0.70383723 | 0.51413489 |
| Sample 77 | 71 | Female | Pancreatic cancer | Training set | 0.94689731 | 0.74299827 |
| Sample 78 | 68 | Male | Pancreatic cancer | Training set | 0.8611596 | 0.25025656 |
| Sample 79 | 73 | Female | Without pancreatic cancer | Training set | 0.05873808 | 0.22573393 |
| Sample 80 | 70 | Male | Pancreatic cancer | Training set | 0.99992248 | 0.98803577 |
| Sample 81 | 59 | Male | Pancreatic cancer | Training set | 0.99775767 | 0.82747569 |
| Sample 82 | 61 | Male | Pancreatic cancer | Training set | 0.77743794 | 0.21115148 |
| Sample 83 | 67 | Female | Pancreatic cancer | Training set | 0.99088643 | 0.61083689 |
| Sample 84 | 64 | Female | Without pancreatic cancer | Training set | 0.21002627 | 0.93001938 |
| Sample 85 | 68 | Female | Without pancreatic cancer | Training set | 0.03174236 | 0.12057433 |
| Sample 86 | 51 | Female | Pancreatic cancer | Training set | 0.84403816 | 0.79429991 |
| Sample 87 | 74 | Male | Pancreatic cancer | Training set | 0.33938673 | 0.62639247 |
| Sample 88 | 61 | Male | Without pancreatic cancer | Training set | 0.13244477 | 0.15772577 |
| Sample 89 | 65 | Male | Without pancreatic cancer | Training set | 0.03756757 | 0.35296481 |
| Sample 90 | 73 | Male | Without pancreatic cancer | Training set | 0.34746229 | 0.75329063 |
| Sample 91 | 83 | Female | Pancreatic cancer | Training set | 1 | 1 |
| Sample 92 | 89 | Male | Pancreatic cancer | Training set | 0.98309756 | 0.66871618 |
| Sample 93 | 72 | Male | Without pancreatic cancer | Training set | 0.27763773 | 0.55045875 |
| Sample 94 | 72 | Male | Pancreatic cancer | Training set | 0.98121663 | 0.89955382 |
| Sample 95 | 51 | Female | Pancreatic cancer | Training set | 0.22552444 | 0.30532686 |
| Sample 96 | 73 | Female | Without pancreatic cancer | Training set | 0.06250196 | 0.0931513 |
| Sample 97 | 62 | Male | Pancreatic cancer | Training set | 0.97247552 | 0.87634912 |
| Sample 98 | 66 | Female | Without pancreatic cancer | Training set | 0.06054158 | 0.09410333 |
| Sample 99 | 64 | Female | Pancreatic cancer | Training set | 0.96160963 | 0.59392248 |
| Sample 100 | 53 | Female | Without pancreatic cancer | Training set | 0.11575779 | 0.08220186 |
| Sample 101 | 58 | Male | Pancreatic cancer | Training set | 0.93663717 | 0.51236157 |

(continued)

| Sample No. | Age | Gender | Sample type | Group | MODEL1 | MODEL2 |
|---|---|---|---|---|---|---|
| Sample 102 | 52 | Female | Without pancreatic cancer | Training set | 0.04815375 | 0.24040156 |
| Sample 103 | 68 | Male | Without pancreatic cancer | Training set | 0.03270634 | 0.13033442 |
| Sample 104 | 66 | Female | Without pancreatic cancer | Training set | 0.07978489 | 0.12384378 |
| Sample 105 | 73 | Male | Pancreatic cancer | Training set | 1 | 1 |
| Sample 106 | 35 | Male | Without pancreatic cancer | Training set | 0.02154563 | 0.25398164 |
| Sample 107 | 52 | Female | Pancreatic cancer | Training set | 0.80951398 | 0.27261042 |
| Sample 108 | 47 | Female | Pancreatic cancer | Training set | 0.2869437 | 0.52668503 |
| Sample 109 | 50 | Male | Without pancreatic cancer | Training set | 0.08096794 | 0.33442612 |
| Sample 110 | 58 | Female | Without pancreatic cancer | Training set | 0.02672282 | 0.22775222 |
| Sample 111 | 61 | Female | Without pancreatic cancer | Training set | 0.02695807 | 0.17228597 |
| Sample 112 | 73 | Male | Without pancreatic cancer | Training set | 0.14341528 | 0.05630292 |
| Sample 113 | 33 | Male | Pancreatic cancer | Training set | 0.99998424 | 0.99707821 |
| Sample 114 | 75 | Female | Pancreatic cancer | Training set | 0.96847927 | 0.34677269 |
| Sample 115 | 74 | Male | Pancreatic cancer | Training set | 0.79780879 | 0.95525211 |
| Sample 116 | 72 | Male | Without pancreatic cancer | Training set | 0.11698831 | 0.29231555 |
| Sample 117 | 73 | Female | Without pancreatic cancer | Training set | 0.09109822 | 0.21886477 |
| Sample 118 | 64 | Male | Pancreatic cancer | Training set | 0.45009795 | 0.53501892 |
| Sample 119 | 66 | Male | Without pancreatic cancer | Training set | 0.01887551 | 0.69044149 |
| Sample 120 | 66 | Female | Pancreatic cancer | Training set | 0.36695883 | 0.38070724 |
| Sample 121 | 68 | Male | Pancreatic cancer | Training set | 0.93044563 | 0.48217866 |
| Sample 122 | 60 | Male | Pancreatic cancer | Training set | 0.98054899 | 0.25490747 |
| Sample 123 | 66 | Female | Pancreatic cancer | Training set | 0.99434139 | 0.66854088 |
| Sample 124 | 66 | Male | Pancreatic cancer | Training set | 0.99787307 | 0.94969532 |
| Sample 125 | 52 | Male | Without pancreatic cancer | Training set | 0.32914335 | 0.41890651 |
| Sample 126 | 61 | Female | Without pancreatic cancer | Training set | 0.04003975 | 0.1934595 |
| Sample 127 | 65 | Male | Pancreatic cancer | Training set | 0.99999807 | 0.99998367 |
| Sample 128 | 35 | Male | Pancreatic cancer | Training set | 0.91754656 | 0.79652187 |
| Sample 129 | 63 | Male | Without pancreatic cancer | Training set | 0.06558267 | 0.08374058 |
| Sample 130 | 68 | Male | Pancreatic cancer | Training set | 0.98035146 | 0.7368831 |
| Sample 131 | 74 | Male | Without pancreatic cancer | Training set | 0.2004795 | 0.11865175 |
| Sample 132 | 78 | Male | Without pancreatic cancer | Training set | 0.04033666 | 0.39760437 |
| Sample 133 | 67 | Male | Without pancreatic cancer | Test set | 0.31006169 | 0.38800437 |
| Sample 134 | 65 | Female | Pancreatic cancer | Test set | 0.99827511 | 0.9801674 |
| Sample 135 | 67 | Female | Without pancreatic cancer | Test set | 0.03456807 | 0.22284357 |
| Sample 136 | 65 | Male | Without pancreatic cancer | Test set | 0.51361932 | 0.47667898 |
| Sample 137 | 73 | Male | Pancreatic cancer | Test set | 0.99984506 | 0.97732774 |
| Sample 138 | 68 | Female | Without pancreatic cancer | Test set | 0.27818339 | 0.12354882 |
| Sample 139 | 49 | Female | Pancreatic cancer | Test set | 0.9765407 | 0.53402888 |

(continued)

| Sample No. | Age | Gender | Sample type | Group | MODEL1 | MODEL2 |
|---|---|---|---|---|---|---|
| Sample 140 | 46 | Female | Without pancreatic cancer | Test set | 0.15208174 | 0.41915306 |
| Sample 141 | 61 | Female | Pancreatic cancer | Test set | 0.99488045 | 0.79092403 |
| Sample 142 | 53 | Female | Pancreatic cancer | Test set | 0.96244763 | 0.84178423 |
| Sample 143 | 79 | Male | Pancreatic cancer | Test set | 0.8251573 | 0.39626533 |
| Sample 144 | 60 | Male | Pancreatic cancer | Test set | 0.96957092 | 0.95724885 |
| Sample 145 | 52 | Male | Without pancreatic cancer | Test set | 0.72047003 | 0.26187496 |
| Sample 146 | 61 | Female | Pancreatic cancer | Test set | 0.95294665 | 0.27935479 |
| Sample 147 | 56 | Female | Pancreatic cancer | Test set | 0.99463814 | 0.8473568 |
| Sample 148 | 68 | Male | Without pancreatic cancer | Test set | 0.05066732 | 0.43004378 |
| Sample 149 | 53 | Male | Without pancreatic cancer | Test set | 0.37611776 | 0.16021398 |
| Sample 150 | 69 | Female | Pancreatic cancer | Test set | 0.98877813 | 0.80583597 |
| Sample 151 | 65 | Male | Without pancreatic cancer | Test set | 0.41874318 | 0.46822312 |
| Sample 152 | 71 | Male | Without pancreatic cancer | Test set | 0.38347822 | 0.17284585 |
| Sample 153 | 64 | Female | Without pancreatic cancer | Test set | 0.34273249 | 0.53256037 |
| Sample 154 | 79 | Male | Without pancreatic cancer | Test set | 0.18189337 | 0.43406318 |
| Sample 155 | 56 | Male | Pancreatic cancer | Test set | 0.99358521 | 0.66992317 |
| Sample 156 | 67 | Male | Pancreatic cancer | Test set | 0.97611604 | 0.9817731 |
| Sample 157 | 67 | Male | Pancreatic cancer | Test set | 0.96612475 | 0.71360917 |
| Sample 158 | 70 | Male | Pancreatic cancer | Test set | 0.98346993 | 0.97165392 |
| Sample 159 | 57 | Female | Without pancreatic cancer | Test set | 0.04987171 | 0.14632569 |
| Sample 160 | 66 | Female | Without pancreatic cancer | Test set | 0.04087084 | 0.22151849 |
| Sample 161 | 51 | Female | Pancreatic cancer | Test set | 0.95558569 | 0.56875071 |
| Sample 162 | 66 | Female | Pancreatic cancer | Test set | 0.97370032 | 0.89306411 |
| Sample 163 | 56 | Female | Without pancreatic cancer | Test set | 0.94431241 | 0.88579486 |
| Sample 164 | 59 | Male | Without pancreatic cancer | Test set | 0.17790901 | 0.2341512 |
| Sample 165 | 65 | Male | Without pancreatic cancer | Test set | 0.04062224 | 0.20341276 |
| Sample 166 | 72 | Male | Without pancreatic cancer | Test set | 0.03634964 | 0.19893791 |
| Sample 167 | 71 | Female | Without pancreatic cancer | Test set | 0.23909528 | 0.36457442 |
| Sample 168 | 72 | Male | Pancreatic cancer | Test set | 0.9895846 | 0.83498032 |
| Sample 169 | 64 | Male | Without pancreatic cancer | Test set | 0.13914154 | 0.37080528 |
| Sample 170 | 66 | Male | Pancreatic cancer | Test set | 0.98637893 | 0.92709594 |
| Sample 171 | 73 | Male | Pancreatic cancer | Test set | 0.99766784 | 0.81383981 |
| Sample 172 | 53 | Female | Without pancreatic cancer | Test set | 0.25548561 | 0.15473561 |
| Sample 173 | 73 | Female | Without pancreatic cancer | Test set | 0.02235891 | 0.17164734 |
| Sample 174 | 65 | Female | Without pancreatic cancer | Test set | 0.06854341 | 0.27990224 |
| Sample 175 | 72 | Male | Pancreatic cancer | Test set | 0.89914897 | 0.79582034 |
| Sample 176 | 68 | Male | Without pancreatic cancer | Test set | 0.07707142 | 0.07000933 |
| Sample 177 | 68 | Male | Pancreatic cancer | Test set | 0.45466364 | 0.61302045 |

(continued)

| Sample No. | Age | Gender | Sample type | Group | MODEL1 | MODEL2 |
|---|---|---|---|---|---|---|
| Sample 178 | 59 | Male | Pancreatic cancer | Test set | 0.31471306 | 0.6957838 |
| Sample 179 | 73 | Male | Pancreatic cancer | Test set | 0.99962696 | 0.99995631 |
| Sample 180 | 58 | Male | Pancreatic cancer | Test set | 0.99453021 | 0.61075525 |
| Sample 181 | 66 | Male | Without pancreatic cancer | Test set | 0.39550559 | 0.33270704 |
| Sample 182 | 55 | Male | Pancreatic cancer | Test set | 0.99819702 | 0.77738821 |
| Sample 183 | 60 | Male | Without pancreatic cancer | Test set | 0.07917567 | 0.14715185 |
| Sample 184 | 80 | Male | Pancreatic cancer | Test set | 0.94788208 | 0.47871498 |
| Sample 185 | 51 | Male | Without pancreatic cancer | Test set | 0.03590508 | 0.15065318 |
| Sample 186 | 73 | Female | Pancreatic cancer | Test set | 0.99095215 | 0.72755814 |
| Sample 187 | 48 | Male | Pancreatic cancer | Test set | 0.47268095 | 0.84275025 |
| Sample 188 | 67 | Male | Without pancreatic cancer | Test set | 0.43555874 | 0.67384984 |
| Sample 189 | 79 | Male | Without pancreatic cancer | Test set | 0.23924567 | 0.11499981 |
| Sample 190 | 58 | Female | Without pancreatic cancer | Test set | 0.14410461 | 0.16051746 |
| Sample 191 | 68 | Female | Pancreatic cancer | Test set | 0.99705838 | 0.77234306 |
| Sample 192 | 64 | Female | Pancreatic cancer | Test set | 0.44505534 | 0.48062547 |
| Sample 193 | 78 | Male | Without pancreatic cancer | Test set | 0.11731827 | 0.25874073 |
| Sample 194 | 64 | Female | Pancreatic cancer | Test set | 0.99383071 | 0.46219981 |
| Sample 195 | 48 | Male | Without pancreatic cancer | Test set | 0.06891145 | 0.29703642 |
| Sample 196 | 70 | Female | Pancreatic cancer | Test set | 0.3089189 | 0.25476156 |
| Sample 197 | 73 | Male | Without pancreatic cancer | Test set | 0.72066945 | 0.19892712 |
| Sample 198 | 70 | Male | Without pancreatic cancer | Test set | 0.10262287 | 0.56600748 |
| Sample 199 | 66 | Female | Without pancreatic cancer | Test set | 0.12578817 | 0.47884671 |
| Sample 200 | 54 | Male | Pancreatic cancer | Test set | 0.96953552 | 0.97468304 |
| Sample 201 | 73 | Female | Pancreatic cancer | Test set | 0.97365073 | 0.88836746 |
| Sample 202 | 61 | Female | Pancreatic cancer | Test set | 0.46276108 | 0.55159466 |
| Sample 203 | 72 | Male | Without pancreatic cancer | Test set | 0.04585753 | 0.62547952 |
| Sample 204 | 67 | Male | Without pancreatic cancer | Test set | 0.10670945 | 0.29937626 |
| Sample 205 | 60 | Male | Without pancreatic cancer | Test set | 0.03488765 | 0.16531538 |
| Sample 206 | 65 | Male | Pancreatic cancer | Test set | 0.84428404 | 0.6670755 |
| Sample 207 | 53 | Male | Pancreatic cancer | Test set | 0.72297536 | 0.66199598 |
| Sample 208 | 64 | Female | Without pancreatic cancer | Test set | 0.15668154 | 0.19992112 |
| Sample 209 | 46 | Male | Without pancreatic cancer | Test set | 0.04448948 | 0.38817245 |
| Sample 210 | 71 | Male | Pancreatic cancer | Test set | 0.97631324 | 0.85352832 |
| Sample 211 | 81 | Male | Pancreatic cancer | Test set | 0.99954334 | 0.99593925 |
| Sample 212 | 63 | Female | Without pancreatic cancer | Test set | 0.1857722 | 0.1456431 |
| Sample 213 | 51 | Female | Without pancreatic cancer | Test set | 0.60012368 | 0.79114585 |
| Sample 214 | 75 | Female | Without pancreatic cancer | Test set | 0.14224736 | 0.53172159 |
| Sample 215 | 43 | Male | Without pancreatic cancer | Test set | 0.08123859 | 0.32490929 |

(continued)

| Sample No. | Age | Gender | Sample type | Group | MODEL1 | MODEL2 |
|---|---|---|---|---|---|---|
| Sample 216 | 78 | Male | Without pancreatic cancer | Test set | 0.4018081 | 0.31747332 |
| Sample 217 | 70 | Female | Pancreatic cancer | Test set | 0.98494418 | 0.6742575 |
| Sample 218 | 73 | Female | Pancreatic cancer | Test set | 0.95639912 | 0.6712826 |
| Sample 219 | 49 | Female | Without pancreatic cancer | Test set | 0.08526009 | 0.11701414 |
| Sample 220 | 67 | Male | Without pancreatic cancer | Test set | 0.18782098 | 0.29893006 |

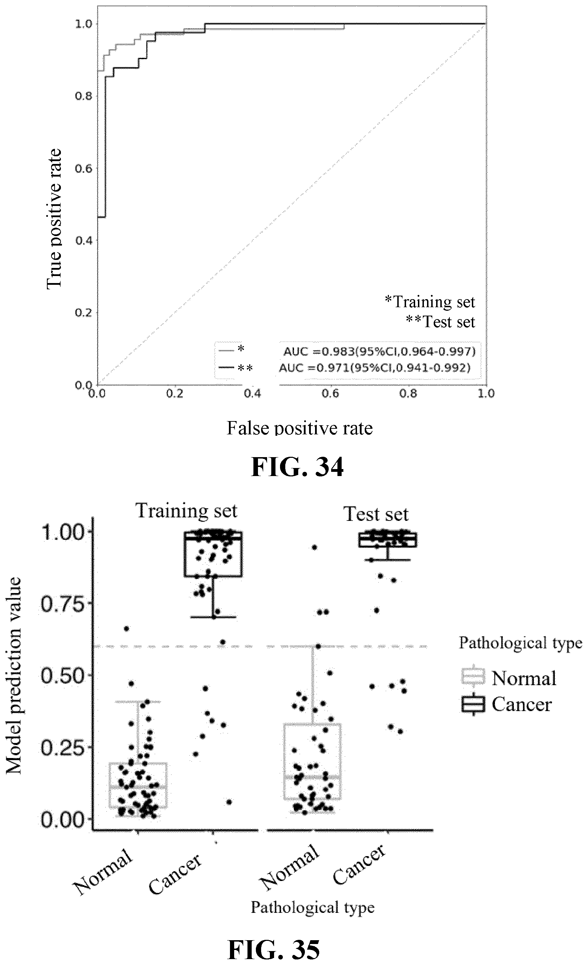

The construction of the DUALMODEL model is similar to Example 3-2, but the MODEL1 prediction values and MODEL2 prediction values are used for the relevant samples. The model scores of DUALMODEL in the training set and the test set are shown in Fig. 33, and the ROC curve of the model is shown in Fig. 34. It can be seen that in the training set and the test set of this model, the scores of samples with pancreatic cancer and those without pancreatic cancer are significantly different from those of other cancer species. In the training set of this model, the AUC for differentiating samples with pancreatic cancer and those without pancreatic cancer samples reached 0.983. In the test set, the AUC for differentiating samples with pancreatic cancer and those without pancreatic cancer samples reached 0.971. The threshold was set to be 0.418, if the score is greater than this value, it is predicted as pancreatic cancer, otherwise it is predicted as absence of pancreatic cancer. Under this threshold, the training set accuracy is 0.939, the training set specificity is 0.984, the training set sensitivity is 0.913, the test set accuracy is 0.909, and the test set specificity is 0.872, the test set sensitivity is 0.951, which indicates that the aggregation model composed of methylation marker combination of the present patent and other patented methylation marker combinations has good performance.

3-8: The prediction effect of ALLMODEL prediction model combining all the target methylation markers and other patented methylation markers

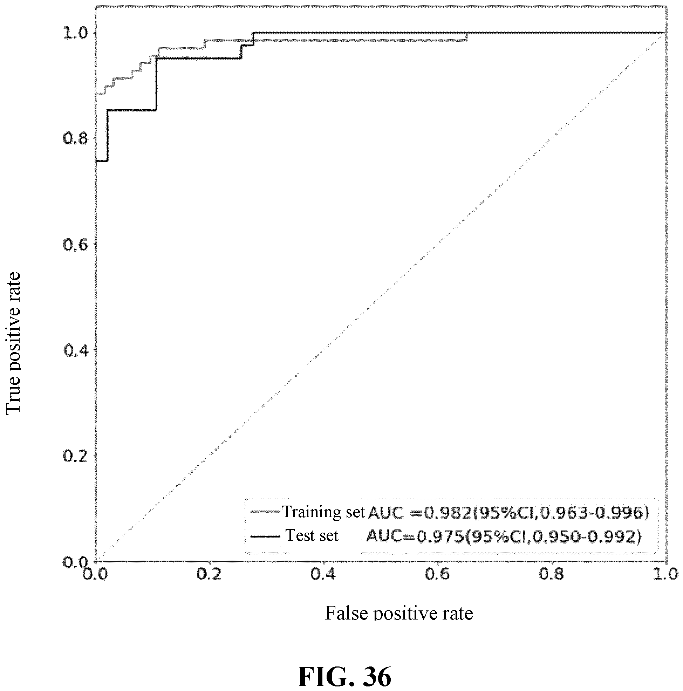

We provided 56 methylation markers in the previous patent application (Patent No.: CN2021106792818), and a logistic regression model ALLMODEL was constructed using the 101 methylation markers in the present application and the 56 methylation markers in the previous patent together. The construction of the ALLMODEL model is similar to Example 3-2, but a total of 157 methylation markers including 101 methylation markers of the present patent and 56 methylation markers of the previous patent are used for the relevant samples. The model scores of ALLMODEL in the training set and the test set are shown in Fig. 35, and the ROC curve of the model is shown in Fig. 36. It can be seen that in the training set and the test set of this model, the scores of samples with pancreatic cancer and those without pancreatic cancer are significantly different from those of other cancer species. In the training set of this model, the AUC for differentiating samples with pancreatic cancer and those without pancreatic cancer samples reached 0.982. In the test set, the AUC for differentiating samples with pancreatic cancer and those without pancreatic cancer samples reached 0.975. The threshold was set to be 0.599, if the score is greater than this value, it is predicted as pancreatic cancer, otherwise it is predicted as absence of pancreatic cancer. Under this threshold, the training set accuracy is 0.939, the training set specificity is 0.984, the training set sensitivity is 0.899, the test set accuracy is 0.886, and the test set specificity is 0.915, the test set sensitivity is 0.854, which indicates that the model constructed using the combination of methylation markers of the present patent and other patented markers has good performance.

**Example 4**

**4-1: Screening of characteristic methylation sites by targeted methylation sequencing**

The inventor collected blood samples from 94 patients with pancreatic cancer and 25 patients with chronic pancreatitis in total, and all the patients signed informed consent forms. The patients with pancreatic cancer had a previous diagnosis of pancreatitis. See the table below for sample information.

| | Training set | Test set |
|---|---|---|
| Number of samples | 80 | 39 |
| Sample type | | |
| Pancreatic cancer | 63 | 31 |

(continued)

| Sample type | | |
|---|---|---|
| Chronic pancreatitis | 17 | 8 |
| Age | | |
| Distribution (mean, maximum and minimum) | 62 (25-80) | 62 (40-79) |
| Gender | | |
| Male | 52 | 23 |
| Female | 28 | 16 |
| Pathological stage | | |
| Chronic pancreatitis | 17 | 8 |
| I | 18 | 7 |
| II | 30 | 14 |
| III or IV | 14 | 9 |
| Unknown | 1 | 1 |
| CA19-9 | | |
| Distribution (mean, maximum and minimum) | 133.84(1-1200) | 86.0(1-1200) |
| >37 | 51 | 23 |
| ≤37 | 21 | 12 |
| NA | 8 | 4 |

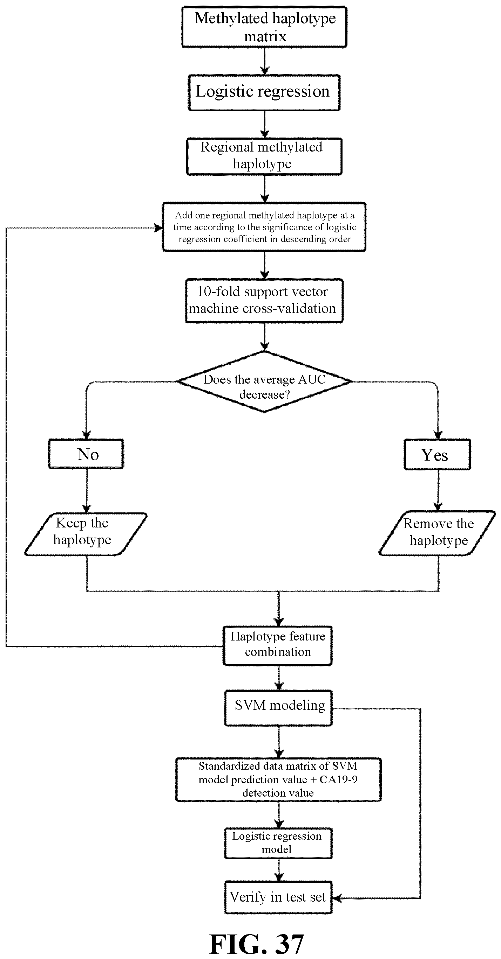

The methylation sequencing data of plasma DNA were obtained by the MethylTitan assay to identify DNA methylation classification markers therein. Refer to Fig. 37 for the process, and the specific process is as follows:

1. Extraction of plasma cfDNA samples

A 2 ml whole blood sample was collected from the patient using a Streck blood collection tube, the plasma was separated by centrifugation timely (within 3 days), transported to the laboratory, and then cfDNA was extracted using the QIAGEN QIAamp Circulating Nucleic Acid Kit according to the instructions.

2. Sequencing and data pre-processing

1) The library was paired-end sequenced using an Illumina Nextseq 500 sequencer.
2) Pear (v0.6.0) software combined the paired-end sequencing data of the same paired-end 150bp sequenced fragment from the Illumina Hiseq X10/ Nextseq 500/Nova seq sequener into one sequence, with the shortest overlapping length of 20 bp and the shortest length of 30bp after combination.
3) Trim_galore v0.6.0 and cutadapt v1.8.1 software were used to perform adapter removal on the combined sequencing data. The adapter sequence "AGATCGGAAGAGCAC" was removed from the 5' end of the sequence, and bases with sequencing quality value lower than 20 at both ends were removed.

3. Sequencing data alignment

The reference genome data used herein were from the UCSC database (UCSC: HG19, hgdownload.soe.ucsc.edu/goldenPath/hg19/bigZips/hg19.fa.gz).

1) First, HG19 was subjected to conversion from cytosine to thymine (CT) and adenine to guanine (GA) using Bismark software, and an index for the converted genome was constructed using Bowtie2 software.
2) The pre-processed data were also subjected to conversions of CT and GA.

3) The converted sequences were aligned to the converted HG19 reference genome using Bowtie2 software. The minimum seed sequence length was 20, and no mismatching was allowed in the seed sequence.

4. Calculation of MHF

For the CpG sites in each target region HG19, the methylation status corresponding to each site was obtained based on the above alignment results. The nucleotide numbering of sites herein corresponds to the nucleotide position numbering of HG19. One target methylated region may have multiple methylated haplotypes. This value needs to be calculated for each methylated haplotype in the target region. An example of the MHF calculation formula is as follows:

$$MHF_{i,h} = \frac{N_{i,h}}{N_i}$$

where i represents the target methylated region, h represents the target methylated haplotype, $N_i$ represents the number of reads located in the target methylated region, and $N_{i,h}$ represents the number of reads containing the target methylated haplotype.

5. Methylation data matrix

    1) The methylation sequencing data of each sample in the training set and the test set were combined into a data matrix, and each site with a depth less than 200 was taken as a missing value.
    2) Sites with a missing value proportion higher than 10% were removed.
    3) For missing values in the data matrix, the KNN algorithm was used to interpolate the missing data.

6. Discovering feature methylated segments based on training set sample group

    1) A logistic regression model was constructed for each methylated segment with regard to the phenotype, and the methylated segment with the most significant regression coefficient was screened out for each amplified target region to form candidate methylated segments.
    2) The training set was randomly divided into ten parts for ten-fold cross-validation incremental feature selection.
    3) The candidate methylated segments in each region are ranked in descending order according to the significance of the regression coefficient, and the data of one methylated segment is added each time to predict the test data (support vector machine (SVM) model).
    4) In step 3), 10 copies of data generated in step 2) were used. For each copy of data, 10 times of calculation were conducted, and the final AUC was the average of 10 calculations. If the AUC of the training data increases, the candidate methylated segment is retained as the feature methylated segment, otherwise it is discarded.

The distribution of the selected characteristic methylation markers in HG19 is as follows: SEQ ID NO: 57 in the SIX3 gene region, SEQ ID NO: 58 in the TLX2 gene region, and SEQ ID NO: 59 in the CILP2 gene region. The levels of the above methylation markers increased or decreased in cfDNA of the patients with pancreatic cancer (Table 4-1). The sequences of the above 3 marker regions are set forth in SEQ ID NOs: 57-59.

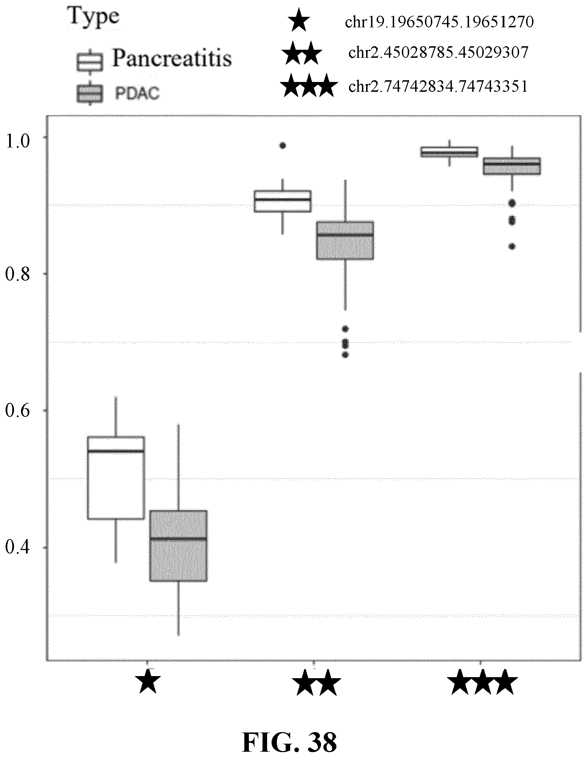

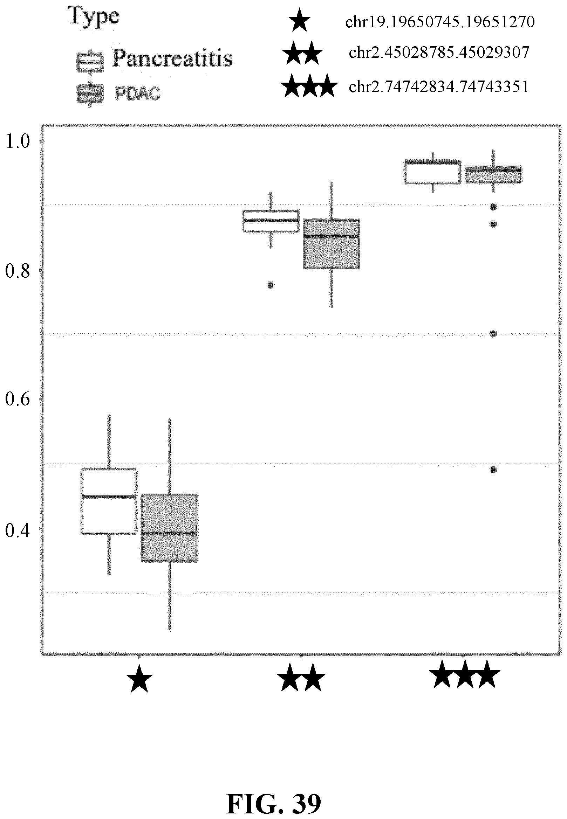

The average methylation levels of methylation markers of people with pancreatic cancer and those with chronic pancreatitis in the training set and the test set are shown in Table 4-1 and Table 4-2, respectively. The distribution of methylation levels of the three methylation markers in the training set and the test set in patients with pancreatic cancer and those with chronic pancreatitis is shown in Fig. 38 and Fig. 39, respectively. As can be seen from the figures and tables, the methylation levels of the methylation markers have significant differences between people with pancreatic cancer and those with chronic pancreatitis, and have good differentiating effects.

**Table 4-1: Methylation levels of DNA methylation markers in the training set**

| Sequence | Marker | Pancreatic cancer | Chronic pancreatitis |
|---|---|---|---|
| SEQ ID NO:57 | chr2:45028785-45029307 | 0.843731054 | 0.909570522 |

(continued)

| Sequence | Marker | Pancreatic cancer | Chronic pancreatitis |
|---|---|---|---|
| SEQ ID NO:58 | chr2:74742834-74743351 | 0.953274962 | 0.978544302 |
| SEQ ID NO:59 | chr19:19650745-19651270 | 0.408843665 | 0.514101315 |

**Table 4-2: Methylation levels of DNA methylation markers in the test set**

| Sequence | Marker | Pancreatic cancer | Chronic pancreatitis |
|---|---|---|---|
| SEQ ID NO:57 | chr2:45028785-45029307 | 0.843896661 | 0.86791556 |
| SEQ ID NO:58 | chr2:74742834-74743351 | 0.926459851 | 0.954493044 |
| SEQ ID NO:59 | chr19:19650745-19651270 | 0.399831579 | 0.44918572 |

**4-2: Construction of classification prediction model based on machine learning**

In order to verify the potential ability of classifying patients with pancreatic cancer and patients with chronic pancreatitis using marker DNA methylation levels (such as methylated haplotype fraction), in the training group, a support vector machine disease classification model pp_model was constructed based on the combination of 3 DNA methylation markers, and a logistic regression disease classification model cpp_model based on the combined data matrix of the support vector machine model prediction score and the CA19-9 measurements was constructed, and the classification prediction effects of the two models were verified in the test group. The training group and the test group were divided according to the proportion, including 80 samples in the training group (samples 1-80) and 39 samples in the test group (samples 80-119).

A support vector machine model was constructed in the training set using the discovered DNA methylation markers.

1) The samples were pre-divided into 2 parts, 1 part was used for training the model and 1 part was used for model testing.

2) To exploit the potential of identifying pancreatic cancer using methylation markers, a disease classification system was developed based on genetic markers. The SVM model was trained using methylation marker levels in the training set. The specific training process is as follows:

a) A training model is constructed using the sklearn software package (v0.23.1) of python software (v3.6.9), command line: pp_model = SVR().

b) The methylation numerical matrix is input to construct an SVM model pp_model.fit (train_df, train_pheno) using the sklearn software package (v0.23.1), where train df represents the methylation numerical matrix of the training set, train_pheno represents the phenotype information of the training set, and pp_model represents the SVM model constructed using three methylation marker numerical matrices.

c) The training set and test set data are brought into the pp_model model respectively to get the prediction score: train_pred = pp_model.predict (train_df)

$$\text{test\_pred} = \text{pp\_model.predict} \left( \text{test\_df} \right)$$

where train_df and test_df are the methylation numerical matrices of the training set and the test set respectively, and train_pred and test_pred are the pp_model model prediction scores of the training set and test set data respectively.

3) In order to improve the ability to differentiate patients with pancreatic cancer and those with pancreatitis, the detection value of CA19-9 was included in the model. The specific process is as follows:

d) The SVM model prediction values of the training set and the corresponding CA19-9 measurement data are combined into a data matrix and standardized:

$$\text{Combine\_scalar\_train} = \text{RobustScaler ().fit( combine\_train\_df )}$$

$$\text{Combine\_scalar\_test} = \text{RobustScaler ().fit( combine\_test\_df )}$$

$$\text{scaled\_combine\_train\_df} = \text{Combine\_scalar\_train.transform (combine\_train\_df)}$$

$$\text{scaled\_combine\_test\_df} = \text{Combine\_scalar\_test.transform(combine\_test\_df)}$$

where combine_train_df and combine_test_df represent the data matrices in which the prediction scores obtained by the pp_model prediction model constructed in this example of the test set samples and the training set samples are combined with CA19-9 respectively; scaled_combine_train_df and scaled_combine_test_df represent the data matrices of the training set and the test set after standardization respectively.

e) A logistic regression model is built using the combined standardized data matrix of the training set pp_model model prediction scores and the CA19-9 measurements, and this model is used to predict the combined standardized data matrix of the test set pp_model model prediction scores and the CA19-9:

$$\text{cpp\_model} = \text{LogisticRegression().fit(scaled\_combine\_train\_df, train\_pheno)}$$

$$\text{combine\_test\_pred} = \text{cpp\_model.predict (scaled\_combine\_test\_df)}$$

where cpp _model represents the logistic regression model fitted using the training set data matrix that incorporates CA19-9 detection values and is standardized; combine_test_pred represents the prediction score of cpp _model in the test set.

In the process of constructing the model, the pancreatic cancer type is coded as 1 and the chronic pancreatitis type is coded as 0. According to the model prediction score distribution, the pp_model and cpp _model thresholds are set to be 0.892 and 0.885 respectively. Based on the two models, when the prediction score is higher than the threshold, the patient is classified as having pancreatic cancer, and otherwise the patient is classified as having pancreatitis.

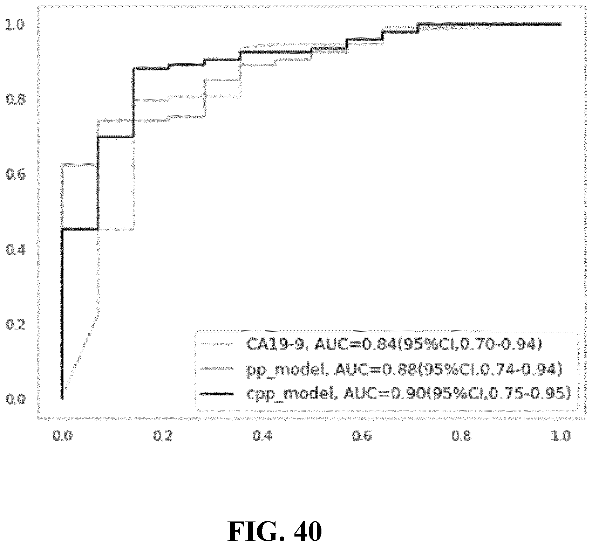

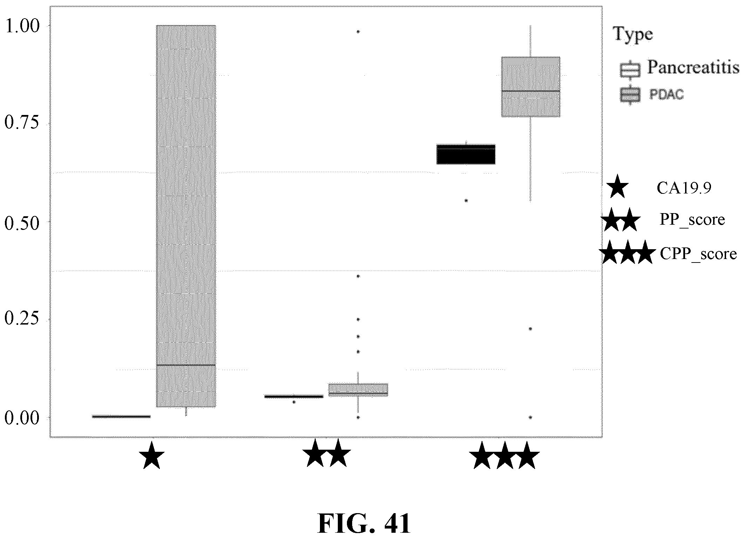

The prediction scores of the two models for the training set and test set samples are shown in Table 4-3 and Table 4-4 respectively. The distribution of the prediction scores is shown in Fig. 40. The ROC curves of the two machine learning models and CA19-9 measurements alone are shown in Fig. 41, where the AUC value of CA19-9 alone is 0.84, the AUC value of pp_model is 0.88, and the AUC value of cpp_model is 0.90. The performance of the SVM model (pp_model) constructed by using three methylation markers is significantly better than that of CA19-9, and the performance of the logistic regression model cpp_model constructed by adding the CA19-9 detection value to the prediction value of the pp_model model is also better than that of pp_model.

The determined threshold is used for statistics in the test set (the recognized threshold of 37 is used for CA19-9). The sensitivity and specificity are shown in Table 4-5. When the specificity in the test set is 100%, the sensitivity of cpp _model to patients with pancreatic cancer can reach 87%, and its performance is better than that of pp_model and CA19-9.

In addition, the performance of the two models in samples identified as negative with respect to CA19-9 (<37) was statistically analyzed. The results are shown in Table 4-6. It can be seen that cpp _model can still reach a sensitivity of 63% and a specificity of 100% for patients with pancreatic cancer patients identified as negative with respect to CA19-9 in the test set.

**Table 4-3: Prediction scores and differentiation results of the two models in the training set**

| Sample | Type | CA19-9 | PP_score | PP call | CPP_score | CPP_call |
|--------|------|--------|----------|---------|-----------|----------|
| Sample 1 | Pancreatitis | 1 | 0.593 | Pancreatitis | 0.306 | Pancreatitis |
| Sample 2 | Pancreatic cancer | 2 | 0.911 | Pancreatic cancer | 0.891 | Pancreatic cancer |
| Sample 3 | Pancreatitis | 2.57 | 0.679 | Pancreatitis | 0.492 | Pancreatitis |

(continued)

| Sample | Type | CA19-9 | PP_score | PP call | CPP_score | CPP_call |
|---|---|---|---|---|---|---|
| Sample 4 | Pancreatitis | 2.61 | 0.815 | Pancreatitis | 0.771 | Pancreatitis |
| Sample 5 | Pancreatic cancer | 3.17 | 0.913 | Pancreatic cancer | 0.893 | Pancreatic cancer |
| Sample 6 | Pancreatic cancer | 3.8 | 0.924 | Pancreatic cancer | 0.902 | Pancreatic cancer |
| Sample 7 | Pancreatic cancer | 4.19 | 0.978 | Pancreatic cancer | 0.938 | Pancreatic cancer |
| Sample 8 | Pancreatitis | 5 | 0.245 | Pancreatitis | 0.018 | Pancreatitis |
| Sample 9 | Pancreatitis | 7 | 0.869 | Pancreatitis | 0.849 | Pancreatitis |
| Sample 10 | Pancreatic cancer | 14.05 | 1.009 | Pancreatic cancer | 0.953 | Pancreatic cancer |
| Sample 11 | Pancreatic cancer | 18.14 | 0.917 | Pancreatic cancer | 0.899 | Pancreatic cancer |
| Sample 12 | Pancreatic cancer | 18.47 | 0.673 | Pancreatitis | 0.485 | Pancreatitis |
| Sample 13 | Pancreatic cancer | 20 | 0.894 | Pancreatic cancer | 0.877 | Pancreatitis |
| Sample 14 | Pancreatic cancer | 21.13 | 0.864 | Pancreatitis | 0.846 | Pancreatitis |
| Sample 15 | Pancreatic cancer | 23.57 | 0.973 | Pancreatic cancer | 0.937 | Pancreatic cancer |
| Sample 16 | Pancreatic cancer | 24.26 | 0.847 | Pancreatitis | 0.824 | Pancreatitis |
| Sample 17 | Pancreatitis | 26.21 | 0.874 | Pancreatitis | 0.858 | Pancreatitis |
| Sample 18 | Pancreatitis | 28.35 | 0.234 | Pancreatitis | 0.017 | Pancreatitis |
| Sample 19 | Pancreatitis | 30.3 | 0.212 | Pancreatitis | 0.014 | Pancreatitis |
| Sample 20 | Pancreatic cancer | 33.99 | 0.898 | Pancreatic cancer | 0.884 | Pancreatitis |
| Sample 21 | Pancreatic cancer | 35 | 1.172 | Pancreatic cancer | 0.989 | Pancreatic cancer |
| Sample 22 | Pancreatic cancer | 37.78 | 0.993 | Pancreatic cancer | 0.948 | Pancreatic cancer |
| Sample 23 | Pancreatic cancer | 39.08 | 0.929 | Pancreatic cancer | 0.911 | Pancreatic cancer |
| Sample 24 | Pancreatic cancer | 42.44 | 0.902 | Pancreatic cancer | 0.889 | Pancreatic cancer |
| Sample 25 | Pancreatic cancer | 52.11 | 0.910 | Pancreatic cancer | 0.897 | Pancreatic cancer |
| Sample 26 | Pancreatic cancer | 54.62 | 0.900 | Pancreatic cancer | 0.889 | Pancreatic cancer |

(continued)

| Sample | Type | CA19-9 | PP_score | PP call | CPP_score | CPP_call |
|---|---|---|---|---|---|---|
| Sample 27 | Pancreatic cancer | 59 | 0.901 | Pancreatic cancer | 0.890 | Pancreatic cancer |
| Sample 28 | Pancreatic cancer | 67.3 | 1.100 | Pancreatic cancer | 0.981 | Pancreatic cancer |
| Sample 29 | Pancreatic cancer | 72.52 | 0.897 | Pancreatic cancer | 0.889 | Pancreatic cancer |
| Sample 30 | Pancreatic cancer | 91.9 | 0.899 | Pancreatic cancer | 0.893 | Pancreatic cancer |
| Sample 31 | Pancreatic cancer | 93.7 | 1.100 | Pancreatic cancer | 0.981 | Pancreatic cancer |
| Sample 32 | Pancreatic cancer | 101.1 | 1.244 | Pancreatic cancer | 0.995 | Pancreatic cancer |
| Sample 33 | Pancreatic cancer | 106 | 0.900 | Pancreatic cancer | 0.896 | Pancreatic cancer |
| Sample 34 | Pancreatic cancer | 115.6 | 1.016 | Pancreatic cancer | 0.962 | Pancreatic cancer |
| Sample 35 | Pancreatic cancer | 129.1 | 0.934 | Pancreatic cancer | 0.924 | Pancreatic cancer |
| Sample 36 | Pancreatic cancer | 130.68 | 1.323 | Pancreatic cancer | 0.998 | Pancreatic cancer |
| Sample 37 | Pancreatic cancer | 137 | 0.892 | Pancreatic cancer | 0.893 | Pancreatic cancer |
| Sample 38 | Pancreatic cancer | 143.77 | 0.865 | Pancreatitis | 0.869 | Pancreatitis |
| Sample 39 | Pancreatic cancer | 144 | 0.943 | Pancreatic cancer | 0.931 | Pancreatic cancer |
| Sample 40 | Pancreatic cancer | 168.47 | 0.896 | Pancreatic cancer | 0.900 | Pancreatic cancer |
| Sample 41 | Pancreatic cancer | 176 | 0.894 | Pancreatic cancer | 0.899 | Pancreatic cancer |
| Sample 42 | Pancreatic cancer | 177.5 | 0.973 | Pancreatic cancer | 0.949 | Pancreatic cancer |
| Sample 43 | Pancreatic cancer | 188.1 | 0.994 | Pancreatic cancer | 0.958 | Pancreatic cancer |
| Sample 44 | Pancreatitis | 216 | 0.899 | Pancreatic cancer | 0.908 | Pancreatic cancer |
| Sample 45 | Pancreatic cancer | 262.77 | 0.899 | Pancreatic cancer | 0.913 | Pancreatic cancer |
| Sample 46 | Pancreatic cancer | 336.99 | 0.906 | Pancreatic cancer | 0.923 | Pancreatic cancer |
| Sample 47 | Pancreatic cancer | 440.56 | 0.947 | Pancreatic cancer | 0.951 | Pancreatic cancer |
| Sample 48 | Pancreatic cancer | 482.61 | 1.037 | Pancreatic cancer | 0.979 | Pancreatic cancer |

(continued)

| Sample | Type | CA19-9 | PP_score | PP call | CPP_score | CPP_call |
|---|---|---|---|---|---|---|
| Sample 49 | Pancreatic cancer | 488 | 0.900 | Pancreatic cancer | 0.929 | Pancreatic cancer |
| Sample 50 | Pancreatic cancer | 535 | 0.898 | Pancreatic cancer | 0.930 | Pancreatic cancer |
| Sample 51 | Pancreatic cancer | 612 | 0.900 | Pancreatic cancer | 0.934 | Pancreatic cancer |
| Sample 52 | Pancreatic cancer | 614.32 | 0.900 | Pancreatic cancer | 0.935 | Pancreatic cancer |
| Sample 53 | Pancreatic cancer | 670 | 0.950 | Pancreatic cancer | 0.959 | Pancreatic cancer |
| Sample 54 | Pancreatic cancer | 683.78 | 0.531 | Pancreatitis | 0.336 | Pancreatitis |
| Sample 55 | Pancreatic cancer | 685.45 | 1.039 | Pancreatic cancer | 0.982 | Pancreatic cancer |
| Sample 56 | Pancreatic cancer | 771 | 0.919 | Pancreatic cancer | 0.949 | Pancreatic cancer |
| Sample 57 | Pancreatic cancer | 836.06 | 0.975 | Pancreatic cancer | 0.970 | Pancreatic cancer |
| Sample 58 | Pancreatic cancer | 849 | 1.001 | Pancreatic cancer | 0.976 | Pancreatic cancer |
| Sample 59 | Pancreatic cancer | 974 | 0.919 | Pancreatic cancer | 0.953 | Pancreatic cancer |
| Sample 60 | Pancreatic cancer | 1149.48 | 1.100 | Pancreatic cancer | 0.991 | Pancreatic cancer |
| Sample 61 | Pancreatic cancer | 1200 | 0.965 | Pancreatic cancer | 0.970 | Pancreatic cancer |
| Sample 62 | Pancreatic cancer | 1200 | 0.905 | Pancreatic cancer | 0.950 | Pancreatic cancer |
| Sample 63 | Pancreatic cancer | 1200 | 0.899 | Pancreatic cancer | 0.947 | Pancreatic cancer |
| Sample 64 | Pancreatitis | 1200 | 0.899 | Pancreatic cancer | 0.947 | Pancreatic cancer |
| Sample 65 | Pancreatic cancer | 1200 | 0.900 | Pancreatic cancer | 0.947 | Pancreatic cancer |
| Sample 66 | Pancreatic cancer | 1200 | 0.887 | Pancreatitis | 0.941 | Pancreatic cancer |
| Sample 67 | Pancreatic cancer | 1200 | 1.035 | Pancreatic cancer | 0.984 | Pancreatic cancer |
| Sample 68 | Pancreatic cancer | 1200 | 0.900 | Pancreatic cancer | 0.948 | Pancreatic cancer |
| Sample 69 | Pancreatic cancer | 1200 | 0.981 | Pancreatic cancer | 0.974 | pancreatic cancer |
| Sample 70 | Pancreatic cancer | 1200 | 0.906 | Pancreatic cancer | 0.950 | Pancreatic cancer |

(continued)

| Sample | Type | CA19-9 | PP_score | PP call | CPP_score | CPP_call |
|---|---|---|---|---|---|---|
| Sample 71 | Pancreatic cancer | 1200 | 1.101 | Pancreatic cancer | 0.991 | Pancreatic cancer |
| Sample 72 | Pancreatic cancer | 1200 | 0.899 | Pancreatic cancer | 0.947 | Pancreatic cancer |
| Sample 73 | Pancreatitis | NA | 0.760 | Pancreatitis | NA | NA |
| Sample 74 | Pancreatitis | NA | 0.888 | Pancreatitis | NA | NA |
| Sample 75 | Pancreatitis | NA | 0.707 | Pancreatitis | NA | NA |
| Sample 76 | Pancreatitis | NA | 0.763 | Pancreatitis | NA | NA |
| Sample 77 | Pancreatitis | NA | 0.820 | Pancreatitis | NA | NA |
| Sample 78 | Pancreatitis | NA | 0.786 | Pancreatitis | NA | NA |
| Sample 79 | Pancreatitis | NA | 0.647 | Pancreatitis | NA | NA |
| Sample 80 | Pancreatic cancer | NA | 0.825 | Pancreatitis | NA | NA |

**Table 4-4: Prediction scores and differentiation results of the two models in the training set**

| Sample | Type | CA19-9 | PP_score | PP_call | CPP_score | CPP_call |
|---|---|---|---|---|---|---|
| Sample 81 | Pancreatitis | NA | 0.610 | Pancreatitis | NA | NA |
| Sample 82 | Pancreatitis | NA | 0.898 | Pancreatic cancer | NA | NA |
| Sample 83 | Pancreatitis | NA | 0.783 | Pancreatitis | NA | NA |
| Sample 84 | Pancreatitis | NA | 0.725 | Pancreatitis | NA | NA |
| Sample 85 | Pancreatic cancer | 1200 | 0.910 | Pancreatic cancer | 0.957 | Pancreatic cancer |
| Sample 86 | Pancreatic cancer | 1200 | 1.355 | Pancreatic cancer | 0.999 | Pancreatic cancer |
| Sample 87 | Pancreatic cancer | 1200 | 0.912 | Pancreatic cancer | 0.953 | Pancreatic cancer |
| Sample 88 | Pancreatic cancer | 1200 | 0.870 | Pancreatitis | 0.932 | Pancreatic cancer |
| Sample 89 | Pancreatic cancer | 1200 | 15.628 | Pancreatic cancer | 1.000 | Pancreatic cancer |
| Sample 90 | Pancreatic cancer | 1200 | 0.970 | Pancreatic cancer | 0.972 | Pancreatic cancer |
| Sample 91 | Pancreatic cancer | 1200 | 0.917 | Pancreatic cancer | 0.955 | Pancreatic cancer |

(continued)

| Sample | Type | CA19-9 | PP_score | PP_call | CPP_score | CPP_call |
|---|---|---|---|---|---|---|
| Sample 92 | Pancreatic cancer | 1200 | 0.818 | Pancreatitis | 0.895 | Pancreatic cancer |
| Sample 93 | Pancreatic cancer | 1200 | 0.921 | Pancreatic cancer | 0.956 | Pancreatic cancer |
| Sample 94 | Pancreatic cancer | 1200 | 0.910 | Pancreatic cancer | 0.952 | Pancreatic cancer |
| Sample 95 | Pancreatic cancer | 768.08 | 3.716 | Pancreatic cancer | 1.000 | Pancreatic cancer |
| Sample 96 | Pancreatic cancer | 373.2 | 0.893 | Pancreatic cancer | 0.917 | Pancreatic cancer |
| Sample 97 | Pancreatic cancer | 343.9 | 0.897 | Pancreatic cancer | 0.918 | Pancreatic cancer |
| Sample 98 | Pancreatic cancer | 224 | 0.923 | Pancreatic cancer | 0.925 | Pancreatic cancer |
| Sample 99 | Pancreatic cancer | 220.5 | 0.998 | Pancreatic cancer | 0.961 | Pancreatic cancer |
| Sample 100 | Pancreatic cancer | 186 | 0.910 | Pancreatic cancer | 0.913 | Pancreatic cancer |
| Sample 101 | Pancreatic cancer | 135 | 0.912 | Pancreatic cancer | 0.909 | Pancreatic cancer |
| Sample 102 | Pancreatic cancer | 86 | 0.901 | Pancreatic cancer | 0.894 | Pancreatic cancer |
| Sample 103 | Pancreatic cancer | 66.68 | 0.956 | Pancreatic cancer | 0.931 | Pancreatic cancer |
| Sample 104 | Pancreatic cancer | 63.8 | 0.966 | Pancreatic cancer | 0.937 | Pancreatic cancer |
| Sample 105 | Pancreatic cancer | 55.9 | 0.765 | Pancreatitis | 0.699 | Pancreatitis |
| Sample 106 | Pancreatic cancer | 52.64 | 1.241 | Pancreatic cancer | 0.995 | Pancreatic cancer |
| Sample 107 | Pancreatic cancer | 41.74 | 1.492 | Pancreatic cancer | 0.999 | Pancreatic cancer |
| Sample 108 | Pancreatic cancer | 30 | 0.914 | Pancreatic cancer | 0.897 | Pancreatic cancer |
| Sample 109 | Pancreatic cancer | 24.78 | 0.879 | Pancreatitis | 0.863 | Pancreatitis |
| Sample 110 | Pancreatic cancer | 24.1 | 1.823 | Pancreatic cancer | 1.000 | Pancreatic cancer |
| Sample 111 | Pancreatic cancer | 21 | 0.934 | Pancreatic cancer | 0.912 | Pancreatic cancer |
| Sample 112 | Pancreatic cancer | 10.29 | 1.079 | Pancreatic cancer | 0.975 | Pancreatic cancer |
| Sample 113 | Pancreatic cancer | 7.41 | 1.069 | Pancreatic cancer | 0.972 | Pancreatic cancer |

(continued)

| Sample | Type | CA19-9 | PP_score | PP_call | CPP_score | CPP_call |
|---|---|---|---|---|---|---|
| Sample 114 | Pancreatic cancer | 7 | 0.730 | Pancreatitis | 0.611 | Pancreatitis |
| Sample 115 | Pancreatitis | 6 | 0.893 | Pancreatic cancer | 0.875 | Pancreatitis |
| Sample 116 | Pancreatitis | 5.56 | 0.899 | Pancreatic cancer | 0.880 | Pancreatitis |
| Sample 117 | Pancreatic cancer | 4.61 | 0.851 | Pancreatitis | 0.825 | Pancreatitis |
| Sample 118 | Pancreatitis | 2.42 | 0.904 | Pancreatic cancer | 0.885 | Pancreatitis |
| Sample 119 | Pancreatitis | 1 | 0.852 | Pancreatitis | 0.826 | Pancreatitis |

**Table 4-5: Sensitivity and specificity of CA19-9 and the two machine learning models**

| Model | Data set | Sensitivity | Specificity |
|---|---|---|---|
| CA19-9 | Training set | 0.79 | 0.80 |
| | Test set | 0.74 | 1.00 |
| pp_model | Training set | 0.90 | 0.80 |
| | Test set | 0.81 | 0.25 |
| cpp_model | Training set | 0.89 | 0.80 |
| | Test set | 0.87 | 1.00 |

**Table 4-6: Performance of two machine learning models in samples identified as negative with respect to CA19-9**

| Model | Data set | Sensitivity | Specificity |
|---|---|---|---|
| pp_model | Training set | 0.77 | 1.00 |
| | Test set | 0.63 | 0.25 |
| cpp_model | Training set | 0.62 | 1.00 |
| | Test set | 0.63 | 1.00 |

This study used the methylation levels of methylation markers in plasma cfDNA to study the differences between the plasma of subjects with chronic pancreatitis and the plasma of those with pancreatic cancer, and screened out 3 DNA methylation markers with significant differences. Based on the above DNA methylation marker cluster in combination of CA19-9 detection values, a malignant pancreatic cancer risk prediction model was established through the support vector machine and logistic regression methods, which can effectively differentiate patients with pancreatic cancer and those with chronic pancreatitis in patients diagnosed with chronic pancreatitis with high sensitivity and specificity, and is suitable for screening and diagnosis of pancreatic cancer in patients with chronic pancreatitis.

**Example 5**

**5-1 Comparing the methylation abundance of pancreatic ductal adenocarcinoma, adjacent tissue and leukocyte DNA samples**

DNA samples were obtained from leukocytes from healthy people with no abnormality in the pancreas, cancer tissues and adjacent tissues from patients with pancreatic ductal adenocarcinoma (including 30 leukocyte samples and 30 cancer tissue samples). Leukocyte DNA was selected as a reference sample because most of the plasma cell-free DNA comes from the DNA released after the rupture of leukocytes, and its background can be a basic background signal of the detection site of plasma cell-free DNA. According to the instructions, leukocyte DNA was extracted using Qiagen QIAamp DNA Mini Kit, and tissue DNA was extracted using Qiagen QIAamp DNA FFPE Tissue Kit. The concentration of cfDNA was detected using Qubit$_{TM}$ dsDNA HS Assay Kit (Thermo, Cat. No.: Q32854).

A 20 ng sample of the DNA obtained in the above step was treated with a bisulfite reagent (MethylCode$_{TM}$ Bisulfite conversion Kit, Thermo, Cat. No.: MECOV50) to obtain converted DNA.

In the PCR reaction system, the final concentration of each primer is 100 nM, and the final concentration of each detection probe is 100 nM. For example, the PCR reaction system can contain 10 $\mu$L to 12.50 $\mu$L of 2x PCR reaction mixture, 0.12 $\mu$L of each of forward primer and reverse primer, 0.04 $\mu$L of probe, 6 $\mu$L of sample DNA (about 10ng), and water making up the total volume of about 20 $\mu$L.

The primer and probe sequences are shown in Table 5-1. For example, the PCR reaction conditions can be as follows: 95°C for 5 min; 95°C for 20 s, and 60°C for 45 s (fluorescence collection), 50 cycles. The ABI 7500 Real-Time PCR System was used to detect different fluorescence in the corresponding fluorescence channel. The Ct values of samples obtained from leukocytes, adjacent tissues and cancer tissues were calculated and compared, methylation level = $2^{-\Delta Ct\ sample\ to\ be\ tested}$/ $2^{-\Delta Ct\ positive\ standard}$ $\times$ 100 %. $\Delta Ct = Ct_{target\ gene} - Ct_{internal\ reference\ gene}$.

Table 5-1 Primer and probe sequences

| SEQ ID NO. | Name | Sequence |
|---|---|---|
| 165 | TLX2 probe 1 | cgGGcgtttcgtTGAtttcgc |
| 166 | TLX2 forward primer 1 | GttTGGTGAGAAGcgAc |
| 167 | TLX2 reverse primer 1 | gCcgTCTaacgCCTAAa |
| 169 | TLX2 probe 2 | CGACCGCTACGACCGCC |
| 170 | TLX2 forward primer 2 | CATCTACAACAAAACGCG |
| 171 | TLX2 reverse primer 2 | GTTTTGTAGCGCGAAGAG |
| 173 | EBF2 probe 1 | AGcgtttcgcgcgttcgG |
| 174 | EBF2 forward primer 1 | cgtTtAtTcgGtttcgtAcg |
| 175 | EBF2 reverse primer 1 | CCTCCCTTATCcgAaaAaaaC |
| 177 | EBF2 probe 2 | TTTCGGATCGCGGCGGAG |
| 178 | EBF2 forward primer 2 | GTTCGTTAGTCGGTAGGG |
| 179 | EBF2 reverse primer 2 | GCAACAAAATATACGCTCGA |
| 181 | KCNA6 probe 1 | ATCCCTTACGCTAACGACGCC |
| 182 | KCNA6 forward primer 1 | AACGCACCTCCGAAAAAA |
| 183 | KCNA6 reverse primer 1 | TGTTTTTTTTTCGGTTTACGG |
| 185 | KCNA6 probe 2 | CCGCGAACCGAAAAAAACGCG |
| 186 | KCNA6 forward primer 2 | ACCAAAACTTTAAAACTCACG |
| 187 | KCNA6 reverse primer 2 | GATATAATTTTTGGAGCGCG |
| 189 | KCNA6 probe 3 | CCGAACACGCTACTCGAAAACCC |
| 190 | KCNA6 forward primer 3 | CAATATCTCCGAACTACGC |
| 191 | KCNA6 reverse primer 3 | GAAGAAGCGGATTCGTCG |

(continued)

| SEQ ID NO. | Name | Sequence |
|---|---|---|
| 193 | CCNA1 probe 1 | cgGtTTtAcgtAGTTGcgtAGGAGt |
| 194 | CCNA1 forward primer 1 | GGttAtAATtTTGGtTTTtttcgGG |
| 195 | CCNA1 reverse primer 1 | gAaAaaTCTTCCCCcgcg |
| 197 | CCNA1 probe 2 | CGCGGTCGGGTCGTTCGTTC |
| 198 | CCNA1 forward primer 2 | TAGGCGTTTGAGTTTTCG |
| 199 | CCNA1 reverse primer 2 | GATAACAACTCTCCGAACT |
| 201 | CCNA1 probe 3 | CGCGACCCGCAAAAACCC |
| 202 | CCNA1 forward primer 3 | CGTAAAAACCTCGAACACG |
| 203 | CCNA1 reverse primer 3 | TGTTGCGTTTTTATCGCG |
| 205 | FOXD3 probe | CGCGAAACCGCCGAAACTACG |
| 206 | FOXD3 forward primer | GTATTTCGTTCGTTTCGTTTA |
| 207 | FOXD3 reverse primer | ACGCAAATTACGATAACCC |
| 209 | TRIM58 probe | CGCGCCGTCCGACTTCTCG |
| 210 | TRIM58 forward primer | GGATTGCGGTTATAGTTTTTG |
| 211 | TRIM58 reverse primer | CGACACTACGAACAAACGT |
| 213 | HOXD 10 probe | ACGCGTCTCTCCCCGCAA |
| 214 | HOXD10 forward primer | TCCCTAACCCAAACTACG |
| 215 | HOXD10 reverse primer | TTAGGATATGGTTAGGCGTTGTC |
| 217 | OLIG3 probe | CACGAAATTAACCGCGTACGC |
| 218 | OLIG3 forward primer | GCCCAAAATAAAATACACCG |
| 219 | OLIG3 reverse primer | GTTATTCGGTCGGTTATTTC |
| 221 | EN2 probe | AACGCGAAACCGCGAACCC |
| 222 | EN2 forward primer | CACTAACAATTCGTTCTACAC |
| 223 | EN2 reverse primer | CGAGGACGTAAATATTATTGAGG |
| 225 | CLEC11A probe | CGTCGTCAAAAACCTACGCCACG |
| 226 | CLEC11A forward primer | GTGGTACGTTCGAGAATTG |
| 227 | CLEC11A reverse primer | CGTAATAAAAACGCCGCTAA |
| 229 | TWIST1 probe | CGCGCTTACCGCTCGACGA |
| 230 | TWIST1 forward primer | CTACTACTACGCCGCTTAC |
| 231 | TWIST1 reverse primer | GCGAGGAAGAGTTAGATCG |
| 161 | ACTB probe | ACCACCACCCAACACACAATAACAAACACA |
| 162 | ACTB forward primer | TGGAGGAGGTTTAGTAAGTTTTTTG |
| 163 | ACTB reverse primer | CCTCCCTTAAAAATTACAAAAACCA |

Summary of sample test results

| | Average ΔCt of cancer tissue | Average ΔCt of adjacent tissue | Average leukocyte ΔCt | p value (cancer tissue vs adjacent tissue) | p value (cancer tissue vs leukocyte) |
|---|---|---|---|---|---|
| TLX2 | 10.5 | 18.2 | 17.9 | 8.0E-08 | 6.4E-08 |
| EBF2 | 4.3 | 6.5 | 10.5 | 5.2E-03 | 5.6E-11 |
| KCNA6 | 12.0 | 19.2 | 19.3 | 5.0E-06 | 3.0E-06 |
| CCNA1 | 11.3 | 19.3 | 20.0 | 1.5E-05 | 3.2E-06 |
| FOXD3 | 3.7 | 8.9 | 6.5 | 7.1E-05 | 8.7E-04 |
| TRIM58 | 3.4 | 12.6 | 7.2 | 1.1E-07 | 4.2E-05 |
| HOXD10 | 5.4 | 10.2 | 7.0 | 1.7E-04 | 3.5E-02 |
| OLIG3 | 5.2 | 12.6 | 7.0 | 6.0E-08 | 1.7E-03 |
| EN2 | 2.7 | 7.3 | 6.6 | 6.9E-07 | 2.5E-08 |
| CLEC11A | 4.4 | 13.3 | 10.8 | 2.0E-07 | 8.8E-07 |
| TWIST1 | 6.2 | 14.0 | 11.4 | 5.1E-07 | 5.0E-06 |

Summary of sample test AUC results

| | AUC of pancreatic ductal adenocarcinoma vs adjacent tissue | AUC of pancreatic ductal adenocarcinoma vs leukocyte genome |
|---|---|---|
| TLX2 | 84 | 81 |
| EBF2 | 49 | 90 |
| KCNA6 | 78 | 78 |
| CCNA1 | 75 | 79 |
| FOXD3 | 81 | 80 |
| TRIM58 | 84 | 81 |
| HOXD10 | 77 | 76 |
| OLIG3 | 85 | 75 |
| EN2 | 84 | 85 |
| CLEC11A | 84 | 56 |
| TWIST1 | 79 | 79 |

The results show that the positive rate of methylation signals in cancer tissues can be much higher than that in leukocyte samples, which also indicates methylation signals in the cancer tissues. Target methylation signals could not detected in most samples of leukocytes. These targets may all have the potential to be used in blood tests for pancreatic cancer. It demonstrates the feasibility and specificity of the selected target markers for tumor tissue.

In the case of greater than 90% specificity, the detection sensitivity statistics of the detection site are shown in the table below. It is proved that the selected target markers have high sensitivity to tumor tissues.

Detection sensitivity of detection site

| Site | Sensitivity | Specificity |
|------|-------------|-------------|
| TLX2 | 69% | 90% |
| EBF2 | 78% | 90% |
| KCNA6 | 62% | 90% |
| CCNA1 | 54% | 96% |
| FOXD3 | 52% | 92% |
| TRIM58 | 65% | 91% |
| HOXD10 | 60% | 95% |
| OLIG3 | 78% | 90% |
| EN2 | 68% | 92% |
| CLEC11A | 60% | 95% |
| TWIST1 | 52% | 96% |

**Comparison of methylation signals in plasma samples from patients with pancreatic ductal adenocarcinoma and those with no abnormality in the pancreas**

The plasma from 100 healthy controls with no abnormality in the pancreas and the plasma from 100 patients with pancreatic ductal adenocarcinoma were selected for testing: extracellular DNA was extracted from the above plasma samples using the commercial QIAamp DNA Mini Kit (QIAGEN, Cat. No.: 51304). Sulfite conversion treatment was performed on the extracted extracellular free DNA using the commercial bisulfite conversion reagent MethylCode$_{TM}$ Bisulfite conversion Kit to obtain converted DNA.

Fluorescent PCR detection was performed using the above PCR reaction system. The primer and probe sequences as shown in Table 5-1 were used and the reference gene ACTB was simultaneously tested as a control. The final concentration of primers is 500 nM and the final concentration of probe is 200 nM. The PCR reaction system contains: 10 $\mu$L of pre-amplification diluted product, 2.5 $\mu$L of primer and probe master mix for the detection site; 12.5 $\mu$L of PCR reagent (Luna$^®$Universal Probe qPCR Master Mix (NEB)).

The fluorescent PCR reaction system is the same as in Example 5-1. PCR reaction conditions are as follows: 95°C for 5 min; 95°C for 15 s, 56°C for 40 s (fluorescence collection), 50 cycles. According to different gene probe modification fluorescence, the corresponding detection fluorescence channel was selected. Methylation level = 2^(-$\Delta$Ct sample to be tested)/2^(-$\Delta$Ct positive standard) $\times$ 100 %. $\Delta$Ct = Ct target gene- Ct internal reference gene.

Summary of sample test results

| | Average plasma $\Delta$Ct of healthy individuals | Average plasma $\Delta$Ct of patients with pancreatic cancer | p value (healthy people vs patients with pancreatic cancer) |
|------|------|------|------|
| TLX2 | 21.5 | 18.0 | 2.4E-02 |
| EBF2 | 23.3 | 16.5 | 8.9E-05 |
| KCNA6 | 34.0 | 31.2 | 2.8E-03 |
| CCNA1 | 34.5 | 33.3 | 3.9E-02 |
| FOXD3 | 10.7 | 7.9 | 6.4E-03 |
| TRIM58 | 23.5 | 16.3 | 4.6E-05 |
| HOXD10 | 5.3 | 4.2 | 8.8E-02 |
| OLIG3 | 13.3 | 10.6 | 2.0E-02 |
| EN2 | 6.8 | 5.7 | 1.7E-02 |
| CLEC11A | 19.6 | 15.8 | 2.8E-02 |
| TWIST1 | 14.8 | 10.8 | 3.6E-03 |

Summary of sample test AUC results

|  | AUC of patients with pancreatic ductal adenocarcinoma vs healthy subjects |
|---|---|
| TLX2 | 65 |
| EBF2 | 71 |
| KCNA6 | 61 |
| CCNA1 | 61 |
| FOXD3 | 69 |
| TRIM58 | 69 |
| HOXD10 | 65 |
| OLIG3 | 72 |
| EN2 | 76 |
| CLEC11A | 68 |
| TWIST1 | 70 |

The results show that all the targets of the present application can be used for blood detection for pancreatic ductal adenocarcinoma. It demonstrates the feasibility and specificity of the selected target markers for tumor tissue.

**Example 6**

**6-1 EBF2 and CCNA1 in combination for prediction of pancreatic cancer**

The present application conducted methylation-specific PCR on the plasma cfDNA of 115 patients with pancreatic cancer and 85 healthy controls, and found that the DNA methylation level of the gene combination of the present application can be used to differentiate between pancreatic cancer plasma and the plasma of normal people.

cfDNA was extracted from the plasma of 115 patients with pancreatic cancer and 85 healthy controls using QIAamp DNA Mini Kit (QIAGEN, Cat. No.: 51304); DNA concentration was detected using Qubit™ dsDNA HS Assay Kit (Thermo, Cat. No.: Q32854); quality inspection was conducted by 1% agarose gel electrophoresis.

The DNA obtained in step 1 was subjected to bisulfite conversion using MethylCode™ Bisulfite conversion Kit (Thermo, Cat. No.: MECOV50). Unmethylated cytosine (C) was converted into uracil (U); methylated cytosine did not change after conversion.

The primer and probe sequences are shown in Table 6-1.

Table 6-1 Primer sequences

| SEQ ID NO. | Name | Sequence |
|---|---|---|
| 173 | EBF2 probe | AGcgtttcgcgcgttcgG |
| 174 | EBF2 forward primer | cgtTtAtTcgGtttcgtAcg |
| 175 | EBF2 reverse primer | CCTCCCTTATCcgAaaAaaaC |
| 193 | CCNA1 probe | cgGtTTtAcgtAGTTGcgtAGGAGt |
| 194 | CCNA1 forward primer | GGttAtAATtTTGGtTTTttcgGG |
| 195 | CCNA1 reverse primer | gAaAaaTCTTCCCCcgcg |
| 161 | ACTB probe | ACCACCACCCAACACACAATAACAAACACA |
| 162 | ACTB forward primer | TGGAGGAGGTTTAGTAAGTTTTTTG |
| 163 | ACTB reverse primer | CCTCCCTTAAAAATTACAAAAACCA |

The multiplex methylation-specific PCR method (Multiplex MSP) was used. The PCR mixture included a PCR reaction solution, a primer mixture, and a probe mixture to prepare single samples. The primer mixture includes a pair of primers for each of the gene combination of the present application and the internal reference gene.

The PCR reaction system is as follows: 5.00 μL of sample cfDNA/positive control/negative control, 3.40 μL of multiplex primer mixture (100 μM), 4.10 μL of water, and 12.5 μL of 2x PCR reaction mixture.

The PCR program was set to be pre-denaturation at 94°C for 2 min, denaturation at 94°C for 30s, annealing at 60°C for 1 min, 45 cycles. Fluorescence signals were collected during the annealing and elongation stage at 60°C.

$$\text{Methylation level} = \text{Ct}_{\text{internal reference gene}} - \text{Ct}_{\text{target gene}}.$$

Binary logistic regression analysis was conducted on the methylation level of the gene combination of the present application, and the equation was fitted. For example, if the score of the exemplary formula is greater than 0, the differentiation result is positive, that is, it is a malignant nodule.

An exemplary fitting equation can be Score=3.54632+EBF2 methylation level×0.04422+CCNA1 methylation level×0.06956.

As analyzed by ROC, the gene combination in the present application has a specificity of 78%, a sensitivity of 62%, and an AUC of 0.689.

The results show the comparison in DNA methylation signals of the combination of detection sites in the present application between control plasma and pancreatic ductal adenocarcinoma plasma. It is proved that the selected target markers have high sensitivity to tumor detection.

### 6-2 KCNA6, TLX2, and EMX1 in combination for pancreatic cancer prediction

The present application conducted methylation-specific PCR on the plasma cfDNA of 115 patients with pancreatic cancer and 85 healthy controls, and found that the DNA methylation level of the gene combination of the present application can be used to differentiate between pancreatic cancer plasma and the plasma of normal people.

cfDNA was extracted from the plasma of 115 patients with pancreatic cancer and 85 healthy controls using QIAamp DNA Mini Kit (QIAGEN, Cat. No.: 51304); DNA concentration was detected using Qubit™ dsDNA HS Assay Kit (Thermo, Cat. No.: Q32854); quality inspection was conducted by 1% agarose gel electrophoresis.

The DNA obtained in step 1 was subjected to bisulfite conversion using MethylCode™ Bisulfite conversion Kit (Thermo, Cat. No.: MECOV50). Unmethylated cytosine (C) was converted into uracil (U); methylated cytosine did not change after conversion.

The primer and probe sequences are shown in Table 6-2.

Table 6-2 Primer sequences

| SEQ ID NO. | Name | Sequence |
|---|---|---|
| 181 | KCNA6 probe | ATCCCTTACGCTAACGACGCC |
| 182 | KCNA6 forward primer | AACGCACCTCCGAAAAAA |
| 183 | KCNA6 reverse primer | TGTTTTTTTTTCGGTTTACGG |
| 165 | TLX2 probe | cgGGcgtttcgtTGAtttcgc |
| 166 | TLX2 forward primer | GttTGGTGAGAAGcgAc |
| 167 | TLX2 reverse primer | gCcgTCTaacgCCTAAa |
| 233 | EMX1 probe | TcgTcgtcgtTGtAGAcgGA |
| 234 | EMX1 forward primer | GTAGcgtTGTTGtTTcgc |
| 235 | EMX1 reverse primer | gTAaAaCcgCcgaaaAacgC |
| 161 | ACTB probe | ACCACCACCCAACACACAATAACAAACACA |
| 162 | ACTB forward primer | TGGAGGAGGTTTAGTAAGTTTTTTG |
| 163 | ACTB reverse primer | CCTCCCTTAAAAAATTACAAAAACCA |

The multiplex methylation-specific PCR method (Multiplex MSP) was used. The PCR mixture included a PCR reaction solution, a primer mixture, and a probe mixture to prepare single samples. The primer mixture includes a pair of primers for each of the gene combination of the present application and the internal reference gene.

The PCR reaction system is as follows: 5.00 μL of sample cfDNA/positive control/negative control, 3.40 μL of multiplex primer mixture (100 μM), 4.10 μL of water, and 12.5 μL of 2x PCR reaction mixture.

The PCR program was set to be pre-denaturation at 94°C for 2 min, denaturation at 94°C for 30s, annealing at 60°C for 1 min, 45 cycles. Fluorescence signals were collected during the annealing and elongation stage at 60°C.

$$\text{Methylation level} = Ct_{\text{internal reference gene}} - Ct_{\text{target gene}}.$$

Binary logistic regression analysis was conducted on the methylation level of the gene combination of the present application, and the equation was fitted. For example, if the score of the exemplary formula is greater than 0, the differentiation result is positive, that is, it is a malignant nodule.

An exemplary fitting equation can be Score=3.48511+KCNA6 methylation level×0.04870+TLX2 methylation level×0.00464+EMX1 methylation level×0.06555.

As analyzed by ROC, the gene combination in the present application has a specificity of 81%, a sensitivity of 63%, and an AUC of 0.735.

The results show the comparison in DNA methylation signals of the combination of detection sites in the present application between control plasma and pancreatic ductal adenocarcinoma plasma. It is proved that the selected target markers have high sensitivity to tumor detection.

### 6-3 TRIM58, TWIST1, FOXD3, and EN2 in combination for pancreatic cancer prediction

The present application conducted methylation-specific PCR on the plasma cfDNA of 115 patients with pancreatic cancer and 85 healthy controls, and found that the DNA methylation level of the gene combination of the present application can be used to differentiate between pancreatic cancer plasma and the plasma of normal people.

cfDNA was extracted from the plasma of 115 patients with pancreatic cancer and 85 healthy controls using QIAamp DNA Mini Kit (QIAGEN, Cat. No.: 51304); DNA concentration was detected using Qubit™ dsDNA HS Assay Kit (Thermo, Cat. No.: Q32854); quality inspection was conducted by 1% agarose gel electrophoresis.

The DNA obtained in step 1 was subjected to bisulfite conversion using MethylCode™ Bisulfite conversion Kit (Thermo, Cat. No.: MECOV50). Unmethylated cytosine (C) was converted into uracil (U); methylated cytosine did not change after conversion.

The primer and probe sequences are shown in Table 6-3.

Table 6-3 Primer sequences

| SEQ ID NO. | Name | Sequence |
| --- | --- | --- |
| 209 | TRIM58 probe | CGCGCCGTCCGACTTCTCG |
| 210 | TRIM58 forward primer | GGATTGCGGTTATAGTTTTTG |
| 211 | TRIM58 reverse primer | CGACACTACGAACAAACGT |
| 229 | TWIST1 probe | CGCGCTTACCGCTCGACGA |
| 230 | TWIST1 forward primer | CTACTACTACGCCGCTTAC |
| 231 | TWIST1 reverse primer | GCGAGGAAGAGTTAGATCG |
| 205 | FOXD3 probe | CGCGAAACCGCCGAAACTACG |
| 206 | FOXD3 forward primer | GTATTTCGTTCGTTTCGTTTA |
| 207 | FOXD3 reverse primer | ACGCAAATTACGATAACCC |
| 221 | EN2 probe | AACGCGAAACCGCGAACCC |
| 222 | EN2 forward primer | CACTAACAATTCGTTCTACAC |
| 223 | EN2 reverse primer | CGAGGACGTAAATATTATTGAGG |
| 161 | ACTB probe | ACCACCACCCAACACACAATAACAAACACA |
| 162 | ACTB forward primer | TGGAGGAGGTTTAGTAAGTTTTTTG |

(continued)

| SEQ ID NO. | Name | Sequence |
|---|---|---|
| 163 | ACTB reverse primer | CCTCCCTTAAAAATTACAAAAACCA |

The multiplex methylation-specific PCR method (Multiplex MSP) was used. The PCR mixture included a PCR reaction solution, a primer mixture, and a probe mixture to prepare single samples. The primer mixture includes a pair of primers for each of the gene combination of the present application and the internal reference gene.

The PCR reaction system is as follows: 5.00 $\mu$L of sample cfDNA/positive control/negative control, 3.40 $\mu$L of multiplex primer mixture (100 $\mu$M), 4.10 $\mu$L of water, and 12.5 $\mu$L of 2x PCR reaction mixture.

The PCR program was set to be pre-denaturation at 94°C for 2 min, denaturation at 94°C for 30s, annealing at 60°C for 1 min, 45 cycles. Fluorescence signals were collected during the annealing and elongation stage at 60°C.

$$\text{Methylation level} = Ct_{\text{internal reference gene}} - Ct_{\text{target gene}}.$$

Binary logistic regression analysis was conducted on the methylation level of the gene combination of the present application, and the equation was fitted. For example, if the score of the exemplary formula is greater than 0, the differentiation result is positive, that is, it is a malignant nodule.

An exemplary fitting equation can be Score=1.76599+TRIM58 methylation leve1×0.03214+TWIST1 methylation level×0.02187+FOXD3 methylation level×0.03075+EN2 methylation level×0.04429.

As analyzed by ROC, the gene combination in the present application has a specificity of 80%, a sensitivity of 64%, and an AUC of 0.735.

The results show the comparison in DNA methylation signals of the combination of detection sites in the present application between control plasma and pancreatic ductal adenocarcinoma plasma. It is proved that the selected target markers have high sensitivity to tumor detection.

**6-4 TRIM58, TWIST1, CLEC11A, HOXD10, and OLIG3 in combination for pancreatic cancer prediction**

The present application conducted methylation-specific PCR on the plasma cfDNA of 115 patients with pancreatic cancer and 85 healthy controls, and found that the DNA methylation level of the gene combination of the present application can be used to differentiate between pancreatic cancer plasma and the plasma of normal people.

cfDNA was extracted from the plasma of 115 patients with pancreatic cancer and 85 healthy controls using QIAamp DNA Mini Kit (QIAGEN, Cat. No.: 51304); DNA concentration was detected using Qubit™ dsDNA HS Assay Kit (Thermo, Cat. No.: Q32854); quality inspection was conducted by 1% agarose gel electrophoresis.

The DNA obtained in step 1 was subjected to bisulfite conversion using MethylCode™ Bisulfite conversion Kit (Thermo, Cat. No.: MECOV50). Unmethylated cytosine (C) was converted into uracil (U); methylated cytosine did not change after conversion.

The primer and probe sequences are shown in Table 6-4.

Table 6-4 Primer sequences

| SEQ ID NO. | Name | Sequence |
|---|---|---|
| 209 | TRIM58 probe | CGCGCCGTCCGACTTCTCG |
| 210 | TRIM58 forward primer | GGATTGCGGTTATAGTTTTTG |
| 211 | TRIM58 reverse primer | CGACACTACGAACAAACGT |
| 229 | TWIST1 probe | CGCGCTTACCGCTCGACGA |
| 230 | TWIST1 forward primer | CTACTACTACGCCGCTTAC |
| 231 | TWIST1 reverse primer | GCGAGGAAGAGTTAGATCG |
| 225 | CLEC11A probe | CGTCGTCAAAAACCTACGCCACG |
| 226 | CLEC11A forward primer | GTGGTACGTTCGAGAATTG |
| 227 | CLEC11A reverse primer | CGTAATAAAAACGCCGCTAA |
| 213 | HOXD10 probe | ACGCGTCTCTCCCCGCAA |

(continued)

| SEQ ID NO. | Name | Sequence |
|---|---|---|
| 214 | HOXD10 forward primer | TCCCTAACCCAAACTACG |
| 215 | HOXD10 reverse primer | TTAGGATATGGTTAGGCGTTGTC |
| 217 | OLIG3 probe | CACGAAATTAACCGCGTACGC |
| 218 | OLIG3 forward primer | GCCCAAAATAAAATACACCG |
| 219 | OLIG3 reverse primer | GTTATTCGGTCGGTTATTTC |
| 161 | ACTB probe | ACCACCACCCAACACACAATAACAAACACA |
| 162 | ACTB forward primer | TGGAGGAGGTTTAGTAAGTTTTTTG |
| 163 | ACTB reverse primer | CCTCCCTTAAAAATTACAAAAACCA |

The multiplex methylation-specific PCR method (Multiplex MSP) was used. The PCR mixture included a PCR reaction solution, a primer mixture, and a probe mixture to prepare single samples. The primer mixture includes a pair of primers for each of the gene combination of the present application and the internal reference gene.

The PCR reaction system is as follows: 5.00 $\mu$L of sample cfDNA/positive control/negative control, 3.40 $\mu$L of multiplex primer mixture (100 $\mu$M), 4.10 $\mu$L of water, and 12.5 $\mu$L of 2x PCR reaction mixture.

The PCR program was set to be pre-denaturation at 94°C for 2 min, denaturation at 94°C for 30s, annealing at 60°C for 1 min, 45 cycles. Fluorescence signals were collected during the annealing and elongation stage at 60°C.

$$\text{Methylation level} = Ct_{\text{internal reference gene}} - Ct_{\text{target gene}}.$$

Binary logistic regression analysis was conducted on the methylation level of the gene combination of the present application, and the equation was fitted. For example, if the score of the exemplary formula is greater than 0, the differentiation result is positive, that is, it is a malignant nodule.

An exemplary fitting equation can be Score = 1.65343 + TRIM58 methylation level $\times$ 0.03638 + TWIST1 methylation level $\times$ 0.02269 + CLEC11A methylation level $\times$ 0.00536 - HOXD10 methylation level $\times$ 0.00435 + OLIG3 methylation level $\times$ 0.02293.

As analyzed by ROC, the gene combination in the present application has a specificity of 90%, a sensitivity of 52%, and an AUC of 0.726.

The results show the comparison in DNA methylation signals of the combination of detection sites in the present application between control plasma and pancreatic ductal adenocarcinoma plasma. It is proved that the selected target markers have high sensitivity to tumor detection.

The foregoing detailed description is provided by way of explanation and example, and is not intended to limit the scope of the appended claims. Various modifications to the embodiments described herein will be apparent to those of ordinary skill in the art and remain within the scope of the appended claims and their equivalents.

## Claims

1.  A method for determining the presence of a pancreatic tumor, assessing the development or risk of development of a pancreatic tumor, and/or assessing the progression of a pancreatic tumor, comprising determining the presence and/or content of modification status of a DNA region with gene EBF2 or a fragment thereof in a sample to be tested.

2.  A method for assessing the methylation status of a DNA region related to a pancreatic tumor, comprising determining the presence and/or content of modification status of a DNA region with gene EBF2 or a fragment thereof in a sample to be tested.

3.  The method of any one of claims 1-2, wherein the DNA region is derived from human chr8:25699246-25907950.

4.  The method of any one of claims 1-3, further comprising obtaining a nucleic acid in the sample to be tested.

5.  The method of claim 4, wherein the nucleic acid includes a cell-free nucleic acid.

**6.** The method of any one of claims 1-5, wherein the sample to be tested includes tissue, cells and/or body fluids.

**7.** The method of any one of claims 1-6, wherein the sample to be tested includes plasma.

**8.** The method of any one of claims 1-7, further comprising converting the DNA region or fragment thereof.

**9.** The method of claim 8, wherein the base with the modification status and the base without the modification status form different substances after conversion.

**10.** The method of any one of claims 1-9, wherein the base with the modification status is substantially unchanged after conversion, and the base without the modification status is changed to other bases different from the base after conversion or is cleaved after conversion.

**11.** The method of any one of claims 9-10, wherein the base includes cytosine.

**12.** The method of any one of claims 1-11, wherein the modification status includes methylation modification.

**13.** The method of any one of claims 10-12, wherein the other base includes uracil.

**14.** The method of any one of claims 8-13, wherein the conversion comprises conversion by a deamination reagent and/or a methylation-sensitive restriction enzyme.

**15.** The method of claim 14, wherein the deamination reagent includes bisulfite or analogues thereof.

**16.** The method of any one of claims 1-15, wherein the method for determining the presence and/or content of modification status comprises determining the presence and/or content of a substance formed after the conversion of a base with the modification status.

**17.** The method of any one of claims 1-16, wherein the method for determining the presence and/or content of modification status comprises determining the presence and/or content of a DNA region with the modification status or a fragment thereof.

**18.** The method of any one of claims 1-17, wherein the presence and/or content of the DNA region with the modification status or fragment thereof is determined by the fluorescence Ct value detected by the fluorescence PCR method.

**19.** The method of any one of claims 1-18, wherein the presence of a pancreatic tumor, or the development or risk of development of a pancreatic tumor is determined by determining the presence of modification status of the DNA region or fragment thereof and/or a higher content of modification status of the DNA region or fragment thereof relative to the reference level.

**20.** The method of any one of claims 1-19, further comprising amplifying the DNA region or fragment thereof in the sample to be tested before determining the presence and/or content of modification status of the DNA region or fragment thereof.

**21.** The method of claim 20, wherein the amplification comprises PCR amplification.

**22.** A method for determining the presence of a disease, assessing the development or risk of development of a disease, and/or assessing the progression of a disease, comprising determining the presence and/or content of modification status of a DNA region selected from the group consisting of DNA regions derived from human chr8:25907849-25907950 and derived from human chr8:25907698-25907894, or a complementary region thereof, or a fragment thereof in a sample to be tested.

**23.** A method for determining the methylation status of a DNA region, comprising determining the presence and/or content of modification status of a DNA region selected from the group consisting of DNA regions derived from human chr8:25907849-25907950 and derived from human chr8:25907698-25907894, or a complementary region thereof, or a fragment thereof in a sample to be tested.

**24.** The method of any one of claims 22-23, comprising providing a nucleic acid capable of binding to a DNA region selected from the group consisting of SEQ ID NO: 172 and SEQ ID NO: 176, or a complementary region thereof, or a converted region thereof, or a fragment thereof.

25. The method of any one of claims 22-24, comprising providing a nucleic acid capable of binding to a DNA region selected from the group consisting of DNA regions derived from human chr8:25907865-25907930 and derived from human chr8:25907698-25907814, or a complementary region thereof, or a converted region thereof, or a fragment thereof.

26. The method of any one of claims 22-25, comprising providing a nucleic acid selected from the group consisting of SEQ ID NO: 173 and SEQ ID NO: 177, or a complementary nucleic acid thereof, or a fragment thereof.

27. The method of any one of claims 22-26, comprising providing a nucleic acid combination selected from the group consisting of SEQ ID NOs: 174 and 175, and SEQ ID NOs: 178 and 179, or a complementary nucleic acid combination thereof, or a fragment thereof.

28. The method of any one of claims 22-27, wherein the disease includes a tumor.

29. The method of any one of claims 22-28, further comprising obtaining a nucleic acid in the sample to be tested.

30. The method of claim 29, wherein the nucleic acid includes a cell-free nucleic acid.

31. The method of any one of claims 22-30, wherein the sample to be tested includes tissue, cells and/or body fluids.

32. The method of any one of claims 22-31, wherein the sample to be tested includes plasma.

33. The method of any one of claims 22-32, further comprising converting the DNA region or fragment thereof.

34. The method of claim 33, wherein the base with the modification status and the base without the modification status form different substances after conversion.

35. The method of any one of claims 22-34, wherein the base with the modification status is substantially unchanged after conversion, and the base without the modification status is changed to other bases different from the base after conversion or is cleaved after conversion.

36. The method of any one of claims 34-35, wherein the base includes cytosine.

37. The method of any one of claims 22-36, wherein the modification status includes methylation modification.

38. The method of any one of claims 35-37, wherein the other base includes uracil.

39. The method of any one of claims 33-38, wherein the conversion comprises conversion by a deamination reagent and/or a methylation-sensitive restriction enzyme.

40. The method of claim 39, wherein the deamination reagent includes bisulfite or analogues thereof.

41. The method of any one of claims 22-40, wherein the method for determining the presence and/or content of modification status comprises determining the presence and/or content of a substance formed by a base with the modification status after the conversion.

42. The method of any one of claims 22-41, wherein the method for determining the presence and/or content of modification status comprises determining the presence and/or content of a DNA region with the modification status or a fragment thereof.

43. The method of any one of claims 22-42, wherein the presence and/or content of the DNA region with the modification status or fragment thereof is determined by the fluorescence Ct value detected by the fluorescence PCR method.

44. The method of any one of claims 22-43, wherein the presence of a pancreatic tumor, or the development or risk of development of a pancreatic tumor is determined by determining the presence of modification status of the DNA region or fragment thereof and/or a higher content of modification status of the DNA region or fragment thereof relative to the reference level.

45. The method of any one of claims 22-44, further comprising amplifying the DNA region or fragment thereof in the sample to be tested before determining the presence and/or content of modification status of the DNA region or fragment thereof.

46. The method of claim 45, wherein the amplification comprises PCR amplification.

47. A nucleic acid comprising a sequence capable of binding to a DNA region with gene EBF2, or a complementary region thereof, or a converted region thereof, or a fragment thereof.

48. A method for preparing a nucleic acid, comprising designing a nucleic acid capable of binding to a DNA region with gene EBF2, or a complementary region thereof, or a converted region thereof, or a fragment thereof, based on the modification status of the DNA region, or complementary region thereof, or converted region thereof, or fragment thereof.

49. A nucleic acid combination comprising a sequence capable of binding to a DNA region with gene EBF2, or a complementary region thereof, or a converted region thereof, or a fragment thereof.

50. A method for preparing a nucleic acid combination, comprising designing a nucleic acid combination capable of amplifying a DNA region with gene EBF2, or a complementary region thereof, or a converted region thereof, or a fragment thereof, based on the modification status of the DNA region, or complementary region thereof, or converted region thereof, or fragment thereof.

51. A kit comprising the nucleic acid of claim 47 and/or the nucleic acid combination of claim 49.

52. Use of the nucleic acid of claim 47, the nucleic acid combination of claim 49 and/or the kit of claim 51 in the preparation of a disease detection product.

53. Use of the nucleic acid of claim 47, the nucleic acid combination of claim 49 and/or the kit of claim 51 in the preparation of a substance for determining the presence of a disease, assessing the development or risk of development of a disease and/or assessing the progression of a disease.

54. Use of the nucleic acid of claim 47, the nucleic acid combination of claim 49 and/or the kit of claim 51 in the preparation of a substance for determining the modification status of the DNA region or fragment thereof.

55. Use of a nucleic acid, a nucleic acid combination and/or a kit for determining the modification status of a DNA region in the preparation of a substance for determining the presence of a pancreatic tumor, assessing the development or risk of development of a pancreatic tumor and/or assessing the progression of a pancreatic tumor, wherein the DNA region for determination includes a DNA region with gene EBF2 or a fragment thereof.

56. Use of a nucleic acid, a nucleic acid combination and/or a kit for determining the modification status of a DNA region in the preparation of a substance for determining the presence of a disease, assessing the development or risk of development of a disease and/or assessing the progression of a disease, wherein the DNA region includes a DNA region selected from the group consisting of DNA regions derived from human chr8:25907849-25907950 and derived from human chr8:25907698-25907894, or a complementary region thereof, or a fragment thereof.

57. Use of nucleic acids of a DNA region with gene EBF2, or a converted region thereof, or a fragment thereof, and a combination of the above-mentioned nucleic acids, in the preparation of a substance for determining the presence of a pancreatic tumor, assessing the development or risk of development of a pancreatic tumor, and/or assessing the progression of a pancreatic tumor.

58. Use of nucleic acids of a DNA region selected from the group consisting of DNA regions derived from human chr8:25907849-25907950 and derived from human chr8:25907698-25907894, or a complementary region thereof, or a converted region thereof, or a fragment thereof, and a combination of the above-mentioned nucleic acids, in the preparation of a substance for determining the presence of a disease, assessing the development or risk of development of a disease and/or assessing the progression of a disease.

59. A storage medium recording a program capable of executing the method as claimed in any one of claims 1-46.

60. A device comprising the storage medium of claim 59.

61. The device of claim 60, further comprising a processor coupled to the storage medium, wherein the processor is configured to execute based on a program stored in the storage medium to implement the method as claimed in any one of claims 1-46.

**FIG. 1**

**FIG. 2**

*Training set
**Test set

AUC=0.927(95%CI,0.885-0.961)
AUC=0.911(95%CI,0.838-0.971)

FIG. 3

FIG. 4

FIG. 5

**FIG. 6**

**FIG. 7**

*Training set
**Test set

| | | |
|---|---|---|
| | * | AUC=0.869(95%CI, 0.812-0.948) |
| | ** | AUC=0.885(95%CI, 0.800-1.000) |

**FIG. 8**

**FIG. 9**

*Training set
**Test set

| | | |
|---|---|---|
| ——— | * | AUC=0.890(95%CI, 0.863-0.919) |
| ——— | ** | AUC=0.881(95%CI, 0.840-0.931) |

**FIG. 10**

**FIG. 11**

**FIG. 12**

*Training set
**Test set

| | | |
|---|---|---|
| — | * | AUC=0.893(95%CI, 0.866-0.923) |
| — | ** | AUC=0.866(95%CI, 0.825-0.915) |

**FIG. 13**

**FIG. 14**

*Training set
**Test set

| | | |
|---|---|---|
| ——— | * | AUC=0.886(95%CI, 0.857-0.920) |
| ——— | ** | AUC=0.862(95%CI, 0.813-0.913) |

**FIG. 15**

*Training set
**Test set

AUC=0.851(95%CI, 0.816-0.885)
AUC=0.859(95%CI, 0.812-0.916)

**FIG. 16**

**FIG. 17**

**Fig. 18**

**FIG. 19**

**FIG. 20**

**FIG. 21**

FIG. 22

**FIG. 23**

220 samples from pancreatic cancer and healthy people

Training set (132 samples)

Test set (88 samples)

Calculate the average AUC of each region by using 3-fold logistic regression cross-validation, and rank in descending order

Add the remaining areas to the feature combination in turn

Yes

No

10-fold logistic regression cross-validation to evaluate whether AUC is improved

Keep the feature

Remove the feature

New feature combination

Train logistic regression model

Verify model effect in test set

**FIG. 24**

**FIG. 25**

**FIG. 26**

**FIG. 27**

**FIG. 28**

**FIG. 29**

**FIG. 30**

**FIG. 31**

FIG. 32

FIG. 33

FIG. 34

FIG. 35

**FIG. 36**

FIG. 37

**FIG. 38**

FIG. 39

**FIG. 40**

FIG. 41

## INTERNATIONAL SEARCH REPORT

| International application No. |
|---|
| **PCT/CN2022/099311** |

| A. | CLASSIFICATION OF SUBJECT MATTER |
|---|---|
| | C12Q 1/6886(2018.01)i; C12Q 1/6883(2018.01)i; G16H 50/20(2018.01)i |

According to International Patent Classification (IPC) or to both national classification and IPC

| B. | FIELDS SEARCHED |
|---|---|

Minimum documentation searched (classification system followed by classification symbols)

C12Q; G16H

Documentation searched other than minimum documentation to the extent that such documents are included in the fields searched

Electronic data base consulted during the international search (name of data base and, where practicable, search terms used)

CNABS, SIPOABS, DWPI, CNTXT, WOTXT, EPTXT, USTXT, CNKI, 百度学术搜索, BAIDU SCHOLAR, WEB OF SCIENCE, PubMed: 胰腺癌, 胰腺导管腺癌, 导管细胞癌, pancreatic cancer, pancreatic ductal adenocarcinoma, ductal adenocarcinoma of pancreas, PDAC, 早期B细胞因子2, EBF2, EIN3-binding F boxprotein 2

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2019194534 A1 (MEDIZEN HUMANCARE INC. et al.) 10 October 2019 (2019-10-10) see abstract, and claims 1-12 | 22, 24-36, 38, 41-43, 45-54, 56, 58-61 |
| X | WO 2013097868 A1 (REGION SYDDANMARK) 04 July 2013 (2013-07-04) see abstract, and claims 1-32 | 23, 37, 39, 40 |
| A | WO 2017165704 A1 (THE UNIVERSITY OF MIAMI) 28 September 2017 (2017-09-28) see abstract, claims 1-71, and description, table 1 | 1-61 |
| A | US 2015141491 A1 (UNIVERSITY OF BIRMINGHAM) 21 May 2015 (2015-05-21) see abstract, claims 1-59, and description, table 2 | 1-61 |
| A | US 2009239214 A1 (DAI, H. Y. et al.) 24 September 2009 (2009-09-24) see abstract, claims 1-41, and description, table 6 | 1-61 |
| A | US 2013274127 A1 (GENOMIC HEALTH INC.) 17 October 2013 (2013-10-17) see abstract, claims 1-17, and description, table 3 | 1-61 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 September 2022** | **21 September 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

<div align="center">

**INTERNATIONAL SEARCH REPORT**

</div>

| | International application No. |
|---|---|
| | **PCT/CN2022/099311** |

**C.    DOCUMENTS CONSIDERED TO BE RELEVANT**

| Category* | Citation of document, with indication, where appropriate, of the relevant passages | Relevant to claim No. |
|:---:|---|:---:|
| A | LI, Mao et al. "MiR-204-5p promotes apoptosis and inhibits migration of osteosarcoma via targeting EBF2" <br> *Biochimie*, Vol. 158, 06 December 2018 (2018-12-06), <br>    pages 224-232, see abstract | 1-61 |

Form PCT/ISA/210 (second sheet) (January 2015)

## INTERNATIONAL SEARCH REPORT

International application No.

**PCT/CN2022/099311**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/CN2022/099311**

| Patent document cited in search report | | | Publication date (day/month/year) | Patent family member(s) | | | Publication date (day/month/year) |
|---|---|---|---|---|---|---|---|
| WO | 2019194534 | A1 | 10 October 2019 | KR | 20190116714 | A | 15 October 2019 |
| WO | 2013097868 | A1 | 04 July 2013 | EP | 2798088 | A1 | 05 November 2014 |
| WO | 2017165704 | A1 | 28 September 2017 | US | 2021154267 | A1 | 27 May 2021 |
| US | 2015141491 | A1 | 21 May 2015 | EP | 3498729 | A2 | 19 June 2019 |
| | | | | US | 2019203293 | A1 | 04 July 2019 |
| | | | | GB | 201212334 | D0 | 22 August 2012 |
| | | | | EP | 2872529 | A2 | 20 May 2015 |
| | | | | WO | 2014009733 | A2 | 16 January 2014 |
| US | 2009239214 | A1 | 24 September 2009 | EP | 1782315 | A2 | 09 May 2007 |
| | | | | CA | 2575557 | A1 | 09 February 2006 |
| | | | | WO | 2006015312 | A2 | 09 February 2006 |
| | | | | AU | 2005267756 | A1 | 09 February 2006 |
| US | 2013274127 | A1 | 17 October 2013 | | None | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910515830

- CN 2021106792818